# EUROPEAN PATENT APPLICATION

(11) **EP 4 691 463 A1**
(43) Date of publication of application: **11.02.2026**
(21) Application number: 24192813.4
(22) Date of filing: 05.08.2024
(51) Int. Cl.: A61K 31/4375, A61K 45/06, A61P 9/00, A61P 9/04

(54) **FINERENONE FOR USE IN A METHOD OF PREVENTING OR TREATING HEART FAILURE IN A PATIENT RECEIVING A CYP3A4 INHIBITOR OR A CYP3A4 INDUCER**

(71) Applicant: Bayer Aktiengesellschaft, 51373 Leverkusen (DE)
(72) Inventor: The designation of the inventor has not yet been filed
(74) Representative: BIP Patents

(57) **Abstract**

The disclosure refers to a method of preventing or treating heart failure in a patient using (4S)-4-(4-cyano-2-methoxyphenyl)-5-ethoxy-2,8-dimethyl-1,4-dihydro-1,6-naphthyridine-3-carboxamide (INN: Finerenone).

## Description

The disclosure refers to a method of preventing or treating heart failure in a patient using (4S)-4-(4-cyano-2-methoxyphenyl)-5-ethoxy-2,8-dimethyl-1,4-dihydro-1,6-naphthyridine-3-carboxamide (INN: finerenone).

Steroidal Mineralocorticoid Receptor Antagonists (MRAs), spironolactone and eplerenone, are recommended in the treatment of patients with heart failure with reduced ejection fraction (HFrEF) and especially those with ejection fraction below 30%, but their role at higher ejection fractions has not been well established. Broad use of steroidal MRAs has further been limited in part due to safety concerns around risks of hyperkalemia and renal dysfunction. These risks may be reduced by the unique pharmacological properties of the non-steroidal MRA finerenone. Heart failure with preserved ejection fraction (HFpEF) which affects around 50% of the overall heart failure population with increasing prevalence has one of the greatest unmet needs in cardiovascular medicine [Butler et al. Developing therapies for heart failure with preserved ejection fraction: current state and future directions, JACC Heart Fail. 2 (2) (2014) 97-112, https://doi.org/10.1016/j.jchf.2013.10.006]. More recently, the steroidal mineralocorticoid receptor antagonist spironolactone was investigated in patients with HFpEF in a trial called TOPCAT [Pitt et al. Spironolactone for heart failure with preserved ejection fraction, N. Engl. J. Med. 370 (15) (2014) 1383-1392, https://doi.org/10.1056/NEJMoa1313731.]. Although spironolactone did not demonstrate benefit with respect to the primary composite endpoint in this global trial there was a nominal benefit in patients recruited in the Americas. Subsequent analyses of TOPCAT have revealed significant regional variation of event rates and drug adherence with different treatment effects [Patel et al., Designing future clinical trials in heart failure with preserved ejection fraction: lessons from TOPCAT, Curr. Heart Fail. Rep. 14 (4) (2017) 217-222, https://doi.org/10.1007/s11897-017-0336-x; Pfeffer MA, Claggett B, Assmann SF, Boineau R, Anand IS, Clausell N, Desai AS, Diaz R, Fleg JL, Gordeev I, Heitner JF, Lewis EF, O'Meara E, Rouleau JL, Probstfield JL, Shaburishvili T, Shah SJ, Solomon SD, Sweitzer NK, McKinlay SM, Pitt B. Regional variation in patients and outcomes in the Treatment of Preserved Cardiac Function Heart Failure With an Aldosterone Antagonist (TOPCAT) trial. Circulation. 2015 Jan 6;131(1):34-42. doi: 10.1161/CIRCULATIONAHA.114.013255. Epub 2014 Nov 18. PMID: 25406305.].

Finerenone is a novel potent and selective non-steroidal MRA. Finerenone's unique binding mode determines potency, selectivity, and blockade of cofactor association with the MR in the nucleus, while its specific physicochemical properties determine tissue penetration and distribution [Kolkhof et al. Nonsteroidal antagonists of the mineralocorticoid receptor, Curr. Opin. Nephrol. Hypertens. 24 (5) (2015) 417-424, https://doi.org/10.1097/MNH.0000000000000147.]. In addition, pharmacokinetic and drug metabolism properties of finerenone yield a short half-life, no active metabolites, and a renal elimination of less than 1% [R. Heinig et al. Pharmacokinetics of the novel nonsteroidal mineralocorticoid receptor antagonist finerenone (BAY 94-8862) in individuals with renal impairment, Clin. Pharmacol. Drug Dev. 5 (6) (2016) 488-501, https://doi.org/10.1002/cpdd.263., M. Gerisch, et al. Biotransformation of finerenone, a novel nonsteroidal mineralocorticoid receptor antagonist, in dogs, rats, and humans, in vivo and in vitro, Drug Metab. Dispos. 46 (11) (2018) 1546-1555, https://doi.org/10.1124/dmd.118.083337.]. The combination of these major parameters translates into a downstream cardiovascular and renal gene expression profile with a deduced MRA pharmacology that clearly differentiates from steroidal MRAs.

Finerenone's mode of action counteracts multiple components which are also key drivers of the HFpEF pathophysiology [Kolkhof, Lawatscheck, Filippatos and Bakris. Int. J. Mol. Sci. 2022;23:9243, Sweeney et al. EMBO Mol Med 2020;12:e1086]. Finerenone has been characterized in several preclinical cardiorenal models which develop heart failure and was shown to demonstrate improvement of systolic and diastolic function [Kolkhof et al. Finerenone, a novel selective nonsteroidal mineralocorticoid receptor antagonist protects from rat cardiorenal injury. J Cardiovasc Pharmacol. 2014 Jul;64(1):69-78. https://doi.org/10.1097/FJC.0000000000000091., Grune et al. Selective Mineralocorticoid Receptor Cofactor Modulation as Molecular Basis for Finerenone's Antifibrotic Activity. Hypertension. 2018 Apr;71(4):599-608. https://doi.org/10.1161/HYPERTENSIONAHA.117.10360., Lachaux et al. Short- and long-term administration of the non-steroidal mineralocorticoid receptor antagonist finerenone opposes metabolic syndrome-related cardio-renal dysfunction. Diabetes Obes Metab. 2018 Oct;20(10):2399-2407. https://doi.org/10.1111/dom.13393., Lima Posada et al. Benefits of the non-steroidal mineralocorticoid receptor antagonist finerenone in metabolic syndrome-related heart failure with preserved ejection fraction. Int. J. Mol. Sci. 2023, Jan 28;24(3):2536.].

However, rodent heart failure models usually develop a mixture of systolic and diastolic dysfunction. There is no rodent animal model which completely recapitulates all aspects of human HFpEF with all aspects [Withaar et al. European Heart Journal (2021) 42, 4420-4430, https://doi.org/10.1093/eurheartj/ehab389]. Therefore, it is currently unknown whether finerenone is able to improve outcomes in human heart failure with preserved ejection fraction (HFpEF), heart failure with mid-range ejection fraction (HFmrEF), heart failure with reduced ejection fraction (HFrEF)or human heart failure (HF) ≥40%. The term heart failure (HF) is sometimes called "cardiac insufficiency" and sometimes HF is still called "cardiac failure". Sometimes HFrEF is referred to as systolic heart failure (and HFpEF as diastolic HF).

Steroidal mineralocorticoid receptor antagonists reduce morbidity and mortality in patients with heart failure and reduced ejection fraction and in myocardial infarction complicated by left ventricular systolic dysfunction, but their efficacy in those with heart failure with mildly reduced or preserved ejection has not been established. According to TOPCAT trail (Treatment of Preserved Cardiac Function Heart Failure With an Aldosterone Antagonist), spironolactone did not reduce the primary endpoint trial. There remains a high unmet need in patients with heart failure and mildly reduced or preserved ejection fraction despite the recent availability of therapeutic options, including sodium-glucose co-transporter-2 inhibitors.

In contrast to the steroidal MRAs spironolactone and eplerenone, finerenone is a highly selective non-steroidal mineralocorticoid receptor antagonist with distinct physiochemical properties, resulting in a more balanced tissue distribution between the heart and kidney compared with steroidal mineralocorticoid receptor antagonists. In two large outcomes trials in participants with chronic kidney disease and Type 2 diabetes, finerenone was well tolerated and reduced kidney disease progression and cardiovascular events, including heart failure hospitalizations, and is approved for the treatment of these patients in many countries. We designed the Study to Evaluate the Efficacy and Safety of Finerenone on Morbidity and Mortality in Participants With Heart Failure and Left Ventricular Ejection Fraction ≥40% (FINEARTS-HF) trial to test the hypothesis that finerenone, in addition to usual therapy, would reduce the composite of cardiovascular death and total heart failure events in patients with heart failure with mildly reduced or preserved ejection fraction. The FINEARTS-HF trial (see example 2) and its outcomes ae described below.An object of the present disclosure is to provide a new method of preventing or treating heart failure.

A further object is to provide a new and/or improved method of treating or preventing symptomatic heart failure.

Another object is to provide a new and/or improved method for reducing the risk of cardiovascular death in patients with heart failure.

A further object is to provide a new and/or improved method of reducing the risk of hospitalization for heart failure in patients.

Another object is to provide a new and/or improved method for reducing the risk of urgent heart failure visits in patients.

A further object is improving symptoms or preventing worsening of symptoms of heart failure. No prior trial of HFmrEF/HFpEF have specifically targeted those with recent worsening heart failure event.

This problem is solved by the method or methods as described below:
The disclosure refers to a method of preventing or treating heart failure in a patient, comprising administering to the patient a therapeutically effective amount of the compound (I) 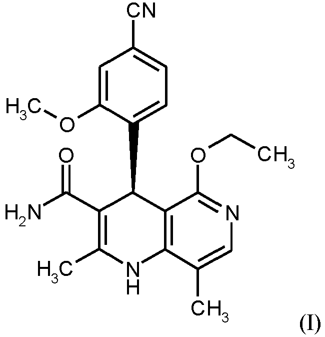 or a hydrate, solvate, pharmaceutically acceptable salt thereof, or a polymorph thereof.

The non-steroidal mineralocorticoid receptor antagonist (MRA) finerenone lessens the effects of mineralocorticoid receptor overactivation by ligands such as aldosterone and cortisol. Finerenone has the chemical name (4S)-4-(4-cyano-2-methoxyphenyl)-5-ethoxy-2,8-dimethyl-1,4-dihydro-1,6-naphthyridine-3-carboxamide, and has the chemical structure of formula (I):

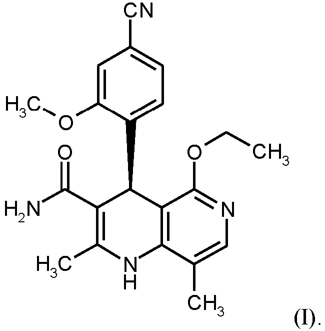

The synthesis, pharmacological properties, and pharmaceutical formulations/dosage forms of finerenone are described in US Patent No. 8,436,180, which is hereby incorporated by reference herein in its entirety. The use described herein refers to finerenone, a polymorph thereof, a solvate thereof, a hydrate thereof, a salt thereof and/or a pharmaceutically acceptable salt thereof.

"Solvates" for the purposes of the disclosure are those forms of the compounds or their salts where solvent molecules form a stoichiometric complex in the solid state and include, but are not limited to for example water, ethanol and methanol.

"Hydrates" are a specific form of solvates, where the solvent molecule is water. Hydrates of the compounds of the disclosure or their salts are stoichiometric compositions of the compounds or salts with water, such as, for example, monohydrate, dihydrates, trihydrate, hemihydrate, sequihydrate.

"Salts" for the purposes of the present disclosure are preferably "pharmaceutically acceptable salts" of finerenone. Suitable pharmaceutically acceptable salts that can be used in the combination according to the disclosure are well known to those skilled in the art and include salts of inorganic acids, organic acids, inorganic bases, alkaline cations, alkaline earth cations and organic bases. In one embodiment the pharmaceutically acceptable salt can be selected from mandelic acid acetate, benzoate, besylate, bromide, camsylate, carbonate, citrate, edisylate, estolate, fumarate, gluceptate, gluconate, glucuronate, hippurate, iodide, isethionate, lactate, lactobionate, malate, maleate, mesylate, methylsulfate, napsylate, nitrate, oxalate, pamoate, phosphate, stearate, succinate, sulfate, tartrate, bitartrate, tosylate, calcium, diolamine, lithium, lysine, magnesium, meglumine, N-methylglucamine, olamine, potassium, tromethamine, tris(hydroxymethyl)aminomethane, benzene sulfonate, ethane sulfonate and zinc. Salts and/complexes can also be formed with at least one of the following acids: hydrochloric acid, hydrobromic acid, sulfuric acid, phosphoric acid, methane sulphonic acid, trifluoromethanesulfonic acid, benzenesulfonic acid, p-toluene sulfonic acid, 1-naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, and/or phenylacetic acid. Furthermore, the compound according to formula (I) can also form co-crystals with the aforementioned acids.

In one embodiment the pharmaceutically acceptable salt can be selected from hydrochloride, sulfate, mesylate, tosylate, tartrate, citrate, benzenesulfonate, ethanesulfonate, maleate, and phosphate.

Polymorphic form of finerenone is disclosed in US Patent 10,399,977, which is hereby incorporated herein by reference in its entirety. In the methods of the present disclosure, the compound to be administered may be finerenone of the formula (I) in crystalline form of polymorph I characterized in that the x-ray diffractogram of the compound exhibits peak maxima of the 2 theta angle at 8.5, 14.1, 17.2, 19.0, 20.5, 25.6, 26.5. In the methods of the present disclosure, the compound to be administered may be finerenone of the formula (I) in crystalline form of polymorph I characterized in that the IR spectrum (IR-ATR) of the compound exhibits band maxima at 3475, 2230, 1681, 1658, 1606, 1572, 1485, 1255, 1136 and 1031 cm-1. In the methods of the present disclosure, the compound to be administered may be finerenone of the formula (I) in crystalline form of polymorph I characterized in that the Raman spectrum of the compound exhibits band maxima at 3074, 2920, 2231, 1601, 1577, 1443, 1327, 1267, 827 and 155 cm⁻¹. Experimental conditions for the measurement of these crystalline form parameters are found in the examples.

In one embodiment, finerenone of the formula (I) in crystalline form of polymorph I is used. In one embodiment, finerenone is the compound of the formula (I) in crystalline form of polymorph I 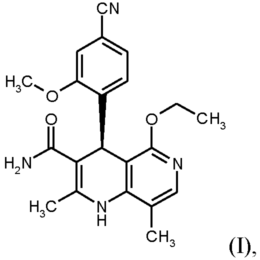 wherein the x-ray diffractogram of the compound exhibits peak maxima of the 2 theta angle at 8.5, 14.1, and 19.0. In one embodiment, finerenone is the compound of the formula (I) in crystalline form of polymorph I, wherein the x-ray diffractogram of the compound further exhibits any one f peak maxima of the 2 theta angle at 17.2, 20.5, 25.6, and 26.5. In one embodiment, finerenone is the compound of the formula (I) in crystalline form of polymorph I, wherein the IR spectrum of the compound exhibits any one of band maxima at 3475, 2230, 1681, 1658, 1606, 1572, 1485, 1255, 1136 and 1031 cm⁻¹. In one embodiment, finerenone is the compound of the formula (I) in crystalline form of polymorph I, wherein the IR spectrum of the compound exhibits band maxima at 3475, 2230, 1681, 1658, 1606, 1572, 1485, 1255, 1136 and 1031 cm-1. In one embodiment, finerenone is the compound of the formula (I) in crystalline form of polymorph I, wherein the Raman spectrum of the compound exhibits any one of band maxima at 3074, 2920, 2231, 1601, 1577, 1443, 1327, 1267, 827 and 155 cm⁻¹. In one embodiment, finerenone is the compound of the formula (I) in crystalline form of polymorph I, wherein the Raman spectrum of the compound exhibits band maxima at 3074, 2920, 2231, 1601, 1577, 1443, 1327, 1267, 827 and 155 cm⁻¹. In one embodiment, finerenone is the compound of the formula (I) in crystalline form of polymorph I, wherein the compound has a melting point of 252° C.

In one embodiment the method according to the disclosure is used for the treatment and/or prevention of heart failure.

In one embodiment, the heart failure is selected from symptomatic heart failure, heart failure with improved ejection fraction (HFimpEF), heart failure with mid-range ejection fraction (HFmrEF), heart failure preserved ejection fraction (HFpEF), heart failure with reduced ejection fraction (HFrEF), chronic heart failure (CHF), congestive heart failure, acute heart failure, chronic heart failure, worsening chronic heart failure (WCHF), hospitalization for heart failure.

In one embodiment, the heart failure is symptomatic heart failure. In one embodiment, the heart failure is heart failure with improved ejection fraction (HFimpEF). In one embodiment, the heart failure is heart failure with mid-range ejection fraction (HFmrEF). In one embodiment, the heart failure is preserved ejection fraction (HFpEF). In one embodiment, the heart failure is heart failure with reduced ejection fraction (HFrEF). In one embodiment, the heart failure is chronic heart failure (CHF). In one embodiment, the heart failure is congestive heart failure. In one embodiment, the heart failure is acute heart failure. In one embodiment, the heart failure is chronic heart failure. In one embodiment, the heart failure is worsening chronic heart failure (WCHF). In one embodiment, the heart failure is hospitalization for heart failure.

In one embodiment, the method according to the disclosure improves symptoms of heart failure. In this embodiment the heart failure can be selected from symptomatic heart failure, heart failure with improved ejection fraction (HFimpEF), heart failure with mid-range ejection fraction (HFmrEF), heart failure preserved ejection fraction (HFpEF), heart failure with reduced ejection fraction (HFrEF), chronic heart failure (CHF), congestive heart failure, acute heart failure, chronic heart failure, worsening chronic heart failure (WCHF), hospitalization for heart failure.

In one embodiment, the method according to the disclosure prevents worsening of symptoms of heart failure. In this embodiment the heart failure can be selected from symptomatic heart failure, heart failure with improved ejection fraction (HFimpEF), heart failure with mid-range ejection fraction (HFmrEF), heart failure preserved ejection fraction (HFpEF), heart failure with reduced ejection fraction (HFrEF), chronic heart failure (CHF), congestive heart failure, acute heart failure, chronic heart failure, worsening chronic heart failure (WCHF), hospitalization for heart failure.

In one embodiment, the hospitalization for heart failure is equal or below 6 months. In one embodiment, the hospitalization for heart failure was equal or above 6 months. In one embodiment, the hospitalization for heart failure is equal or below 12 months. In one embodiment, the hospitalization for heart failure is equal or above 12 months. In one embodiment, the hospitalization for heart failure s any prior heart failure hospitalization.

In one embodiment, the hospitalization for heart failure is equal or below 6 months prior to randomization. In one embodiment, the hospitalization for heart failure was equal or above 6 months prior to randomization. In one embodiment, the hospitalization for heart failure is equal or below 12 months prior to randomization. In one embodiment, the hospitalization for heart failure is equal or above 12 months prior to randomization. In one embodiment, the hospitalization for heart failure s any prior heart failure hospitalization prior to randomization.

In one embodiment, the hospitalization for heart failure is equal or below 6 months prior to treatment with finerenone. In one embodiment, the hospitalization for heart failure was equal or above 6 months prior to treatment with finerenone. In one embodiment, the hospitalization for heart failure is equal or below 12 months prior to treatment with finerenone. In one embodiment, the hospitalization for heart failure is equal or above 12 months prior to treatment with finerenone. In one embodiment, the hospitalization for heart failure s any prior heart failure hospitalization prior to treatment with finerenone.

Unless otherwise stated, the terms used herein are used in the manner customary to those skilled in the art. The chemical definitions are used according to IUPAC.

In one embodiment, the heart failure is classified by the definition of the New York Heart Association (NYHA) Functional Classification (The Criteria Committee of the New York Heart Association. (1994). Nomenclature and Criteria for Diagnosis of Diseases of the Heart and Great Vessels (9th ed.). Boston: Little, Brown & Co. pp. 253-256.). This definition is regarded as a way of describing the extent of functional limitation due to symptoms caused by HF. It places patients in one of four categories based on how much they are limited during physical activity; the limitations/symptoms are in regard to normal breathing and varying degrees in shortness of breath and/or angina. In practice, sometimes only two symptoms are considered relevant.

NYHA Class I (Symptoms): Presence of cardiac disease. No limitation of physical activity. Ordinary physical activity does not cause undue fatigue, palpitation, dyspnea (shortness of breath). NYHA Class II (Symptoms): Slight limitation of physical activity. Comfortable at rest. Ordinary physical activity results in fatigue, palpitation, dyspnea. NYHA Class III (Symptoms): Marked limitation of physical activity. Comfortable at rest. Less than ordinary activity causes fatigue, palpitation, or dyspnea. NYHA Class I (Symptoms): Unable to carry on any physical activity without discomfort. Symptoms of heart failure at rest. If any physical activity is undertaken, discomfort increases.

In one embodiment, the heart failure is selected from New York Heart Association (NYHA) class I, II, III and IV. In one embodiment, the heart failure is selected from NYHA class II, III and IV. In one embodiment, the heart failure is NYHA class II. In one embodiment, the heart failure is NYHA class III. In one embodiment, the heart failure is NYHA class IV.

In one embodiment, method for treating symptomatic heart failure in a patient, comprising administering to the patient a therapeutically effective amount of the compound (I) 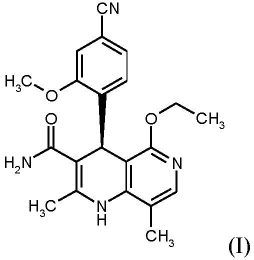 or a hydrate, solvate, pharmaceutically acceptable salt thereof, or a polymorph thereof.

In one embodiment, method for preventing symptomatic heart failure in a patient, comprising administering to the patient a therapeutically effective amount of the compound (I) 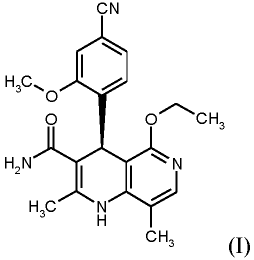 or a hydrate, solvate, pharmaceutically acceptable salt thereof, or a polymorph thereof.

In one embodiment, method for lowering the risk of symptomatic heart failure in a patient, comprising administering to the patient a therapeutically effective amount of the compound (I) 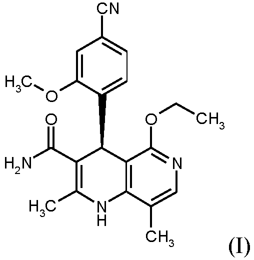 or a hydrate, solvate, pharmaceutically acceptable salt thereof, or a polymorph thereof.

In one embodiment, method for treating symptomatic heart failure with left ventricular ejection fraction (LVEF) equal or above 30% in a patient, comprising administering to the patient a therapeutically effective amount of the compound (I) 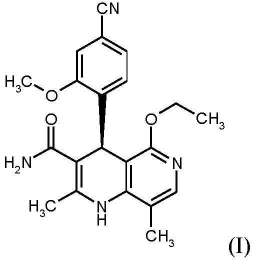 or a hydrate, solvate, pharmaceutically acceptable salt thereof, or a polymorph thereof.

In one embodiment, method for preventing symptomatic heart failure with left ventricular ejection fraction (LVEF) equal or above 30% in a patient, comprising administering to the patient a therapeutically effective amount of the compound (I) 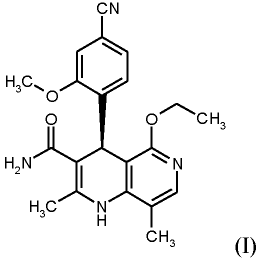 or a hydrate, solvate, pharmaceutically acceptable salt thereof, or a polymorph thereof.

In one embodiment, method for lowering the risk of symptomatic heart failure with left ventricular ejection fraction (LVEF) equal or above 30% in a patient, comprising administering to the patient a therapeutically effective amount of the compound (I) 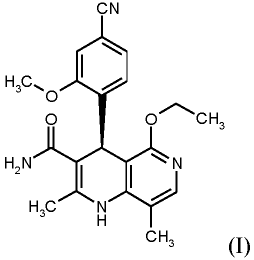 or a hydrate, solvate, pharmaceutically acceptable salt thereof, or a polymorph thereof.

In one embodiment, method for treating symptomatic heart failure with left ventricular ejection fraction (LVEF) of equal or above 40% in a patient, comprising administering to the patient a therapeutically effective amount of the compound (I) 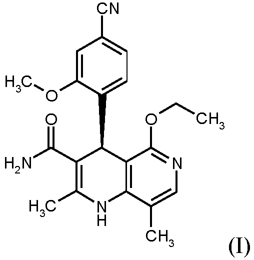 or a hydrate, solvate, pharmaceutically acceptable salt thereof, or a polymorph thereof.

In one embodiment, method for preventing symptomatic heart failure with left ventricular ejection fraction (LVEF) equal or above 40% in a patient, comprising administering to the patient a therapeutically effective amount of the compound (I) 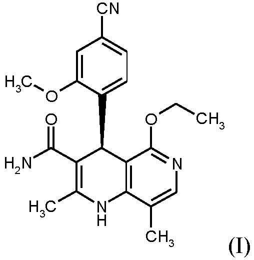 or a hydrate, solvate, pharmaceutically acceptable salt thereof, or a polymorph thereof.

In one embodiment, method for lowering the risk of symptomatic heart failure with left ventricular ejection fraction (LVEF) equal or above 40% in a patient, comprising administering to the patient a therapeutically effective amount of the compound (I) 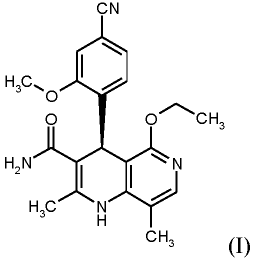 or a hydrate, solvate, pharmaceutically acceptable salt thereof, or a polymorph thereof.

In one embodiment, the method is for reducing the risk of cardiovascular death, hospitalization for heart failure, and/or urgent heart failure visits in patients with heart failure, comprising administering to the patient a therapeutically effective amount of the compound (I) 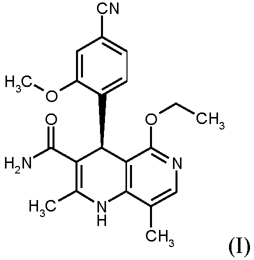 or a hydrate, solvate, pharmaceutically acceptable salt thereof, or a polymorph thereof.

In one embodiment, the method is for reducing the risk of cardiovascular death, hospitalization for heart failure, and/or urgent heart failure visits in patients with heart failure with left ventricular ejection fraction (LVEF) equal or above 30 %, comprising administering to the patient a therapeutically effective amount of the compound (I) 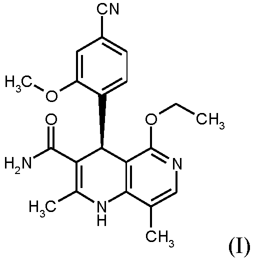 or a hydrate, solvate, pharmaceutically acceptable salt thereof, or a polymorph thereof.

In one embodiment, the method is for reducing the risk of cardiovascular death, hospitalization for heart failure, and/or urgent heart failure visits in patients with heart failure with left ventricular ejection fraction (LVEF) equal or above 40%, comprising administering to the patient a therapeutically effective amount of the compound (I) 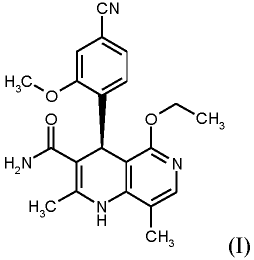 or a hydrate, solvate, pharmaceutically acceptable salt thereof, or a polymorph thereof.

In one embodiment, the method is for reducing the risk of cardiovascular death in patients with heart failure, comprising administering to the patient a therapeutically effective amount of the compound (I) 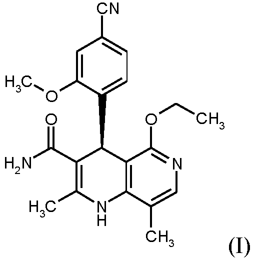 or a hydrate, solvate, pharmaceutically acceptable salt thereof, or a polymorph thereof.

In one embodiment, the method is for reducing the risk of cardiovascular death in patients with heart failure with left ventricular ejection fraction (LVEF) equal or above 30 %, comprising administering to the patient a therapeutically effective amount of the compound (I) 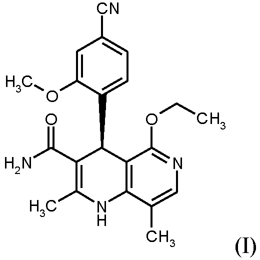 or a hydrate, solvate, pharmaceutically acceptable salt thereof, or a polymorph thereof.

In one embodiment, the method is for reducing the risk of cardiovascular death in patients with heart failure with left ventricular ejection fraction (LVEF) equal or above 40%, comprising administering to the patient a therapeutically effective amount of the compound (I) 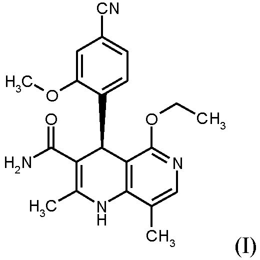 or a hydrate, solvate, pharmaceutically acceptable salt thereof, or a polymorph thereof.

In one embodiment, the method is for reducing the risk of hospitalization for heart failure in patients with heart failure, comprising administering to the patient a therapeutically effective amount of the compound (I) 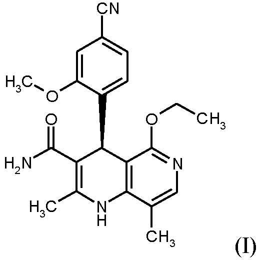 or a hydrate, solvate, pharmaceutically acceptable salt thereof, or a polymorph thereof.

In one embodiment, the method is for reducing the risk of hospitalization for heart failure in patients with heart failure with left ventricular ejection fraction (LVEF) equal or above 30 %, comprising administering to the patient a therapeutically effective amount of the compound (I) 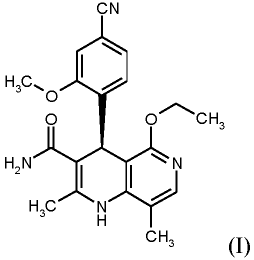 or a hydrate, solvate, pharmaceutically acceptable salt thereof, or a polymorph thereof.

In one embodiment, the method is for reducing the risk of hospitalization for heart failure in patients with heart failure with left ventricular ejection fraction (LVEF) equal or above 40%, comprising administering to the patient a therapeutically effective amount of the compound (I) 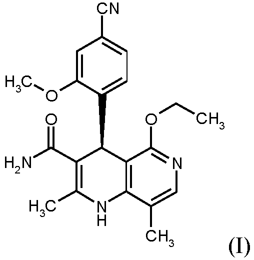 or a hydrate, solvate, pharmaceutically acceptable salt thereof, or a polymorph thereof.

In one embodiment, the method is for reducing the risk of urgent heart failure visits in patients with heart failure, comprising administering to the patient a therapeutically effective amount of the compound (I) 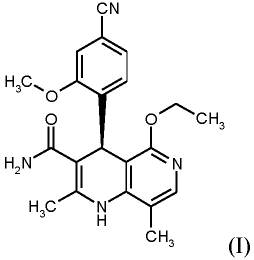 or a hydrate, solvate, pharmaceutically acceptable salt thereof, or a polymorph thereof.

In one embodiment, the method is for reducing the risk of urgent heart failure visits in patients with heart failure with left ventricular ejection fraction (LVEF) of equal or above 30 %, comprising administering to the patient a therapeutically effective amount of the compound (I) 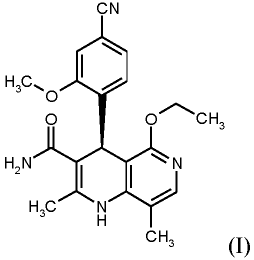 or a hydrate, solvate, pharmaceutically acceptable salt thereof, or a polymorph thereof.

In one embodiment, the method is for reducing the risk of urgent heart failure visits in patients with heart failure with left ventricular ejection fraction (LVEF) of equal or above 40%, comprising administering to the patient a therapeutically effective amount of the compound (I) 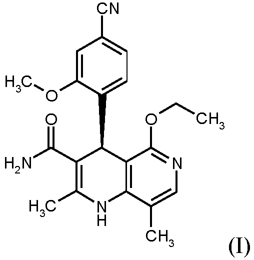 or a hydrate, solvate, pharmaceutically acceptable salt thereof, or a polymorph thereof.

In one embodiment, the method according to the disclosure improves symptoms of heart failure with left ventricular ejection fraction (LVEF) equal or above 40%, comprising administering to the patient a therapeutically effective amount of the compound (I) 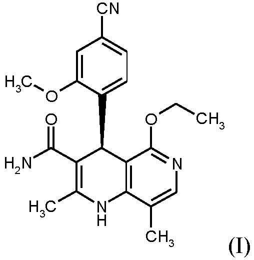 or a hydrate, solvate, pharmaceutically acceptable salt thereof, or a polymorph thereof, the heart failure is selected from symptomatic heart failure, heart failure with improved ejection fraction (HFimpEF), heart failure with mid-range ejection fraction (HFmrEF), heart failure preserved ejection fraction (HFpEF), heart failure with reduced ejection fraction (HFrEF), chronic heart failure (CHF), congestive heart failure, acute heart failure, chronic heart failure, worsening chronic heart failure (WCHF), hospitalization for heart failure.

In one embodiment, the method according to the disclosure prevents worsening of symptoms of heart failure with left ventricular ejection fraction (LVEF) equal or above 40%, comprising administering to the patient a therapeutically effective amount of the compound (I) 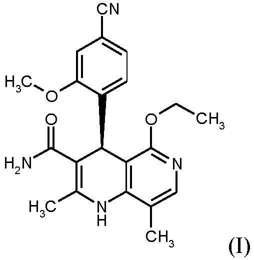 or a hydrate, solvate, pharmaceutically acceptable salt thereof, or a polymorph thereof, the heart failure is selected from symptomatic heart failure, heart failure with improved ejection fraction (HFimpEF), heart failure with mid-range ejection fraction (HFmrEF), heart failure preserved ejection fraction (HFpEF), heart failure with reduced ejection fraction (HFrEF), chronic heart failure (CHF), congestive heart failure, acute heart failure, chronic heart failure, worsening chronic heart failure (WCHF), hospitalization for heart failure.

In one embodiment, the method is for reducing the risk of cardiovascular death, hospitalization for heart failure, and/or urgent heart failure visits in adults with heart failure with left ventricular ejection fraction (LVEF) of ≥ 40%

In one embodiment, the patient has heart failure with left ventricular ejection fraction (LVEF) of equal or below 30 %.

Left ventricular ejection fraction (LVEF) is the central measure of left ventricular function. This indication of how well your heart is pumping out blood can help to diagnose and track heart failure. LVEF is the fraction of chamber volume ejected in systole (stroke volume) in relation to the volume of the blood in the ventricle at the end of diastole (end-diastolic volume). It is a measurement, expressed as a percentage (%), of how much blood the left ventricle pumps out with each contraction. An LVEF of 60 percent means that 60 percent of the total amount of blood in the left ventricle is pushed out with each heartbeat. A normal heart's LVEF is between 55 and 70 percent. The simplest classification as per the American College of Cardiology (ACC) that is used clinically as follows:
- Hyperdynamic = LVEF greater than 70%
- Normal = LVEF 50% to 70% (midpoint 60%)
- Mild dysfunction = LVEF 40% to 49% (midpoint 45%)
- Moderate dysfunction = LVEF 30% to 39% (midpoint 35%)
- Severe dysfunction = LVEF less than 30%

In one embodiment, the patient has a LVEF selected from less than 30%, 30% to 39%, 40% to 49%, 45 %, 50% to 70%, 60 %, and greater than 70%. In one embodiment, the patient has a LVEF of selected from equal or above 70%, equal or above 50 % to equal or below 70 %, equal or above 60%, equal or below 60 %, equal or above 50 % to equal or below 60 %, equal or above 45 %, equal or below 45 %, and equal or above 40 % to equal or below 49 %.

In one embodiment, the patient has a LVEF of equal or above 30%, 35%, 39%, 40%, 45 %, 49%, 50 %, 55%, 65 % or 70 %. In one embodiment, the patient has a LVEF equal or above 30 %. In one embodiment, the patient has a LVEF equal or above 35 %. In one embodiment, the patient has a LVEF equal or above 39 %. In one embodiment, the patient has a LVEF equal or above 40 %. In one embodiment, the patient has a LVEF equal or above 45 %. In one embodiment, the patient has a LVEF equal or above 49 %. In one embodiment, the patient has a LVEF equal or above 50 %. In one embodiment, the patient has a LVEF equal or above 55 %. In one embodiment, the patient has a LVEF equal or above 59 %. In one embodiment, the patient has a LVEF equal or above 60 %. In one embodiment, the patient has a LVEF equal or above 65 %. In one embodiment, the patient has a LVEF equal or above 70 %. In one embodiment, the patient has a LVEF equal or above 75 %.

In one embodiment, the patient has a LVEF equal or below 30%, 35%, 39%, 40%, 45 %, 49%, 50 %, 55%, 65 % or 70 %. In one embodiment, the patient has a LVEF equal or below 30 %. In one embodiment, the patient has a LVEF equal or below 35 %. In one embodiment, the patient has a LVEF equal or below 39 %. In one embodiment, the patient has a LVEF equal or below 40 %. In one embodiment, the patient has a LVEF equal or below 45 %. In one embodiment, the patient has a LVEF equal or below 49 %. In one embodiment, the patient has a LVEF equal or below 50 %. In one embodiment, the patient has a LVEF equal or below 55 %. In one embodiment, the patient has a LVEF equal or below 59 %. In one embodiment, the patient has a LVEF equal or below 60 %. In one embodiment, the patient has a LVEF equal or below 65 %. In one embodiment, the patient has a LVEF equal or below 70 %. In one embodiment, the patient has a LVEF equal or below 75 %.

In one embodiment, the patient has a LVEF selected from 44+/-3 %, equal or above 41 to equal or below 47 %, equal or above 41 to equal or below 44 %, equal or above 44 to equal or below 47 %, 54+/-3 %, equal or above 51 to equal or below 57 %, equal or above 51 to equal or below 54 %, equal or above 54 to equal or below 57 %, 64+/-5 %, equal or above 59 to equal or below 69 %, equal or above 59 to equal or below 64 %, equal or above 64 to equal or below 69 %, 53+/-8 %, equal or above 45 to equal or below 61 %, equal or above 45 to equal or below 53 %, and equal or above 53 to equal or below 61 %.

In one embodiment, the patient has heart failure according to NYHA class II, III or IV, and a LVEF of equal or above 30%, 35%, 39%, 40%, 45 %, 49%, 50 %, 55%, 65 % or 70 %. In one embodiment, the patient has heart failure according to NYHA class II, III or IV, and a LVEF of equal or below 30%, 35%, 39%, 40%, 45 %, 49%, 50 %, 55%, 65 % or 70 %.

In one embodiment, the patient has heart failure according to NYHA class II, and a LVEF of equal or above 30%, 35%, 39%, 40%, 45 %, 49%, 50 %, 55%, 60, 65 % or 70 %. In one embodiment, the patient has heart failure according to NYHA class II, and a LVEF of equal or above 30%. In one embodiment, the patient has heart failure according to NYHA class II, and a LVEF equal or above 35%. In one embodiment, the patient has heart failure according to NYHA class II, and a LVEF equal or above 39%. In one embodiment, the patient has heart failure according to NYHA class II, and a LVEF of equal or above 40%. In one embodiment, the patient has heart failure according to NYHA class II, and a LVEF of equal or above 45 %. In one embodiment, the patient has heart failure according to NYHA class II, and a LVEF of equal or above 49%. In one embodiment, the patient has heart failure according to NYHA class II, and a LVEF of equal or above 50 %. In one embodiment, the patient has heart failure according to NYHA class II, and a LVEF of equal or above 55%. In one embodiment, the patient has heart failure according to NYHA class II, and a LVEF of equal or above 60%. In one embodiment, the patient has heart failure according to NYHA class II, and a LVEF of equal or above 65 %. In one embodiment, the patient has heart failure according to NYHA class II, and a LVEF of equal or above 70 %.

In one embodiment, the patient has heart failure according to NYHA class II, and a LVEF of equal or below 30%, 35%, 39%, 40%, 45 %, 49%, 50 %, 55%, 60, 65 % or 70 %. In one embodiment, the patient has heart failure according to NYHA class II, and a LVEF of equal or below 30%. In one embodiment, the patient has heart failure according to NYHA class II, and a LVEF of equal or below 35%. In one embodiment, the patient has heart failure according to NYHA class II, and a LVEF of equal or below 39%. In one embodiment, the patient has heart failure according to NYHA class II, and a LVEF of equal or below 40%. In one embodiment, the patient has heart failure according to NYHA class II, and a LVEF of equal or below 45 %. In one embodiment, the patient has heart failure according to NYHA class II, and a LVEF of equal or below 49%. In one embodiment, the patient has heart failure according to NYHA class II, and a LVEF of equal or below 50 %. In one embodiment, the patient has heart failure according to NYHA class II, and a LVEF of equal or below 55%. In one embodiment, the patient has heart failure according to NYHA class II, and a LVEF of equal or below 60%. In one embodiment, the patient has heart failure according to NYHA class II, and a LVEF of equal or below 65 %. In one embodiment, the patient has heart failure according to NYHA class II, and a LVEF of equal or below 70 %.

In one embodiment, the patient has heart failure according to NYHA class III, and a LVEF of equal or above 30%, 35%, 39%, 40%, 45 %, 49%, 50 %, 55%, 60, 65 % or 70 %. In one embodiment, the patient has heart failure according to NYHA class III, and a LVEF of equal or above 30%. In one embodiment, the patient has heart failure according to NYHA class III, and a LVEF of equal or above 35%. In one embodiment, the patient has heart failure according to NYHA class III, and a LVEF of equal or above 39%. In one embodiment, the patient has heart failure according to NYHA class III, and a LVEF of equal or above 40%. In one embodiment, the patient has heart failure according to NYHA class III, and a LVEF of equal or above 45 %. In one embodiment, the patient has heart failure according to NYHA class III, and a LVEF of equal or above 49%. In one embodiment, the patient has heart failure according to NYHA class III, and a LVEF of equal or above 50 %. In one embodiment, the patient has heart failure according to NYHA class III, and a LVEF of equal or above 55%. In one embodiment, the patient has heart failure according to NYHA class III, and a LVEF of equal or above 60%. In one embodiment, the patient has heart failure according to NYHA class III, and a LVEF of equal or above 65 %. In one embodiment, the patient has heart failure according to NYHA class III, and a LVEF of equal or above 70 %.

In one embodiment, the patient has heart failure according to NYHA class III, and a LVEF of equal or below 30%, 35%, 39%, 40%, 45 %, 49%, 50 %, 55%, 60, 65 % or 70 %. In one embodiment, the patient has heart failure according to NYHA class III, and a LVEF of equal or below 30%. In one embodiment, the patient has heart failure according to NYHA class III, and a LVEF of equal or below 35%. In one embodiment, the patient has heart failure according to NYHA class III, and a LVEF of equal or below 39%. In one embodiment, the patient has heart failure according to NYHA class III, and a LVEF of equal or below 40%. In one embodiment, the patient has heart failure according to NYHA class III, and a LVEF of equal or below 45 %. In one embodiment, the patient has heart failure according to NYHA class III, and a LVEF of equal or below 49%. In one embodiment, the patient has heart failure according to NYHA class III, and a LVEF of equal or below 50 %. In one embodiment, the patient has heart failure according to NYHA class III, and a LVEF of equal or below 55%. In one embodiment, the patient has heart failure according to NYHA class III, and a LVEF of equal or below 60%. In one embodiment, the patient has heart failure according to NYHA class III, and a LVEF of equal or below 65 %. In one embodiment, the patient has heart failure according to NYHA class III, and a LVEF of equal or below 70 %.

In one embodiment, the patient has heart failure according to NYHA class IV, and a LVEF of equal or above 30%, 35%, 39%, 40%, 45 %, 49%, 50 %, 55%, 60, 65 % or 70 %. In one embodiment, the patient has heart failure according to NYHA class IV, and a LVEF of equal or above 30%. In one embodiment, the patient has heart failure according to NYHA class IV, and a LVEF of equal or above 35%. In one embodiment, the patient has heart failure according to NYHA class IV, and a LVEF of equal or above 39%. In one embodiment, the patient has heart failure according to NYHA class IV, and a LVEF of equal or above 40%. In one embodiment, the patient has heart failure according to NYHA class IV, and a LVEF of equal or above 45 %. In one embodiment, the patient has heart failure according to NYHA class IV, and a LVEF of equal or above 49%. In one embodiment, the patient has heart failure according to NYHA class IV, and a LVEF of equal or above 50 %. In one embodiment, the patient has heart failure according to NYHA class IV, and a LVEF of equal or above 55%. In one embodiment, the patient has heart failure according to NYHA class IV, and a LVEF of equal or above 60%. In one embodiment, the patient has heart failure according to NYHA class IV, and a LVEF of equal or above 65 %. In one embodiment, the patient has heart failure according to NYHA class IV, and a LVEF of equal or above 70 %.

In one embodiment, the patient has heart failure according to NYHA class IV, and a LVEF of equal or below 30%, 35%, 39%, 40%, 45 %, 49%, 50 %, 55%, 60, 65 % or 70 %. In one embodiment, the patient has heart failure according to NYHA class IV, and a LVEF of equal or below 30%. In one embodiment, the patient has heart failure according to NYHA class IV, and a LVEF of equal or below 35%. In one embodiment, the patient has heart failure according to NYHA class IV, and a LVEF of equal or below 39%. In one embodiment, the patient has heart failure according to NYHA class IV, and a LVEF of equal or below 40%. In one embodiment, the patient has heart failure according to NYHA class IV, and a LVEF of equal or below 45 %. In one embodiment, the patient has heart failure according to NYHA class IV, and a LVEF of equal or below 49%. In one embodiment, the patient has heart failure according to NYHA class IV, and a LVEF of equal or below 50 %. In one embodiment, the patient has heart failure according to NYHA class IV, and a LVEF of equal or below 55%. In one embodiment, the patient has heart failure according to NYHA class IV, and a LVEF of equal or below 60%. In one embodiment, the patient has heart failure according to NYHA class IV, and a LVEF of equal or below 65 %. In one embodiment, the patient has heart failure according to NYHA class IV, and a LVEF of equal or below 70 %.

In one embodiment, the patient has heart failure according to
- NYHA II and LVEF equal or above 50 % to equal or below 60 %
- NYHA II and LVEF equal or above 40 % to equal or below 60 %,
- NYHA II and LVEF equal or above 40 % to equal or below 50 %,
- NYHA II and LVEF 50% to 70%,
- NYHA II and LVEF 40% to 49%,
- NYHA II and LVEF 30% to 39%,
- NYHA III and LVEF equal or above 50 % to equal or below 60 %
- NYHA III and LVEF equal or above 40 % to equal or below 60 %,
- NYHA III and LVEF equal or above 40 % to equal or below 50 %,
- NYHA III and LVEF 50% to 70%,
- NYHA III and LVEF 40% to 49%,
- NYHA III and LVEF 30% to 39%,
- NYHA IV and LVEF equal or above 50 % to equal or below 60 %
- NYHA IV and LVEF equal or above 40 % to equal or below 60 %,
- NYHA IV and LVEF equal or above 40 % to equal or below 50 %,
- NYHA IV and LVEF 50% to 70%,
- NYHA IV and LVEF 40% to 49%, or
- NYHA IV and LVEF 30% to 39%.

In one embodiment, the patient has an estimated glomerular filtration rate (eGFR) equal or above 15 mL/min/1.73m².

Glomerular filtration rate (GFR) is the volume of fluid filtered from the renal (kidney) glomerular capillaries into the Bowman's capsule per unit time. The GFR is typically recorded in units of volume per time, e.g., milliliters per minute (mL/min). eGFR is estimated GFR (eGFR) and is a mathematically derived entity based on a patient's serum creatinine level, age, sex and race. This is usually calculated by the laboratory analyzing the blood sample and reported along with the serum creatinine result. "Normal" GFR is approximately 100 but you will often see it reported as >90 ("greater than 90") or >60 ("greater than 60"). It is for this reason that patients (and some doctors) sometimes quote the eGFR as a percentage of normal kidney function. eGFR is expressed as mL/min/1.73m².

In one embodiment, the patient has an eGFR of selected from equal or above 15, 20, 25, 30, 35, 40, 45, 50, 55, 60, 65, 70, 75, 80, 85, and 90 mL/min/1.73m². In one embodiment, the patient has an eGFR of equal or above 15 mL/min/1.73m². In one embodiment, the patient has an eGFR of equal or above 20 mL/min/1.73m². In one embodiment, the patient has an eGFR of equal or above 25 mL/min/1.73m². In one embodiment, the patient has an eGFR of equal or above 30 mL/min/1.73m². In one embodiment, the patient has an eGFR of equal or above 35 mL/min/1.73m². In one embodiment, the patient has an eGFR of equal or above 40 mL/min/1.73m². In one embodiment, the patient has an eGFR of equal or above 45 mL/min/1.73m². In one embodiment, the patient has an eGFR of equal or above 50 mL/min/1.73m². In one embodiment, the patient has an eGFR of equal or above 55 mL/min/1.73m². In one embodiment, the patient has an eGFR of equal or above 60 mL/min/1.73m². In one embodiment, the patient has an eGFR of equal or above 65 mL/min/1.73m². In one embodiment, the patient has an eGFR of equal or above 70 mL/min/1.73m². In one embodiment, the patient has an eGFR of equal or above 75 mL/min/1.73m². In one embodiment, the patient has an eGFR of equal or above 80 mL/min/1.73m². In one embodiment, the patient has an eGFR of equal or above 85 mL/min/1.73m². In one embodiment, the patient has an eGFR of equal or above 90 mL/min/1.73m².

In one embodiment, the patient has an eGFR of selected from equal or below 15, 20, 25, 30, 35, 40, 45, 50, 55, 60, 65, 70, 75, 80, 85, and 90 mL/min/1.73m². In one embodiment, the patient has an eGFR of equal or below 15 mL/min/1.73m². In one embodiment, the patient has an eGFR of equal or below 20 mL/min/1.73m². In one embodiment, the patient has an eGFR of equal or below 25 mL/min/1.73m². In one embodiment, the patient has an eGFR of equal or below 30 mL/min/1.73m². In one embodiment, the patient has an eGFR of equal or below 35 mL/min/1.73m². In one embodiment, the patient has an eGFR of equal or below 40 mL/min/1.73m². In one embodiment, the patient has an eGFR of equal or below 45 mL/min/1.73m². In one embodiment, the patient has an eGFR of equal or below 50 mL/min/1.73m². In one embodiment, the patient has an eGFR of equal or below 55 mL/min/1.73m². In one embodiment, the patient has an eGFR of equal or below 60 mL/min/1.73m². In one embodiment, the patient has an eGFR of equal or below 65 mL/min/1.73m². In one embodiment, the patient has an eGFR of equal or below 70 mL/min/1.73m². In one embodiment, the patient has an eGFR of equal or below 75 mL/min/1.73m². In one embodiment, the patient has an eGFR of equal or below 80 mL/min/1.73m². In one embodiment, the patient has an eGFR of equal or below 85 mL/min/1.73m². In one embodiment, the patient has an eGFR of equal or below 90 mL/min/1.73m².

In one embodiment, the patient has an eGFR of equal or above 15 to equal or below 90 mL/min/1.73m². In one embodiment, the patient has an eGFR of equal or above 15 to equal or below 60 mL/min/1.73m². In one embodiment, the patient has an eGFR of equal or above 15 to equal or below 45 mL/min/1.73m². In one embodiment, the patient has an eGFR of equal or above 15 to equal or below 30 mL/min/1.73m². In one embodiment, the patient has an eGFR of equal or above 25 to equal or below 90 mL/min/1.73m². In one embodiment, the patient has an eGFR of equal or above 25 to equal or below 60 mL/min/1.73m². In one embodiment, the patient has an eGFR of equal or above 25 to equal or below 45 mL/min/1.73m². In one embodiment, the patient has an eGFR of equal or above 25 to equal or below 30 mL/min/1.73m². In one embodiment, the patient has an eGFR of equal or above 30 to equal or below 90 mL/min/1.73m².

In one embodiment, the patient has an eGFR of equal or above 30 to equal or below 60 mL/min/1.73m². In one embodiment, the patient has an eGFR of equal or above 30 to equal or below 45 mL/min/1.73m². In one embodiment, the patient has an eGFR of equal or above 45 to equal or below 90 mL/min/1.73m². In one embodiment, the patient has an eGFR of equal or above 45 to equal or below 60 mL/min/1.73m². In one embodiment, the patient has an eGFR of equal or above 60 to equal or below 90 mL/min/1,73m².

In one embodiment, the patient has an Urine Albumin to Creatinine Ratio (UACR) of equal or above 5 mg/g.

The Urine Albumin to Creatinine Ratio ratio is a result of measuring albumin in urine. The albumin concentration is not related to the urine volume, but to the creatinine concentration in the urine. The unit is "mg/g". The usual ranges are as follows: "normal": below 30 mg/g; Microalbuminuria: 30 to 300 mg/g; Macroalbuminuria: 300 to 3000 mg/g; "Major proteinuria": above 3,000 mg/g. Albuminuria, as assessed via UACR estimations, serves as an early marker of cardiovascular and kidney disease as well as an important marker in predicting the risk for development and progression of HF.

In one embodiment, the patient has an UACR of equal or above 10 mg/g. In one embodiment, the patient has an UACR of equal or above 15mg/g. In one embodiment, the patient has an UACR of equal or above 20 mg/g. In one embodiment, the patient has an UACR of equal or above 25 mg/g. In one embodiment, the patient has an UACR of equal or above 30mg/g. In one embodiment, the patient has an UACR of equal or above 35mg/g. In one embodiment, the patient has an UACR of equal or above 40 mg/g. In one embodiment, the patient has an UACR of equal or above 45mg/g. In one embodiment, the patient has an UACR of equal or above 50mg/g. In one embodiment, the patient has an UACR of equal or above 55mg/g. In one embodiment, the patient has an UACR of equal or above 60mg/g. In one embodiment, the patient has an UACR of equal or above 65mg/g. In one embodiment, the patient has an UACR of equal or above 70mg/g. In one embodiment, the patient has an UACR of equal or above 75mg/g. In one embodiment, the patient has an UACR of equal or above 80mg/g. In one embodiment, the patient has an UACR of equal or above 90mg/g. In one embodiment, the patient has an UACR of equal or above 95mg/g. In one embodiment, the patient has an UACR of equal or above 100mg/g. In one embodiment, the patient has an UACR of equal or above 105mg/g. In one embodiment, the patient has an UACR of equal or above 1 10mg/g. In one embodiment, the patient has an UACR of equal or above 125mg/g. In one embodiment, the patient has an UACR of equal or above 130mg/g. In one embodiment, the patient has an UACR of equal or above 135mg/g. In one embodiment, the patient has an UACR of equal or above 140mg/g. In one embodiment, the patient has an UACR of equal or above 145mg/g. In one embodiment, the patient has an UACR of equal or above 150mg/g. In one embodiment, the patient has an UACR of equal or above 155mg/g. In one embodiment, the patient has an UACR of equal or above 160mg/g. In one embodiment, the patient has an UACR of equal or above 165mg/g. In one embodiment, the patient has an UACR of equal or above 170mg/g. In one embodiment, the patient has an UACR of equal or above 175mg/g. In one embodiment, the patient has an UACR of equal or above 180mg/g. In one embodiment, the patient has an UACR of equal or above 185mg/g. In one embodiment, the patient has an UACR of equal or above 190mg/g. In one embodiment, the patient has an UACR of equal or above 195mg/g. In one embodiment, the patient has an UACR of equal or above 200mg/g. In one embodiment, the patient has an UACR of equal or above 205mg/g. In one embodiment, the patient has an UACR of equal or above 210mg/g. In one embodiment, the patient has an UACR of equal or above 225mg/g. In one embodiment, the patient has an UACR of equal or above 230mg/g. In one embodiment, the patient has an UACR of equal or above 235mg/g. In one embodiment, the patient has an UACR of equal or above 240mg/g. In one embodiment, the patient has an UACR of equal or above 245mg/g. In one embodiment, the patient has an UACR of equal or above 250mg/g. In one embodiment, the patient has an UACR of equal or above 255mg/g. In one embodiment, the patient has an UACR of equal or above 260mg/g. In one embodiment, the patient has an UACR of equal or above 265mg/g. In one embodiment, the patient has an UACR of equal or above 270mg/g. In one embodiment, the patient has an UACR of equal or above 275mg/g. In one embodiment, the patient has an UACR of equal or above 280mg/g. In one embodiment, the patient has an UACR of equal or above 285mg/g. In one embodiment, the patient has an UACR of equal or above 290mg/g. In one embodiment, the patient has an UACR of equal or above 295mg/g. In one embodiment, the patient has an UACR of equal or above 300mg/g. In one embodiment, the patient has an UACR of equal or above 305mg/g. In one embodiment, the patient has an UACR of equal or above 310mg/g. In one embodiment, the patient has an UACR of equal or above 325mg/g. In one embodiment, the patient has an UACR of equal or above 330mg/g. In one embodiment, the patient has an UACR of equal or above 335mg/g. In one embodiment, the patient has an UACR of equal or above 340mg/g. In one embodiment, the patient has an UACR of equal or above 345mg/g. In one embodiment, the patient has an UACR of equal or above 350mg/g. In one embodiment, the patient has an UACR of equal or above 355mg/g. In one embodiment, the patient has an UACR of equal or above 360mg/g. In one embodiment, the patient has an UACR of equal or above 365mg/g. In one embodiment, the patient has an UACR of equal or above 370mg/g. In one embodiment, the patient has an UACR of equal or above 375mg/g. In one embodiment, the patient has an UACR of equal or above 380mg/g. In one embodiment, the patient has an UACR of equal or above 385mg/g. In one embodiment, the patient has an UACR of equal or above 390mg/g. In one embodiment, the patient has an UACR of equal or above 395mg/g. In one embodiment, the patient has an UACR of equal or above 400mg/g. In one embodiment, the patient has an UACR of equal or above 405mg/g. In one embodiment, the patient has an UACR of equal or above 410mg/g. In one embodiment, the patient has an UACR of equal or above 425mg/g. In one embodiment, the patient has an UACR of equal or above 430mg/g. In one embodiment, the patient has an UACR of equal or above 435mg/g. In one embodiment, the patient has an UACR of equal or above 440mg/g. In one embodiment, the patient has an UACR of equal or above 445mg/g. In one embodiment, the patient has an UACR of equal or above 450mg/g. In one embodiment, the patient has an UACR of equal or above 455mg/g. In one embodiment, the patient has an UACR of equal or above 460mg/g. In one embodiment, the patient has an UACR of equal or above 465mg/g. In one embodiment, the patient has an UACR of equal or above 470mg/g. In one embodiment, the patient has an UACR of equal or above 475mg/g. In one embodiment, the patient has an UACR of equal or above 480mg/g. In one embodiment, the patient has an UACR of equal or above 485mg/g. In one embodiment, the patient has an UACR of equal or above 490mg/g. In one embodiment, the patient has an UACR of equal or above 495mg/g. In one embodiment, the patient has an UACR of equal or above 500mg/g. In one embodiment, the patient has an UACR of equal or above 505mg/g. In one embodiment, the patient has an UACR of equal or above 510mg/g. In one embodiment, the patient has an UACR of equal or above 515mg/g. In one embodiment, the patient has an UACR of equal or above 520mg/g. In one embodiment, the patient has an UACR of equal or above 525mg/g. In one embodiment, the patient has an UACR of equal or above 530mg/g. In one embodiment, the patient has an UACR of equal or above 535mg/g. In one embodiment, the patient has an UACR of equal or above 540mg/g. In one embodiment, the patient has an UACR of equal or above 550mg/g. In one embodiment, the patient has an UACR of equal or above 555mg/g. In one embodiment, the patient has an UACR of equal or above 560mg/g. In one embodiment, the patient has an UACR of equal or above 565mg/g. In one embodiment, the patient has an UACR of equal or above 570mg/g. In one embodiment, the patient has an UACR of equal or above 575mg/g. In one embodiment, the patient has an UACR of equal or above 580mg/g. In one embodiment, the patient has an UACR of equal or above 585mg/g. In one embodiment, the patient has an UACR of equal or above 590mg/g. In one embodiment, the patient has an UACR of equal or above 595 mg/g. In one embodiment, the patient has an UACR of equal or above 600 mg/g. In one embodiment, the patient has an UACR of equal or above 700 mg/g.

In one embodiment, the patient has an UACR of equal or below 5 mg/g. In one embodiment, the patient has an UACR of equal or below 10mg/g. In one embodiment, the patient has an UACR of equal or below 15mg/g. In one embodiment, the patient has an UACR of equal or below 20mg/g. In one embodiment, the patient has an UACR of equal or below 25mg/g. In one embodiment, the patient has an UACR of equal or below 30mg/g. In one embodiment, the patient has an UACR of equal or below 35mg/g. In one embodiment, the patient has an UACR of equal or below 40mg/g. In one embodiment, the patient has an UACR of equal or below 45mg/g. In one embodiment, the patient has an UACR of equal or below 50mg/g. In one embodiment, the patient has an UACR of equal or below 55mg/g. In one embodiment, the patient has an UACR of equal or below 60mg/g. In one embodiment, the patient has an UACR of equal or below 65mg/g. In one embodiment, the patient has an UACR of equal or below 70mg/g. In one embodiment, the patient has an UACR of equal or below 75mg/g. In one embodiment, the patient has an UACR of equal or below 80mg/g. In one embodiment, the patient has an UACR of equal or below 90mg/g. In one embodiment, the patient has an UACR of equal or below 95mg/g. In one embodiment, the patient has an UACR of equal or below 100mg/g. In one embodiment, the patient has an UACR of equal or below 105mg/g. In one embodiment, the patient has an UACR of equal or below 1 10mg/g. In one embodiment, the patient has an UACR of equal or below 125mg/g. In one embodiment, the patient has an UACR of equal or below 130mg/g. In one embodiment, the patient has an UACR of equal or below 135mg/g. In one embodiment, the patient has an UACR of equal or below 140mg/g. In one embodiment, the patient has an UACR of equal or below 145mg/g. In one embodiment, the patient has an UACR of equal or below 150mg/g. In one embodiment, the patient has an UACR of equal or below 155mg/g. In one embodiment, the patient has an UACR of equal or below 160mg/g. In one embodiment, the patient has an UACR of equal or below 165mg/g. In one embodiment, the patient has an UACR of equal or below 170mg/g. In one embodiment, the patient has an UACR of equal or below 175mg/g. In one embodiment, the patient has an UACR of equal or below 180mg/g. In one embodiment, the patient has an UACR of equal or below 185mg/g. In one embodiment, the patient has an UACR of equal or below 190mg/g. In one embodiment, the patient has an UACR of equal or below 195mg/g. In one embodiment, the patient has an UACR of equal or below 200mg/g. In one embodiment, the patient has an UACR of equal or below 205mg/g. In one embodiment, the patient has an UACR of equal or below 210mg/g. In one embodiment, the patient has an UACR of equal or below 225mg/g. In one embodiment, the patient has an UACR of equal or below 230mg/g. In one embodiment, the patient has an UACR of equal or below 235mg/g. In one embodiment, the patient has an UACR of equal or below 240mg/g. In one embodiment, the patient has an UACR of equal or below 245mg/g. In one embodiment, the patient has an UACR of equal or below 250mg/g. In one embodiment, the patient has an UACR of equal or below 255mg/g. In one embodiment, the patient has an UACR of equal or below 260mg/g. In one embodiment, the patient has an UACR of equal or below 265mg/g. In one embodiment, the patient has an UACR of equal or below 270mg/g. In one embodiment, the patient has an UACR of equal or below 275mg/g. In one embodiment, the patient has an UACR of equal or below 280mg/g. In one embodiment, the patient has an UACR of equal or below 285mg/g. In one embodiment, the patient has an UACR of equal or below 290mg/g. In one embodiment, the patient has an UACR of equal or below 295mg/g. In one embodiment, the patient has an UACR of equal or below 300mg/g. In one embodiment, the patient has an UACR of equal or below 305mg/g. In one embodiment, the patient has an UACR of equal or below 310mg/g. In one embodiment, the patient has an UACR of equal or below 325mg/g. In one embodiment, the patient has an UACR of equal or below 330mg/g. In one embodiment, the patient has an UACR of equal or below 335mg/g. In one embodiment, the patient has an UACR of equal or below 340mg/g. In one embodiment, the patient has an UACR of equal or below 345mg/g. In one embodiment, the patient has an UACR of equal or below 350mg/g. In one embodiment, the patient has an UACR of equal or below 355mg/g. In one embodiment, the patient has an UACR of equal or below 360mg/g. In one embodiment, the patient has an UACR of equal or below 365mg/g. In one embodiment, the patient has an UACR of equal or below 370mg/g. In one embodiment, the patient has an UACR of equal or below 375mg/g. In one embodiment, the patient has an UACR of equal or below 380mg/g. In one embodiment, the patient has an UACR of equal or below 385mg/g. In one embodiment, the patient has an UACR of equal or below 390mg/g. In one embodiment, the patient has an UACR of equal or below 395mg/g. In one embodiment, the patient has an UACR of equal or below 400mg/g. In one embodiment, the patient has an UACR of equal or below 405mg/g. In one embodiment, the patient has an UACR of equal or below 410mg/g. In one embodiment, the patient has an UACR of equal or below 425mg/g. In one embodiment, the patient has an UACR of equal or below 430mg/g. In one embodiment, the patient has an UACR of equal or below 435mg/g. In one embodiment, the patient has an UACR of equal or below 440mg/g. In one embodiment, the patient has an UACR of equal or below 445mg/g. In one embodiment, the patient has an UACR of equal or below 450mg/g. In one embodiment, the patient has an UACR of equal or below 455mg/g. In one embodiment, the patient has an UACR of equal or below 460mg/g. In one embodiment, the patient has an UACR of equal or below 465mg/g. In one embodiment, the patient has an UACR of equal or below 470mg/g. In one embodiment, the patient has an UACR of equal or below 475mg/g. In one embodiment, the patient has an UACR of equal or below 480mg/g. In one embodiment, the patient has an UACR of equal or below 485mg/g. In one embodiment, the patient has an UACR of equal or below 490mg/g. In one embodiment, the patient has an UACR of equal or below 495mg/g. In one embodiment, the patient has an UACR of equal or below 500mg/g. In one embodiment, the patient has an UACR of equal or below 505mg/g. In one embodiment, the patient has an UACR of equal or below 5 10mg/g. In one embodiment, the patient has an UACR of equal or below 515mg/g. In one embodiment, the patient has an UACR of equal or below 520mg/g. In one embodiment, the patient has an UACR of equal or below 525mg/g. In one embodiment, the patient has an UACR of equal or below 530mg/g. In one embodiment, the patient has an UACR of equal or below 535mg/g. In one embodiment, the patient has an UACR of equal or below 540mg/g. In one embodiment, the patient has an UACR of equal or below 550mg/g. In one embodiment, the patient has an UACR of equal or below 555mg/g. In one embodiment, the patient has an UACR of equal or below 560mg/g. In one embodiment, the patient has an UACR of equal or below 565mg/g. In one embodiment, the patient has an UACR of equal or below 570mg/g. In one embodiment, the patient has an UACR of equal or below 575mg/g. In one embodiment, the patient has an UACR of equal or below 580mg/g. In one embodiment, the patient has an UACR of equal or below 585mg/g. In one embodiment, the patient has an UACR of equal or below 590mg/g. In one embodiment, the patient has an UACR of equal or below 595 mg/g. In one embodiment, the patient has an UACR of equal or below 600 mg/g. In one embodiment, the patient has an UACR of equal or below 700 mg/g. In one embodiment, the patient has an UACR of equal or below 800 mg/g. In one embodiment, the patient has an UACR of equal or below 900 mg/g. In one embodiment, the patient has an UACR of equal or below 1000 mg/g. In one embodiment, the patient has an UACR of equal or below 2000 mg/g. In one embodiment, the patient has an UACR of equal or below 3000 mg/g. In one embodiment, the patient has an UACR of equal or below 4000 mg/g. In one embodiment, the patient has an UACR of equal or below 5000 mg/g.

In one embodiment, the patient has an UACR of equal or above 5 to equal or below 5000 mg/g. In one embodiment, the patient has an UACR of equal or above 5 to equal or below 4000 mg/g. In one embodiment, the patient has an UACR of equal or above 5 to equal or below 3000 mg/g. In one embodiment, the patient has an UACR of equal or above 5 to equal or below 2000 mg/g. In one embodiment, the patient has an UACR of equal or above 5 to equal or below 1000 mg/g. In one embodiment, the patient has an UACR of equal or above 5 to equal or below 500 mg/g. In one embodiment, the patient has an UACR of equal or above 5 to equal or below 400 mg/g. In one embodiment, the patient has an UACR of equal or above 5 to equal or below 300 mg/g. In one embodiment, the patient has an UACR of equal or above 5 to equal or below 200 mg/g. In one embodiment, the patient has an UACR of equal or above 5 to equal or below 100 mg/g. In one embodiment, the patient has an UACR of equal or above 10 to equal or below 5000 mg/g. In one embodiment, the patient has an UACR of equal or above 10 to equal or below 4000 mg/g. In one embodiment, the patient has an UACR of equal or above 10 to equal or below 3000 mg/g. In one embodiment, the patient has an UACR of equal or above 10 to equal or below 2000 mg/g. In one embodiment, the patient has an UACR of equal or above 10 to equal or below 1000 mg/g. In one embodiment, the patient has an UACR of equal or above 10 to equal or below 500 mg/g. In one embodiment, the patient has an UACR of equal or above 10 to equal or below 400 mg/g. In one embodiment, the patient has an UACR of equal or above 10 to equal or below 300 mg/g. In one embodiment, the patient has an UACR of equal or above 10 to equal or below 200 mg/g. In one embodiment, the patient has an UACR of equal or above 10 to equal or below 100 mg/g. In one embodiment, the patient has an UACR of equal or above 100 to equal or below 5000 mg/g. In one embodiment, the patient has an UACR of equal or above 100 to equal or below 4000 mg/g. In one embodiment, the patient has an UACR of equal or above 100 to equal or below 3000 mg/g. In one embodiment, the patient has an UACR of equal or above 100 to equal or below 2000 mg/g. In one embodiment, the patient has an UACR of equal or above 100 to equal or below 1000 mg/g. In one embodiment, the patient has an UACR of equal or above 100 to equal or below 500 mg/g. In one embodiment, the patient has an UACR of equal or above 100 to equal or below 400 mg/g. In one embodiment, the patient has an UACR of equal or above 100 to equal or below 300 mg/g. In one embodiment, the patient has an UACR of equal or above 100 to equal or below 200 mg/g. In one embodiment, the patient has an UACR of equal or above 200 to equal or below 5000 mg/g. In one embodiment, the patient has an UACR of equal or above 200 to equal or below 4000 mg/g. In one embodiment, the patient has an UACR of equal or above 200 to equal or below 3000 mg/g. In one embodiment, the patient has an UACR of equal or above 200 to equal or below 2000 mg/g. In one embodiment, the patient has an UACR of equal or above 200 to equal or below 1000 mg/g. In one embodiment, the patient has an UACR of equal or above 200 to equal or below 500 mg/g. In one embodiment, the patient has an UACR of equal or above 200 to equal or below 400 mg/g. In one embodiment, the patient has an UACR of equal or above 200 to equal or below 300 mg/g. In one embodiment, the patient has an UACR of equal or above 300 to equal or below 5000 mg/g. In one embodiment, the patient has an UACR of equal or above 300 to equal or below 4000 mg/g. In one embodiment, the patient has an UACR of equal or above 300 to equal or below 3000 mg/g. In one embodiment, the patient has an UACR of equal or above 300 to equal or below 2000 mg/g. In one embodiment, the patient has an UACR of equal or above 300 to equal or below 1000 mg/g. In one embodiment, the patient has an UACR of equal or above 300 to equal or below 500 mg/g. In one embodiment, the patient has an UACR of equal or above 300 to equal or below 400 mg/g. In one embodiment, the patient has an UACR of equal or above 400 to equal or below 5000 mg/g. In one embodiment, the patient has an UACR of equal or above 400 to equal or below 4000 mg/g. In one embodiment, the patient has an UACR of equal or above 400 to equal or below 3000 mg/g. In one embodiment, the patient has an UACR of equal or above 400 to equal or below 2000 mg/g. In one embodiment, the patient has an UACR of equal or above 400 to equal or below 1000 mg/g. In one embodiment, the patient has an UACR of equal or above 400 to equal or below 500 mg/g. In one embodiment, the patient has an UACR of equal or above 500 to equal or below 5000 mg/g. In one embodiment, the patient has an UACR of equal or above 500 to equal or below 4000 mg/g. In one embodiment, the patient has an UACR of equal or above 500 to equal or below 3000 mg/g. In one embodiment, the patient has an UACR of equal or above 500 to equal or below 2000 mg/g. In one embodiment, the patient has an UACR of equal or above 500 to equal or below 1000 mg/g. In one embodiment, the patient has an UACR of equal or above 600 to equal or below 5000 mg/g. In one embodiment, the patient has an UACR of equal or above 600 to equal or below 4000 mg/g. In one embodiment, the patient has an UACR of equal or above 600 to equal or below 3000 mg/g. In one embodiment, the patient has an UACR of equal or above 600 to equal or below 2000 mg/g. In one embodiment, the patient has an UACR of equal or above 600 to equal or below 1000 mg/g. In one embodiment, the patient has an UACR of equal or above 700 to equal or below 5000 mg/g. In one embodiment, the patient has an UACR of equal or above 700 to equal or below 4000 mg/g. In one embodiment, the patient has an UACR of equal or above 700 to equal or below 3000 mg/g. In one embodiment, the patient has an UACR of equal or above 700 to equal or below 2000 mg/g. In one embodiment, the patient has an UACR of equal or above 700 to equal or below 1000 mg/g. In one embodiment, the patient has an UACR of equal or above 800 to equal or below 5000 mg/g. In one embodiment, the patient has an UACR of equal or above 800 to equal or below 4000 mg/g. In one embodiment, the patient has an UACR of equal or above 800 to equal or below 3000 mg/g. In one embodiment, the patient has an UACR of equal or above 800 to equal or below 2000 mg/g. In one embodiment, the patient has an UACR of equal or above 800 to equal or below 1000 mg/g. In one embodiment, the patient has an UACR of equal or above 900 to equal or below 5000 mg/g. In one embodiment, the patient has an UACR of equal or above 900 to equal or below 4000 mg/g. In one embodiment, the patient has an UACR of equal or above 900 to equal or below 3000 mg/g. In one embodiment, the patient has an UACR of equal or above 900 to equal or below 2000 mg/g. In one embodiment, the patient has an UACR of equal or above 900 to equal or below 1000 mg/g. In one embodiment, the patient has an UACR of equal or above 1000 to equal or below 5000 mg/g. In one embodiment, the patient has an UACR of equal or above 1000 to equal or below 4000 mg/g. In one embodiment, the patient has an UACR of equal or above 1000 to equal or below 3000 mg/g. In one embodiment, the patient has an UACR of equal or above 1000 to equal or below 2000 mg/g. In one embodiment, the patient has an UACR of equal or above 2000 to equal or below 5000 mg/g. In one embodiment, the patient has an UACR of equal or above 2000 to equal or below 4000 mg/g. In one embodiment, the patient has an UACR of equal or above 2000 to equal or below 3000 mg/g. one embodiment, the patient has an UACR of equal or above 3000 to equal or below 4000 mg/g. In one embodiment, the patient has an UACR of equal or above 4000 to equal or below 5000 mg/g.

In one embodiment, the patient has an UACR selected from of equal or above 5, 10, 15, 20, 25, 30, 35, 40, 45, 50, 55, 60, 65, 70, 75, 80, 90, 95, 100, 105, 110, 125, 130, 135, 140, 145, 150, 155, 160, 165, 170, 175, 180, 185, 190, 195, 200, 205, 210, 225, 230, 235, 240, 245, 250, 255, 260, 265, 270, 275, 280, 285, 290, 295, 300, 305, 310, 325, 330, 335, 340, 345, 350, 355, 360, 365, 370, 375, 380, 385, 390, 395, 400, 405, 410, 425, 430, 435, 440, 445, 450, 455, 460, 465, 470, 475, 480, 485, 490, 495, 500, 505, 510, 515, 520, 525, 530, 535, 540, 550, 555, 560, 565, 570, 575, 580, 585, 590, 595 and 600 mg/g.

In one embodiment, the patient has an UACR selected from of equal or below 5, 10, 15, 20, 25, 30, 35, 40, 45, 50, 55, 60, 65, 70, 75, 80, 90, 95, 100, 105, 110, 125, 130, 135, 140, 145, 150, 155, 160, 165, 170, 175, 180, 185, 190, 195, 200, 205, 210, 225, 230, 235, 240, 245, 250, 255, 260, 265, 270, 275, 280, 285, 290, 295, 300, 305, 310, 325, 330, 335, 340, 345, 350, 355, 360, 365, 370, 375, 380, 385, 390, 395, 400, 405, 410, 425, 430, 435, 440, 445, 450, 455, 460, 465, 470, 475, 480, 485, 490, 495, 500, 505, 510, 515, 520, 525, 530, 535, 540, 550, 555, 560, 565, 570, 575, 580, 585, 590, 595 and 600 mg/g.

In one embodiment, the patient has an UACR of equal or above 5 to equal or below 500 mg/g. In one embodiment, the patient has an UACR of equal or above 5 to equal or below 300 mg/g. In one embodiment, the patient has an UACR of equal or above 5 to equal or below 300 mg/g. In one embodiment, the patient has an UACR of equal or above 5 to equal or below 250 mg/g. In one embodiment, the patient has an UACR of equal or above 5 to equal or below 200 mg/g. In one embodiment, the patient has an UACR of equal or above 5 to equal or below 150 mg/g. In one embodiment, the patient has an UACR of equal or above 5 to equal or below 100 mg/g. In one embodiment, the patient has an UACR of equal or above 5 to equal or below 90 mg/g. In one embodiment, the patient has an UACR of equal or above 5 to equal or below 80 mg/g. In one embodiment, the patient has an UACR of equal or above 5 to equal or below 75 mg/g. In one embodiment, the patient has an UACR of equal or above 5 to equal or below 70 mg/g. In one embodiment, the patient has an UACR of equal or above 5 to equal or below 60 mg/g. In one embodiment, the patient has an UACR of equal or above 5 to equal or below 55 mg/g. In one embodiment, the patient has an UACR of equal or above 10 to equal or below 500 mg/g. In one embodiment, the patient has an UACR of equal or above 10 to equal or below 300 mg/g. In one embodiment, the patient has an UACR of equal or above 10 to equal or below 300 mg/g. In one embodiment, the patient has an UACR of equal or above 10 to equal or below 250 mg/g. In one embodiment, the patient has an UACR of equal or above 10 to equal or below 200 mg/g. In one embodiment, the patient has an UACR of equal or above 10 to equal or below 150 mg/g. In one embodiment, the patient has an UACR of equal or above 10 to equal or below 100 mg/g. In one embodiment, the patient has an UACR of equal or above 10 to equal or below 90 mg/g. In one embodiment, the patient has an UACR of equal or above 10 to equal or below 80 mg/g. In one embodiment, the patient has an UACR of equal or above 10 to equal or below 75 mg/g. In one embodiment, the patient has an UACR of equal or above 10 to equal or below 70 mg/g. In one embodiment, the patient has an UACR of equal or above 10 to equal or below 60 mg/g. In one embodiment, the patient has an UACR of equal or above 10 to equal or below 55 mg/g.

The patient can be characterized by KDIGO Risk Categories as described in The Kidney Disease: Improving Global Outcomes (KDIGO) Clinical Practice Guideline for the Management of Glomerular Diseases. KDIGO is the global nonprofit organization developing and implementing evidence-based clinical practice guidelines in kidney disease. Chronic Kidney Disease (CKD) is defined as abnormalities of kidney structure or function, present for more than 3 months, with implications for health. CKD is classified based on Cause, GFR category (G1: Normal or high; G2: Mildly decreased; G3a: Mildly to moderately decreased; G3b: Moderately to severely decreased; G4: Severely decreased; G5: Kidney failure), and Albuminuria category (A1: normal; A2: mildly; A3: Severely increased).

In one embodiment, the patient has an eGFR of equal or below 15 mL/min/1.73m² and a UACR of equal or below 30 mg/g. In one embodiment, the patient has an eGFR of equal or below 15 mL/min/1.73m² and a UACR of equal or above 30 to equal or below 300 mg/g. In one embodiment, the patient has an eGFR of equal or below 15 mL/min/1.73m² and a UACR of equal or above 300 mg/g. In one embodiment, the patient has an eGFR of equal or above 15 to equal or below 29 mL/min/1.73m² and a UACR of equal or below 30 mg/g. In one embodiment, the patient has an eGFR of equal or above 15 to equal or below 29 mL/min/1.73m² and a UACR of equal or above 30 to equal or below 300 mg/g. In one embodiment, the patient has an eGFR equal or above 15 to equal or below 29 mL/min/1.73m² and a UACR of equal or below 300 mg/g. In one embodiment, the patient has an eGFR of equal or above 15 to equal or below 30 mL/min/1.73m² and a UACR of equal or below 30 mg/g. In one embodiment, the patient has an eGFR of equal or above 15 to equal or below 30 mL/min/1.73m² and a UACR of equal or above 30 to equal or below 300 mg/g. In one embodiment, the patient has an eGFR equal or above 15 to equal or below 30 mL/min/1.73m² and a UACR of equal or below 300 mg/g. In one embodiment, the patient has an eGFR of equal or above 30 to equal or below 44 mL/min/1.73m² and a UACR of equal or below 30 mg/g. In one embodiment, the patient has an eGFR of equal or above 30 to equal or below 44 mL/min/1.73m² and a UACR of equal or above 30 to equal or below 300 mg/g. In one embodiment, the patient has an eGFR of equal or above 30 to equal or below 44 mL/min/1.73m² and a UACR of equal or below 300 mg/g. In one embodiment, the patient has an eGFR of equal or above 30 to equal or below 45 mL/min/1.73m² and a UACR of equal or below 30 mg/g. In one embodiment, the patient has an eGFR of equal or above 30 to equal or below 45 mL/min/1.73m² and a UACR of equal or above 30 to equal or below 300 mg/g. In one embodiment, the patient has an eGFR of equal or above 30 to equal or below 45 mL/min/1.73m² and a UACR of equal or below 300 mg/g. In one embodiment, the patient has an eGFR of equal or above 45 to equal or below 59 mL/min/1.73m² and a UACR of equal or below 30 mg/g. In one embodiment, the patient has an eGFR of equal or above 45 to equal or below 59 mL/min/1.73m² and a UACR of equal or above 30 to equal or below 300 mg/g. In one embodiment, the patient has an eGFR of equal or above 45 to equal or below 59 mL/min/1.73m² and a UACR of equal or below 300 mg/g. In one embodiment, the patient has an eGFR of equal or above 45 to equal or below 60 mL/min/1.73m² and a UACR of equal or below 30 mg/g. In one embodiment, the patient has an eGFR of equal or above 45 to equal or below 60 mL/min/1.73m² and a UACR of equal or above 30 to equal or below 300 mg/g. In one embodiment, the patient has an eGFR of equal or above 45 to equal or below 60 mL/min/1.73m² and a UACR of equal or below 300 mg/g. In one embodiment, the patient has an eGFR of equal or above 60 to equal or below 89 mL/min/1.73m² and a UACR of equal or below 30 mg/g. In one embodiment, the patient has an eGFR of equal or above 60 to equal or below 89 mL/min/1.73m² and a UACR of equal or above 30 to equal or below 300 mg/g. In one embodiment, the patient has an eGFR of equal or above 60 to equal or below 89 mL/min/1.73m² and a UACR of equal or below 300 mg/g. In one embodiment, the patient has an eGFR of equal or above 60 to equal or below 90 mL/min/1.73m² and a UACR of equal or below 30 mg/g. In one embodiment, the patient has an eGFR of equal or above 60 to equal or below 90 mL/min/1.73m² and a UACR of equal or above 30 to equal or below 300 mg/g. In one embodiment, the patient has an eGFR of equal or above 60 to equal or below 90 mL/min/1.73m² and a UACR of equal or below 300 mg/g. In one embodiment, the patient has an eGFR of equal or above 90 mL/min/1.73m² and a UACR of equal or below 30 mg/g. In one embodiment, the patient has an eGFR of equal or above 90 mL/min/1.73m² and a UACR of equal or above 30 to equal or below 300 mg/g. In one embodiment, the patient has an eGFR of equal or above 90 mL/min/1.73m² and a UACR of equal or below 300 mg/g.

The patient can be characterized by potassium level. It is expressed in "mmol/L". The potassium level and distribution among the body compartments depend on a complex interplay of multiple factors including renal and gastrointestinal function; diet, medications, and supplements; neurohormonal status; and acid-base balance. Under normal conditions, the kidneys are responsible for up to 90-95% of potassium elimination, with the colon being responsible for the remainder. Because cardiac repolarization relies on potassium influx, hypokalemia lengthens the action potential and increases QT dispersion. Hyperkalemia leads to a shortening of the repolarization time, which may lead to QT interval shortening. Both hypo- and hyperkalemia may be life-threatening conditions by increasing the risk of ventricular arrhythmia and sudden cardiac death. Dyskalemia in HF has important prognostic implications. Critical comorbidities include CKD, diabetes mellitus (DM), frailty, and aging. In HF, as in other conditions, for example, myocardial infarction, hypertension, kidney disease, or in the general population, the relationship between diabetes mellitus concentrations and adverse outcomes appears to be U-shaped, where both low- and high-potassium levels are associated with adverse outcomes, although it remains unclear to what extent dyskalemia is a risk factor itself versus a risk marker representing the patients' overall clinical status, other comorbidities, and/or use or nonuse of HF medications. Correction of both hypokalemia and hyperkalemia offsets their associated risks.

In one embodiment, the patient has a potassium level of above 3 mmol/L. In one embodiment, the patient has a potassium level of above 3.5 mmol/L. In one embodiment, the patient has a potassium level of above 4 mmol/L. In one embodiment, the patient has a potassium level of above 4.5 mmol/L. In one embodiment, the patient has a potassium level of above 4.8 mmol/L. In one embodiment, the patient has a potassium level of above 5 mmol/L. In one embodiment, the patient has a potassium level of above 5.5 mmol/L. In one embodiment, the patient has a potassium level of above 6 mmol/L. In one embodiment, the patient has a potassium level of above 6.5 mmol/L. In one embodiment, the patient has a potassium level of above 7 mmol/L. In one embodiment, the patient has a potassium level of above 7.5 mmol/L. In one embodiment, the patient has a potassium level equal or above 3 mmol/L. In one embodiment, the patient has a potassium level equal or above 3.5 mmol/L. In one embodiment, the patient has a potassium level equal or above 4 mmol/L. In one embodiment, the patient has a potassium level equal or above 4.5 mmol/L. In one embodiment, the patient has a potassium level equal or above 4.8 mmol/L. In one embodiment, the patient has a potassium level equal or above 5 mmol/L. In one embodiment, the patient has a potassium level equal or above 5.5 mmol/L. In one embodiment, the patient has a potassium level equal or above 6 mmol/L. In one embodiment, the patient has a potassium level equal or above 6.5 mmol/L. In one embodiment, the patient has a potassium level equal or above 7 mmol/L. In one embodiment, the patient has a potassium level equal or above 7.5 mmol/L. In one embodiment, the patient has a potassium level below 3 mmol/L. In one embodiment, the patient has a potassium level below 3.5 mmol/L. In one embodiment, the patient has a potassium level below 4 mmol/L. In one embodiment, the patient has a potassium level below 4.5 mmol/L. In one embodiment, the patient has a potassium level below 4.8 mmol/L. In one embodiment, the patient has a potassium level below 5 mmol/L. In one embodiment, the patient has a potassium level below 5.5 mmol/L. In one embodiment, the patient has a potassium level below 6 mmol/L. In one embodiment, the patient has a potassium level below 6.5 mmol/L. In one embodiment, the patient has a potassium level below 7 mmol/L. In one embodiment, the patient has a potassium level below 7.5 mmol/L. In one embodiment, the patient has a potassium level equal or below 3 mmol/L. In one embodiment, the patient has a potassium level equal or below 3.5 mmol/L. In one embodiment, the patient has a potassium level equal or below 4 mmol/L. In one embodiment, the patient has a potassium level equal or below 4.5 mmol/L. In one embodiment, the patient has a potassium level equal or below 4.8 mmol/L. In one embodiment, the patient has a potassium level equal or below 5 mmol/L. In one embodiment, the patient has a potassium level equal or below 5.5 mmol/L. In one embodiment, the patient has a potassium level equal or below 6 mmol/L. In one embodiment, the patient has a potassium level equal or below 6.5 mmol/L. In one embodiment, the patient has a potassium level equal or below7 mmol/L. In one embodiment, the patient has a potassium level equal or below 7.5 mmol/L.

In one embodiment, the patient has a potassium level selected from of above 3, 3.5, 4, 4.5, 4.8, 5, 5.5, 6, 6.5, 7 or 7.5 mmol/L. In one embodiment, the patient has a potassium level selected from of equal or above 3, 3.5, 4, 4.5, 4.8, 5, 5.5, 6, 6.5, 7 or 7.5 mmol/L. In one embodiment, the patient has a potassium level selected from of below 3, 3.5, 4, 4.5, 4.8, 5, 5.5, 6, 6.5, 7 or 7.5 mmol/L. In one embodiment, the patient has a potassium level selected from of equal or below 3, 3.5, 4, 4.5, 4.8, 5, 5.5, 6, 6.5, 7 or 7.5 mmol/L.

In one embodiment, the patient has a potassium level of equal or above 3 to equal or below 3.5 mmol/L. In one embodiment, the patient has a potassium level of equal or above 3 to equal or below 4 mmol/L. In one embodiment, the patient has a potassium level of equal or above 3 to equal or below 4.5 mmol/L. In one embodiment, the patient has a potassium level of equal or above 3 to equal or below 4.8 mmol/L. In one embodiment, the patient has a potassium level of equal or above 3 to equal or below 5 mmol/L. In one embodiment, the patient has a potassium level of equal or above 3 to equal or below 5.5 mmol/L. In one embodiment, the patient has a potassium level of equal or above 3 to equal or below 6 mmol/L. In one embodiment, the patient has a potassium level of equal or above 3 to equal or below 6.5 mmol/L. In one embodiment, the patient has a potassium level of equal or above 3 to equal or below 7 mmol/L. In one embodiment, the patient has a potassium level of equal or above 3 to equal or below 7.5 mmol/L. In one embodiment, the patient has a potassium level of equal or above 3.5 to equal or below 4 mmol/L. In one embodiment, the patient has a potassium level of equal or above 3.5 to equal or below 4.5 mmol/L. In one embodiment, the patient has a potassium level of equal or above 3.5 to equal or below 4.8 mmol/L. In one embodiment, the patient has a potassium level of equal or above 3.5 to equal or below 5 mmol/L. In one embodiment, the patient has a potassium level of equal or above 3.5 to equal or below 5.5 mmol/L. In one embodiment, the patient has a potassium level of equal or above 3.5 to equal or below 6 mmol/L. In one embodiment, the patient has a potassium level of equal or above 3.5 to equal or below 6.5 mmol/L. In one embodiment, the patient has a potassium level of equal or above 3.5 to equal or below 7 mmol/L. In one embodiment, the patient has a potassium level of equal or above 3.5 to equal or below 7.5 mmol/L. In one embodiment, the patient has a potassium level of equal or above 4 to equal or below 4.5 mmol/L. In one embodiment, the patient has a potassium level of equal or above 4 to equal or below 4.8 mmol/L. In one embodiment, the patient has a potassium level of equal or above 4 to equal or below 5 mmol/L. In one embodiment, the patient has a potassium level of equal or above 4 to equal or below 5.5 mmol/L. In one embodiment, the patient has a potassium level of equal or above 4 to equal or below 6 mmol/L. In one embodiment, the patient has a potassium level of equal or above 4 to equal or below 6.5 mmol/L. In one embodiment, the patient has a potassium level of equal or above 4 to equal or below 7 mmol/L. In one embodiment, the patient has a potassium level of equal or above 4 to equal or below 7.5 mmol/L. In one embodiment, the patient has a potassium level of equal or above 4.5 to equal or below 4.8 mmol/L. In one embodiment, the patient has a potassium level of equal or above 4.5 to equal or below 5 mmol/L. In one embodiment, the patient has a potassium level of equal or above 4.5 to equal or below 5.5 mmol/L. In one embodiment, the patient has a potassium level of equal or above 4.5 to equal or below 6 mmol/L. In one embodiment, the patient has a potassium level of equal or above 4.5 to equal or below 6.5 mmol/L. In one embodiment, the patient has a potassium level of equal or above 4.5 to equal or below 7 mmol/L. In one embodiment, the patient has a potassium level of equal or above 4.5 to equal or below 7.5 mmol/L. In one embodiment, the patient has a potassium level of equal or above 4.8 to equal or below 5 mmol/L. In one embodiment, the patient has a potassium level of equal or above 4.8 to equal or below 5.5 mmol/L. In one embodiment, the patient has a potassium level of equal or above 4.8 to equal or below 6 mmol/L. In one embodiment, the patient has a potassium level of equal or above 4.8 to equal or below 6.5 mmol/L. In one embodiment, the patient has a potassium level of equal or above 4.8 to equal or below 7 mmol/L. In one embodiment, the patient has a potassium level of equal or above 4.8 to equal or below 7.5 mmol/L. In one embodiment, the patient has a potassium level of equal or above 5 to equal or below 5.5 mmol/L. In one embodiment, the patient has a potassium level of equal or above 5 to equal or below 6 mmol/L. In one embodiment, the patient has a potassium level of equal or above 5 to equal or below 6.5 mmol/L. In one embodiment, the patient has a potassium level of equal or above 5 to equal or below 7 mmol/L. In one embodiment, the patient has a potassium level of equal or above 5 to equal or below 7.5 mmol/L. In one embodiment, the patient has a potassium level of equal or above 5.5 to equal or below 6 mmol/L. In one embodiment, the patient has a potassium level of equal or above 5.5 to equal or below 6.5 mmol/L. In one embodiment, the patient has a potassium level of equal or above 5.5 to equal or below 7 mmol/L. In one embodiment, the patient has a potassium level of equal or above 5.5 to equal or below 7.5 mmol/L. In one embodiment, the patient has a potassium level of equal or above 6 to equal or below 6.5 mmol/L. In one embodiment, the patient has a potassium level of equal or above 6 to equal or below 7 mmol/L. In one embodiment, the patient has a potassium level of equal or above 6 to equal or below 7.5 mmol/L. In one embodiment, the patient has a potassium level of equal or above 6.5 to equal or below 7 mmol/L. In one embodiment, the patient has a potassium level of equal or above 6.5 to equal or below 7.5 mmol/L. In one embodiment, the patient has a potassium level of equal or above 7 to equal or below 7.5 mmol/L.

Measurement of serum sodium is routine in assessing electrolyte, acid-base, and water balance, as well as renal function. The sodium level is expressed in mmol/L. The reference range for serum sodium is 135-147 mmol/L, although different assays establish their own reference ranges, which may differ slightly.

Sodium excretion via the urine ('natriuresis') is a particular therapeutic target for cardiovascular diseases. For example, neurohumoral stimulation by the renin-angiotensin-aldosterone system (RAAS) contributes to permanent sodium retention and an associated extracellular volume load in the development of chronic heart and kidney failure. In particular, neurohumoral stimulation by the renin-angiotensin-aldosterone system (RAAS) or elevation of sodium-glucose-transporter-2 (SGLT2) expression in the proximal tubule contributes to permanent sodium retention and an associated extracellular volume load in the development of chronic heart and kidney failure. The body is no longer able to perform effective natriuresis, especially when there is impaired kidney function, which is often associated with heart failure. Therefore, maintaining an effective natriuresis would be useful in treatment and/or prevention of cardiovascular and/or renal diseases.

The patient can be characterized by the sodium level.

In one embodiment, the patient has a sodium level selected from of equal or above 100, 110, 115, 117, 120, 125, 130, 135, 138, 140, 141, 144, 145, 150, 154 and 155 mmol/L. In one embodiment, the patient has a sodium level selected from of equal or below 100, 110, 115, 117, 120, 125, 130, 135, 138, 140, 144, 145, 150, 154 and 155 mmol/L.

In one embodiment, the patient has a sodium level of equal or above 100 mmol/L. In one embodiment, the patient has a sodium level of equal or above 110 mmol/L. In one embodiment, the patient has a sodium level of equal or above 115 mmol/L. In one embodiment, the patient has a sodium level of equal or above 120 mmol/L. In one embodiment, the patient has a sodium level of equal or above 125 mmol/L. In one embodiment, the patient has a sodium level of equal or above 130 mmol/L. In one embodiment, the patient has a sodium level of equal or above 135 mmol/L. In one embodiment, the patient has a sodium level of equal or above 138 mmol/L. In one embodiment, the patient has a sodium level of equal or above 140 mmol/L. In one embodiment, the patient has a sodium level of equal or above 144 mmol/L. In one embodiment, the patient has a sodium level of equal or above 145 mmol/L. In one embodiment, the patient has a sodium level of equal or above 150 mmol/L. In one embodiment, the patient has a sodium level of equal or below 154 mmol/L. In one embodiment, the patient has a sodium level of equal or above 155 mmol/L. In one embodiment, the patient has a sodium level of equal or below 100 mmol/L. In one embodiment, the patient has a sodium level of equal or below 110 mmol/L. In one embodiment, the patient has a sodium level of equal or below 115 mmol/L. In one embodiment, the patient has a sodium level of equal or below 120 mmol/L. In one embodiment, the patient has a sodium level of equal or below 125 mmol/L. In one embodiment, the patient has a sodium level of equal or below 130 mmol/L. In one embodiment, the patient has a sodium level of equal or below 135 mmol/L. In one embodiment, the patient has a sodium level of equal or below 138 mmol/L. In one embodiment, the patient has a sodium level of equal or below 140 mmol/L. In one embodiment, the patient has a sodium level of equal or below 144 mmol/L. In one embodiment, the patient has a sodium level of equal or below 145 mmol/L. In one embodiment, the patient has a sodium level of equal or below 150 mmol/L. In one embodiment, the patient has a sodium level of equal or below 154 mmol/L. In one embodiment, the patient has a sodium level of equal or below 155 mmol/L.

In one embodiment, the patient has a sodium level of equal or above 100 to equal or below 110 mmol/L. In one embodiment, the patient has a sodium level of equal or above 100 to equal or below 115 mmol/L. In one embodiment, the patient has a sodium level of equal or above 100 to equal or below 117 mmol/L. In one embodiment, the patient has a sodium level of equal or above 100 to equal or below 120 mmol/L. In one embodiment, the patient has a sodium level of equal or above 100 to equal or below 125 mmol/L. In one embodiment, the patient has a sodium level of equal or above 100 to equal or below 130 mmol/L. In one embodiment, the patient has a sodium level of equal or above 100 to equal or below 135 mmol/L. In one embodiment, the patient has a sodium level of equal or above 100 to equal or below 138 mmol/L. In one embodiment, the patient has a sodium level of equal or above 100 to equal or below 140 mmol/L. In one embodiment, the patient has a sodium level of equal or above 100 to equal or below 141 mmol/L. In one embodiment, the patient has a sodium level of equal or above 100 to equal or below 144 mmol/L. In one embodiment, the patient has a sodium level of equal or above 100 to equal or below 145 mmol/L. In one embodiment, the patient has a sodium level of equal or above 100 to equal or below 150 mmol/L. In one embodiment, the patient has a sodium level of equal or above 100 to equal or below 154 mmol/L. In one embodiment, the patient has a sodium level of equal or above 100 to equal or below 155 mmol/L. In one embodiment, the patient has a sodium level of equal or above 110 to equal or below 115 mmol/L. In one embodiment, the patient has a sodium level of equal or above 110 to equal or below 117 mmol/L. In one embodiment, the patient has a sodium level of equal or above 110 to equal or below 120 mmol/L. In one embodiment, the patient has a sodium level of equal or above 110 to equal or below 125 mmol/L. In one embodiment, the patient has a sodium level of equal or above 110 to equal or below 130 mmol/L. In one embodiment, the patient has a sodium level of equal or above 110 to equal or below 135 mmol/L. In one embodiment, the patient has a sodium level of equal or above 110 to equal or below 138 mmol/L. In one embodiment, the patient has a sodium level of equal or above 110 to equal or below 140 mmol/L. In one embodiment, the patient has a sodium level of equal or above 110 to equal or below 141 mmol/L. In one embodiment, the patient has a sodium level of equal or above 110 to equal or below 144 mmol/L. In one embodiment, the patient has a sodium level of equal or above 110 to equal or below 145 mmol/L. In one embodiment, the patient has a sodium level of equal or above 110 to equal or below 150 mmol/L. In one embodiment, the patient has a sodium level of equal or above 110 to equal or below 154 mmol/L. In one embodiment, the patient has a sodium level of equal or above 110 to equal or below 155 mmol/L. In one embodiment, the patient has a sodium level of equal or above 117 to equal or below 120 mmol/L. In one embodiment, the patient has a sodium level of equal or above 117 to equal or below 125 mmol/L. In one embodiment, the patient has a sodium level of equal or above 117 to equal or below 130 mmol/L. In one embodiment, the patient has a sodium level of equal or above 117 to equal or below 135 mmol/L. In one embodiment, the patient has a sodium level of equal or above 117 to equal or below 138 mmol/L. In one embodiment, the patient has a sodium level of equal or above 117 to equal or below 140 mmol/L. In one embodiment, the patient has a sodium level of equal or above 117 to equal or below 141 mmol/L. In one embodiment, the patient has a sodium level of equal or above 117 to equal or below 144 mmol/L.

In one embodiment, the patient has a sodium level of equal or above 117 to equal or below 145 mmol/L. In one embodiment, the patient has a sodium level of equal or above 117 to equal or below 150 mmol/L. In one embodiment, the patient has a sodium level of equal or above 117 to equal or below 154 mmol/L. In one embodiment, the patient has a sodium level of equal or above 117 to equal or below 155 mmol/L.In one embodiment, the patient has a sodium level of equal or above 120 to equal or below 125 mmol/L. In one embodiment, the patient has a sodium level of equal or above 120 to equal or below 130 mmol/L. In one embodiment, the patient has a sodium level of equal or above 120 to equal or below 135 mmol/L. In one embodiment, the patient has a sodium level of equal or above 120 to equal or below 138 mmol/L. In one embodiment, the patient has a sodium level of equal or above 120 to equal or below 140 mmol/L. In one embodiment, the patient has a sodium level of equal or above 120 to equal or below 141 mmol/L. In one embodiment, the patient has a sodium level of equal or above 120 to equal or below 144 mmol/L. In one embodiment, the patient has a sodium level of equal or above 120 to equal or below 145 mmol/L. In one embodiment, the patient has a sodium level of equal or above 120 to equal or below 150 mmol/L. In one embodiment, the patient has a sodium level of equal or above 120 to equal or below 154 mmol/L. In one embodiment, the patient has a sodium level of equal or above 120 to equal or below 155 mmol/L. In one embodiment, the patient has a sodium level of equal or above 125 to equal or below 130 mmol/L. In one embodiment, the patient has a sodium level of equal or above 125 to equal or below 135 mmol/L. In one embodiment, the patient has a sodium level of equal or above 125 to equal or below 138 mmol/L. In one embodiment, the patient has a sodium level of equal or above 125 to equal or below 140 mmol/L. In one embodiment, the patient has a sodium level of equal or above 125 to equal or below 141 mmol/L. In one embodiment, the patient has a sodium level of equal or above 125 to equal or below 144 mmol/L. In one embodiment, the patient has a sodium level of equal or above 125 to equal or below 145 mmol/L. In one embodiment, the patient has a sodium level of equal or above 125 to equal or below 150 mmol/L. In one embodiment, the patient has a sodium level of equal or above 125 to equal or below 154 mmol/L. In one embodiment, the patient has a sodium level of equal or above 125 to equal or below 155 mmol/L. In one embodiment, the patient has a sodium level of equal or above 130 to equal or below 135 mmol/L. In one embodiment, the patient has a sodium level of equal or above 130 to equal or below 138 mmol/L. In one embodiment, the patient has a sodium level of equal or above 130 to equal or below 140 mmol/L. In one embodiment, the patient has a sodium level of equal or above 130 to equal or below 141 mmol/L. In one embodiment, the patient has a sodium level of equal or above 130 to equal or below 144 mmol/L. In one embodiment, the patient has a sodium level of equal or above 130 to equal or below 145 mmol/L. In one embodiment, the patient has a sodium level of equal or above 130 to equal or below 150 mmol/L. In one embodiment, the patient has a sodium level of equal or above 130 to equal or below 154 mmol/L. In one embodiment, the patient has a sodium level of equal or above 130 to equal or below 155 mmol/L. In one embodiment, the patient has a sodium level of equal or above 135 to equal or below 138 mmol/L. In one embodiment, the patient has a sodium level of equal or above 135 to equal or below 140 mmol/L. In one embodiment, the patient has a sodium level of equal or above 135 to equal or below 141 mmol/L. In one embodiment, the patient has a sodium level of equal or above 135 to equal or below 144 mmol/L. In one embodiment, the patient has a sodium level of equal or above 135 to equal or below 145 mmol/L. In one embodiment, the patient has a sodium level of equal or above 135 to equal or below 150 mmol/L. In one embodiment, the patient has a sodium level of equal or above 135 to equal or below 154 mmol/L. In one embodiment, the patient has a sodium level of equal or above 135 to equal or below 155 mmol/L.In one embodiment, the patient has a sodium level of equal or above 138 to equal or below 140 mmol/L. In one embodiment, the patient has a sodium level of equal or above 138 to equal or below 141 mmol/L. In one embodiment, the patient has a sodium level of equal or above 138 to equal or below 144 mmol/L. In one embodiment, the patient has a sodium level of equal or above 138 to equal or below 145 mmol/L. In one embodiment, the patient has a sodium level of equal or above 138 to equal or below 150 mmol/L. In one embodiment, the patient has a sodium level of equal or above 138 to equal or below 154 mmol/L. In one embodiment, the patient has a sodium level of equal or above 138 to equal or below 155 mmol/L. In one embodiment, the patient has a sodium level of equal or above 140 to equal or below 141 mmol/L. In one embodiment, the patient has a sodium level of equal or above 140 to equal or below 144 mmol/L. In one embodiment, the patient has a sodium level of equal or above 140 to equal or below 145 mmol/L. In one embodiment, the patient has a sodium level of equal or above 140 to equal or below 150 mmol/L. In one embodiment, the patient has a sodium level of equal or above 140 to equal or below 154 mmol/L. In one embodiment, the patient has a sodium level of equal or above 140 to equal or below 155 mmol/L. In one embodiment, the patient has a sodium level of equal or above 141 to equal or below 144 mmol/L. In one embodiment, the patient has a sodium level of equal or above 141 to equal or below 145 mmol/L. In one embodiment, the patient has a sodium level of equal or above 141 to equal or below 150 mmol/L. In one embodiment, the patient has a sodium level of equal or above 141 to equal or below 154 mmol/L. In one embodiment, the patient has a sodium level of equal or above 141 to equal or below 155 mmol/L. In one embodiment, the patient has a sodium level of equal or above 144 to equal or below 145 mmol/L. In one embodiment, the patient has a sodium level of equal or above 144 to equal or below 150 mmol/L. In one embodiment, the patient has a sodium level of equal or above 144 to equal or below 154 mmol/L. In one embodiment, the patient has a sodium level of equal or above 144 to equal or below 155 mmol/L. In one embodiment, the patient has a sodium level of equal or above 145 to equal or below 150 mmol/L. In one embodiment, the patient has a sodium level of equal or above 145 to equal or below 154 mmol/L. In one embodiment, the patient has a sodium level of equal or above 145 to equal or below 155 mmol/L. In one embodiment, the patient has a sodium level of equal or above 150 to equal or below 154 mmol/L. In one embodiment, the patient has a sodium level of equal or above 150 to equal or below 155 mmol/L. In one embodiment, the patient has a sodium level of equal or above 154 to equal or below 155 mmol/L.

The chloride level can be used as a prognostic marker in HF. Chloride is among the major electrolytes that play a unique role in fluid homeostasis and is associated with cardiorenal and neurohormonal systems. Hypochloremia (low serum chloride level) can be an independent predictor of adverse outcomes in acute or chronic HF. Various HF therapies may cause hypochloremia, and hypochloremia itself can initiate and exacerbate diuretic resistance in HF. The chloride concentration is also used to calculate the anion gap, the difference between the main cation sodium and the two main anions chloride and bicarbonate.

The chloride level is expressed in mmol/L.

Lower chloride levels are associated with a worse prognosis in HF. It may have a prominent contribution to the pathophysiology. Chloride, rather than sodium, modulates the renin secretion and tubuloglomerular feedback in the kidney and regulate sodium transport pathway in the loop of Henle and distal convoluted tube. The relationship between chloride levels and prognosis was observed in both HF with reduced ejection fraction (HFrEF) and HF with mildly reduced or preserved ejection fraction (HFmrEF/HFpEF), and cam have a contribution to the pathophysiology and prognosis in HF. Higher chloride levels may potentially alleviatie hypochloremia-related HF symptoms and prognosis. In one embodiment, the patient has a chloride level selected from of equal or above 90, 95, 100, 105, 110 mmol/L. In one embodiment, the patient has a chloride level selected from of equal or below 90, 95, 100, 105, 110 mmol/L.

In one embodiment, the patient has a chloride level of equal or below 90 mmol/L. In one embodiment, the patient has a chloride level of equal or below 95 mmol/L. In one embodiment, the patient has a chloride level of equal or below 100 mmol/L. In one embodiment, the patient has a chloride level of equal or below 105 mmol/L. In one embodiment, the patient has a chloride level of equal or below 110 mmol/L. In one embodiment, the patient has a chloride level of equal or above 90 mmol/L. In one embodiment, the patient has a chloride level of equal or above 95 mmol/L. In one embodiment, the patient has a chloride level of equal or above 100 mmol/L. In one embodiment, the patient has a chloride level of equal or above 105 mmol/L. In one embodiment, the patient has a chloride level of equal or above 110 mmol/L.

In one embodiment, the patient has a chloride level of equal or above 90 to equal or below 110 mmol/L. In one embodiment, the patient has a chloride level of equal or above 90 to equal or below 105 mmol/L. In one embodiment, the patient has a chloride level of equal or above 90 to equal or below 100 mmol/L. In one embodiment, the patient has a chloride level of equal or above 90 to equal or below 95 mmol/L.

Blood urea refers to the concentration of urea, a waste product produced when proteins break down in the bloodstream. It is a frequently measured parameter in clinical settings to evaluate the overall health and function of the kidneys. The kidneys remove urea from the blood after it is created in the liver as a byproduct of protein breakdown. Blood urea levels offer important information about kidney function. The ranges can vary slightly depending on age, gender, and health status or depending on the lab performing the test.

The urea level in serum is expressed in mg/dL.

In one embodiment the patient has a urea level of equal or above 6 to equal or below 24 mg/dL. In one embodiment the patient has a urea level of equal or above 6 to equal or below 21 mg/dL. In one embodiment the patient has a urea level of equal or above 6 mg/dL. In one embodiment the patient has a urea level of equal or below 6 mg/dL. In one embodiment the patient has a urea level of equal or above 21 mg/dL. In one embodiment the patient has a urea level of equal or below 21 mg/dL. In one embodiment the patient has a urea level of equal or above 24 mg/dL. In one embodiment the patient has a urea level of equal or below 24 mg/dL.

In one embodiment, the patient has a urea level selected from of equal or below 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 48, 49, 50, 55, 60, 65, 70, 75, 80, 85, 90, 94, 95 and 100 mg/dL. In one embodiment, the patient has a urea level selected from of equal or above 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 48, 49, 50, 55, 60, 65, 70, 75, 80, 85, 90, 94, 95 and 100 mg/dL.

In one embodiment the patient has a urea level of equal or above 4 to equal or below 94 mg/dL.

In one embodiment the patient has a urea level of equal or above 4 mg/dL. In one embodiment the patient has a urea level of equal or above 6 mg/dL. In one embodiment the patient has a urea level of equal or above 8 mg/dL. In one embodiment the patient has a urea level of equal or above 13 mg/dL. In one embodiment the patient has a urea level of equal or above 16 mg/dL. In one embodiment the patient has a urea level of equal or above 20 mg/dL. In one embodiment the patient has a urea level of equal or above 23 mg/dL. In one embodiment the patient has a urea level of equal or above 27 mg/dL. In one embodiment the patient has a urea level of equal or above 33 mg/dL. In one embodiment the patient has a urea level of equal or above 35 mg/dL. In one embodiment the patient has a urea level of equal or above 40 mg/dL. In one embodiment the patient has a urea level of equal or above 45 mg/dL. In one embodiment the patient has a urea level of equal or above 50 mg/dL. In one embodiment the patient has a urea level of equal or above 55 mg/dL. In one embodiment the patient has a urea level of equal or above 60 mg/dL. In one embodiment the patient has a urea level of equal or above 65 mg/dL. In one embodiment the patient has a urea level of equal or above 70 mg/dL. In one embodiment the patient has a urea level of equal or above 75 mg/dL. In one embodiment the patient has a urea level of equal or above 80 mg/dL. In one embodiment the patient has a urea level of equal or above 85 mg/dL. In one embodiment the patient has a urea level of equal or above 90 mg/dL. In one embodiment the patient has a urea level of equal or above 94 mg/dL. In one embodiment the patient has a urea level of equal or above 95 mg/dL.

In one embodiment the patient has a urea level of equal or below 4 mg/dL. In one embodiment the patient has a urea level of equal or below 6 mg/dL. In one embodiment the patient has a urea level of equal or below 8 mg/dL. In one embodiment the patient has a urea level of equal or below 13 mg/dL. In one embodiment the patient has a urea level of equal or below 16 mg/dL. In one embodiment the patient has a urea level of equal or below 20 mg/dL. In one embodiment the patient has a urea level of equal or below 23 mg/dL. In one embodiment the patient has a urea level of equal or below 27 mg/dL. In one embodiment the patient has a urea level of equal or below 33 mg/dL. In one embodiment the patient has a urea level of equal or below 35 mg/dL. In one embodiment the patient has a urea level of equal or below 40 mg/dL. In one embodiment the patient has a urea level of equal or below 45 mg/dL. In one embodiment the patient has a urea level of equal or below 50 mg/dL. In one embodiment the patient has a urea level of equal or below 55 mg/dL. In one embodiment the patient has a urea level of equal or below 60 mg/dL. In one embodiment the patient has a urea level of equal or below 65 mg/dL. In one embodiment the patient has a urea level of equal or below 70 mg/dL. In one embodiment the patient has a urea level of equal or below 75 mg/dL. In one embodiment the patient has a urea level of equal or below 80 mg/dL. In one embodiment the patient has a urea level of equal or below 85 mg/dL. In one embodiment the patient has a urea level of equal or below 90 mg/dL. In one embodiment the patient has a urea level of equal or below 94 mg/dL. In one embodiment the patient has a urea level of equal or below 95 mg/dL.

Blood urea nitrogen (BUN) is a medical test that measures the amount of urea nitrogen found in blood. The test for measuring urea in the blood is similar worldwide, but the results are reported in 2 different ways depending on the country doing the lab testing. In some instances, the urea level in plasma or serum is measured as nitrogen and is called "Blood Urea Nitrogen" or BUN. The unit used for BUN is mg/dL. In other instances, the whole urea molecule is measured, not just the nitrogen, and is reported in Standard International units of mmol/L. This measurement is about twice as high as the BUN measurement because BUN only measures the nitrogen part of the molecule (a Molecular Weight of 28), while urea measures the whole molecule (a Molecular weight of 60). Thus, urea is approximately twice that of BUN (60/28 = 2.14). For example, a BUN result of 10 mg/dL is equal to a urea result of 21.4 mg/dL. Converting BUN (mg/dl) to Urea (mmol/L). The liver produces urea in the urea cycle as a waste product of the digestion of protein. Normal human adult blood should contain 6 to 20 mg/dL (2.1 to 7.1 mmol/L) of urea nitrogen. Individual laboratories will have different reference ranges, as the assay used can vary between laboratories. The test is used to detect renal problems. It is not considered as reliable as creatinine or BUN/creatinine ratio blood studies.

Troponin is a protein found in the body, specifically in heart muscle cells. The three main types of cardiac troponin proteins are I, T, and C. During e.g. a heart attack, troponin spills into the bloodstream and it is a biomarker that can indicate cardiac injury. The high-sensitivity cardiac troponin test allows for detection of very low levels of troponin T, helping to diagnose e.g. heart attacks, diagnose other heart-related conditions, Obstructive coronary artery disease (CAD), stable angina, congestive heart failure (CHF), cardiomyopathy and the like. Since the first use of troponin testing, several generations of more refined and more reliable tests have been developed and used to help diagnose heart attack more rapidly and accurately.

Assessed values of hs-Troponin T in patients with chronic heart failure have recently emerged as an independent predictor of all cause mortality, cardiovascular mortality as well as hospitalization for cardiovascular causes. The prognostic value of hs-Troponin T is unaffected by heart failure etiology. (Aimo et al. Prognostic value of high-sensitivity Troponin-T in chronic heart failure: an individual patient data meta-analysis. Circulation. 2018; 137:286-297)

### High-sensitivity Troponin T is expressed in ng/L.

In one embodiment, the patient has a High-sensitivity Troponin T level from equal or above 5 to equal or below 95000 ng/L. In one embodiment, the patient has a High-sensitivity Troponin T level from equal or above 5 to equal or below 8000 ng/L. In one embodiment, the patient has a High-sensitivity Troponin T level from equal or above 5 to equal or below 7000 ng/L. In one embodiment, the patient has a High-sensitivity Troponin T level from equal or above 5 to equal or below 6000 ng/L. In one embodiment, the patient has a High-sensitivity Troponin T level from equal or above 5 to equal or below 5000 ng/L. In one embodiment, the patient has a High-sensitivity Troponin T level from equal or above 5 to equal or below 4000 ng/L. In one embodiment, the patient has a High-sensitivity Troponin T level from equal or above 5 to equal or below 3000 ng/L. In one embodiment, the patient has a High-sensitivity Troponin T level from equal or above 5 to equal or below 2000 ng/L. In one embodiment, the patient has a High-sensitivity Troponin T level from equal or above 5 to equal or below 1000 ng/L. In one embodiment, the patient has a High-sensitivity Troponin T level from equal or above 5 to equal or below 900 ng/L. In one embodiment, the patient has a High-sensitivity Troponin T level from equal or above 5 to equal or below 800 ng/L. In one embodiment, the patient has a High-sensitivity Troponin T level from equal or above 5 to equal or below 700 ng/L. In one embodiment, the patient has a High-sensitivity Troponin T level from equal or above 5 to equal or below 600 ng/L. In one embodiment, the patient has a High-sensitivity Troponin T level from equal or above 5 to equal or below 500 ng/L. In one embodiment, the patient has a High-sensitivity Troponin T level from equal or above 5 to equal or below 400 ng/L. In one embodiment, the patient has a High-sensitivity Troponin T level from equal or above 5 to equal or below 300 ng/L. In one embodiment, the patient has a High-sensitivity Troponin T level from equal or above 5 to equal or below 200 ng/L. In one embodiment, the patient has a High-sensitivity Troponin T level from equal or above 5 to equal or below 100 ng/L. In one embodiment, the patient has a High-sensitivity Troponin T level from equal or above 5 to equal or below 500 ng/L. In one embodiment, the patient has a High-sensitivity Troponin T level from equal or above 5 to equal or below 40 ng/L. In one embodiment, the patient has a High-sensitivity Troponin T level from equal or above 5 to equal or below 30 ng/L. In one embodiment, the patient has a High-sensitivity Troponin T level from equal or above 5 to equal or below 20 ng/L. In one embodiment, the patient has a High-sensitivity Troponin T level from equal or above 5 to equal or below 10 ng/L.

In one embodiment, the patient has a High-sensitivity Troponin T level from equal or above 100 to equal or below 95000 ng/L. In one embodiment, the patient has a High-sensitivity Troponin T level from equal or above 100 to equal or below 8000 ng/L. In one embodiment, the patient has a High-sensitivity Troponin T level from equal or above 100 to equal or below 7000 ng/L. In one embodiment, the patient has a High-sensitivity Troponin T level from equal or above 100 to equal or below 6000 ng/L. In one embodiment, the patient has a High-sensitivity Troponin T level from equal or above 100 to equal or below 5000 ng/L. In one embodiment, the patient has a High-sensitivity Troponin T level from equal or above 100 to equal or below 4000 ng/L. In one embodiment, the patient has a High-sensitivity Troponin T level from equal or above 100 to equal or below 3000 ng/L. In one embodiment, the patient has a High-sensitivity Troponin T level from equal or above 100 to equal or below 2000 ng/L. In one embodiment, the patient has a High-sensitivity Troponin T level from equal or above 100 to equal or below 1000 ng/L. In one embodiment, the patient has a High-sensitivity Troponin T level from equal or above 100 to equal or below 900 ng/L. In one embodiment, the patient has a High-sensitivity Troponin T level from equal or above 100 to equal or below 800 ng/L. In one embodiment, the patient has a High-sensitivity Troponin T level from equal or above 100 to equal or below 700 ng/L. In one embodiment, the patient has a High-sensitivity Troponin T level from equal or above 100 to equal or below 600 ng/L. In one embodiment, the patient has a High-sensitivity Troponin T level from equal or above 100 to equal or below 500 ng/L. In one embodiment, the patient has a High-sensitivity Troponin T level from equal or above 100 to equal or below 400 ng/L. In one embodiment, the patient has a High-sensitivity Troponin T level from equal or above 100 to equal or below 300 ng/L. In one embodiment, the patient has a High-sensitivity Troponin T level from equal or above 100 to equal or below 200 ng/L.

In one embodiment, the patient has a High-sensitivity Troponin T level from equal or above 1000 to equal or below 95000 ng/L. In one embodiment, the patient has a High-sensitivity Troponin T level from equal or above 1000 to equal or below 8000 ng/L. In one embodiment, the patient has a High-sensitivity Troponin T level from equal or above 1000 to equal or below 7000 ng/L. In one embodiment, the patient has a High-sensitivity Troponin T level from equal or above 1000 to equal or below 6000 ng/L. In one embodiment, the patient has a High-sensitivity Troponin T level from equal or above 1000 to equal or below 5000 ng/L. In one embodiment, the patient has a High-sensitivity Troponin T level from equal or above 1000 to equal or below 4000 ng/L. In one embodiment, the patient has a High-sensitivity Troponin T level from equal or above 1000 to equal or below 3000 ng/L. In one embodiment, the patient has a High-sensitivity Troponin T level from equal or above 1000 to equal or below 2000 ng/L.

In one embodiment, the patient has a High-sensitivity Troponin T level from equal or above 6 to equal or below 30 ng/L. In one embodiment, the patient has a High-sensitivity Troponin T level from equal or above 6.5 to equal or below 30 ng/L.

In one embodiment, the patient has a High-sensitivity Troponin T level selected from of equal or above 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100, 20, 300, 400, 500, 600, 700, 900, 1000, 1500, 2000, 2500, 3000, 3500, 4000, 4500, 5000, 5500, 6000, 6500, 7000, 7500, 8000, 8500, 9000 and 9500 ng/L. In one embodiment, the patient has a High-sensitivity Troponin T level selected from of equal or above 5, 6, 7, 8, 9, 10, 11, 12, 13, 14, 15, 16, 17, 18, 19, 20, 21, 22, 23, 24, 25, 26, 27, 28, 29, and 30 ng/L
In one embodiment, the patient has a High-sensitivity Troponin T level selected from of 6.5 ng/L, 17.7 ng/L, 26.1 ng/L, 28.2 ng/L, and 7610.0 ng/L
The N-terminal prohormone of brain natriuretic peptide (NT-proBNP) is a prohormone with a 76 amino acid N-terminal inactive protein that is cleaved from the molecule to release brain natriuretic peptide 32 (BNP, also known as B-type natriuretic peptide). Both BNP and NT-proBNP levels in the blood are used for screening, diagnosis and prognosis of CHF in both acute and non-acute settings. Elevated plasma concentrations of NT-proBNP correlate with the onset of heart failure hospitalization and cardiovascular death (Mueller et al "Heart Failure association of the European Society of cardiology practical guidance on the use of natriuretic peptide concentrations. European Journal of Heart Failure 2019; 21, 715-731).

NT-proBNP is expressed in pg/mL.

In one embodiment, the patient has a N-terminal prohormone of brain natriuretic peptide (NT-proBNP) level of equal or above 300 pg/mL.

In one embodiment, the patient has a NT-proBNP level selected from of equal or above 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 400, 4100, 4200, 4300, 4400 or 4500 pg/mL.

In one embodiment, the patient has a NT-proBNP level selected from of equal or below 300, 350, 400, 450, 500, 550, 600, 650, 700, 750, 800, 850, 900, 950, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 400, 4100, 4200, 4300, 4400 or 4500 pg/mL.

In one embodiment, the patient has a NT-proBNP level of equal or above 300 to equal or below 4300 pg/mL. In one embodiment, the patient has a NT-proBNP level of equal or above 300 to equal or below 2200 pg/mL. In one embodiment, the patient has a NT-proBNP level of equal or above 300 to equal or below 2000 pg/mL. In one embodiment, the patient has a NT-proBNP level of equal or above 300 to equal or below 1900 pg/mL. In one embodiment, the patient has a NT-proBNP level of equal or above 300 to equal or below 1600 pg/mL. In one embodiment, the patient has a NT-proBNP level of equal or above 500 to equal or below 3000 pg/mL.

In one embodiment, the patient has a Brain natriuretic Peptide (BNP) of equal or above 100 pg/mL. In one embodiment, the patient has a Brain natriuretic Peptide (BNP) of equal or above 300 pg/mL.

Blood pressure (BP) is the pressure of circulating blood against the walls of blood vessels. Most of this pressure results from the heart pumping blood through the circulatory system. When used without qualification, the term "blood pressure" refers to the pressure in a brachial artery, where it is most commonly measured. Blood pressure is usually expressed in terms of the systolic pressure (maximum pressure during one heartbeat) over diastolic pressure (minimum pressure between two heartbeats) in the cardiac cycle. It is expressed in millimeters of mercury (mmHg) above the surrounding atmospheric pressure, or in kilopascals (kPa). The difference between the systolic and diastolic pressures is known as pulse pressure, while the average pressure during a cardiac cycle is known as mean arterial pressure.

In one embodiment, the patient has a systolic blood pressure selected from equal or above 40, 45, 50, 55, 60, 65, 70, 75, 80, 85, 90, 95, 100, 105, 110, 114, 115, 120, 125, 129, 130, 135, 140, 144, 145, 150, 155, 160, 165, 170, 175, 180, 185, and 190 mmHg. In one embodiment, the patient has a systolic blood pressure selected from equal or below 40, 45, 50, 55, 60, 65, 70, 75, 80, 85, 90, 95, 100, 105, 110, 114, 115, 120, 125, 129, 130, 135, 140, 144, 145, 150, 155, 160, 165, 170, 175, 180, 185, and 190 mmHg.

In one embodiment, the patient has a systolic blood pressure of equal or above 40 mmHg. In one embodiment, the patient has a systolic blood pressure of equal or above 45 mmHg. In one embodiment, the patient has a systolic blood pressure of equal or above 50 mmHg. In one embodiment, the patient has a systolic blood pressure of equal or above 55 mmHg. In one embodiment, the patient has a systolic blood pressure of equal or above 60 mmHg. In one embodiment, the patient has a systolic blood pressure of equal or above 65 mmHg. In one embodiment, the patient has a systolic blood pressure of equal or above 70 mmHg. In one embodiment, the patient has a systolic blood pressure of equal or above 75 mmHg. In one embodiment, the patient has a systolic blood pressure of equal or above 80 mmHg. In one embodiment, the patient has a systolic blood pressure of equal or above 85 mmHg. In one embodiment, the patient has a systolic blood pressure of equal or above 90 mmHg. In one embodiment, the patient has a systolic blood pressure of equal or above 100 mmHg. In one embodiment, the patient has a systolic blood pressure of equal or above 105 mmHg. In one embodiment, the patient has a systolic blood pressure of equal or above 110 mmHg. In one embodiment, the patient has a systolic blood pressure of equal or above 114 mmHg. In one embodiment, the patient has a systolic blood pressure of equal or above 115 mmHg. In one embodiment, the patient has a systolic blood pressure of equal or above 120 mmHg. In one embodiment, the patient has a systolic blood pressure of equal or above 125 mmHg. In one embodiment, the patient has a systolic blood pressure of equal or above 129 mmHg. In one embodiment, the patient has a systolic blood pressure of equal or above 130 mmHg. In one embodiment, the patient has a systolic blood pressure of equal or above 135 mmHg. In one embodiment, the patient has a systolic blood pressure of equal or above 140 mmHg. In one embodiment, the patient has a systolic blood pressure of equal or above 144 mmHg. In one embodiment, the patient has a systolic blood pressure of equal or above 145 mmHg. In one embodiment, the patient has a systolic blood pressure of equal or above 150 mmHg. In one embodiment, the patient has a systolic blood pressure of equal or above 155 mmHg. In one embodiment, the patient has a systolic blood pressure of equal or above 160 mmHg. In one embodiment, the patient has a systolic blood pressure of equal or above 165 mmHg. In one embodiment, the patient has a systolic blood pressure of equal or above 170 mmHg. In one embodiment, the patient has a systolic blood pressure of equal or above 175 mmHg. In one embodiment, the patient has a systolic blood pressure of equal or above 180 mmHg. In one embodiment, the patient has a systolic blood pressure of equal or above 185 mmHg. In one embodiment, the patient has a systolic blood pressure of equal or above 190 mmHg.

In one embodiment, the patient has a systolic blood pressure of equal or below 40 mmHg. In one embodiment, the patient has a systolic blood pressure of equal or below 45 mmHg. In one embodiment, the patient has a systolic blood pressure of equal or below 50 mmHg. In one embodiment, the patient has a systolic blood pressure of equal or below 55 mmHg. In one embodiment, the patient has a systolic blood pressure of equal or below 60 mmHg. In one embodiment, the patient has a systolic blood pressure of equal or below 65 mmHg. In one embodiment, the patient has a systolic blood pressure of equal or below 70 mmHg. In one embodiment, the patient has a systolic blood pressure of equal or below 75 mmHg. In one embodiment, the patient has a systolic blood pressure of equal or below 80 mmHg. In one embodiment, the patient has a systolic blood pressure of equal or below 85 mmHg. In one embodiment, the patient has a systolic blood pressure of equal or below 90 mmHg. In one embodiment, the patient has a systolic blood pressure of equal or below 100 mmHg. In one embodiment, the patient has a systolic blood pressure of equal or below 105 mmHg. In one embodiment, the patient has a systolic blood pressure of equal or below 110 mmHg. In one embodiment, the patient has a systolic blood pressure of equal or below 114 mmHg. In one embodiment, the patient has a systolic blood pressure of equal or below 115 mmHg. In one embodiment, the patient has a systolic blood pressure of equal or below 120 mmHg. In one embodiment, the patient has a systolic blood pressure of equal or below 125 mmHg. In one embodiment, the patient has a systolic blood pressure of equal or below 129 mmHg. In one embodiment, the patient has a systolic blood pressure of equal or below 130 mmHg. In one embodiment, the patient has a systolic blood pressure of equal or below 135 mmHg. In one embodiment, the patient has a systolic blood pressure of equal or below 140 mmHg. In one embodiment, the patient has a systolic blood pressure of equal or below 144 mmHg. In one embodiment, the patient has a systolic blood pressure of equal or below 145 mmHg. In one embodiment, the patient has a systolic blood pressure of equal or below 150 mmHg. In one embodiment, the patient has a systolic blood pressure of equal or below 155 mmHg. In one embodiment, the patient has a systolic blood pressure of equal or below 160 mmHg. In one embodiment, the patient has a systolic blood pressure of equal or below 165 mmHg. In one embodiment, the patient has a systolic blood pressure of equal or below 170 mmHg. In one embodiment, the patient has a systolic blood pressure of equal or below 175 mmHg. In one embodiment, the patient has a systolic blood pressure of equal or below 180 mmHg. In one embodiment, the patient has a systolic blood pressure of equal or below 185 mmHg. In one embodiment, the patient has a systolic blood pressure of equal or below 190 mmHg.

In one embodiment, the patient has a systolic blood pressure of 129±15 mmHg. In one embodiment, the patient has a systolic blood pressure of equal or above 114 to equal or below 144 mmHg. In one embodiment, the patient has a systolic blood pressure of equal or above 114 to equal or below 129 mmHg. In one embodiment, the patient has a systolic blood pressure of equal or above 129 to equal or below 144 mmHg. In one embodiment, the patient has a systolic blood pressure of equal or above 110 to equal or below 114 mmHg. In one embodiment, the patient has a systolic blood pressure of equal or above 100 to equal or below 110 mmHg. In one embodiment, the patient has a systolic blood pressure of equal or above 90 to equal or below 100 mmHg.

As such, finerenone presents a new opportunity to modify risk of adverse events in patients with HF, including among those with cardio-kidney-metabolic multimorbidity. The patient to be treated may be multimorbid. He may have one or more other diseases. The patient may have a medical history, or various medical histories. The medical terms used herein are known to those skilled in the art and are used as common in the art.

In one embodiment, the patient has a history of prior MRA use. In one embodiment, the patient has a history of prior MRA use. In one embodiment, the patient has a history of atrial flutter. In one embodiment, the patient has a history of atrial fibrillation. In one embodiment, the patient has a history of coronary artery disease (CAD). In one embodiment, the patient has a history of stroke. In one embodiment, the patient has a history of transient ischemic attack (TIA). In one embodiment, the patient has a history of chronic obstructive pulmonary disease (COPD). In one embodiment, the patient has a history of metabolic syndrome. In one embodiment, the patient has a history of frailty. In one embodiment, the patient has a history of peripheral artery disease (PAD). In one embodiment, the patient has a history of obstructive sleep apnea (OSA). In one embodiment, the patient has a history of cancer. In one embodiment, the patient has a history of Chronic obstructive pulmonary disease (COPD). In one embodiment, the patient has a history of resistant hypertension. In one embodiment, the patient has a history of Diabetes. In one embodiment, the patient has a history of atrial flutter. In one embodiment, the patient has a history of atrial fibrillation. In one embodiment, the patient has a history of hypertension. In one embodiment, the patient has a history of myocardial infarction. In one embodiment, the patient has a history of stroke. In one embodiment, the patient has a history of LVEF equal or below 40%. In one embodiment, the patient was hospitalized prior to treatment or prevention. In one embodiment, the patient was hospitalized for hearth failure (HHF). In one embodiment, the patient has atherosclerotic cardiovascular disease (ASCVD). In one embodiment, the patient has a history of Diabetes. In one embodiment, the patient has type 2 diabetes (T2D). In one embodiment, the patient has chronic kidney disease. In one embodiment, the patient has a history of chronic kidney disease (CKD). In one embodiment, the patient has chronic kidney disease (CKD) with an eGFR equal or above 60mL/min/1.73m². In one embodiment, the patient a combination of atherosclerotic cardiovascular disease (ASCVD), type 2 diabetes (T2D), and/or chronic kidney disease (CKD; defined as eGFR<60mL/min/1.73m2). In one embodiment, the patient has sinus rhythm or normal heart rhythm. I one embodiment, the patient has a history of structural heart disease. The structural heart disease can be indicated by increased left atrial size or left ventricular hypertrophy.

Heart rate (or pulse rate) is the frequency of the heartbeat measured by the number of contractions of the heart per minute (beats per minute, beats/min or bpm). The American Heart Association states the normal resting adult human heart rate is 60-100 bpm. Tachycardia is a high heart rate, defined as above 100 bpm at rest. Bradycardia is a low heart rate, defined as below 60 bpm at rest. When the heart is not beating in a regular pattern, this is referred to as an arrhythmia. Abnormalities of heart rate sometimes indicate disease.

In one embodiment, the patient has an average heart rate (resting) selected from of equal or above 40, 45, 50, 55, 59, 60, 65, 70, 71, 75, 80, 83, 85, 90, 95, 100, 105, 110, 115, and 120 beats/min.

The average heart rate is a resting heart rate.

In one embodiment, the patient has an average (resting) heart rate of 71 +/- 12 beats/min. In one embodiment, the patient has an average (resting) heart rate of equal or above 45 to equal or below 59 beats/min. In one embodiment, the patient has an average (resting) heart rate of equal or above 59 to equal or below 83 beats/min. In one embodiment, the patient has an average (resting) heart rate of equal or above 59 to equal or below 71 beats/min. In one embodiment, the patient has an average (resting) heart rate of equal or above 71 to equal or below 83 beats/min. In one embodiment, the patient has an average (resting) heart rate of equal or above 83 beats/min to equal or below 110 beats/min. In one embodiment, the patient has an average (resting) heart rate of equal or above 90 beats/min to equal or below 110 beats/min. In one embodiment, the patient has an average (resting) heart rate of equal or above 90 beats/min to equal or below 100 beats/min.

The patient can receive further medication or comedication. This can be e.g. the case if the patients is multimorbid.

In one embodiment, the patient further receives any one of the medicaments selected from potassium-sparing diuretic, osmotic diuretic, carbonic anhydrase inhibitor, digoxin, nitrates, loop diuretics, thiazide diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), Sodium-glucose Cotransporter-1 Inhibitor (SGLT-1i), potassium supplements, potassium lowering agents, potassium binders, centrally acting antihypertensives, cytochrome P450 isoenzyme 3A4 (CYP3A4) inhibitors, CYP3A4 inducers, aspirin, statins, organic anion transporting polypeptides (OATP) substrates, anti-diabetic drugs, insulin, insulin analogues, dipeptidyl peptidase 4 inhibitors, glucagon-like peptide-1 (GLP-1) agonists, biguanides, sulfonylureas, alpha glucosidase inhibitors, metiglinides, thiazolidinediones, CYP3A4 inducer, CYP3A4 inhibitor and mixtures thereof.

In one embodiment, the patient further receives a potassium-sparing diuretic. In one embodiment, the patient further receives an osmotic diuretic. In one embodiment, the patient further receives a carbonic anhydrase inhibitor. In one embodiment, the patient further receives digoxin. In one embodiment, the patient further receives a nitrate. In one embodiment, the patient further receives a loop diuretic. In one embodiment, the patient further receives a thiazide diuretic. In one embodiment, the patient further receives a beta-blocker. In one embodiment, the patient further receives an ACE inhibitor (ACEi). In one embodiment, the patient further receives an angiotension-receptor-blocker (ARB). In one embodiment, the patient further receives an Angiotensin Receptor-Neprilysin Inhibitor (ARNI). In one embodiment, the patient further receives a Calcium Channel Blockers. In one embodiment, the patient further receives a Sodium-glucose Cotransporter-2 Inhibitor (SGLT-2i). In one embodiment, the patient further receives a Sodium-glucose Cotransporter-1 Inhibitor (SGLT-1i). In one embodiment, the patient further receives a potassium supplement. In one embodiment, the patient further receives a potassium lowering agent. In one embodiment, the patient further receives a potassium binder. In one embodiment, the patient further receives a antihypertensive agent. In one embodiment, the patient further receives a centrally acting antihypertensive. In one embodiment, the patient further receives acytochrome P450 isoenzyme 3A4 (CYP3A4) inhibitor. In one embodiment, the patient further receives a CYP3A4 inducer. In one embodiment, the patient further receives aspirin. In one embodiment, the patient further receives a statine. In one embodiment, the patient further receives an organic anion transporting polypeptides (OATP) substrate. In one embodiment, the patient further receives an anti-diabetic drug. In one embodiment, the patient further receives insulin. In one embodiment, the patient further receives an insulin analogue. In one embodiment, the patient further receives a dipeptidyl peptidase 4 inhibitor. In one embodiment, the patient further receives a glucagon-like peptide-1 agonists. In one embodiment, the patient further receives a biguanide. In one embodiment, the patient further receives a sulfonylurea. In one embodiment, the patient further receives an alpha glucosidase inhibitor. In one embodiment, the patient further receives a metiglinide. In one embodiment, the patient further receives a thiazolidinedione.

In one embodiment, the patient further receives an alpha blocker. In one embodiment, the patient further receives a beta blocker. In one embodiment the alpha blocker and/or beta blocker is selected from acebutolol, amlodipine, bisoprolol, arotinolol, atenolol, betaxolol, bimatoprost, timolol, bisoprolol, perindopril, bromonidine, brinzolamide, carteolol, lantanoprost, carvediol, ivabradine, dorzolamide, esmolol, hydrochlorothiazide, metroprolol, nebivolol, labetalol, landiolol, nadolol, nebiviolol, pilocarpine, propranolol, sotalol, tafluprost, timolol, travoprost, the pharmaceutical acceptable salts thereof and combinations thereof.

In one embodiment, the patient further receives an angiotensin-converting enzyme (ACE) inhibitor. In one embodiment the ACE inhibitor is selected from ramipril, alacepril, amlodipine, perindopril, benazepril, captopril, enalapril, fosinopril, lisinopril, moexipril, perindopril, ramipril, zofenopril, clizapril, imidapril, quinapril, trandolapril, the pharmaceutical acceptable salts thereof and combinations thereof.

In one embodiment, the patient further receives an angiotensin receptor blockers (ARBs) selected from allisartan, azilsartan, candesartan, olmesartan, valsartan, irbesartan, losartan, telmisartan, candesartan, eprosartan, fimasartan, the pharmaceutical acceptable salts thereof and combinations thereof.

In one embodiment, the patient further receives an angiotensin receptor neprilysin inhibitors (ARNIs). In one embodiment the ARNI is sacubitril or the pharmaceutical acceptable salts thereof. In one embodiment the patient receives sacubitril, valsartan or the pharmaceutical acceptable salts thereof.

In one embodiment, the patient further receives atorvastatin the pharmaceutical acceptable salts thereof. In one embodiment the patient receives a further medication selected from acetylsalicylic acid, valsartan, clopidogrel, ramipril, perindopril, ezetimibe, the pharmaceutical acceptable salts thereof and combinations thereof.

In one embodiment, the patient further receives a biguanide. In one embodiment the patient receives a medication selected from alogliptin, metformin, canagliflozin, dapagliflozin, empagliflozin, ertugliflozin, gemigliptin, glibenclamide, gliciclazide, linagliptide, pioglitazone, sitagliptin, teneligliptin, vildagliptin, vogliibose, pioglitazin, the pharmaceutical acceptable salts thereof and combinations thereof.

In one embodiment, the patient further receives a calcium channel blocker. In one embodiment, the patient further receives a calcium channel blocker selected from amlodipine, azelnidipine, barnidipine, benidipine, bepridil, cilnidipine, diltiazem, efonidipine, lacidinipine, lercanidipine, levamlodipine, manidipine, nicardipine, nifedipine, nilvadepine, nimodipine,nisoldipine, nitrendipine, perhexiline, verapamil, the pharmaceutical acceptable salts thereof and combinations thereof. In one embodiment, the patient further receives a medication selected from amlodipine, atorvastatin, candesartan, hydrochlorotiazide (HCT), perindopril, azilsartan, benazepril, ramipril, Olmesartan, valsartn, indapipamide, perindopril, irbesartan, lisinopril, lorsartan, azelnidipine, barnidipine, benidipine, bepridil, cilnidipine, diltiazem, efonidipine, lacidinipine, lercanidipine, levamlodipine, manidipine, nicardipine, nifedipine, nilvadepine, nimodipine, nisoldipine, nitrendipine, perhexiline, verapamil, perindopril, ramipril, rosuvastatin, telmisartan, atorvastatin, benazepril, the pharmaceutical acceptable salts thereof and combinations thereof.

In one embodiment, the patient further receives a dipeptidyl peptidase-4 (DDP4) inhibitor. In one embodiment, the DDP4 inhibitor is selected from alogliptin, anagliptin, tenegliptin, saxagliptin, linagliptin, gemigliptin, gosogliptin, sitagliptin, tenegliptin, vildagliptin, the pharmaceutical acceptable salts thereof and combinations thereof. In one embodiment, the patient reeives a medication selected from alogliptin, anagliptin, tenegliptin, saxagliptin, linagliptin, gemigliptin, gosogliptin, sitagliptin, tenegliptin, vildagliptin, metformin, pioglitazone, cangliflozin, dapagliflozin, empagliflozin, ipragliflozin, the pharmaceutical acceptable salts thereof and combinations thereof.

In one embodiment, the patient further receives a diuretic. In one embodiment, the diuretic is selected from tropolone, acetazolamide, altizide, sprionolacton, amiloride, hydrochlorotiaazide, clopamide,
chlorthalidone, indapamide, furosemide, azosemide, glycerol, cendroflumethiazide, brimonidine, bumetanide, butizide, chlorothiazide, isosorbide, triamterene, dorzolamide, metolazone, protheobromide, torasemide, tolvaptan, trochloromethiazid, xipamide, theobromide, hydrocortisone, pharmaceutical acceptable salts thereof and combinations thereof. In one embodiment, the patient further receives a medicationelected from tropolone, acetazolamide, altizide, sprionolacton, amiloride, hydrochlorotiaazide, chlorthalidone, indapamide, furosemide, azosemide, glycerol, cendroflumethiazide, brimonidine, bumetanide, butizide, chlorothiazide, isosorbide, triamterene, dorzolamide, metolazone, protheobromide, torasemide, tolvaptan, trochloromethiazid, xipamide, theobromide, hydrocortisone, tolvaptan, torasemide, trichloromethiazide, urea, xipamide, amlodipine, olmesatran, perindopril, atenolol, azosemide, benazepril, potassium chloride, bisoprololm timolol, candesartan, captopril, cetomacrogel, cilapril, clobetasol, clopamide, clotrimazole, timolol, enalapril, etacrynic acid, irbesartan, losartan, telmisartan, nebivolol, zofenopril, rosuvastatin, the pharmaceutical acceptable salts thereof and combinations thereof.

In one embodiment, the patient further receives a stain. In one embodiment, the statin is selected from fluvastatin, atorvastatin, lovastatin, pitavastatin, pravastatin, rosuvastatin, simvastatin, astaxanthin, the pharmaceutical acceptable salts thereof and combinations thereof.

In one embodiment, the patient further receives a glucagon-like peptide 1 (GLP-1) agonist. In one embodiment, the GLP-1 agonist is selected from dulaglutide, exenatide, liraglutide, lixisenatine, semaglutine, tirzepatide, the pharmaceutical acceptable salts thereof and combinations thereof.

In one embodiment, the patient further receives a sodium-glucose cotransporter-2 (SGLT2) inhibitor. In one embodiment the SGLT2 inhibitor is selected from canagliflozin, dapagliflozin, empagliflozin, ertigliflozin, ipragliflozin, luseogliflozin, remogliflozin, tofogliflozin, the pharmaceutical acceptable salts thereof and combinations thereof.

In one embodiment, the patient further receives a sulphonamide. In one embodiment the sulfonamide is selected from glibenclamide, gliclazide, glimepiride, glipizide, gliquidone, tolbutamide, the pharmaceutical acceptable salts thereof and combinations thereof.

In one embodiment, the patient further receives a potassium binder. In one embodiment, the potassium binder is selected from potassium polystytrene, calcium polystyrene sulfonate, patiromer calcium, patiromer, sorbitex calcium, sodium polystyrene sulfonate, sodium zirconium cyclosilicate, the pharmaceutical acceptable salts thereof and combinations thereof.

In one embodiment, the patient further receives a mineralocorticoid receptor antagonists (MRAs). In one embodiment the MRA is selected from spironolactone, canrenone, eplerenone, potassium canrenoate, the pharmaceutical acceptable salts thereof and combinations thereof.

In one embodiment, the patient further receives a vabradine or the pharmaceutical acceptable salts thereof.

In one embodiment, the patient further receives an insulin. In one embodiment, the insulin is selected from a natural, synthetic, modified, procine, bovine, insuline derivatve, insuline analogouea, long acting, short acting, a pharmaceutical acceptable salt thereof and combinations thereof. In one embodiment, the insulin is selected from insuline aspart, aspart protamine, protamine, dediglec, bovine, procine, glargine, glargine biosimiliar, glusine, human, lispro, lispro bioaimilia, lispro protamine, human, zinc suspension, analogues, the pharmaceutical acceptable salts thereof and combinations thereof.

In one embodiment, the patient further receives an oral antidiabetic. In one embodiment, the oral antidiabetic is selected from alpha-glucosidase inhibitors, amylin analogs, dipeptidyl peptidase 4 inhibitors, incretin mimetics, insulin,meglitinides, non-sulfonylureas, SGLT-2, sulfonylureas, and thiazolidinediones, the pharmaceutical acceptable salts thereof and combinations thereof. In one embodiment, the oral antidiabetic is selected from acarbose, miglitol, pramlintide, alogliptan, linagliptan, saxagliptin, sitagliptin, albiglutide, dulaglutide, exenatide, liraglutide, lixisenatide, insulins, nateglinide, repaglinide, metformin, canagliflozin, dapagliflozin, empagliflozin, chlorpropamide, glimepiride, glipizide, glyburide, tolazamide, tolbutamide, rosiglitazone, pioglitazone, the pharmaceutical acceptable salts thereof and combinations thereof. In one embodiment, the further medication is selected from (RS)-3-METHYL-2-OXOVALERIANIC ACID CALCIUM;(RS)-3-METHYL-2-OXOBUTYRIC ACID CALCIUM;CALCIUM (RS)-4-METHYL-2-OXOVALERIANAT;CALCIUM 2-OXO-3-PHENYLPROPIONATE, DESMENINOL CALCIUM, HISTIDINE, LYSINE ACETATE, THREONINE, TRYPTOPHAN, TYROSINE, ACARBOSE, ACETYLCYSTEINE, ALOGLIPTIN, ALOGLIPTIN BENZOATE, ALOGLIPTIN BENZOATE, METFORMIN HYDROCHLORIDE, ALOGLIPTIN BENZOATE, PIOGLITAZONE HYDROCHLORIDE, ANAGLIPTIN, ATORVASTATIN, ATORVASTATIN CALCIUM, BIGUANIDE, CALCIUM CARBONATE, MAGNESIUM CARBONATE, POTASSIUM CARBONATE;SODIUM BICARBONATE, SODIUM PHOSPHATE, CALCIUM DOBESILATE, CANAGLIFLOZIN, CANAGLIFLOZIN HEMIHYDRATE, CANAGLIFLOZIN HEMIHYDRATE, METFORMIN, CANAGLIFLOZIN HEMIHYDRATE, TENELIGLIPTIN HYDROBROMIDE, CHLORPHENAMINE MALEATE, PHENYLEPHRINE HYDROCHLORIDE, PHENYLPROPANOLAMINE HYDROCHLORIDE;PHENYLTOLOXAMINE CITRATE, CYSTEINE, GLYCINE, GLYCYRRHIZIC ACID, AMMONIUM SALT, DAPAGLIFLOZIN, SAXAGLIPTIN, DEXAMETHASONE, DIGOXIN, DULAGLUTIDE, EMPAGLIFLOZIN, ENOXAPARIN, ERTUGLIFLOZ1N, EXENATIDE, GEMIGLIPTIN TARTRATE, GEMIGLIPTIN TARTRATE, GLIBENCLAMIDE, GLICLAZIDE, GLIMEPIRIDE, GLIPIZIDE, GLIQUIDONE, GLUCAGON, GLUCOSE, GOSOGLIPTIN, HYDROCHLOROTHIAZIDE, IMEGLIMIN, INSULIN ASPART, INSULIN ASPART PROTAMINE (CRYSTALLINE), INSULIN DEGLUDEC, INSULIN GLARGINE, INSULIN HUMAN, INSULIN HUMAN INJECTION, ISOPHANE, INSULIN HUMAN, INSULIN LISPRO PROTAMINE SUSPENSION, INSULIN LISPRO, IPRAGLIFLOZIN L-PROLINE, IPRAGLIFLOZIN L-PROLINE;SITAGLIPTIN PHOSPHATE MONOHYDRATE, KALLIDINOGENASE, LEVOTHYROXINE SODIUM, LINAGLIPTIN, LIRAGLUTIDE, LIRAGLUTIDE, LIXISENATIDE, LIXISENATIDE, LUSEOGLIFLOZIN, MACROGOL, MECOBALAMIN, MELILOTUS OFFICINALIS EXTRACT, METFORMIN, MIGLITOL, MITIGLINIDE, NATEGLINIDE, , PIOGLITAZONE, PITAVASTATIN, QUETIAPINE FUMARATE, REMOGLIFLOZIN ETABONATE, REPAGLINIDE, ROSUVASTATIN, SAXAGLIPTIN, SEMAGLUTIDE, SITAGLIPTIN, SULTAMICILLIN, TELMISARTAN, THIOCTIC ACID, TIRZEPATIDE, TOFOGLIFLOZIN, TOLBUTAMIDE, VILDAGLIPTIN, VOGLIBOSE, the pharmaceutical acceptable salts thereof and combinations thereof.

In one embodiment, the patient further receives a CYP3A4 inducer. The patient can receive the CYP3A4 inducer prior and/or during the treatment. In one embodiment, the patient further receives a weak CYP3A4 inducer. In one embodiment, the patient further receives a moderate CYP3A4 inducer. In one embodiment, the patient further receives a strong CYP3A4 inducer. In one embodiment, the patient further receives a CYP3A4 inducer of unspecified potency. Strong and moderate CYP3A4 inducers are drugs that decrease the AUC of sensitive (index) substrates of a given pathway where CYP3A4 is involved by ≥80 percent and ≥50 to <80 percent, respectively. Weak inducers decrease the AUC by ≥20 to <50 percent.

Examples, but not limited to, strong CYP3A4 inducers can be carbamazepine, antiandrogens (enzalutamide, apalutamide), primidone, phenytoin (anticonvulsant), rifampin, rifampicin, rifapentine, rifabutin, alogliptin, pioglitazone, aprepitant, carbamazepine, clobazam, eleutherocococcus senticosus, hypericum perforatum, St John's wort, enzalutamide, apalutamide, methformin, oxcarbazepine, prednisone, quercetin, avasimibe, fosphenytoin, ivosidenib, lumacaftor, and/or mitotane.

In one embodiment, the strong CYP3A4 inducer and/or a combination that comprises at least one strong CYP3A4 inducer is selected from:
- CARBAMAZEPINE
- ELEUTHEROCOCCUS SENTICOSUS ROOT WITH RHIZOME;HYPERICUM PERFORATUM HERB
- ENZALUTAMIDE
- HYPERICUM PERFORATUM EXTRACT
- PHENYTOIN
- PHENYTOIN SODIUM
- RIFAMPICIN
- RIFAMPICIN SODIUM
and mixtures thereof. In one embodiment thereof, the patient receives an amount of the compound according to formula (1) of 5 to 80 mg. In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inducer. The adjustment can be up-titration, down-titration, maintenance or interruption of the treatment with the compound according to formula (1). Multiple embodiments of dose adjustments are described in detail below. These can be applied based on the co-medication with a CYP3A4 inducer. These can also be applied based on the amount and/or type of the CYP3A4 inducer the patient receives.

In one embodiment, the strong CYP3A4 inducer and/or a combination that comprises at least one strong CYP3A4 inducer is selected from:
- CARBAMAZEPINE
- PHENYTOIN SODIUM
and mixtures thereof, wherein the patient receive an amount of the compound according to formula (1) of 10 mg.

In one embodiment, the strong CYP3A4 inducer and/or a combination that comprises at least one strong CYP3A4 inducer is selected from:
- CARBAMAZEPINE
- ENZALUTAMIDE
- HYPERICUM PERFORATUM EXTRACT
- PHENYTOIN
- PHENYTOIN SODIUM
- RIFAMPICIN
and mixtures thereof, wherein the patient receive an amount of the compound according to formula (1) of 20 mg.

In one embodiment, the strong CYP3A4 inducer and/or a combination that comprises at least one strong CYP3A4 inducer is selected from:
- CARBAMAZEPINE
- PHENYTOIN
- RIFAMPICIN
and mixtures thereof, wherein the patient receive an amount of the compound according to formula (1) of 40 mg.

Examples, but not limited to, moderate CYP3A4 inducers can be upadacitinib, benzalkomium chloride, dexamethasons, phenylmercuric nitrate, dexamethasone, modafinil, nafcillin, asunaprevir, beclabuvir, daclatasvir, bosentan, cenobamate, dabrafenib, efavirenz, elagolix, etravirine, lersivirine, lesinurad, lopinavir, lorlatinib, metamizole (dipyrone), mitapivat, pexidartinib, phenobarbital, primidone, repotrectinib, rifabutin, semagacestat, sotorasib, talviraline, telotristat ethyl, thioridazine.

In one embodiment, the moderate CYP3A4 inducer and/or a combination that comprises at least one moderate CYP3A4 inducer is selected from:
- BENZALKONIUM CHLORIDE;DEXAMETHASONE;PHENYLMERCURIC NITRATE
- BENZALKONIUM CHLORIDE;DEXAMETHASONE;RESORCINOL;TETRACAINE HYDROCHLORIDE
- BENZETHONIUM CHLORIDE;CETYLPYRIDINIUM CHLORIDE;DEXAMETHASONE;HEXYLRESORCINOL
- BORIC ACID;DEXAMETHASONE SODIUM PHOSPHATE;SODIUM BORATE;THIOMERSAL
- CARMELLOSE;DEXAMETHASONE SODIUM METASULFOBENZOATE
- CHLORAMPHENICOL;DEXAMETHASONE
- CIPROFLOXACIN HYDROCHLORIDE;DEXAMETHASONE
- CYANOCOBALAMIN;DEXAMETHASONE SODIUM PHOSPHATE;PYRIDOXINE HYDROCHLORIDE;THIAMINE HYDROCHLORIDE
- DEXAMETHASONE
- DEXAMETHASONE ACETATE
- DEXAMETHASONE DIPROPIONATE
- DEXAMETHASONE PHOSPHATE
- DEXAMETHASONE PHOSPHATE;POLYMYXIN B SULFATE;TRIMETHOPRIM
- DEXAMETHASONE PHOSPHATE;TOBRAMYCIN
- DEXAMETHASONE SODIUM METASULFOBENZOATE
- DEXAMETHASONE SODIUM PHOSPHATE
- DEXAMETHASONE SODIUM PHOSPHATE;LEVOFLOXACIN HEMIHYDRATE
- DEXAMETHASONE SODIUM PHOSPHATE;NEOMYCIN SULFATE
- DEXAMETHASONE SODIUM PHOSPHATE;NEOMYCIN SULFATE;POLYMYXIN B SULFATE
- DEXAMETHASONE SODIUM PHOSPHATE;NETILMICIN SULFATE
- DEXAMETHASONE SODIUM PHOSPHATE;TOBRAMYCIN
- DEXAMETHASONE SODIUM SUCCINATE
- DEXAMETHASONE VALERATE
- DEXAMETHASONE;FRAMYCETIN SULFATE;GRAMICIDIN
- DEXAMETHASONE;GENTAMICIN
- DEXAMETHASONE;MOXIFLOXACIN
- DEXAMETHASONE;MOXIFLOXACIN HYDROCHLORIDE
- DEXAMETHASONE;NEOMYCIN SULFATE;POLYMYXIN B SULFATE
- DEXAMETHASONE;NEOMYCIN;POLYMYXIN B
- DEXAMETHASONE;TOBRAMYCIN
- MODAFINIL
- NAFCILLIN
and mixtures thereof. In one embodiment thereof, the patient receives an amount of the compound according to formula (1) of 5 to 80 mg. In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inducer. The adjustment can be up-titration, down-titration, maintenance or interruption of the treatment with the compound according to formula (1). Multiple embodiments of dose adjustments are described in detail below. These can be applied based on the co-medication with a CYP3A4 inducer. These can also be applied based on the amount and/or type of the CYP3A4 inducer the patient receives.

In one embodiment, the moderate CYP3A4 inducer and/or a combination that comprises at least one moderate CYP3A4 inducer is selected from:
- BENZALKONIUM CHLORIDE;DEXAMETHASONE;RESORCINOL;TETRACAINE HYDROCHLORIDE
- DEXAMETHASONE
- DEXAMETHASONE ACETATE
- DEXAMETHASONE DIPROPIONATE
- DEXAMETHASONE PHOSPHATE
- DEXAMETHASONE SODIUM PHOSPHATE
- DEXAMETHASONE SODIUM PHOSPHATE;NEOMYCIN SULFATE
- DEXAMETHASONE SODIUM PHOSPHATE;NETILMICIN SULFATE
- DEXAMETHASONE SODIUM PHOSPHATE;TOBRAMYCIN
- DEXAMETHASONE;NEOMYCIN SULFATE;POLYMYXIN B SULFATE
- DEXAMETHASONE;TOBRAMYCIN
- MODAFINIL
and mixtures thereof, wherein the patient receive an amount of the compound according to formula (1) of 10 mg.

In one embodiment, the moderate CYP3A4 inducer and/or a combination that comprises at least one moderate CYP3A4 inducer is selected from:
- BENZETHONIUM CHLORIDE;CETYLPYRIDINIUM CHLORIDE;DEXAMETHASONE;HEXYLRESORCINOL
- CHLORAMPHENICOL;DEXAMETHASONE
- CYANOCOBALAMIN;DEXAMETHASONE SODIUM PHOSPHATE;PYRIDOXINE HYDROCHLORIDE;THIAMINE HYDROCHLORIDE
- DEXAMETHASONE
- DEXAMETHASONE ACETATE
- DEXAMETHASONE DIPROPIONATE
- DEXAMETHASONE PHOSPHATE
- DEXAMETHASONE PHOSPHATE;TOBRAMYCIN
- DEXAMETHASONE SODIUM PHOSPHATE
- DEXAMETHASONE SODIUM PHOSPHATE;NEOMYCIN SULFATE
- DEXAMETHASONE SODIUM PHOSPHATE;NEOMYCIN SULFATE;POLYMYXIN B SULFATE
- DEXAMETHASONE SODIUM PHOSPHATE;NETILMICIN SULFATE
- DEXAMETHASONE SODIUM SUCCINATE
- DEXAMETHASONE VALERATE
- DEXAMETHASONE;FRAMYCETIN SULFATE;GRAMICIDIN
- DEXAMETHASONE;GENTAMICIN
- DEXAMETHASONE;MOXIFLOXACIN
- DEXAMETHASONE;MOXIFLOXACIN HYDROCHLORIDE
- DEXAMETHASONE;NEOMYCIN SULFATE;POLYMYXIN B SULFATE
- DEXAMETHASONE;NEOMYCIN;POLYMYXIN B
- DEXAMETHASONE;TOBRAMYCIN
- MODAFINIL
and mixtures thereof, wherein the patient receive an amount of the compound according to formula (1) of 20 mg.

In one embodiment, the moderate CYP3A4 inducer and/or a combination that comprises at least one moderate CYP3A4 inducer is selected from:
- BENZALKONIUM CHLORIDE;DEXAMETHASONE;PHENYLMERCURIC NITRATE
- CARMELLOSE;DEXAMETHASONE SODIUM METASULFOBENZOATE
- CIPROFLOXACIN HYDROCHLORIDE;DEXAMETHASONE
- DEXAMETHASONE
- DEXAMETHASONE ACETATE
- DEXAMETHASONE DIPROPIONATE
- DEXAMETHASONE PHOSPHATE
- DEXAMETHASONE PHOSPHATE;POLYMYXIN B SULFATE;TRIMETHOPRIM
- DEXAMETHASONE SODIUM METASULFOBENZOATE
- DEXAMETHASONE SODIUM PHOSPHATE
- DEXAMETHASONE SODIUM PHOSPHATE;NEOMYCIN SULFATE
- DEXAMETHASONE VALERATE
- DEXAMETHASONE;FRAMYCETIN SULFATE;GRAMICIDIN
- DEXAMETHASONE;MOXIFLOXACIN HYDROCHLORIDE
- DEXAMETHASONE;NEOMYCIN SULFATE;POLYMYXIN B SULFATE
- DEXAMETHASONE;TOBRAMYCIN
and mixtures thereof, wherein the patient receive an amount of the compound according to formula (1) of 40 mg.

In one embodiment, the moderate CYP3A4 inducer and/or a combination that comprises at least one moderate CYP3A4 inducer is selected from:
- DEXAMETHASONE;TOBRAMYCIN
and mixtures thereof, wherein the patient receive an amount of the compound according to formula (1) of 80 mg.

Examples, but not limited to, CYP3A4 inducers of unspecified potency can be anticonvulsants, mood stabilizers (oxcarbazepine, topiramate), barbiturates (phenobarbital, butalbital), bactericidals (rifampicin, rifabutin), non-nucleoside reverse-transcriptase inhibitors (efavirenz, nevirapine), troglitazone (hypoglycemic), glucocorticoids (blood glucose increase, immunosuppressive), capsaicin, brigatinib, clobazam, dabrafenib, elagolix, eslicarbazepine, letermovir, lorlatinib, oritavancin, perampanel, and/or telotristat.

In one embodiment, the CYP3A4 inducer of unspecified potency and/or a combination that comprises at least one CYP3A4 inducer of unspecified potency is selected from:
- AMOBARBITAL;CAFFEINE;CODEINE PHOSPHATE;OCTIBENZONIUM BROMIDE;PARACETAMOL
- CLOTRIMAZOLE;HEXAMIDINE ISETIONATE;PREDNISOLONE ACETATE
- CYPROTERONE
- GINKGO BILOBA LEAF;LIGUSTRAZINE PHOSPHATE
- LIGUSTRAZINE HYDROCHLORIDE
- LIGUSTRAZINE HYDROCHLORIDE;SALVIA MILTIORRHIZA
- LIGUSTRAZINE HYDROCHLORIDE;SALVIA MILTIORRHIZA ROOT WITH RHIZOME
- LIGUSTRAZINE PHOSPHATE
- PHENYLBUTAZONE;PREDNISOLONE
- PREDNISOLONE
- PREDNISOLONE ACETATE
- PREDNISOLONE ACETATE;SULFACETAMIDE SODIUM
- PREDNISOLONE PHOSPHATE
- PREDNISOLONE SODIUM PHOSPHATE
- PREDNISOLONE SODIUM SUCCINATE
- PREDNISOLONE STEAGLATE
- PREDNISOLONE VALEROACETATE
- PREDNISOLONE;TETRYZOLINE HYDROCHLORIDE
- RELUGOLIX
- RIFAMYCIN SODIUM
and mixtures thereof. In one embodiment thereof, the patient receives an amount of the compound according to formula (1) of 5 to 80 mg. In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inducer. The adjustment can be up-titration, down-titration, maintenance or interruption of the treatment with the compound according to formula (1). Multiple embodiments of dose adjustments are described in detail below. These can be applied based on the co-medication with a CYP3A4 inducer. These can also be applied based on the amount and/or type of the CYP3A4 inducer the patient receives.

In one embodiment, the CYP3A4 inducer of unspecified potency and/or a combination that comprises at least one CYP3A4 inducer of unspecified potency is selected from:
- LIGUSTRAZINE HYDROCHLORIDE;SALVIA MILTIORRHIZA
- LIGUSTRAZINE HYDROCHLORIDE;SALVIA MILTIORRHIZA ROOT WITH RHIZOME
- PREDNISOLONE
- PREDNISOLONE ACETATE
- PREDNISOLONE SODIUM PHOSPHATE
- PREDNISOLONE SODIUM SUCCINATE
- PREDNISOLONE VALEROACETATE
- PREDNISOLONE;TETRYZOLINE HYDROCHLORIDE
and mixtures thereof, wherein the patient receive an amount of the compound according to formula (1) of 10 mg.

In one embodiment, the CYP3A4 inducer of unspecified potency and/or a combination that comprises at least one CYP3A4 inducer of unspecified potency is selected from:
- CYPROTERONE
- PHENYLBUTAZONE;PREDNISOLONE
- PREDNISOLONE
- PREDNISOLONE ACETATE
- PREDNISOLONE SODIUM PHOSPHATE
- PREDNISOLONE VALEROACETATE
- PREDNISOLONE;TETRYZOLINE HYDROCHLORIDE
and mixtures thereof, wherein the patient receive an amount of the compound according to formula (1) of 20 mg.

In one embodiment, the CYP3A4 inducer of unspecified potency and/or a combination that comprises at least one CYP3A4 inducer of unspecified potency is selected from:
- CYPROTERONE
- LIGUSTRAZINE HYDROCHLORIDE
- LIGUSTRAZINE HYDROCHLORIDE;SALVIA MILTIORRHIZA ROOT WITH RHIZOME
- LIGUSTRAZINE PHOSPHATE
- PREDNISOLONE
- PREDNISOLONE ACETATE
- PREDNISOLONE SODIUM PHOSPHATE
- PREDNISOLONE STEAGLATE
- PREDNISOLONE VALEROACETATE
and mixtures thereof, wherein the patient receive an amount of the compound according to formula (1) of 40 mg.

Examples, but not limited to, weak CYP3A4 inducers can be alogliptin, pioglitazone, aprepitant, clobazam, metformin, oxcabazepine, prednisone, quercetin.

In one embodiment, the weak CYP3A4 inducer and/or a combination that comprises at least one weak CYP3A4 inducer is selected from:
- ALOGLIPTIN BENZOATE;PIOGLITAZONE HYDROCHLORIDE
- APREPITANT
- CLOBAZAM
- METFORMIN HYDROCHLORIDE;PIOGLITAZONE HYDROCHLORIDE
- METFORMIN;PIOGLITAZONE
- OXCARBAZEPINE
- PIOGLITAZONE
- PIOGLITAZONE HYDROCHLORIDE
- PREDNISONE
- PREDNISONE ACETATE
- QUERCETIN
and mixtures thereof. In one embodiment thereof, the patient receives an amount of the compound according to formula (1) of 5 to 80 mg. In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inducer. The adjustment can be up-titration, down-titration, maintenance or interruption of the treatment with the compound according to formula (1). Multiple embodiments of dose adjustments are described in detail below. These can be applied based on the co-medication with a CYP3A4 inducer. These can also be applied based on the amount and/or type of the CYP3A4 inducer the patient receives.

In one embodiment, the weak CYP3A4 inducer and/or a combination that comprises at least one weak CYP3A4 inducer is selected from:
- PREDNISONE
wherein the patient receive an amount of the compound according to formula (1) of 5 mg.

In one embodiment, the weak CYP3A4 inducer and/or a combination that comprises at least one weak CYP3A4 inducer is selected from:
- ALOGLIPTIN BENZOATE;PIOGLITAZONE HYDROCHLORIDE
- METFORMIN HYDROCHLORIDE;PIOGLITAZONE HYDROCHLORIDE
- OXCARBAZEPINE
- PIOGLITAZONE
- PIOGLITAZONE HYDROCHLORIDE
- PREDNISONE
- PREDNISONE ACETATE
- QUERCETIN
and mixtures thereof, wherein the patient receive an amount of the compound according to formula (1) of 10 mg.

In one embodiment, the weak CYP3A4 inducer and/or a combination that comprises at least one weak CYP3A4 inducer is selected from:
- ALOGLIPTIN BENZOATE;PIOGLITAZONE HYDROCHLORIDE
- APREPITANT
- CLOBAZAM
- METFORMIN HYDROCHLORIDE;PIOGLITAZONE HYDROCHLORIDE
- OXCARBAZEPINE
- PIOGLITAZONE
- PIOGLITAZONE HYDROCHLORIDE
- PREDNISONE
- PREDNISONE ACETATE
- QUERCETIN
and mixtures thereof, wherein the patient receive an amount of the compound according to formula (1) of 20 mg.

In one embodiment, the weak CYP3A4 inducer and/or a combination that comprises at least one weak CYP3A4 inducer is selected from:
- METFORMIN HYDROCHLORIDE;PIOGLITAZONE HYDROCHLORIDE
- METFORMIN;PIOGLITAZONE
- OXCARBAZEPINE
- PIOGLITAZONE
- PIOGLITAZONE HYDROCHLORIDE
- PREDNISONE
- PREDNISONE ACETATE
- QUERCETIN
and mixtures thereof, wherein the patient receive an amount of the compound according to formula (1) of 40 mg.

In one embodiment, the patient further receives a CYP3A4 inhibitor. The patient can receive the CYP3A4 inhibitor prior and/or during the treatment. In one embodiment, the patient further receives a weak CYP3A4 inhibitor. In one embodiment, the patient further receives a moderate CYP3A4 inhibitor. In one embodiment, the patient further receives a strong CYP3A4 inhibitor.

Inhibitors of CYP3A4 are classified by potency. A strong inhibitor causes at least a 5-fold increase in the plasma AUC values, or equal or more than 80% decrease in clearance, of CYP3A4 sensitive (index) substrate. A moderate inhibitor causes at least a 2-fold increase in the plasma AUC values, or equal or more than 50-80% decrease in clearance. A weak inhibitor causes at least a 1.25-fold but less than 2-fold increase in the plasma AUC values, or equal or more than 20-50% decrease in clearance.

Examples, but not limited to, strong CYP3A4 inhibitors can be boceprevir, protease inhibitors (ritonavir [and combination drugs thereof], indinavir, nelfinavir, saquinavir, amprenavir, atazanavir, darunavir), cobicistat, clarithromycin, telithromycin, nefazodone, ceritinib, mibefradil, ribociclib, tucatinib, chloramphenicol (antibiotic), some azole antifungals (ketoconazole, itraconazole, posaconazole, voriconazole), green tea extract, grape seed extract, dillapiole, apigenin, Artemisia annua, aluminium chlorhydrate, micoconazol, hydrocortisone, josamycin, mazipredone, nirmatrelvir, , adagrasib, conivaptan, delavirdine, ensitrelvir, grapefruit, idelalisib, indinavir, levoketoconazole, lonafamib, lopinavir, mifepristone, nelfinavir, saquinavir, telaprevir, tipranavir, and/or troleandomycin.

In one embodiment, the strong CYP3A4 inhibitor and/or a combination that comprises at least one strong CYP3A4 inhibitor is selected from:
- ALUMINIUM CHLOROHYDRATE;MICONAZOLE
- CLARITHROMYCIN
- CLARITHROMYCIN LACTOBIONATE
- HYDROCORTISONE;MICONAZOLE
- HYDROCORTISONE;MICONAZOLE NITRATE
- ITRACONAZOLE
- JOSAMYCIN
- KETOCONAZOLE
- MAZIPREDONE HYDROCHLORIDE;MICONAZOLE NITRATE
- MICONAZOLE
- MICONAZOLE NITRATE
- NIRMATRELVIR;RITONAVIR
- RITONAVIR
- VORICONAZOLE
and mixtures thereof. In one embodiment thereof, the patient receives an amount of the compound according to formula (1) of 5 to 80 mg. In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inhibitor. The adjustment can be up-titration, down-titration, maintenance or interruption of the treatment with the compound according to formula (1). Multiple embodiments of dose adjustments are described in detail below. These can be applied based on the co-medication with a CYP3A4 inhibitor. These can also be applied based on the amount and/or type of the CYP3A4 inhibitor the patient receives.

In one embodiment, the strong CYP3A4 inhibitor and/or a combination that comprises at least one strong CYP3A4 inhibitor is selected from:
- ALUMINIUM CHLOROHYDRATE;MICONAZOLE
- CLARITHROMYCIN
- ITRACONAZOLE
- KETOCONAZOLE
- MICONAZOLE NITRATE
- RITONAVIR
- VORICONAZOLE
and mixtures thereof, wherein the patient receive an amount of the compound according to formula (1) of 10 mg.

In one embodiment, the strong CYP3A4 inhibitor and/or a combination that comprises at least one strong CYP3A4 inhibitor is selected from:
- ALUMINIUM CHLOROHYDRATE;MICONAZOLE
- CLARITHROMYCIN
- CLARITHROMYCIN LACTOBIONATE
- HYDROCORTISONE;MICONAZOLE
- HYDROCORTISONE;MICONAZOLE NITRATE
- ITRACONAZOLE
- KETOCONAZOLE
- MAZIPREDONE HYDROCHLORIDE;MICONAZOLE NITRATE
- MICONAZOLE
- MICONAZOLE NITRATE
- NIRMATRELVIR;RITONAVIR
- RITONAVIR
and mixtures thereof, wherein the patient receive an amount of the compound according to formula (1) of 20 mg.

In one embodiment, the strong CYP3A4 inhibitor and/or a combination that comprises at least one strong CYP3A4 inhibitor is selected from:
- CLARITHROMYCIN
- HYDROCORTISONE;MICONAZOLE
- HYDROCORTISONE;MICONAZOLE NITRATE
- ITRACONAZOLE
- KETOCONAZOLE
- MICONAZOLE
- NIRMATRELVIR;RITONAVIR
- RITONAVIR
- VORICONAZOLE
and mixtures thereof, wherein the patient receive an amount of the compound according to formula (1) of 40 mg.

Examples, but not limited to, moderate CYP3A4 inhibitors can be aprepitant, ciprofloxacin, conivaptan, crizotinib, rutin, tofisopam, some calcium channel blockers (verapamil, diltiazem), some azole antifungals (fluconazole, miconazole), bergamottin, cyclosporine, donedarone, fluvoxamine, imatinib, valerian, and/or erythromycin.

In one embodiment, the moderate CYP3A4 inhibitor and/or a combination that comprises at least one moderate CYP3A4 inhibitor is selected from:
- APREPITANT
- ATAZANAVIR
- ATAZANAVIR SULFATE
- BETAMETHASONE DIPROPIONATE;CLOTRIMAZOLE
- BETAMETHASONE DIPROPIONATE;CLOTRIMAZOLE;GENTAMICIN SULFATE
- BETAMETHASONE DIPROPIONATE;CLOTRIMAZOLE;NEOMYCIN SULFATE
- BETAMETHASONE;CHLORAMPHENICOL
- BETAMETHASONE;CLOTRIMAZOLE
- CHLORAMPHENICOL
- CHLORAMPHENICOL PALMITATE
- CHLORAMPHENICOL;COLLAGENASE
- CHLORAMPHENICOL;DEXAMETHASONE
- CHLORAMPHENICOL;METHYLURACIL
- CICLOSPORIN
- CIMETIDINE
- CIPROFLOXACIN
- CIPROFLOXACIN HYDROCHLORIDE
- CIPROFLOXACIN HYDROCHLORIDE MONOHYDRATE
- CIPROFLOXACIN HYDROCHLORIDE;DEXAMETHASONE
- CIPROFLOXACIN HYDROCHLORIDE;FLUOCINOLONE ACETONIDE
- CIPROFLOXACIN HYDROCHLORIDE;T1NIDAZOLE
- CIPROFLOXACIN LACTATE
- CIPROFLOXACIN;FLUOCINOLONE ACETONIDE
- CLOTRIMAZOLE
- CLOTRIMAZOLE;HEXAMIDINE ISETIONATE;PREDNISOLONE ACETATE
- CLOTRIMAZOLE;HYDROCORTISONE
- CLOTRIMAZOLE;HYDROCORTISONE;ZINC OXIDE
- COLISTIMETHATE SODIUM;ERYTHROMYCIN LACTOBIONATE
- COLISTIN;ERYTHROMYCIN LACTOBIONATE
- DILTIAZEM
- DILTIAZEM HYDROCHLORIDE
- DRONEDARONE
- DRONEDARONE HYDROCHLORIDE
- ERYTHROMYCIN
- ERYTHROMYCIN ETHYLSUCCINATE
- ERYTHROMYCIN LACTOBIONATE
- ERYTHROMYCIN PROPIONATE
- FLUCONAZOLE
- FLUVOXAMINE
- IMATINIB MESILATE
- TOFISOPAM
- TRANDOLAPRIL;VERAPAMIL HYDROCHLORIDE
- VERAPAMIL
- VERAPAMIL HYDROCHLORIDE
and mixtures thereof. In one embodiment thereof, the patient receives an amount of the compound according to formula (1) of 5 to 80 mg. In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inhibitor. The adjustment can be up-titration, down-titration, maintenance or interruption of the treatment with the compound according to formula (1). Multiple embodiments of dose adjustments are described in detail below. These can be applied based on the co-medication with a CYP3A4 inhibitor. These can also be applied based on the amount and/or type of the CYP3A4 inhibitor the patient receives.

In one embodiment, the moderate CYP3A4 inhibitor and/or a combination that comprises at least one moderate CYP3A4 inhibitor is selected from:
- BETAMETHASONE;CHLORAMPHENICOL
- BETAMETHASONE;CLOTRIMAZOLE
- CHLORAMPHENICOL
- CICLOSPORIN
- CIMETIDINE
- CIPROFLOXACIN
- CIPROFLOXACIN HYDROCHLORIDE
- CLOTRIMAZOLE
- DILTIAZEM
- DILTIAZEM HYDROCHLORIDE
- DRONEDARONE
- DRONEDARONE HYDROCHLORIDE
- ERYTHROMYCIN
- ERYTHROMYCIN PROPIONATE
- FLUCONAZOLE
- FLUVOXAMINE
- TOFISOPAM
- TRANDOLAPRIL;VERAPAMIL HYDROCHLORIDE
- VERAPAMIL
- VERAPAMIL HYDROCHLORIDE
and mixtures thereof, wherein the patient receive an amount of the compound according to formula (1) of 10 mg.

In one embodiment, the moderate CYP3A4 inhibitor and/or a combination that comprises at least one moderate CYP3A4 inhibitor is selected from:
- APREPITANT
- BETAMETHASONE DIPROPIONATE;CLOTRIMAZOLE
- BETAMETHASONE DIPROPIONATE;CLOTRIMAZOLE;NEOMYCIN SULFATE
- BETAMETHASONE;CLOTRIMAZOLE
- CHLORAMPHENICOL
- CHLORAMPHENICOL PALMITATE
- CHLORAMPHENICOL;COLLAGENASE
- CHLORAMPHENICOL;DEXAMETHASONE
- CICLOSPORIN
- CIMETIDINE
- CIPROFLOXACIN
- CIPROFLOXACIN HYDROCHLORIDE
- CIPROFLOXACIN HYDROCHLORIDE;FLUOCINOLONE ACETONIDE
- CIPROFLOXACIN HYDROCHLORIDE;TINIDAZOLE
- CIPROFLOXACIN LACTATE
- CIPROFLOXACIN;FLUOCINOLONE ACETONIDE
- CLOTRIMAZOLE
- COLISTIMETHATE SODIUM;ERYTHROMYCIN LACTOBIONATE
- COLISTIN;ERYTHROMYCIN LACTOBIONATE
- DILTIAZEM
- DILTIAZEM HYDROCHLORIDE
- DRONEDARONE HYDROCHLORIDE
- ERYTHROMYCIN
- ERYTHROMYCIN ETHYLSUCCINATE
- FLUCONAZOLE
- FLUVOXAMINE
- IMATINIB MESILATE
- TOFISOPAM
- VERAPAMIL
- VERAPAMIL HYDROCHLORIDE
and mixtures thereof, wherein the patient receive an amount of the compound according to formula (1) of 20 mg.

In one embodiment, the moderate CYP3A4 inhibitor and/or a combination that comprises at least one moderate CYP3A4 inhibitor is selected from:
- BETAMETHASONE DIPROPIONATE;CLOTRIMAZOLE
- BETAMETHASONE DIPROPIONATE;CLOTRIMAZOLE;NEOMYCIN SULFATE
- CHLORAMPHENICOL
- CHLORAMPHENICOL;COLLAGENASE
- CHLORAMPHENICOL;METHYLURACIL
- CICLOSPORIN
- CIPROFLOXACIN
- CIPROFLOXACIN HYDROCHLORIDE
- CIPROFLOXACIN HYDROCHLORIDE MONOHYDRATE
- CIPROFLOXACIN HYDROCHLORIDE;DEXAMETHASONE
- CIPROFLOXACIN HYDROCHLORIDE;FLUOCINOLONE ACETONIDE
- CIPROFLOXACIN LACTATE
- CLOTRIMAZOLE
- CLOTRIMAZOLE;HYDROCORTISONE
- COLISTIMETHATE SODIUM;ERYTHROMYCIN LACTOBIONATE
- COLISTIN;ERYTHROMYCIN LACTOBIONATE
- DILTIAZEM
- DILTIAZEM HYDROCHLORIDE
- DRONEDARONE HYDROCHLORIDE
- ERYTHROMYCIN
- FLUCONAZOLE
- VERAPAMIL
- VERAPAMIL HYDROCHLORIDE
and mixtures thereof, wherein the patient receive an amount of the compound according to formula (1) of 40 mg.

Examples, but not limited to, weak CYP3A4 inhibitors can be berberine, buprenorphine, cafestol, cilostazol, cimetidine, fosaprepitant, lomitapide, orphenadrine, omeprazole, quercetin, ranitidine, ranolazine, tacrolimus, ticagrelor, valproic acid, amlodipine, azithromycin, cimetidine, fluvoxamine, and/or amiodarone.

In one embodiment, the weak CYP3A4 inhibitor and/or a combination that comprises at least one weak CYP3A4 inhibitor is selected from:
- ACETYLSALICYLIC ACID;ATORVASTATIN
- ACETYLSALICYLIC ACID;ATORVASTATIN CALCIUM
- ACETYLSALICYLIC ACID;ATORVASTATIN CALCIUM;CLOPIDOGREL BISULFATE
- ACETYLSALICYLIC ACID;ATORVASTATIN CALCIUM;RAMIPRIL
- ALLIUM SATIVUM BULB;BLUMEA BALSAMIFERA LEAF;CRATAEGUS PINNATIFIDA FRUIT;ERIGERON BREVISCAPUS WHOLE PLANT;GINKGO BILOBA LEAF;GYNOSTEMMA PENTAPHYLLUM HERB;PANAX NOTOGINSENG ROOT WITH RHIZOME; SALVIA MILTIORRHIZA ROOT WITH RHIZOME
- ALLIUM SPP. BULB;BUXUS SPP. LEAF WITH TWIG;CINNAMOMUM MIGAO FRUIT;GINKGO BILOBA LEAF;SALVIA MILTIORRHIZA ROOT WITH RHIZOME
- ALPRAZOLAM
- AMBROXOL HYDROCHLORIDE;ROXITHROMYCIN
- AMIODARONE
- AMIODARONE HYDROCHLORIDE
- AMLODIPINE
- AMLODIPINE BENZOATE
- AMLODIPINE BESILATE
- AMLODIPINE BESILATE;ATORVASTATIN CALCIUM
- AMLODIPINE BESILATE;ATORVASTATIN CALCIUM TRIHYDRATE;PERINDOPRIL ARGININE
- AMLODIPINE BESILATE;ATORVASTATIN CALCIUM;PERINDOPRIL ARGININE
- AMLODIPINE BESILATE;ATORVASTATIN L-LYSINE
- AMLODIPINE BESILATE;AZILSARTAN
- AMLODIPINE BESILATE;BENAZEPRIL HYDROCHLORIDE
- AMLODIPINE BESILATE;BISOPROLOL FUMARATE
- AMLODIPINE BESILATE;CANDESARTAN CILEXETIL
- AMLODIPINE BESILATE;HYDROCHLOROTHIAZIDE;OLMESARTAN MEDOXOMIL
- AMLODIPINE BESILATE;HYDROCHLOROTHIAZIDE;VALSARTAN
- AMLODIPINE BESILATE;INDAPAMIDE
- AMLODIPINE BESILA TE;INDAP AMIDE;PERINDOPRIL ARGININE
- AMLODIPINE BESILA TE;INDAP AMIDE;PERINDOPRIL ERBUMINE
- AMLODIPINE BESILATE;IRBESARTAN
- AMLODIPINE BESILATE;LISINOPRIL
- AMLODIPINE BESILATE;LISINOPRIL DIHYDRATE
- AMLODIPINE BESILATE;LOSARTAN POTASSIUM
- AMLODIPINE BESILATE;OLMESARTAN MEDOXOMIL
- AMLODIPINE BESILATE;PERINDOPRIL ARGININE
- AMLODIPINE BESILATE;PERINDOPRIL ERBUMINE
- AMLODIPINE BESILATE;PERINDOPRIL TOSILATE
- AMLODIPINE BESILATE;RAMIPRIL
- AMLODIPINE BESILATE;ROSUVASTATIN CALCIUM;TELMISARTAN
- AMLODIPINE BESILATE;TELMISARTAN
- AMLODIPINE BESILATE;VALSARTAN
- AMLODIPINE CAMSILATE
- AMLODIPINE MALEATE
- AMLODIPINE MESILATE
- AMLODIPINE;ATORVASTATIN CALCIUM
- AMLODIPINE;BENAZEPRIL HYDROCHLORIDE
- AMLODIPINE;HYDROCHLOROTHIAZIDE;OLMESARTAN
- AMLODIPINE;HYDROCHLOROTHIAZIDE;VALSARTAN
- AMLODIPINE;INDAPAMIDE
- AMLODIPINE;PERINDOPRIL
- AMLODIPINE;RAMIPRIL
- AMLODIPINE;VALSARTAN
- ASTAXANTHIN;BERBERINE;FOLIC ACID;MONASCUS PURPUREUS;POLICOSANOL;UBIDECARENONE
- ATORVASTATIN
- ATORVASTATIN CALCIUM
- ATORVASTATIN CALCIUM TRIHYDRATE
- ATORVASTATIN CALCIUM TRIHYDRATE;EZETIMIBE
- ATORVASTATIN CALCIUM;EZETIMIBE
- ATORVASTATIN CALCIUM;PERINDOPRIL ARGININE
- ATORVASTATIN;EZETIMIBE
- BACOPA MONNIERI;CITICOLINE SODIUM;GINKGO BILOBA;PHOSPHATIDYL SERINE;SALVIA OFFICINALIS;THIOCTIC ACID
- BERBERINE
- BERBERINE CHLORIDE HYDRATE
- BERBERINE HYDROCHLORIDE
- BERBERINE HYDROCHLORIDE;DOLOMIAEA COSTUS ROOT;TETRADIUM RUTICARPUM FRUIT
- BERBERINE HYDROCHLORIDE;RHEUM SPP. ROOT WITH RHIZOME;SCUTELLARIA BAICALENSIS ROOT
- BERBERINE;CINNAMOMUM VERUM;GYMNEMA SYLVESTRE;LAGERSTROEMIA SPECIOSA;PICRASMA EXCELSA;SYZYGIUM CUMINI;URTICA DIOICA;V ACCINIUM MYRTILLUS
- BICALUTAMIDE
- BORNEOL;GINKGO BILOBA LEAF;GYNOSTEMMA PENTAPHYLLUM WHOLE PLANT; SALVIA YUNNANENSIS ROOT
- CAFFEINE;CHLORZOXAZONE;PARACETAMOL;THIAMINE
- CHLORZOXAZONE
- CHLORZOXAZONE;PARACETAMOL
- CHOLINE;GINKGO BILOBA LEAF
- CILOSTAZOL
- CYANOCOBALAMIN;FISH OIL;FOLIC ACID;GINKGO BILOBA LEAF;PHOSPHATIDYL SERINE;TOCOPHEROL
- DIPYRIDAMOLE;GINKGO BILOBA LEAF EXTRACT
- ELEUTHEROCOCCUS SENTICOSUS;EUPHRASIA OFFICINALIS;GINKGO BILOBA;VACC1NIUM MYRTILLUS
- EVEROLIMUS
- FLUOXETINE
- FLUOXETINE HYDROCHLORIDE
- GINKGO BILOBA
- GINKGO BILOBA EXTRACT
- GINKGO BILOBA LEAF
- GINKGO BILOBA LEAF EXTRACT
- GINKGO BILOBA LEAF;LIGUSTRAZINE PHOSPHATE
- GINKGO BILOBA;GLUCOSAMINE
- ISONIAZID
- LEVAMLODIPINE
- LEVAMLODIPINE BESILATE
- LEVAMLODIPINE MALEATE
- PROPIVERINE
- PROPIVERINE HYDROCHLORIDE
- RANITIDINE
- RANITIDINE HYDROCHLORIDE
- RANITIDINE HYDROCHLORIDE;SUCRALFATE;TRIPOTASSIUM DICITRATOBISMUTHATE
- RANOLAZINE
- ROXITHROMYCIN
- TACROLIMUS
- TACROLIMUS MONOHYDRATE
- TICAGRELOR
- TOLVAPTAN
- TOLVAPTAN DISODIUM PHOSPHATE
and mixtures thereof. In one embodiment thereof, the patient receives an amount of the compound according to formula (1) of 5 to 80 mg. In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inhibitor. The adjustment can be up-titration, down-titration, maintenance or interruption of the treatment with the compound according to formula (1). Multiple embodiments of dose adjustments are described in detail below. These can be applied based on the co-medication with a CYP3A4 inhibitor. These can also be applied based on the amount and/or type of the CYP3A4 inhibitor the patient receives.

In one embodiment, the weak CYP3A4 inhibitor and/or a combination that comprises at least one weak CYP3A4 inhibitor is selected from:
- ATORVASTATIN
and mixtures thereof, wherein the patient receive an amount of the compound according to formula (1) of 5 mg.

In one embodiment, the weak CYP3A4 inhibitor and/or a combination that comprises at least one weak CYP3A4 inhibitor is selected from:
- ACETYLSALICYLIC ACID;ATORVASTATIN
- ACETYLSALICYLIC ACID;ATORVASTATIN CALCIUM;CLOPIDOGREL BISULFATE
- ALPRAZOLAM
- AMIODARONE
- AMIODARONE HYDROCHLORIDE
- AMLODIPINE
- AMLODIPINE BESILATE
- AMLODIPINE BESILATE;ATORVASTATIN CALCIUM
- AMLODIPINE BESILATE;ATORVASTATIN CALCIUM TRIHYDRATE;PERINDOPRIL ARGININE
- AMLODIPINE BESILATE;AZILSARTAN
- AMLODIPINE BESILATE;BENAZEPRIL HYDROCHLORIDE
- AMLODIPINE BESILATE;HYDROCHLOROTHIAZIDE;OLMESARTAN MEDOXOMIL
- AMLODIPINE BESILATE;HYDROCHLOROTHIAZIDE;VALSARTAN
- AMLODIPINE BESILATE;INDAPAMIDE
- AMLODIPINE BESILATE;INDAPAMIDE;PERINDOPRIL ARGININE
- AMLODIPINE BESILATE;INDAPAMIDE;PERINDOPRIL ERBUMINE
- AMLODIPINE BESILATE;IRBESARTAN
- AMLODIPINE BESILATE;LISINOPRIL
- AMLODIPINE BESILATE;LISINOPRIL DIHYDRATE
- AMLODIPINE BESILATE;LOSARTAN POTASSIUM
- AMLODIPINE BESILATE;OLMESARTAN MEDOXOMIL
- AMLODIPINE BESILATE;PERINDOPRIL ARGININE
- AMLODIPINE BESILATE;PERINDOPRIL ERBUMINE
- AMLODIPINE BESILATE;PERINDOPRIL TOSILATE
- AMLODIPINE BESILATE;RAMIPRIL
- AMLODIPINE BESILATE;ROSUVASTATIN CALCIUM;TELMISARTAN
- AMLODIPINE BESILATE;TELMISARTAN
- AMLODIPINE BESILATE;VALSARTAN
- AMLODIPINE MALEATE
- AMLODIPINE MESILATE
- AMLODIPINE;ATORVASTATIN CALCIUM
- AMLODIPINE;INDAP AMIDE
- AMLODIPINE;VALSARTAN
- ATORVASTATIN
- ATORVASTATIN CALCIUM
- ATORVASTATIN CALCIUM TRIHYDRATE
- ATORVASTATIN CALCIUM TRIHYDRATE;EZETIMIBE
- ATORVASTATIN CALCIUM;EZETIMIBE
- ATORVASTATIN;EZETIMIBE
- BERBERINE HYDROCHLORIDE;DOLOMIAEA COSTUS ROOT;TETRADIUM RUTICARPUM FRUIT
- BICALUTAMIDE
- BORNEOL;GINKGO BILOBA LEAF;GYNOSTEMMA PENTAPHYLLUM WHOLE PLANT; SALVIA YUNNANENSIS ROOT
- CHLORZOXAZONE
- CHLORZOXAZONE;PARACETAMOL
- CILOSTAZOL
- EVEROLIMUS
- FLUOXETINE
- FLUOXETINE HYDROCHLORIDE
- GINKGO BILOBA
- GINKGO BILOBA EXTRACT
- GINKGO BILOBA LEAF EXTRACT
- GINKGO BILOBA;GLUCOSAMINE
- ISONIAZID
- LEVAMLODIPINE
- LEVAMLODIPINE BESILATE
- LEVAMLODIPINE MALEATE
- PROPIVERINE HYDROCHLORIDE
- RANITIDINE
- RANITIDINE HYDROCHLORIDE
- RANOLAZINE
- ROXITHROMYCIN
- TACROLIMUS
- TACROLIMUS MONOHYDRATE
- TICAGRELOR
- TOLVAPTAN
- TOLVAPTAN DISODIUM PHOSPHATE
and mixtures thereof, wherein the patient receive an amount of the compound according to formula (1) of 10 mg.

In one embodiment, the weak CYP3A4 inhibitor and/or a combination that comprises at least one weak CYP3A4 inhibitor is selected from:
- ACETYLSALICYLIC ACID;ATORVASTATIN
- ACETYLSALICYLIC ACID;ATORVASTATIN CALCIUM;CLOPIDOGREL BISULFATE
- ACETYLSALICYLIC ACID;ATORVASTATIN CALCIUM;RAMIPRIL
- ALPRAZOLAM
- AMIODARONE
- AMIODARONE HYDROCHLORIDE
- AMLODIPINE
- AMLODIPINE BENZOATE
- AMLODIPINE BESILATE
- AMLODIPINE BESILATE;ATORVASTATIN CALCIUM
- AMLODIPINE BESILATE;ATORVASTATIN CALCIUM TRIHYDRATE;PERINDOPRIL ARGININE
- AMLODIPINE BESILATE;ATORVASTATIN CALCIUM;PERINDOPRIL ARGININE
- AMLODIPINE BESILATE;ATORVASTATIN L-LYSINE
- AMLODIPINE BESILATE;BENAZEPRIL HYDROCHLORIDE
- AMLODIPINE BESILATE;BISOPROLOL FUMARATE
- AMLODIPINE BESILATE;HYDROCHLOROTHIAZIDE;OLMESARTAN MEDOXOMIL
- AMLODIPINE BESILATE;HYDROCHLOROTHIAZIDE;VALSARTAN
- AMLODIPINE BESILATE;INDAPAMIDE
- AMLODIPINE BESILATE;INDAPAMIDE;PERINDOPRIL ARGININE
- AMLODIPINE BESILATE;INDAPAMIDE;PERINDOPRIL ERBUMINE
- AMLODIPINE BESILATE;IRBESARTAN
- AMLODIPINE BESILATE;LISINOPRIL
- AMLODIPINE BESILATE;LISINOPRIL DIHYDRATE
- AMLODIPINE BESILATE;LOSARTAN POTASSIUM
- AMLODIPINE BESILATE;OLMESARTAN MEDOXOMIL
- AMLODIPINE BESILATE;PERINDOPRIL ARGININE
- AMLODIPINE BESILATE;PERINDOPRIL ERBUMINE
- AMLODIPINE BESILATE;PERINDOPRIL TOSILATE
- AMLODIPINE BESILATE;RAMIPRIL
- AMLODIPINE BESILATE;ROSUVASTATIN CALCIUM;TELMISARTAN
- AMLODIPINE BESILATE;TELMISARTAN
- AMLODIPINE BESILATE;VALSARTAN
- AMLODIPINE CAMSILATE
- AMLODIPINE MALEATE
- AMLODIPINE MESILATE
- AMLODIPINE;ATORVASTATIN CALCIUM
- AMLODIPINE;HYDROCHLOROTHIAZIDE;OLMESARTAN
- AMLODIPINE;HYDROCHLOROTHIAZIDE;VALSARTAN
- AMLODIPINE;PERINDOPRIL
- AMLODIPINE;VALSARTAN
- ASTAXANTHIN;BERBERINE;FOLIC ACID;MONASCUS PURPUREUS;POLICOSANOL;UBIDECARENONE
- ATORVASTATIN
- ATORVASTATIN CALCIUM
- ATORVASTATIN CALCIUM TRIHYDRATE
- ATORVASTATIN CALCIUM TRIHYDRATE;EZETIMIBE
- ATORVASTATIN CALCIUM;EZETIMIBE
- ATORVASTATIN CALCIUM;PERINDOPRIL ARGININE
- ATORVASTATIN;EZETIMIBE
- BACOPA MONNIERI;CITICOLINE SODIUM;GINKGO BILOBA;PHOSPHATIDYL SERINE;SALVIA OFFICINALIS;THIOCTIC ACID
- BERBERINE CHLORIDE HYDRATE
- BERBERINE HYDROCHLORIDE
- BERBERINE;CINNAMOMUM VERUM;GYMNEMA SYLVESTRE;LAGERSTROEMIA SPECIOSA;PICRASMA EXCELSA;SYZYGIUM CUMINI;URTICA DIOICA;VACCINIUM MYRTILLUS
- BICALUTAMIDE
- BORNEOL;GINKGO BILOBA LEAF;GYNOSTEMMA PENTAPHYLLUM WHOLE PLANT; SALVIA YUNNANENSIS ROOT
- CAFFEINE;CHLORZOXAZONE;PARACETAMOL;THIAMINE
- CHLORZOXAZONE
- CHLORZOXAZONE;PARACETAMOL
- CHOLINE;GINKGO BILOBA LEAF
- CILOSTAZOL
- ELEUTHEROCOCCUS SENTICOSUS;EUPHRASIA OFFICINALIS;GINKGO BILOBA;VACCINIUM MYRTILLUS
- EVEROLIMUS
- FLUOXETINE
- FLUOXETINE HYDROCHLORIDE
- GINKGO BILOBA
- GINKGO BILOBA EXTRACT
- GINKGO BILOBA LEAF
- GINKGO BILOBA LEAF EXTRACT
- GINKGO BILOBA;GLUCOSAMINE
- ISONIAZID
- LEVAMLODIPINE
- LEVAMLODIPINE BESILATE
- LEVAMLODIPINE MALEATE
- PROPIVERINE HYDROCHLORIDE
- RANITIDINE
- RANITIDINE HYDROCHLORIDE
- RANOLAZINE
- ROXITHROMYCIN
- TACROLIMUS
- TACROLIMUS MONOHYDRATE
- TICAGRELOR
- TOLVAPTAN
- TOLVAPTAN DISODIUM PHOSPHATE
and mixtures thereof, wherein the patient receive an amount of the compound according to formula (1) of 20 mg.

In one embodiment, the weak CYP3A4 inhibitor and/or a combination that comprises at least one weak CYP3A4 inhibitor is selected from:
- ALPRAZOLAM
- ATORVASTATIN;EZETIMIBE
and mixtures thereof, wherein the patient receive an amount of the compound according to formula (1) of 30 mg.

In one embodiment, the weak CYP3A4 inhibitor and/or a combination that comprises at least one weak CYP3A4 inhibitor is selected from:
- ACETYLSALICYLIC ACID;ATORVASTATIN CALCIUM;RAMIPRIL
- ALLIUM SPP. BULB;BUXUS SPP. LEAF WITH TWIG;CINNAMOMUM MIGAO FRUIT;GINKGO BILOBA LEAF;SALVIA MILTIORRHIZA ROOT WITH RHIZOME
- ALPRAZOLAM
- AMBROXOL HYDROCHLORIDE;ROXITHROMYCIN
- AMIODARONE
- AMIODARONE HYDROCHLORIDE
- AMLODIPINE
- AMLODIPINE BESILATE
- AMLODIPINE BESILATE;ATORVASTATIN CALCIUM
- AMLODIPINE BESILATE;ATORVASTATIN CALCIUM;PERINDOPRIL ARGININE
- AMLODIPINE BESILATE;ATORVASTATIN L-LYSINE
- AMLODIPINE BESILATE;BENAZEPRIL HYDROCHLORIDE
- AMLODIPINE BESILATE;BISOPROLOL FUMARATE
- AMLODIPINE BESILATE;CANDESARTAN CILEXETIL
- AMLODIPINE BESILATE;HYDROCHLOROTHIAZIDE;OLMESARTAN MEDOXOMIL
- AMLODIPINE BESILATE;HYDROCHLOROTHIAZIDE;VALSARTAN
- AMLODIPINE BESILATE;INDAPAMIDE
- AMLODIPINE BESILATE;INDAPAMIDE;PERINDOPRIL ARGININE
- AMLODIPINE BESILATE;INDAPAMIDE;PERINDOPRIL ERBUMINE
- AMLODIPINE BESILATE;LISINOPRIL
- AMLODIPINE BESILATE;LISINOPRIL DIHYDRATE
- AMLODIPINE BESILATE;OLMESARTAN MEDOXOMIL
- AMLODIPINE BESILATE;PERINDOPRIL ARGININE
- AMLODIPINE BESILATE;PERINDOPRIL ERBUMINE
- AMLODIPINE BESILATE;RAMIPRIL
- AMLODIPINE BESILATE;TELMISARTAN
- AMLODIPINE BESILATE;VALSARTAN
- AMLODIPINE CAMSILATE
- AMLODIPINE MALEATE
- AMLODIPINE MESILATE
- AMLODIPINE;ATORVASTATIN CALCIUM
- AMLODIPINE;HYDROCHLOROTHIAZIDE;OLMESARTAN
- AMLODIPINE;HYDROCHLOROTHIAZIDE;VALSARTAN
- AMLODIPINE;RAMIPRIL
- AMLODIPINE;VALSARTAN
- ASTAXANTHIN;BERBERINE;FOLIC ACID;MONASCUS PURPUREUS;POLICOSANOL;UBIDECARENONE
- ATORVASTATIN
- ATORVASTATIN CALCIUM
- ATORVASTATIN CALCIUM TRIHYDRATE
- ATORVASTATIN CALCIUM;EZETIMIBE
- ATORVASTATIN CALCIUM;PERINDOPRIL ARGININE
- ATORVASTATIN;EZETIMIBE
- BERBERINE
- BERBERINE HYDROCHLORIDE
- BERBERINE HYDROCHLORIDE;RHEUM SPP. ROOT WITH RHIZOME;SCUTELLARIA BAICALENSIS ROOT
- BICALUTAMIDE
- CHLORZOXAZONE
- CHLORZOXAZONE;PARACETAMOL
- CILOSTAZOL
- CYANOCOBALAMIN;FISH OIL;FOLIC ACID;GINKGO BILOBA LEAF;PHOSPHATIDYL SERINE;TOCOPHEROL
- ELEUTHEROCOCCUS SENTICOSUS;EUPHRASIA OFFICINALIS;GINKGO BILOBA;VACCINIUM MYRTILLUS
- FLUOXETINE
- FLUOXETINE HYDROCHLORIDE
- GINKGO BILOBA
- GINKGO BILOBA EXTRACT
- GINKGO BILOBA LEAF
- GINKGO BILOBA LEAF EXTRACT
- LEVAMLODIPINE BESILATE
- LEVAMLODIPINE MALEATE
- PROPIVERINE
- PROPIVERINE HYDROCHLORIDE
- RANITIDINE
- RANITIDINE HYDROCHLORIDE
- RANITIDINE HYDROCHLORIDE; SUCRALFATE;TRIPOTASSIUM DICITRATOBISMUTHATE
- RANOLAZINE
- ROXITHROMYCIN
- TACROLIMUS
- TICAGRELOR
- TOLVAPTAN
and mixtures thereof, wherein the patient receive an amount of the compound according to formula (1) of 40 mg.

In one embodiment, the weak CYP3A4 inhibitor and/or a combination that comprises at least one weak CYP3A4 inhibitor is selected from:
- ATORVASTATIN
wherein the patient receive an amount of the compound according to formula (1) of 60 mg.

In one embodiment, the weak CYP3A4 inhibitor and/or a combination that comprises at least one weak CYP3A4 inhibitor is selected from:
- AMIODARONE HYDROCHLORIDE
- AMLODIPINE
- AMLODIPINE BESILATE;HYDROCHLOROTHIAZIDE;VALSARTAN
- ATORVASTATIN
- ATORVASTATIN CALCIUM
- ATORVASTATIN CALCIUM TRIHYDRATE
- BICALUTAMIDE
- TOLVAPTAN
and mixtures thereof, wherein the patient receive an amount of the compound according to formula (1) of 80 mg.

Examples, but limited to, CYP3A4 inhibitors of unspecified potency can be bergaptol (a furocoumarin in citrus), cannabidiol, dithiocarbamate (functional group), flavonoids, mifepristone, norfloxacin, some non-nucleoside reverse-transcriptase inhibitors (delavirdine), gestodene, star fruit, milk thistle, niacin, nicotinic acid, niacinamide (nicotinamide), Vitamin B3 (complex), ginkgo biloba, sesamin, piperine, isoniazid, serenoa.

In one embodiment, the CYP3A4 inhibitor of unspecified potency and/or a combination that comprises at least one CYP3A4 inhibitor of unspecified potency is selected from:
- ALTIZIDE;SPIRONOLACTONE
- BILOBALIDE;GINKGOLIDE A;GINKGOLIDE B;GINKGOLIDE C
- BUTIZIDE;SPIRONOLACTONE
- CAFFEINE;ERGOTAMINE TARTRATE;METAMIZOLE SODIUM
- DESOGESTREL;ETHINYLESTRADIOL
- ERGOTAMINE TARTRATE
- ETHINYLESTRADIOL;NORGESTIMATE
- FEXUPRAZAN HYDROCHLORIDE
- FUROSEMIDE;SPIRONOLACTONE
- HYDROCHLOROTHIAZIDE;SPIRONOLACTONE
- MIDAZOLAM
- MIDAZOLAM HYDROCHLORIDE
- NORFLOXACIN
- SERTRALINE
- SERTRALINE HYDROCHLORIDE
- SPIRONOLACTONE
- SPIRONOLACTONE;TORASEMIDE
and mixtures thereof. In one embodiment thereof, the patient receives an amount of the compound according to formula (1) of 5 to 80 mg In one embodiment thereof, the patient receives an amount of the compound according to formula (1) of 5 mg. In one embodiment thereof, the patient receives an amount of the compound according to formula (1) of 10 mg. In one embodiment thereof, the patient receives an amount of the compound according to formula (1) of 20 mg. In one embodiment thereof, the patient receives an amount of the compound according to formula (1) of 30 mg. In one embodiment thereof, the patient receives an amount of the compound according to formula (1) of 40 mg. In one embodiment thereof, the patient receives an amount of the compound according to formula (1) of 60 mg. In one embodiment thereof, the patient receives an amount of the compound according to formula (1) of 80 mg. The amount of the compound according to formula (I) in these embodiments can be adjusted based on the CYP3A4 inhibitor. The adjustment can be up-titration, down-titration, maintenance or interruption of the treatment with the compound according to formula (1). Multiple embodiments of dose adjustments are described in detail below. These can be applied based on the co-medication with a CYP3A4 inhibitor. These can also be applied based on the amount and/or type of the CYP3A4 inhibitor the patient receives.

In one embodiment, the CYP3A4 inhibitor of unspecified potency and/or a combination that comprises at least one CYP3A4 inhibitor of unspecified potency is selected from:
- CAFFEINE;ERGOTAMINE TARTRATE;METAMIZOLE SODIUM
- ERGOTAMINE TARTRATE
- ETHINYLESTRADIOL;NORGESTIMATE
- FEXUPRAZAN HYDROCHLORIDE
- FUROSEMIDE;SPIRONOLACTONE
- HYDROCHLOROTHIAZIDE;SPIRONOLACTONE
- MIDAZOLAM
- MIDAZOLAM HYDROCHLORIDE
- NORFLOXACIN
- SERTRALINE
- SERTRALINE HYDROCHLORIDE
- SPIRONOLACTONE
and mixtures thereof, wherein the patient receive an amount of the compound according to formula (1) of 10 mg.

In one embodiment, the CYP3A4 inhibitor of unspecified potency and/or a combination that comprises at least one CYP3A4 inhibitor of unspecified potency is selected from:
- BILOBALIDE;GINKGOLIDE A;GINKGOLIDE B;GINKGOLIDE C
- CAFFEINE;ERGOTAMINE TARTRATE;METAMIZOLE SODIUM
- ERGOTAMINE TARTRATE
- FEXUPRAZAN HYDROCHLORIDE
- FUROSEMIDE;SPIRONOLACTONE
- MIDAZOLAM
- MIDAZOLAM HYDROCHLORIDE
- NORFLOXACIN
- SERTRALINE
- SERTRALINE HYDROCHLORIDE
- SPIRONOLACTONE
and mixtures thereof, wherein the patient receive an amount of the compound according to formula (1) of 20 mg.

In one embodiment, the CYP3A4 inhibitor of unspecified potency and/or a combination that comprises at least one CYP3A4 inhibitor of unspecified potency is selected from:
- DESOGESTREL;ETHINYLESTRADIOL
- FEXUPRAZAN HYDROCHLORIDE
- HYDROCHLOROTHIAZIDE;SPIRONOLACTONE
- MIDAZOLAM
- MIDAZOLAM HYDROCHLORIDE
- NORFLOXACIN
- SERTRALINE
- SERTRALINE HYDROCHLORIDE
- SPIRONOLACTONE
- SPIRONOLACTONE;TORASEMIDE
and mixtures thereof, wherein the patient receive an amount of the compound according to formula (1) of 40 mg.

In one embodiment, the patient receives the compound according to formula (I) and at least one further medicament. In one embodiment, the patient receives the compound according to formula (I) and equal or below 5 medications. In one embodiment, the patient receives the compound according to formula (I) and equal or above 5 medications. In one embodiment, the patient receives the compound according to formula (I) and equal or above 5 to equal or below 9 medications. In one embodiment, the patient receives the compound according to formula (I) and equal or above 5 medications. In one embodiment, the patient receives the compound according to formula (I) and equal or below 9 medications. In one embodiment, the patient receives the compound according to formula (I) and equal or above 5 medications. In one embodiment, the patient receives the compound according to formula (I) and equal or above 9 medications. In one embodiment, the patient receives equal or above 10 medications. Examples of co-medications are described above and below in detail. Examples of co-medications are loop diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), glucagon-like peptide-1 (GLP-1) agonists, CYP3A4 inducer, CYP3A4 inhibitor and/or mixtures thereof.

These multiple medications may be stated as: "non-polypharmacy": <5 medications; "polypharmacy": 5 to 9 medications; and "hyperpolypharmacy": ≥10 medications.

Kansas City Cardiomyopathy Questionnaire (KCCQ) is a standardized patient reported outcomes (PRO) questionnaire which assesses symptoms, physical and social limitations, and quality of life in patients with heart failure (HF). Patients are asked to recall how their HF impacted their life over a 2-week recall period. The KCCQ is composed of different score domains including the Total symptom score (TSS), which depicts patients perceptions on their symptoms related to heart failure (Green CP, Porter CB, Bresnahan DR, Spertus JA. Development and evaluation of the Kansas City Cardiomyopathy Questionnaire: a new health status measure for heart failure. J Am Coll Cardiol. 2000 Apr;35(5): 1245-55.)

In one embodiment, the patient has a KCCQ-TSS of equal or above 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85 ,86, 87, 88.89. 90. 91, 92, 93, 94, 95, 96, 97, 98, 99, and 100. In one embodiment, the patient has a KCCQ-TSS of equal or below 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85 ,86, 87, 88.89. 90. 91, 92, 93, 94, 95, 96, 97, 98, 99, and 100.

In one embodiment, the patient has a KCCQ-TSS of equal or above 43 to equal or below 91. In one embodiment, the patient has a KCCQ-TSS of equal or above 43 to equal or below 67. In one embodiment, the patient has a KCCQ-TSS of equal or above 67 to equal or below 91. In one embodiment, the patient has a KCCQ-TSS of 69+/- 24. In one embodiment, the patient has a KCCQ-TSS of equal or above 45 to equal or below 93. In one embodiment, the patient has a KCCQ-TSS of qual or above 45 to equal or below 69. In one embodiment, the patient has a KCCQ-TSS of equal or above 69 to equal or below 93. In one embodiment, the patient has a KCCQ-TSS of 66+/- 24. In one embodiment, the patient has a KCCQ-TSS of equal or above 42 to equal or below 90. In one embodiment, the patient has a KCCQ-TSS of equal or above 42 to equal or below 66. In one embodiment, the patient has a KCCQ-TSS of equal or above 66 to equal or below 90. In one embodiment, the patient has a KCCQ-TSS of 68.7+/-23.1. In one embodiment, the patient has a KCCQ-TSS of 71.2 +/- 22.5. In one embodiment, the patient has a KCCQ-TSS of 66.0 +/-24.5. In one embodiment, the patient has a KCCQ-TSS of 65.3 ± 23.8. In one embodiment, the patient has a KCCQ-TSS of 68.5+/- 23.7. In one embodiment, the patient has a KCCQ-TSS of 67.8+/-22.7. In one embodiment, the patient has a KCCQ-TSS of 62.3+/- 25.4. In one embodiment, the patient has a KCCQ-TSS of 63.8+/- 25.5.

Body mass index (BMI) is a value derived from the mass (weight) and height of a person. The BMI is defined as the body mass divided by the square of the body height, and is expressed in units of kg/m², resulting from mass in kilograms (kg) and height in metres (m).

In one embodiment, the patient has a body-mass-index (BMI) of equal or above 15 kg/m².

In one embodiment, the patient has a body-mass-index (BMI) selected from of equal or above 15, 16, 17, 18, 18.5, 19, 20, 21, 22, 23, 24, 24.5, 25, 26, 27, 28, 29, 30, 31, 32, 33, 34, 34.5, 35, 36, 37, 38, 39, 40, 41, 42, 43, 44, 45, 46, 47, 48, 49, and 50 kg/m². In one embodiment, the patient has a body-mass-index (BMI) selected from of equal or below 15, 16, 17, 18, 18.5, 19, 20, 21, 22, 23, 24, 24.5, 25, 26, 27, 28, 29, 30, 31, 32, 33, 34, 34.5, 35, 36, 37, 38, 39, 40, 41, 42, 43, 44, 45, 46, 47, 48, 49, and 50 kg/m². In one embodiment, the patient has a body-mass-index (BMI) equal or above 15 kg/m². In one embodiment, the patient has a body-mass-index (BMI) equal or above 20 kg/m². In one embodiment, the patient has a body-mass-index (BMI) equal or above 25 kg/m². In one embodiment, the patient has a body-mass-index (BMI) equal or above 30 kg/m². In one embodiment, the patient has a body-mass-index (BMI) equal or above 35 kg/m². In one embodiment, the patient has a body-mass-index (BMI) equal or above 40 kg/m². In one embodiment, the patient has a body-mass-index (BMI) equal or above 45 kg/m². In one embodiment, the patient has a body-mass-index (BMI) equal or above 50 kg/m². In one embodiment, the patient has a body-mass-index (BMI) equal or below 15 kg/m2. In one embodiment, the patient has a body-mass-index (BMI) equal or below 20 kg/m2. In one embodiment, the patient has a body-mass-index (BMI) equal or below 25 kg/m2. In one embodiment, the patient has a body-mass-index (BMI) equal or below 30 kg/m2. In one embodiment, the patient has a body-mass-index (BMI) equal or below 35 kg/m2. In one embodiment, the patient has a body-mass-index (BMI) equal or below 40 kg/m2. In one embodiment, the patient has a body-mass-index (BMI) equal or below 45 kg/m2. In one embodiment, the patient has a body-mass-index (BMI) equal or below 50 kg/m2.

In one embodiment, the patient has a body-mass-index (BMI) of 30+/-6 kg/m². In one embodiment, the patient has a body-mass-index (BMI) of equal or above 24 to equal or below 36 kg/m². In one embodiment, the patient has a body-mass-index (BMI) of equal or above 24 to equal or below 30 kg/m². In one embodiment, the patient has a body-mass-index (BMI) of equal or above 30 to equal or below 36 kg/m². In one embodiment, the patient has a body-mass-index (BMI) of equal or above 30 kg/m². In one embodiment, the patient has a body-mass-index (BMI) of equal or above 15 to equal or below 18.4 kg/m². In one embodiment, the patient has a body-mass-index (BMI) of equal or above 18.5 to equal or below 24.9 kg/m². In one embodiment, the patient has a body-mass-index (BMI) of equal or above 25 to equal or below 29.9 kg/m². In one embodiment, the patient has a body-mass-index (BMI) of equal or above 30 to equal or below 34.9 kg/m². In one embodiment, the patient has a body-mass-index (BMI) of equal or above 35 to equal or below 39.9 kg/m². In one embodiment, the patient has a body-mass-index (BMI) of equal or above 40 to equal or below 50 kg/m².

In one embodiment, the patient was hospitalized for heart failure (HHF) and has at least one of
- KCCQ-TSS: 53±24, or 70±23, or 71±22,
- eGFR (mL/min/1.73m²): 60±20, or 63±20, or 62±19,
- NT-proBNP (ng/L) (median): 1790, or 1691, or 1322,
- History of Atrial Flutter
- History of Atrial fibrillation,
- History of Diabetes.
- History of coronary artery disease (CAD)
- History of chronic kidney disease (CKD)
- History of stroke
- History of transient ischemic attack (TIA)
- History of chronic obstructive pulmonary disease (COPD)
- History of metabolic syndrome
- History of frailty
- History of peripheral artery disease (PAD), and/or
- History of obstructive sleep apnea (OSA)

In one embodiment, the patient is selected from one ore more of the following subgroups:
- Potassium subgroups are specified above according to the following cutpoints (≤4.5, >4.5 mmol/L). Additional subgroups can be defined by median, quantiles, and other standard definitions of hypo-, hyper- and normokalemia.
- Sodium subgroups defined by the median, quantiles, and standard definitions of hypo-, hyper- and normonatremia.
- Chloride subgroups defined by the median, quantiles, and standard definitions of hypo-, hyper- and normo-chloremia.
- Hemoglobin and hematocrit subgroups defined by the median, quantiles, and standard definitions of anemia.
- eGFR subgroups can be specified above according to the following cutpoints <60, ≥60 mL/min/1.73 m².
- Focused examination of Stage 4 CKD (if eGFR was less than 30mL/min/1.73m²)
- UACR subgroups are specified above according to the following cutpoints (<30 vs. ≥30 mg/g).
- BUN and BUN/creatinine ratio subgroups defined by the median and quantiles.
- Patients suspected to have primary hyperaldosteronism based on renin and aldosterone levels
- BMI subgroups are specified above according to the following cutpoints (<30kg/m2≥30kg/m²)
- Subgroups defined by standard nutritional indices e.g., the geriatric nutritional risk index (GNRI), prognostic nutritional index (PNI), and controlling nutritional status (CONUT), examining these as categorical variables using recognized cut points, medians, quantiles, and as continuous variables.
- Age subgroups are specified above according to the following cutpoints (median age).
- Systolic blood pressure subgroups are specified above according to the following cutpoints (median age). SBP and DBP can be assessed at the median, quantiles, and various global definitions of hypertension (based on both SBP and DBP).
- Patients with apparent treatment resistant hypertension
- Atrial fibrillation
- Atrial fibrillation or atrial flutter on baseline ECG
- a previous history of atrial fibrillation/flutter, optionally together with the overlap between medical history and baseline ECG and derived history of persistent/permanent AF versus paroxysmal AF.
- Patients with improved/recovered LVEF (those who had LVEF ≤40% at any time prior to randomization)
- LVEF at the following cutpoint: <60%, ≥60%), ≤ 49%, 50 to 59%, ≥60% - to examine those with HF with mildly reduced ejection fraction.
- two-way interaction between sex and LVEF will be examined.
- Other anthropometric indices e.g., waist-to-hip ratio, waist-to-height ratio using quantiles and recognized cutpoints
- Time from prior HF hospitalization
- Time from index HF diagnosis
- Patients with COPD
- Patients with OSA
- Patients with cirrhosis
- Patients with any atherosclerotic cardiovascular disease
- Patients with history of stroke/TIA
- Patients with history of coronary artery disease / prior MI
- Patients with history of coronary artery bypass graft surgery
- Patients with peripheral artery disease
- Patients with metabolic syndrome (using standard definitions)
- Subgroups based on baseline use and dosing of diuretics
- Patients with multimorbidity and frailty
- Patients with cardio-renal-metabolic overlap (intersection of atherosclerotic cardiovascular disease, CKD, and diabetes)
- Total baseline medications will be categorized as ("non-polypharmacy": <5 medications; "polypharmacy": 5 to 9 medications; and "hyperpolypharmacy": ≥10 medications)
- Number of blood pressure lowering therapies at baseline
- Patients with baseline risk as determined by the Meta-Analysis Global Group in Chronic (MAGGIC) Heart Failure Risk Score and other HF risk scores
- Patients with baseline risk as determined by the Kidney Failure Risk Equation
- Subgroups based on baseline evidence of congestion and congestion scores
- Regional subgroups based on socioeconomic differences based on the GINI coefficient
- Subgroups based on KCCQ-TSS and other KCCQ domains at baseline.
- Subgroups based on EQ-5D-5L VAS and index scores at baseline.
- Time from diagnosis of heart failure, analyzed as both a continuous and a categorical variable
- Time from HF hospitalization continuously and focused examination for those never hospitalized
- NT-proBNP and high-sensitivity troponin will be examined at median, quantiles, and as continuous easures
- History of any prior MRA use prior to randomization
- Background HF therapies including focused examination of patients on various combinations of therapies (including the Heart Failure Collaboratory score)
- Patients on or off betablockers
- Patients on or off potassium supplements
- Patients on or off potassium lowering therapies
- Patients with phenotypic characteristics of amyloid cardiomyopathy, based on validated clinical models/scores

In the following, embodiments of the dose, dosing scheme, titration, up-titration and/or down-titration of the compound according to formula (I) are described. These embodiments also apply to embodiments, in which a hydrate, solvate, pharmaceutically acceptable salt thereof, a polymorph thereof or polymorph (I) is used.

The term "dose" is used as typically used in the art. The term "dose" is usually applied to the quantity of a drug or other agent administered for therapeutic purposes to the body. The term "dose" and the term "the amount the patient receives" can be used synonymously. In one example, a dose of 10 mg of the compound according to formula (I) is administered to the patient. In other words: the patient receives an amount of 10 mg of the compound of formula (1).

The term "dose" can include a quantity of the drug that the patient takes at the same time. The term "dose" can include a quantity of the drug that the patient takes consecutively. The respective amount of the drug can be taken immediately one after the other or they can be taken at a time interval. This interval can be the same or different.

The "dose" or the "amount the patient receives" can also refer to a single dose, a daily dose or multiple daily doses. For example, if the patient receives 10 mg of the compound according to formula (1) twice a day, this is a single dose of 10 mg, but a daily dose of 20 mg. The term "dose" / "the amount the patient receives" can, thus, refer to the single dose (10 mg) or the total daily dose (20 mg).

A dose can be a once daily, twice daily, four times daily or multiple daily dose.

The amount or dose can be delivered to the patient, for example, using one pharmaceutical composition or one or more pharmaceutical compositions. Pharmaceutical compositions are described in detail below.

For example, a dose of 40 mg of the compound according to formula (I) can be administered to the patient using four tablets comprising 10 mg each. These four tablets can be taken at the same time or consecutively. In another example, a dose of 40 mg of the compound according to formula (I) can be administered to the patient using two tablets comprising 20 mg each. For example, a dose of 40 mg of the compound according to formula (I) can be administered to the patient using two tablets comprising 10 mg each and one tablet comprising 20 mg.

In one embodiment, the compound according to formula (I) is used in an amount of 0.25 mg to 80 mg. In one embodiment, the compound according to formula (I) is used in an amount of 0.25 mg to 40 mg. In one embodiment, the compound according to formula (I) is used in an amount of 0.25 mg to 20 mg. In one embodiment, the compound according to formula (I) is used in an amount of 0.25 mg to 10 mg. In one embodiment, the compound according to formula (I) is used in an amount of 0.25 mg to 5 mg. In one embodiment, the compound according to formula (I) is used in an amount of 5 to 80 mg. In one embodiment, the compound according to formula (I) is used in an amount of 5 to 80 mg, 5 to 70 mg, 5 to 60 mg, 5 to 50 mg, 5 to 40 mg, 10 to 80 mg, 10 to 70 mg, 10 to 60 mg, 10 to 50 mg or 10 to 40 mg. In one embodiment, the compound according to formula (I) is used in an amount of 10 to 40 mg. In one embodiment, the compound according to formula (I) is used in an amount of 20 to 40 mg. In one embodiment, the compound according to formula (I) is used in an amount of 0.25, 0.5, 0.75, 1, 1.25, 1.5, 1.75, 2, 2.25, 2.5, 2.75, 3, 3.25, 3.5, 3.75, 4, 4.25, 4.5, 4.75, 5, 10, 15, 20, 25, 30, 35, 40, 45, 50, 55, 60, 65, 70, 75 or 80 mg. In one embodiment, the compound according to formula (I) is used in an amount of 5, 10, 15, 20, 25, 30, 35, 40, 45, 50, 55, 60, 65, 70, 75 or 80 mg. In one embodiment, the compound according to formula (I) is used in an amount of 5 mg. In one embodiment, the compound according to formula (1) is used in an amount of 10 mg. In one embodiment, the compound according to formula (I) is used in an amount of 15 mg. In one embodiment, the compound according to formula (I) is used in an amount of 20 mg. In one embodiment, the compound according to formula (I) is used in an amount of 25 mg. In one embodiment, the compound according to formula (I) is used in an amount of 30 mg. In one embodiment, the compound according to formula (I) is used in an amount of 35 mg. In one embodiment, the compound according to formula (I) is used in an amount of 40 mg. In one embodiment, the compound according to formula (I) is used in an amount of 5 mg, 10 mg, 15 mg, 20 mg, 25 mg, 30 mg, 35 mg or 40 mg. In one embodiment, the compound according to formula (I) is used in an amount of 5 mg, 10 mg, 20 mg, 30 mg or 40 mg.

The amount of the compound according to formula (I) can be adjusted. In one embodiment thereof, the dose is adjusted by up-titration. In one embodiment thereof, the dose is adjusted by down-titration. In one embodiment thereof, the dose is continued. In one embodiment thereof, the treatment is interrupted.

In one embodiment the treatment or prevention is interrupted due to hyperkalaemia, renal impairment, diarrhoea, nausea, asthenia, dyspnoea, hypotension, sepsis, septic shock, COVID-19, Cardiac arrest, COVID-19 pneumonia, Cardiac failure, Respiratory failure, Lung neoplasm malignant, Pneumonia, Atrial fibrillation, COVID-19, Acute kidney injury, Angina unstable, Anaemia, COVID-19 pneumonia, Urinary tract infection, Syncope, Chest pain, Chronic obstructive pulmonary disease, Angina pectoris, Cellulitis, Sepsis, Coronary artery disease, Cardiac failure, Sudden death, Femur fracture, Fall, Gastrointestinal haemorrhage, hypokalemia and/or Hyperkalaemia.

In one embodiment the dose is adjusted based on one or more of the factors selected from LVEF, NYAH, eGFR, UACR, Potassium level, sodium level, chloride level, urea level, blood urea nitrogen (BUN), high-sensitivity Troponin T level, natriuretic peptide (NT-proBNP), blood pressure (BP), systolic blood pressure, average heart rate (resting), previous diseases, prior MRA use, heart rate (or pulse rate), comedication, KCCQ-TSS, Body mass index (BMI), comedication, CYP3A4 inhibitor, CYP3A4 inducer, loop diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), and mixtures thereof. In one embodiment thereof, the dose is adjusted by up-titration. In one embodiment thereof, the dose is adjusted by down-titration. In one embodiment thereof, the dose is continued (or maintained). In one embodiment thereof, the treatment is interrupted.

The dose can be adjusted based on the eGFR.

In one embodiment thereof, the dose is adjusted by up-titration. In one embodiment thereof, the dose is adjusted by down-titration. In one embodiment thereof, the dose is continued. In one embodiment thereof, the treatment is interrupted.

In one embodiment, the patient receives the compound according to formula (I) in an amount of 10 mg, if the patient has an eGFR of equal or below 60 mL/min/1.73 m². In one embodiment, the patient receives the compound according to formula (I) starting dose in an amount of 10 mg, if the patient has an eGFR of equal or below 60 mL/min/1.73 m².

In one embodiment, the patient receives the compound according to formula (I) in an amount of 20 mg, if the patient has an eGFR of equal or above 60 mL/min/1.73 m². In one embodiment, the patient receives the compound according to formula (I) starting dose in an amount of 20 mg, if the patient has an eGFR of equal or above 60 mL/min/1.73 m².

In one embodiment, the patient receives the compound according to formula (I) in an amount of 40 mg, if the patient has an eGFR of equal or above 60 mL/min/1.73 m². In one embodiment, the patient receives the compound according to formula (I) as starting dose in an amount of 40 mg, if the patient has an eGFR of equal or above 60 mL/min/1.73 m².

In one embodiment, the patient receives a lower dose of the compound according to formula (I), and patient has an eGFR of equal or below 60 mL/min/1.73 m², then the dose is up-titrated to the next higher dose. In one embodiment, the patient receives a higher dose of the compound according to formula (I), and patient has an eGFR of equal or above 60 mL/min/1.73 m², then the dose is down-titrated to the next lower dose. Further examples for "lower dose" are described below.

In one embodiment, the dose the patient is receiving is up-titrated.

In one embodiment, the dose the patient is receiving is up-titrated depending on the potassium level.

In one embodiment, the dose the patient is receiving is up-titrated, if the potassium level is equal or below 3, 3.5, 4, 4.5, 4.8, 5 or 5.5 mmol/L. In one embodiment, the dose the patient is receiving is up-titrated, if the potassium level is equal or below 3, 3.5, 4, 4.5, 4.7, 4.8, 4.9, 5, 5.1, 5.4, 5.5, or 5.6 mmol/L. In one embodiment, the dose the patient is receiving is up-titrated, if the potassium level is equal or above 3, 3.5, 4, 4.5, 4.8, 5 or 5.5 mmol/L. In one embodiment, the dose the patient is receiving is up-titrated, if the potassium level is equal or above 3, 3.5, 4, 4.5, 4.8, or 5 or 5.5 mmol/L, but below 5.5 mmol/L.

In one embodiment, the dose the patient is receiving is up-titrated, if the potassium level is equal or below 4.0 mmol/L. In one embodiment, the dose the patient is receiving is up-titrated, if the potassium level is equal or below 4.5 mmol/L. In one embodiment, the dose the patient is receiving is up-titrated, if the potassium level is equal or below 4.8 mmol/L. In one embodiment, the dose the patient is receiving is up-titrated, if the potassium level is equal or below 5 mmol/L. In one embodiment, the dose the patient is receiving is up-titrated, if the potassium level is equal or below 5.5 mmol/L.

In one embodiment, the dose the patient is receiving is up-titrated, if the potassium level is equal or above 4.5 mmol/L to equal or below to below 5.5 mmol/L. In one embodiment, the dose the patient is receiving is up-titrated, if the potassium level is equal or above 4.8 mmol/L to below 5.5 mmol/L. In one embodiment, the dose the patient is receiving is up-titrated, if the potassium level is equal or above 5 mmol/L to below 5.5 mmol/L. In one embodiment, the dose the patient is receiving is up-titrated, if the potassium level is equal or above 4.5 mmol/L to equal or below to equal or below 5 mmol/L. In one embodiment, the dose the patient is receiving is up-titrated, if the potassium level is equal or above 4.8 mmol/L to equal or equal or below 5 mmol/L.

In one embodiment, the dose the patient is receiving is maintained of the potassium level is equal or above 5 to below 5.5 mmol/L. A synonym for "maintained" is for example "continued".

In one embodiment, the dose the patient is receiving is continued.

In one embodiment, the dose the patient is receiving is continued is depending on the potassium level.

In one embodiment, the dose the patient is receiving is continued, if the potassium level is equal or below 3, 3.5, 4, 4.5, 4.8, 5 or 5.5 mmol/L. In one embodiment, the dose the patient is receiving is continued, if the potassium level is equal or above 3, 3.5, 4, 4.5, 4.8, 5 or 5.5 mmol/L. In one embodiment, the dose the patient is receiving is continued, if the potassium level is equal or above 3, 3.5, 4, 4.5, 4.8, or 5 or 5.5 mmol/L, but below 5.5 mmol/L. The dose the patient is receiving can be selected from 10 mg, 20 mg and 40 mg. The dose the patient is receiving can be 10 mg. The dose the patient is receiving can be 20 mg. The dose the patient is receiving can be 40 mg.

In one embodiment, the dose the patient is receiving is continued, if the potassium level is equal or below 4.5, 4.8, 5 or 5.5 mmol/L. In one embodiment, the dose the patient is receiving is continued, if the potassium level is equal or above 4.5, 4.8, 5 or 5.5 mmol/L. In one embodiment, the dose the patient is receiving is continued, if the potassium level is equal or above 4.5, 4.8, or 5 or 5.5 mmol/L, but below 5.5 mmol/L. The dose the patient is receiving can be selected from 10 mg, 20 mg and 40 mg. The dose the patient is receiving can be 10 mg. The dose the patient is receiving can be 20 mg. The dose the patient is receiving can be 40 mg.

In one embodiment, the dose the patient is receiving is continued, if the potassium level is equal or below 4.8, 5 or 5.5 mmol/L. In one embodiment, the dose the patient is receiving is continued, if the potassium level is equal or above 4.8, 5 or 5.5 mmol/L. In one embodiment, the dose the patient is receiving is continued, if the potassium level is equal or above 4.8, or 5 or 5.5 mmol/L, but below 5.5 mmol/L. The dose the patient is receiving can be selected from 10 mg, 20 mg and 40 mg. The dose the patient is receiving can be 10 mg. The dose the patient is receiving can be 20 mg. The dose the patient is receiving can be 40 mg.

In one embodiment, the dose the patient is receiving is continued, if the potassium level is equal or below 4.8 mmol/L. In one embodiment, the dose the patient is receiving is continued, if the potassium level is equal or above 4.8 mmol/L. In one embodiment, the dose the patient is receiving is continued, if the potassium level is equal or above 4.8 mmol/L, but below 5.5 mmol/L. The dose the patient is receiving can be selected from 10 mg, 20 mg and 40 mg. The dose the patient is receiving can be 10 mg. The dose the patient is receiving can be 20 mg. The dose the patient is receiving can be 40 mg.

In one embodiment, the dose the patient is receiving is continued, if the potassium level is equal or below 5 mmol/L. In one embodiment, the dose the patient is receiving is continued, if the potassium level is equal or above 5 mmol/L. In one embodiment, the dose the patient is receiving is continued, if the potassium level is equal or above 5 mmol/L, but below 5.5 mmol/L. The dose the patient is receiving can be selected from 10 mg, 20 mg and 40 mg. The dose the patient is receiving can be 10 mg. The dose the patient is receiving can be 20 mg. The dose the patient is receiving can be 40 mg.

In one embodiment, the dose the patient is receiving is continued, if the potassium level is equal or below 5.5 mmol/L. In one embodiment, the dose the patient is receiving is continued, if the potassium level is equal or above 5.5 mmol/L. The dose the patient is receiving can be selected from 10 mg, 20 mg and 40 mg. The dose the patient is receiving can be 10 mg. The dose the patient is receiving can be 20 mg. The dose the patient is receiving can be 40 mg.

In one embodiment, the patient the treatment of the patient is interrupted. In one embodiment, the patient the treatment of the patient is interrupted, if the potassium level is equal or above 5, 5.5 or 6 mmol/L. In one embodiment, the patient the treatment of the patient is interrupted, if the potassium level is equal or above 5.5 or 6 mmol/L. In one embodiment, the patient the treatment of the patient is interrupted, if the potassium level is equal or above 5 mmol/L. In one embodiment, the patient the treatment of the patient is interrupted, if the potassium level is equal or above 5.5 mmol/L. In one embodiment, the patient the treatment of the patient is interrupted, if the potassium level is equal or above 6 mmol/L. In one embodiment, the patient the treatment of the patient is interrupted, if the potassium level is equal or above 6.5 mmol/L.

In one embodiment, the patient the treatment of the patient is interrupted, if the potassium level is between 5 to 6.5 mmol/L. In one embodiment, the patient the treatment of the patient is interrupted, if the potassium level is between 5 to 6 mmol/L. In one embodiment, the patient the treatment of the patient is interrupted, if the potassium level is between 5.5 to 6.5 mmol/L. In one embodiment, the patient the treatment of the patient is interrupted, if the potassium level is between 5.5 to 6 mmol/L.

In one embodiment, the treatment is interrupted, if
- the patient receives a dose selected from 10, 20, 30 and 40 mg; and
- the potassium level is equal or above 5, 5.5 or 6 mmol/L.

In one embodiment, the treatment is interrupted, if
- the patient receives a dose selected from 10, 20, 30 and 40 mg; and
- the potassium level is equal or above 5, 5.5, 5.9, 6, or 6.1 mmol/L.

In one embodiment, the treatment is interrupted, if
- the patient receives a dose selected from 10, 20, 30 and 40 mg; and
- the potassium level is equal or above 5.5 or 6 mmol/L.

In one embodiment, the treatment is interrupted, if
- the patient receives a dose selected from 10, 20, 30 and 40 mg; and
- the potassium level is equal or above 5.5 mmol/L.

In one embodiment, the treatment is interrupted, if
- the patient receives a dose selected from 10, 20, 30 and 40 mg; and
- the potassium level is equal or above 6 mmol/L.

In one embodiment, the treatment is interrupted, if
- the patient receives a dose selected from 10, 20, 30 and 40 mg; and
- the potassium level is between equal or above 5 to 6.5 mmol/L.

In one embodiment, the treatment is interrupted, if
- the patient receives a dose selected from 10, 20, 30 and 40 mg; and
- the potassium level is between equal or above 5.5 to 6 mmol/L.

In one embodiment, the treatment is interrupted, if
- the patient receives a dose of 10 mg; and
- the potassium level is equal or above 5, 5.5 or 6 mmol/L.

In one embodiment, the treatment is interrupted, if
- the patient receives a dose of 20 mg; and
- the potassium level is equal or above 5, 5.5 or 6 mmol/L.

In one embodiment, the treatment is interrupted, if
- the patient receives a dose of 30 mg; and
- the potassium level is equal or above 5, 5.5 or 6 mmol/L.

In one embodiment, the treatment is interrupted, if
- the patient receives a dose of 40 mg; and
- the potassium level is equal or above 5, 5.5 or 6 mmol/L.

In one embodiment, the treatment is interrupted, if
- the patient receives a dose selected from 10 mg; and
- the potassium level is equal or above 5.5 mmol/L.

In one embodiment, the treatment is interrupted, if
- the patient receives a dose selected from 10 mg; and
- the potassium level is equal or above 6 mmol/L.

In one embodiment, the treatment is interrupted, if
- the patient receives a dose selected from 10 mg; and
- the potassium level is between equal or above 5 to 6.5 mmol/L.

In one embodiment, the treatment is interrupted, if
- the patient receives a dose selected from 10 mg; and
- the potassium level is between equal or above 5.5 to 6 mmol/L.

In one embodiment, the treatment is interrupted, if
- the patient receives a dose selected from 20 mg; and
- the potassium level is equal or above 5.5 mmol/L.

In one embodiment, the treatment is interrupted, if
- the patient receives a dose selected from 20 mg; and
- the potassium level is equal or above 6 mmol/L.

In one embodiment, the treatment is interrupted, if
- the patient receives a dose selected from 20 mg; and
- the potassium level is between equal or above 5 to 6.5 mmol/L.

In one embodiment, the treatment is interrupted, if
- the patient receives a dose selected from 20 mg; and
- the potassium level is between equal or above 5.5 to 6 mmol/L.

In one embodiment, the treatment is interrupted, if
- the patient receives a dose selected from 30 mg; and
- the potassium level is equal or above 5.5 mmol/L.

In one embodiment, the treatment is interrupted, if
- the patient receives a dose selected from 30 mg; and
- the potassium level is equal or above 6 mmol/L.

In one embodiment, the treatment is interrupted, if
- the patient receives a dose selected from 30 mg; and
- the potassium level is between equal or above 5 to 6.5 mmol/L.

In one embodiment, the treatment is interrupted, if
- the patient receives a dose selected from 30 mg; and
- the potassium level is between equal or above 5.5 to 6 mmol/L.

In one embodiment, the treatment is interrupted, if
- the patient receives a dose selected from 40 mg; and
- the potassium level is equal or above 5.5 mmol/L.

In one embodiment, the treatment is interrupted, if
- the patient receives a dose selected from 40 mg; and
- the potassium level is equal or above 6 mmol/L.

In one embodiment, the treatment is interrupted, if
- the patient receives a dose selected from 40 mg; and
- the potassium level is between equal or above 5 to 6.5 mmol/L.

In one embodiment, the treatment is interrupted, if
- the patient receives a dose selected from 40 mg; and
- the potassium level is between equal or above 5.5 to 6 mmol/L.

The treatment of the patient can be interrupted and then restarted. In one embodiment, the treatment of the patient is restarted.

In one embodiment, the dose with which the treatment is restarted is selected from 10 mg, 20 mg and 40 mg. In one embodiment, the dose with which the treatment is restarted is 10 mg. In one embodiment, the dose with which the treatment is restarted is 20 mg. In one embodiment, the dose with which the treatment is restarted is 40 mg.

In one embodiment, the treatment of the patient is restarted, if the potassium level is equal or below 3, 3.5, 4, 4.5, 4.8, 5 or 5.5 mmol/L. In one embodiment, the treatment of the patient is restarted, if the potassium level is equal or above 3, 3.5, 4, 4.5, 4.8, 5 or 5.5 mmol/L. In one embodiment, the treatment of the patient is restarted, if the potassium level is equal or above 3, 3.5, 4, 4.5, 4.8, or 5 or 5.5 mmol/L, but below 5.5 mmol/L. The dose with which the treatment is restarted can be selected from 10 mg, 20 mg and 40 mg. The dose with which the treatment is restarted can be 10 mg. The dose with which the treatment is restarted can be 20 mg. The dose with which the treatment is restarted can be 40 mg.

In one embodiment, the treatment of the patient is restarted, if the potassium level is equal or below 4.5, 4.8, 5 or 5.5 mmol/L. In one embodiment, the treatment of the patient is restarted, if the potassium level is equal or above 4.5, 4.8, 5 or 5.5 mmol/L. In one embodiment, the treatment of the patient is restarted, if the potassium level is equal or above 4.5, 4.8, or 5 or 5.5 mmol/L, but below 5.5 mmol/L. The dose with which the treatment is restarted can be selected from 10 mg, 20 mg and 40 mg. The dose with which the treatment is restarted can be 10 mg. The dose with which the treatment is restarted can be 20 mg. The dose with which the treatment is restarted can be 40 mg.

In one embodiment, the treatment of the patient is restarted, if the potassium level is equal or below 4.8, 5 or 5.5 mmol/L. In one embodiment, the treatment of the patient is restarted, if the potassium level is equal or above 4.8, 5 or 5.5 mmol/L. In one embodiment, the treatment of the patient is restarted, if the potassium level is equal or above 4.8, or 5 or 5.5 mmol/L, but below 5.5 mmol/L. The dose with which the treatment is restarted can be selected from 10 mg, 20 mg and 40 mg. The dose with which the treatment is restarted can be 10 mg. The dose with which the treatment is restarted can be 20 mg. The dose with which the treatment is restarted can be 40 mg.

In one embodiment, the treatment of the patient is restarted, if the potassium level is equal or below 4.8 mmol/L. In one embodiment, the treatment of the patient is restarted, if the potassium level is equal or above 4.8 mmol/L. In one embodiment, the treatment of the patient is restarted, if the potassium level is equal or above 4.8 mmol/L, but below 5.5 mmol/L. The dose with which the treatment is restarted can be selected from 10 mg, 20 mg and 40 mg. The dose with which the treatment is restarted can be 10 mg. The dose with which the treatment is restarted can be 20 mg. The dose with which the treatment is restarted can be 40 mg.

In one embodiment, the treatment of the patient is restarted, if the potassium level is equal or below 5 mmol/L. In one embodiment, the treatment of the patient is restarted, if the potassium level is equal or above 5 mmol/L. In one embodiment, the treatment of the patient is restarted, if the potassium level is equal or above 5 mmol/L, but below 5.5 mmol/L. The dose with which the treatment is restarted can be selected from 10 mg, 20 mg and 40 mg. The dose with which the treatment is restarted can be 10 mg. The dose with which the treatment is restarted can be 20 mg. The dose with which the treatment is restarted can be 40 mg.

In one embodiment, the treatment of the patient is restarted, if the potassium level is equal or below 5.5 mmol/L. In one embodiment, the treatment of the patient is restarted, if the potassium level is equal or above 5.5 mmol/L. The dose with which the treatment is restarted can be selected from 10 mg, 20 mg and 40 mg. The dose with which the treatment is restarted can be 10 mg. The dose with which the treatment is restarted can be 20 mg. The dose with which the treatment is restarted can be 40 mg.

In one embodiment, the treatment is restarted, wherein
- the patient receives a dose selected from 10, 20, 30, and 40 mg;
- the potassium level is equal or below 4.5, 4.8, 5, 5.5, 6 or 6.5 mmol/L.

In one embodiment, the treatment is restarted, wherein
- the patient receives a dose selected from 10, 20, 30, and 40 mg;
- the potassium level is equal or below 5 mmol/L.

In one embodiment, the treatment is restarted, wherein
- the patient receives a dose selected from 10, 20, 30, and 40 mg;
- the potassium level is equal or below 5.5 mmol/L.

In one embodiment, the treatment is restarted, wherein
- the patient receives a dose selected from 10, 20, 30, and 40 mg;
- the potassium level is equal or below 6 mmol/L.

In one embodiment, the treatment is restarted, wherein
- the patient receives a dose of 10 mg;
- the potassium level is equal or below 5 mmol/L.

In one embodiment, the treatment is restarted, wherein
- the patient receives a dose of 20 mg;
- the potassium level is equal or below 5 mmol/L.

In one embodiment, the treatment is restarted, wherein
- the patient receives a dose of 30 mg;
- the potassium level is equal or below 5mmol/L.

In one embodiment, the treatment is restarted, wherein
- the patient receives a dose of 40 mg;
- the potassium level is equal or below 5 mmol/L.

In one embodiment, the treatment is restarted, wherein
- the patient receives a dose of 10 mg;
- the potassium level is equal or below 5.5 mmol/L.

In one embodiment, the treatment is restarted, wherein
- the patient receives a dose of 20 mg;
- the potassium level is equal or below 5.5 mmol/L.

In one embodiment, the treatment is restarted, wherein
- the patient receives a dose of 30 mg;
- the potassium level is equal or below 5.5 mmol/L.

In one embodiment, the treatment is restarted, wherein
- the patient receives a dose of 40 mg;
- the potassium level is equal or below 5.5 mmol/L.

Further consideration might apply to the dose adjustment:
In case the participant receives the lowest dose, a no further decrease might be possible after interruption. The dose level can be 10, 20, 30 or 40 mg. The treatment can be restarted at the same dose level once potassium level is equal or below 4.5, 4.8, 5, 5.5, 6 or 6.5 mmol/L. The treatment can be restarted at the same dose level once potassium level is equal or below 4.5, 4.8, 5, or 5.5 mmol/L. The treatment can be restarted at the same dose level once potassium level is equal or below 5.5 mmol/L. Serum/plasma potassium can be measured at a safety visit after re-starting treatment or dose adjustment. Serum/plasma potassium is to be measured at a safety visit 4 weeks ± 7 days after re-starting treatment or dose adjustment.

In case the participant receives the lowest dose, but hyperkalemia recurs soon after a previous event of hyperkalemia leading to interruption of study intervention, and there is no explanation for the recurring hyperkalemia event other than intake of the dose, the treatment can be premature and/or permanent discontinued.

In the following, dosing schemes are described. In some embodiment, for the dosing schemes for up-titration and/or down-titration described herein the following embodiments can apply:
In some embodiments, The amount of the compound according to formula (I) is adjusted by up-titration. Here, the patient receives a lower dose, which is up-titrated to a higher dose. The patient can receive a lower dose, which is up-titrated to the next higher dose. In some embodiments, The amount of the compound according to formula (I) is adjusted by down-titration. Here, the patient receives a higher dose, which is down-titrated to a lower dose. The patient can receive a higher dose, which is down-titrated to the next lower dose.

In one embodiment, the "lower dose" of the compound according to formula (I) is between 0.5 to 80 mg. In one embodiment, the "lower dose" of the compound according to formula (I) is between 5 to 80 mg. In one embodiment, the "lower dose" of the compound according to formula (1) is selected from 5 mg, 10 mg, 20 mg, 30 mg, 40 mg, 60 mg, and 80 mg. In one embodiment, the "lower dose" is selected from 10 mg, 20 mg, and 30 mg. In one embodiment, the "lower dose" is 5 mg. In one embodiment, the "lower dose" is 10 mg. In one embodiment, the "lower dose" is 20 mg. In one embodiment, the "lower dose" is 30 mg.

The "next lower dose" can be a dose that is lower than the dose the patient is currently receiving. For example, if the currently receiving or the higher dose is 40 mg, the next lower dose can be for example 30 mg, 20 mg, 10 mg, or 5 mg. In one example the higher dose is 40 mg and the next lower dose is 30 mg. In one example the higher dose is 40 mg and the next lower dose is 20 mg. In one example the higher dose is 40 mg and the next lower dose is 10 mg. In one example the higher dose is 40 mg and the next lower dose is 5 mg.

In one embodiment, "lower dose" and "next lower dose" are synonyms.

In one embodiment, the "next lower dose" is selected from 5 mg, 10 mg, 20 mg, 30 mg, 40 mg, 60 mg, and 80 mg. In one embodiment, the "next lower dose" is selected from 10 mg, 20 mg, and 30 mg. In one embodiment, the "next lower dose" is 5 mg. In one embodiment, the "next lower dose" is 10 mg. In one embodiment, the "next lower dose" is 20 mg. In one embodiment, the "next lower dose" is 30 mg. In one embodiment, the "next lower dose" is 40 mg. In one embodiment, the "next lower dose" is 60 mg.

In one embodiment, the "higher dose" of the compound according to formula (I) is between 0.5 to 80 mg. In one embodiment, the "higher dose" of the compound according to formula (I) is between 5 to 80 mg. In one embodiment, the "higher dose" of the compound according to formula (I) is selected from 5 mg, 10 mg, 20 mg, 30 mg, 40 mg, 60 mg, and 80 mg. In one embodiment, the "higher dose" is selected from 20 mg, 30 mg, and 40 mg. In one embodiment, the "higher dose" is 20 mg. In one embodiment, the "higher dose" is 30 mg. In one embodiment, the "higher dose" is 40 mg.

The "next higher dose" can be a dose that is higher than the dose the patient is currently receiving. For example, if the currently receiving or the lower dose is 10 mg, the next higher dose can be for example 20 mg, 30 mg or 40 mg. In one example the lower dose 10 mg and the next higher dose is 20 mg. In one example the lower dose 10 mg and the next higher dose is 30 mg. In one example the lower dose 20 mg and the next higher dose is 30 mg. In one example the lower dose 20 mg and the next higher dose is 40 mg.

In one embodiment, "higher dose" and "next higher dose" are synonyms.

In one embodiment, the "next higher dose" is selected from 5 mg, 10 mg, 20 mg, 30 mg, 40 mg, 60 mg, and 80 mg. In one embodiment, the "next higher dose" is selected from 20 mg, 30 mg, and 40 mg. In one embodiment, the "next higher dose" is 20 mg. In one embodiment, the "next higher dose" is 30 mg. In one embodiment, the "next higher dose" is 40 mg. In one embodiment, the "next higher dose" is 60 mg. In one embodiment, the "next higher dose" is 80 mg.

In one embodiment, the "next lower dose" is selected from 10 mg, 20 mg, and 30 mg. In one embodiment, the "next lower dose" is 10 mg. In one embodiment, the "next lower dose" is 20 mg. In one embodiment, the "next lower dose" is 30 mg.

In some embodiments, The amount of the compound according to formula (I) is adjusted by up-titration. Here, the patient receives a lower dose, which is up-titrated to a higher dose:
In one embodiment, the "lower dose" is 5 mg, and the higher dose is selected from 10 mg, 20 mg, 30 mg, 40 mg, 60 mg and 80 mg. In one embodiment, the "lower dose" is 10 mg, and the higher dose is selected from 20 mg, 30 mg, 40 mg, 60 mg and 80 mg. In one embodiment, the "lower dose" is 20 mg, and the higher dose is selected from 30 mg, 40 mg, 60 mg and 80 mg. In one embodiment, the "lower dose" is 30 mg, and the higher dose is selected from 40 mg, 60 mg and 80 mg. In one embodiment, the "lower dose" is 40 mg, and the higher dose is selected from 60 mg and 80 mg. In one embodiment, the "lower dose" is 60 mg, and the higher dose is 80 mg. In these embodiments, the "higher dose" can be the "next higher dose".

In one embodiment, the "lower dose" is 5 mg, and the "higher dose" is 10 mg. In one embodiment, the "lower dose" is 5 mg, and the "higher dose" is 20 mg. In one embodiment, the "lower dose" is 5 mg, and the "higher dose" is 30 mg. In one embodiment, the "lower dose" is 5 mg, and the "higher dose" is 40 mg. In one embodiment, the "lower dose" is 5 mg, and the "higher dose" is 60 mg. In one embodiment, the "lower dose" is 5 mg, and the "higher dose" is 80 mg. In one embodiment, the "lower dose" is 10 mg, and the "higher dose" is 20 mg. In one embodiment, the "lower dose" is 10 mg, and the "higher dose" is 30 mg. In one embodiment, the "lower dose" is 10 mg, and the "higher dose" is 40 mg. In one embodiment, the "lower dose" is 10 mg, and the "higher dose" is 60 mg. In one embodiment, the "lower dose" is 10 mg, and the "higher dose" is 80 mg. In one embodiment, the "lower dose" is 10 mg and the "next higher dose" is 20 mg. In one embodiment, the "lower dose" is 10 mg and the "next higher dose" is 40 mg. In one embodiment, the "lower dose" is 20 mg, and the "higher dose" is 40 mg. In one embodiment, the "lower dose" is 20 mg, and the "higher dose" is 30 mg. In one embodiment, the "lower dose" is 20 mg, and the "next higher dose" is 40 mg. In one embodiment, the "lower dose" is 20 mg, and the "higher dose" is 60 mg. In one embodiment, the "lower dose" is 20 mg, and the "higher dose" is 80 mg. In one embodiment, the "lower dose" is 30 mg, and the "next higher dose" is 40 mg. In one embodiment, the "lower dose" is 30 mg, and the "higher dose" is 60 mg. In one embodiment, the "lower dose" is 30 mg, and the "higher dose" is 80 mg. In one embodiment, the "lower dose" is 40 mg, and the "higher dose" is 60 mg. In one embodiment, the "lower dose" is 40 mg, and the "higher dose" is 80 mg. In one embodiment, the "lower dose" is 60 mg, and the "higher dose" is 80 mg. In these embodiments, the "higher dose" can be the "next higher dose".

In some embodiments, The amount of the compound according to formula (I) is adjusted by down-titration. Here, the patient receives a higher dose, which is down-titrated to a lower dose:
In one embodiment, the "higher dose" is 80 mg, and the "lower dose" is selected from 5 mg, 10 mg, 20 mg, 40 mg, and 60 mg. In one embodiment, the "higher dose" is 60 mg, and the "lower dose" is selected from 5 mg, 10 mg, 20 mg, and 40 mg. In one embodiment, the "higher dose" is 40 mg, and the "lower dose" is selected from 5 mg, 10 mg, and 20 mg. In one embodiment, the "higher dose" is 20 mg, and the "lower dose" is selected from 5 mg, and 10 mg. In one embodiment, the "higher dose" is 10 mg, and the "lower dose" is 5 mg. In these embodiments, the "lower dose" can be the "next lower dose".

In one embodiment, the "higher dose" is 80 mg, and the "lower dose" is 60 mg. In one embodiment, the "higher dose" is 80 mg, and the "lower dose" is 40 mg. In one embodiment, the "higher dose" is 80 mg, and the "lower dose" is 30 mg. In one embodiment, the "higher dose" is 80 mg, and the "lower dose" is 20 mg. In one embodiment, the "higher dose" is 80 mg, and the "lower dose" is 10 mg. In one embodiment, the "higher dose" is 80 mg, and the "lower dose" is 5 mg. In one embodiment, the "higher dose" is 60 mg, and the "lower dose" is 40 mg. In one embodiment, the "higher dose" is 60 mg, and the "lower dose" is 30 mg. In one embodiment, the "higher dose" is 60 mg, and the "lower dose" is 20 mg. In one embodiment, the "higher dose" is 60 mg, and the "lower dose" is 10 mg. In one embodiment, the "higher dose" is 60 mg, and the "lower dose" is 5 mg. In one embodiment, the "higher dose" is 40 mg and the "lower dose" is 30 mg. In one embodiment, the "higher dose" is 40 mg and the "lower dose" is 20 mg. In one embodiment, the "higher dose" is 40 mg and the "lower dose" is 10 mg. In one embodiment, the "higher dose" is 40 mg and the "lower dose" is 5 mg. In one embodiment, the "higher dose" is 20 mg and the "lower dose" is 10 mg. In one embodiment, the "higher dose" is 20 mg and the "lower dose" is 5 mg. In one embodiment, the "higher dose" is 10 mg and the "lower dose" is 5 mg. In these embodiments, the "lower dose" can be the "next lower dose".

In one embodiment, the "higher dose" is 40 mg and the "next lower dose" is 20 mg. In one embodiment, the "higher dose" is 40 mg and the "next lower dose" is 10 mg.

In some embodiments that dose can be adjusted stepwise.

In one embodiment, the dose the patient is receiving is up-titrated stepwise. Stepwise can mean, that the titration is performed in one step. For example, in case that the "lower dose" is 10 mg, and the "higher dose" is 20 mg, than the dose is up-titrated for example from 10 mg to 20 mg. Stepwise can mean, that the titration is performed in at least two steps. For example, in case that the "lower does" is 10 mg, and "the "higher dose" is 40 mg, than the dose is up-titrated for example in the first step from 10 mg to 20 mg and in the second step from 20 mg to 40 mg. Stepwise can mean, that the up-titration is performed in three steps. For example, in case that the "lower does" is 10 mg, and "the "higher dose" is 40 mg, than the dose is up-titrated for example in the first step from 10 mg to 20 mg and in the second step from 20 mg to 30 mg, and in the third step from 30 mg to 40 mg.

In some embodiments that dose can be adjusted stepwise. In one embodiment, the dose the patient is receiving is down-titrated stepwise. In one embodiment, the dose the patient is receiving is down-titrated stepwise. Stepwise can mean, that the titration is performed in one step. For example, in case that the "lower dose" is 10 mg, and the "higher dose" is 20 mg, than the dose is down-titrated for example from 20 mg to 10 mg. Stepwise can mean, that the titration is performed in at least two steps. For example, in case that the "lower does" is 10 mg, and "the "higher dose" is 40 mg, than the dose is down-titrated for example in the first step from 40 mg to 20 mg and in the second step from 20 mg to 10 mg. Stepwise can mean, that the up-titration is performed in three steps. For example, in case that the "lower does" is 10 mg, and "the "higher dose" is 40 mg, than the dose is down-titrated for example in the first step from 40 mg to 30 mg and in the second step from 30 mg to 10 mg, and in the third step from 30 mg to 40 mg.

In one embodiment, the patient receives a lower dose of the compound according to formula (I), and
- the potassium level is equal or below 4.5 mmol/L,
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.5 mmol/L,
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.5 mmol/L,
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 4.8 mmol/L,
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.8 mmol/L,
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.8 mmol/L,
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5 mmol/L,
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5.5 mmol/L,
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
then the dose is up-titrated to the next higher dose. In this embodiment, the lower dose can be selected from 10 mg and 20 mg; and the next higher dose can be selected from 20 mg and 40 mg.

In one embodiment, the patient receives a dose of the compound according to formula (I) in an amount of 10 mg, and the potassium level is equal or below 4.5 mmol/L, then the dose is up-titrated to 20 mg. In one embodiment, the patient receives a dose of the compound according to formula (I) in an amount of 10 mg, and the potassium level is equal or below 4.8 mmol/L, then the dose is up-titrated to 20 mg. In one embodiment, the patient receives a dose of the compound according to formula (I) in an amount of 10 mg, and the potassium level is equal or below 5 mmol/L, then the dose is up-titrated to 20 mg. In one embodiment, the patient receives a dose of the compound according to formula (I) in an amount of 10 mg, and the potassium level is equal or below 5.5 mmol/L, then the dose is up-titrated to 20 mg.

In one embodiment, the patient receives a dose of the compound according to formula (I) in an amount of 10 mg, and the potassium level is equal or below 4.5 mmol/L, then the dose is up-titrated to 40 mg. In one embodiment, the patient receives a dose of the compound according to formula (I) in an amount of 10 mg, and the potassium level is equal or below 4.8 mmol/L, then the dose is up-titrated to 40 mg. In one embodiment, the patient receives a dose of the compound according to formula (I) in an amount of 10 mg, and the potassium level is equal or below 5 mmol/L, then the dose is up-titrated to 40 mg. In one embodiment, the patient receives a dose of the compound according to formula (I) in an amount of 10 mg, and the potassium level is equal or below 5.5 mmol/L, then the dose is up-titrated to 40 mg.

In one embodiment, the patient receives a dose of the compound according to formula (I) in an amount of 10 mg, and the potassium level is equal or below 4.5 mmol/L, then the dose is up-titrated to 40 mg as follows:

| | |
|---|---|
| Step 1 | from 10 mg to 20 mg |
| Step 2 | from 20 mg to 40 mg. |

In one embodiment, the patient receives a dose of the compound according to formula (I) in an amount of 10 mg, and the potassium level is equal or below 4.8 mmol/L, then the dose is up-titrated to 40 mg as follows:

| | |
|---|---|
| Step 1 | from 10 mg to 20 mg |
| Step 2 | from 20 mg to 40 mg. |

In one embodiment, the patient receives a dose of the compound according to formula (I) in an amount of 10 mg, and the potassium level is equal or below 5 mmol/L, then the dose is up-titrated to 40 mg as follows:

| | |
|---|---|
| Step 1 | from 10 mg to 20 mg |
| Step 2 | from 20 mg to 40 mg. |

In one embodiment, the patient receives a dose of the compound according to formula (I) in an amount of 10 mg, and the potassium level is equal or below 5.5 mmol/L, then the dose is up-titrated to 40 mg as follows:

| | |
|---|---|
| Step 1 | from 10 mg to 20 mg |
| Step 2 | from 20 mg to 40 mg. |

In one embodiment, the patient receives a dose of the compound according to formula (I) in an amount of 20 mg, and the potassium level is equal or below 4.5 mmol/L, then the dose is up-titrated to 40 mg. In one embodiment, the patient receives a dose of the compound according to formula (I) in an amount of 20 mg, and the potassium level is equal or below 4.8 mmol/L, then the dose is up-titrated to 40 mg. In one embodiment, the patient receives a dose of the compound according to formula (I) in an amount of 20 mg, and the potassium level is equal or below 5 mmol/L, then the dose is up-titrated to 40 mg. In one embodiment, the patient receives a dose of the compound according to formula (I) in an amount of 20 mg, and the potassium level is equal or below 5.5 mmol/L, then the dose is up-titrated to 40 mg.

In one embodiment, the dose the patient is receiving is up-titrated depending on the eGFR decrease. In one embodiment, the dose the patient is receiving is up-titrated depending on the eGFR decrease, based on the relative eGFR change from baseline.

In one embodiment, the dose the patient is receiving is up-titrated, if the eGFR decrease is equal or below 5, 10, 15, 20, 25, 30, 35, 40, 45, 50, 57 or 60 %, based on the relative eGFR change from baseline. In one embodiment, the dose the patient is receiving is up-titrated, if the eGFR decrease is equal or above 5, 10, 15, 20, 25, 30, 35, 40, 45, 50, 57 or 60 %, based on the relative eGFR change from baseline. In one embodiment, the dose the patient is receiving is up-titrated, if the eGFR decrease is equal or below 5, 10, 15, 20, 25, 30, 35, or 40 %, based on the relative eGFR change from baseline. In one embodiment, the dose the patient is receiving is up-titrated, if the eGFR decrease is equal or above 5, 10, 15, 20, 25, 30, 35, or 40 %, based on the relative eGFR change from baseline. In one embodiment, the dose the patient is receiving is up-titrated, if the eGFR decrease is equal or below 5, 10, 15, 20, 25, or 30 %, based on the relative eGFR change from baseline. In one embodiment, the dose the patient is receiving is up-titrated, if the eGFR decrease is equal or above 5, 10, 15, 20, 25, or 30 40 %, based on the relative eGFR change from baseline.

In one embodiment, the dose the patient is receiving is up-titrated, if the eGFR decrease is equal or below 25 %, based on the relative eGFR change from baseline. In one embodiment, the dose the patient is receiving is up-titrated, if the eGFR decrease is equal or below 30 %, based on the relative eGFR change from baseline. In one embodiment, the dose the patient is receiving is up-titrated, if the eGFR decrease is equal or below 40 %, based on the relative eGFR change from baseline. In one embodiment, the dose the patient is receiving is up-titrated, if the eGFR decrease is equal or below 50 %, based on the relative eGFR change from baseline. In one embodiment, the dose the patient is receiving is up-titrated, if the eGFR decrease is equal or below 57 %, based on the relative eGFR change from baseline.

In one embodiment, the dose the patient is receiving is up-titrated, if the eGFR decrease is equal or above 25 %, based on the relative eGFR change from baseline. In one embodiment, the dose the patient is receiving is up-titrated, if the eGFR decrease is equal or above 30 %, based on the relative eGFR change from baseline. In one embodiment, the dose the patient is receiving is up-titrated, if the eGFR decrease is equal or above 40 %, based on the relative eGFR change from baseline. In one embodiment, the dose the patient is receiving is up-titrated, if the eGFR decrease is equal or above 50 %, based on the relative eGFR change from baseline. In one embodiment, the dose the patient is receiving is up-titrated, if the eGFR decrease is equal or above 57 %, based on the relative eGFR change from baseline.

In one embodiment, the dose the patient is receiving is up-titrated, if the eGFR decrease is equal or above 25 % to equal or below 57 %, based on the relative eGFR change from baseline. In one embodiment, the dose the patient is receiving is up-titrated, if the eGFR decrease is equal or above 25 % to equal or below 50 %, based on the relative eGFR change from baseline. In one embodiment, the dose the patient is receiving is up-titrated, if the eGFR decrease is equal or above 25 % to equal or below 40 %, based on the relative eGFR change from baseline. In one embodiment, the dose the patient is receiving is up-titrated, if the eGFR decrease is equal or above 25 % to equal or below 30%, based on the relative eGFR change from baseline. In one embodiment, the dose the patient is receiving is up-titrated, if the eGFR decrease is equal or above 30 % to equal or below 57 %, based on the relative eGFR change from baseline. In one embodiment, the dose the patient is receiving is up-titrated, if the eGFR decrease is equal or above 30 % to equal or below 50 %, based on the relative eGFR change from baseline. In one embodiment, the dose the patient is receiving is up-titrated, if the eGFR decrease is equal or above 30 % to equal or below 40 %, based on the relative eGFR change from baseline. In one embodiment, the dose the patient is receiving is up-titrated, if the eGFR decrease is equal or above 40 % to equal or below 57 %, based on the relative eGFR change from baseline. In one embodiment, the dose the patient is receiving is up-titrated, if the eGFR decrease is equal or above 40 % to equal or below 50 %, based on the relative eGFR change from baseline. In one embodiment, the dose the patient is receiving is up-titrated, if the eGFR decrease is equal or above 50 % to equal or below 57 %, based on the relative eGFR change from baseline.

In one embodiment, the dose can be adjusted based on the potassium level and/or the eGFR decrease.

In one embodiment, the patient receives a lower dose of the compound according to formula (I), and
- the potassium level is equal or below 4.5 mmol/L,
and if the eGFR decrease is less than 30 %, based on the relative eGFR change from baseline, then the dose is up-titrated to a higher dose. In this embodiment, the lower dose can be selected from 10 mg and 20 mg; and the higher dos can be selected from 20 mg and 40 mg.

In one embodiment, the patient receives a lower dose of the compound according to formula (I), and
- the potassium level is equal or below 4.5 mmol/L,
and if the eGFR decrease is equal or below 30 %, based on the relative eGFR change from baseline, then the dose is up-titrated to a higher dose stepwise. In this embodiment, the lower dose can be selected from 10 mg and 20 mg; and the higher dos can be selected from 20 mg and 40 mg.

In one embodiment, the patient receives a lower dose of the compound according to formula (I), and
- the potassium level is equal or below 4.5 mmol/L,
and if the eGFR decrease is equal below 30 %, based on the relative eGFR change from baseline, then the dose is up-titrated to the next higher dose. In this embodiment, the lower dose can be selected from 10 mg and 20 mg; and the next higher dos can be selected from 20 mg and 40 mg.

In one embodiment, the patient receives a lower dose of the compound according to formula (I), and
- the potassium level is equal or below 4.8 mmol/L,
and if the eGFR decrease is equal or below 30%, based on the relative eGFR change from baseline, then the dose is up-titrated to a higher dose. In this embodiment, the lower dose can be selected from 10 mg and 20 mg; and the higher dos can be selected from 20 mg and 40 mg.

In one embodiment, the patient receives a lower dose of the compound according to formula (I), and
- the potassium level is equal or below 4.8 mmol/L,
and if the eGFR decrease is equal or below 30 %, based on the relative eGFR change from baseline, then the dose is up-titrated to a higher dose stepwise. In this embodiment, the lower dose can be selected from 10 mg and 20 mg; and the higher dos can be selected from 20 mg and 40 mg.

In one embodiment, the patient receives a lower dose of the compound according to formula (I), and
- the potassium level is equal or below 4.8 mmol/L,
and if the eGFR decrease is equal or below 30%, based on the relative eGFR change from baseline, then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5 mmol/L,
and if the eGFR decrease is equal or below 30%, based on the relative eGFR change from baseline, then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
and if the eGFR decrease is equal or below 30%, based on the relative eGFR change from baseline, then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5.5 mmol/L,
and if the eGFR decrease is equal or below 30%, based on the relative eGFR change from baseline, then the dose is up-titrated to the next higher dose. In this embodiment, the lower dose can be selected from 10 mg and 20 mg; and the next higher dose can be selected from 20 mg and 40 mg.

In one embodiment, the patient receives a dose of 10 mg of the compound according to formula (I), and
- the potassium level is equal or below 4.5 mmol/L,
and if the eGFR decrease is equal or below 30%, based on the relative eGFR change from baseline, then the dose is up-titrated to 20mg.

In one embodiment, the patient receives a dose of 10 mg of the compound according to formula (I), and
- the potassium level is equal or below 4.8 mmol/L,
and if the eGFR decrease is equal or below 30%, based on the relative eGFR change from baseline, then the dose is up-titrated to 20mg.

In one embodiment, the patient receives a dose of 10 mg of the compound according to formula (I), and
- the potassium level is equal or below 5 mmol/L,
and if the eGFR decrease is equal or below 30%, based on the relative eGFR change from baseline, then the dose is up-titrated to 20mg.

In one embodiment, the patient receives a dose of 10 mg of the compound according to formula (I), and
- the potassium level is equal or below 5.5 mmol/L,
and if the eGFR decrease is equal or below 30%, based on the relative eGFR change from baseline, then the dose is up-titrated to 20mg.

In one embodiment, the patient receives a dose of 10 mg of the compound according to formula (I), and
- the potassium level is equal or below 4.5 mmol/L,
and if the eGFR decrease is equal or below 30%, based on the relative eGFR change from baseline, then the dose is up-titrated to 40 mg.

In one embodiment, the patient receives a dose of 10 mg of the compound according to formula (I), and
- the potassium level is equal or below 4.8 mmol/L,
and if the eGFR decrease is equal or below 30%, based on the relative eGFR change from baseline, then the dose is up-titrated to 40 mg.

In one embodiment, the patient receives a dose of 10 mg of the compound according to formula (I), and
- the potassium level is equal or below 5 mmol/L,
and if the eGFR decrease is equal or below 30%, based on the relative eGFR change from baseline, then the dose is up-titrated to 40 mg.

In one embodiment, the patient receives a dose of 10 mg of the compound according to formula (I), and
- the potassium level is equal or below 5.5 mmol/L,
and if the eGFR decrease is equal or below 30%, based on the relative eGFR change from baseline, then the dose is up-titrated to 40 mg.

In one embodiment, the patient receives a dose of 20 mg of the compound according to formula (I), and
- the potassium level is equal or below 4.5 mmol/L,
and if the eGFR decrease is equal or below 30%, based on the relative eGFR change from baseline, then the dose is up-titrated to 40 mg.

In one embodiment, the patient receives a dose of 20 mg of the compound according to formula (I), and
- the potassium level is equal or below 4.8 mmol/L,
and if the eGFR decrease is equal or below 30%, based on the relative eGFR change from baseline, then the dose is up-titrated to 40 mg.

In one embodiment, the patient receives a dose of 20 mg of the compound according to formula (I), and
- the potassium level is equal or below 5 mmol/L,
and if the eGFR decrease is equal or below 30%, based on the relative eGFR change from baseline, then the dose is up-titrated to 40 mg.

In one embodiment, the patient receives a dose of 20 mg of the compound according to formula (I), and
- the potassium level is equal or below 5.5 mmol/L,
and if the eGFR decrease is equal or below 30%, based on the relative eGFR change from baseline, then the dose is up-titrated to 40 mg.

In one embodiment, the patient receives a dose of 10 mg of the compound according to formula (I), and
- the potassium level is equal or below 4.5 mmol/L,
and if the eGFR decrease is equal or below 30%, based on the relative eGFR change from baseline, then the dose is up-titrated to 40 mg as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 4.8 mmol/L,
and if the eGFR decrease is equal or below 30%, based on the relative eGFR change from baseline, then the dose is up-titrated to 40 mg as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 5 mmol/L,
and if the eGFR decrease is equal or below 30%, based on the relative eGFR change from baseline, then the dose is up-titrated to 40 mg as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 5.5 mmol/L,
and if the eGFR decrease is equal or below 30%, based on the relative eGFR change from baseline, then the dose is up-titrated to 40 mg as follows:

| | |
|---|---|
| Step 1 | 10 mg to 20 mg |
| Step 2 | 20 mg to 40 mg. |

In one embodiment, the dose can be adjusted based on the eGFR.

In one embodiment, the patient receives a lower dose and the patient has an eGFR of equal or below 60 mL/min/1.73 m², the dose is up-titrated to a higher dose. In one embodiment, the patient receives a dose of 10 mg and the patient has an equal or below 60 mL/min/1.73 m², then the dose is up-titrated to a dose of 20 mg. In one embodiment, the patient receives a dose of 10 mg and the patient has an equal or below 60 mL/min/1.73 m², then the dose is up-titrated to a dose of 40 mg. In one embodiment, the patient receives a dose of 20 mg and the patient has an equal or below 60 mL/min/1.73 m²,then the dose is up-titrated to a dose of 40 mg.

In one embodiment, the patient receives a lower dose and the patient has an eGFR of equal or above 60 mL/min/1.73 m², the dose is up-titrated to a higher dose. In one embodiment, the patient receives a dose of 10 mg and the patient has an equal or above 60 mL/min/1.73 m², the dose is up-titrated to a higher dose of 20 mg. In one embodiment, the patient receives a dose of 10 mg and the patient has an equal or above 60 mL/min/1.73 m², the dose is up-titrated to a higher dose of 40 mg. In one embodiment, the patient receives a dose of 20 mg and the patient has an equal or above 60 mL/min/1.73 m², the dose is up-titrated to a higher dose of 40 mg.

In one embodiment, the dose is adjusted based on potassium level and/or eGFR.

In one embodiment, the patient receives a lower dose of the compound according to formula (I), and
- the potassium level is equal or below 4.5 mmol/L,
- the patient has an eGFR of equal or below 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.5 mmol/L,
- the patient has an eGFR of equal or below 60 mL/min/1.73 m²
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 4.8 mmol/L,
- the patient has an eGFR of equal or below 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.8 mmol/L,
- the patient has an eGFR of equal or below 60 mL/min/1.73 m²
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5 mmol/L,
- the patient has an eGFR of equal or below 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
- the patient has an eGFR of equal or below 60 mL/min/1.73 m²
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5.5 mmol/L,
- the patient has an eGFR of equal or below 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
- the patient has an eGFR of equal or below 60 mL/min/1.73 m²
then the dose is up-titrated to the next higher dose. In this embodiment, the lower dose can be selected from 10 mg and 20 mg; and the next higher dose can be selected from 20 mg and 40 mg.

In one embodiment, the patient receives a dose of 10 mg of the compound according to formula (I), and
- the potassium level is equal or below 4.5 mmol/L,
- the patient has an eGFR of equal or below 60 mL/min/1.73 m²
then the dose is up-titrated to a dose of 20 mg.

In one embodiment, the patient receives a dose of 10 mg of the compound according to formula (I), and
- the potassium level is equal or below 4.8 mmol/L,
- the patient has an eGFR of equal or below 60 mL/min/1.73 m²
then the dose is up-titrated to a dose of 20 mg.

In one embodiment, the patient receives a dose of 10 mg of the compound according to formula (I), and
- the potassium level is equal or below 5 mmol/L,
- the patient has an eGFR of equal or below 60 mL/min/1.73 m²
then the dose is up-titrated to dose of 20 mg.

In one embodiment, the patient receives a dose of 10 mg of the compound according to formula (I), and
- the potassium level is equal or below 5.5 mmol/L,
- the patient has an eGFR of equal or below 60 mL/min/1.73 m²
then the dose is up-titrated to dose of 20 mg.

In one embodiment, the patient receives a lower dose of the compound according to formula (I), and
- the potassium level is equal or below 4.5 mmol/L,
- the patient has an eGFR of equal or above 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.5 mmol/L,
- the patient has an eGFR of equal or above 60 mL/min/1.73 m²
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.5 mmol/L,
- the patient has an eGFR of equal or above 60 mL/min/1.73 m²
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 4.8 mmol/L,
- the patient has an eGFR of equal or above 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.8 mmol/L,
- the patient has an eGFR of equal or above 60 mL/min/1.73 m²
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.8 mmol/L,
- the patient has an eGFR of equal or above 60 mL/min/1.73 m²
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5 mmol/L,
- the patient has an eGFR of equal or above 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
- the patient has an eGFR of equal or above 60 mL/min/1.73 m²
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
- the patient has an eGFR of equal or above 60 mL/min/1.73 m²
then the dose is up-titrated to the next higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
- the patient has an eGFR of equal or above 60 mL/min/1.73 m²
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
- the patient has an eGFR of equal or above 60 mL/min/1.73 m²
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
- the patient has an eGFR of equal or above 60 mL/min/1.73 m²
then the dose is up-titrated to the next higher dose. In this embodiment, the lower dose can be selected from 10 mg and 20 mg; and the higher dose can be selected from 20 mg and 40 mg.

In one embodiment, the patient receives a dose of 10 mg of the compound according to formula (I), and
- the potassium level is equal or below 4.5 mmol/L,
- the patient has an eGFR of equal or above 60 mL/min/1.73 m²
then the dose is up-titrated to a dose of 20 mg.

In one embodiment, the patient receives a dose of 10 mg of the compound according to formula (I), and
- the potassium level is equal or below 4.8 mmol/L,
- the patient has an eGFR of equal or above 60 mL/min/1.73 m²
then the dose is up-titrated to a dose of 20 mg.

In one embodiment, the patient receives a dose of 10 mg of the compound according to formula (I), and
- the potassium level is equal or below 5 mmol/L,
- the patient has an eGFR of equal or above 60 mL/min/1.73 m²
then the dose is up-titrated to a dose of 20 mg.

In one embodiment, the patient receives a dose of 10 mg of the compound according to formula (I), and
- the potassium level is equal or below 5.5 mmol/L,
- the patient has an eGFR of equal or above 60 mL/min/1.73 m²
then the dose is up-titrated to a dose of 20 mg.

In one embodiment, the patient receives a dose of 10 mg of the compound according to formula (I), and
- the potassium level is equal or below 4.5 mmol/L,
- the patient has an eGFR of equal or above 60 mL/min/1.73 m²
then the dose is up-titrated to a dose of 40 mg.

In one embodiment, the patient receives a dose of 10 mg of the compound according to formula (I), and
- the potassium level is equal or below 4.5 mmol/L,
- the patient has an eGFR of equal or above 60 mL/min/1.73 m²
then the dose is up-titrated to a dose of 40 mg as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 4.8 mmol/L,
- the patient has an eGFR of equal or above 60 mL/min/1.73 m²
then the dose is up-titrated to a dose of 40 mg.

In one embodiment, the patient receives a dose of 10 mg of the compound according to formula (I), and
- the potassium level is equal or below 4.8 mmol/L,
- the patient has an eGFR of equal or above 60 mL/min/1.73 m²
then the dose is up-titrated to a dose of 40 mg as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 5 mmol/L,
- the patient has an eGFR of equal or above 60 mL/min/1.73 m²
then the dose is up-titrated to a dose of 40 mg.

In one embodiment, the patient receives a dose of 10 mg of the compound according to formula (I), and
- the potassium level is equal or below 5 mmol/L,
- the patient has an eGFR of equal or above 60 mL/min/1.73 m²
then the dose is up-titrated to a dose of 40 mg. as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 5.5 mmol/L,
- the patient has an eGFR of equal or above 60 mL/min/1.73 m²
then the dose is up-titrated to a dose of 40 mg.

In one embodiment, the patient receives a dose of 10 mg of the compound according to formula (I), and
- the potassium level is equal or below 5.5 mmol/L,
- the patient has an eGFR of equal or above 60 mL/min/1.73 m²
then the dose is up-titrated to a dose of 40 mg. as follows:

| | |
|---|---|
| Step 1 | 10 mg to 20 mg |
| Step 2 | 20 mg to 40 mg. |

In one embodiment, the patient receives a dose of 20 mg of the compound according to formula (I), and
- the potassium level is equal or below 4.5 mmol/L,
- the patient has an eGFR of equal or above 60 mL/min/1.73 m²
then the dose is up-titrated to a dose of 40 mg.

In one embodiment, the patient receives a dose of 20 mg of the compound according to formula (I), and
- the potassium level is equal or below 4.8 mmol/L,
- the patient has an eGFR of equal or above 60 mL/min/1.73 m²
then the dose is up-titrated to a dose of 40 mg.

In one embodiment, the patient receives a dose of 20 mg of the compound according to formula (I), and
- the potassium level is equal or below 5 mmol/L,
- the patient has an eGFR of equal or above 60 mL/min/1.73 m²
then the dose is up-titrated to a dose of 40 mg.

In one embodiment, the patient receives a dose of 20 mg of the compound according to formula (I), and
- the potassium level is equal or below 5.5 mmol/L,
- the patient has an eGFR of equal or above 60 mL/min/1.73 m²
then the dose is up-titrated to a dose of 40 mg.

In one embodiment, the dose is adjusted based on potassium level, eGFR and/or eGFR decrease.

In one embodiment, the patient receives a lower dose of the compound according to formula (I), and
- the potassium level is equal or below 4.5 mmol/L,
- the patient has an eGFR of equal or above 60 mL/min/1.73 m², and
- the eGFR decrease is equal or below 30%, based on the relative eGFR change from baseline, then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.5 mmol/L,
- the patient has an eGFR of equal or above 60 mL/min/1.73 m², and
- the eGFR decrease is equal or below 30%, based on the relative eGFR change from baseline, then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.5 mmol/L,
- the patient has an eGFR of equal or above 60 mL/min/1.73 m², and
- the eGFR decrease is equal or below 30%, based on the relative eGFR change from baseline,
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 4.8 mmol/L,
- the patient has an eGFR of equal or above 60 mL/min/1.73 m², and
- the eGFR decrease is equal or below 30%, based on the relative eGFR change from baseline,
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.8 mmol/L,
- the patient has an eGFR of equal or above 60 mL/min/1.73 m², and
- the eGFR decrease is equal or below 30%, based on the relative eGFR change from baseline,
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 4.8 mmol/L,
- the patient has an eGFR of equal or above 60 mL/min/1.73 m², and
- the eGFR decrease is equal or below 30%, based on the relative eGFR change from baseline,
then the dose is up-titrated to the next higher dose. In this embodiment, the lower dose can be selected from 10 mg and 20 mg; and the next higher dose can be selected from 20 mg and 40 mg.

In one embodiment, the patient receives a lower dose of the compound according to formula (I), and
- the potassium level is equal or below 5 mmol/L,
- the patient has an eGFR of equal or above 60 mL/min/1.73 m², and
- the eGFR decrease is equal or below 30%, based on the relative eGFR change from baseline,
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
- the patient has an eGFR of equal or above 60 mL/min/1.73 m², and
- the eGFR decrease is equal or below 30%, based on the relative eGFR change from baseline,
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5 mmol/L,
- the patient has an eGFR of equal or above 60 mL/min/1.73 m², and
- the eGFR decrease is equal or below 30%, based on the relative eGFR change from baseline,
then the dose is up-titrated to the next higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
- the patient has an eGFR of equal or above 60 mL/min/1.73 m², and
- the eGFR decrease is equal or below 30%, based on the relative eGFR change from baseline,
then the dose is up-titrated to a higher dose. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
- the patient has an eGFR of equal or above 60 mL/min/1.73 m², and
- the eGFR decrease is equal or below 30%, based on the relative eGFR change from baseline,
then the dose is up-titrated to a higher dose stepwise. In this embodiment, the lower dose can be selected from 10 mg and 20 mg; and the higher dose can be selected from 20 mg and 40 mg.

In one embodiment, the patient receives a lower dose of the compound according to formula (I), and
- the potassium level is equal or below 5.5 mmol/L,
- the patient has an eGFR of equal or above 60 mL/min/1.73 m², and
- the eGFR decrease is equal or below 30%, based on the relative eGFR change from baseline,
then the dose is up-titrated to the next higher dose. In this embodiment, the lower dose can be selected from 10 mg and 20 mg; and the higher dose can be selected from 20 mg and 40 mg.

In one embodiment, the patient receives a dose of 10 mg of the compound according to formula (I), and
- the potassium level is equal or below 4.5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 20 mg.

In one embodiment, the patient receives a dose of 10 mg of the compound according to formula (I), and
- the potassium level is equal or below 4.8 mmol/L,
- the patient has an eGFR of equal or above 60 mL/min/1.73 m², and
- the eGFR decrease is equal or below 30%, based on the relative eGFR change from baseline,
then the dose is up-titrated to a dose of 20 mg.

In one embodiment, the patient receives a dose of 10 mg of the compound according to formula (I), and
- the potassium level is equal or below 5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 20 mg.

In one embodiment, the patient receives a dose of 10 mg of the compound according to formula (I), and
- the potassium level is equal or below 5.5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 20 mg.

In one embodiment, the patient receives a dose of 10 mg of the compound according to formula (I), and
- the potassium level is equal or below 4.5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the patient receives a dose of 10 mg of the compound according to formula (I), and
- the potassium level is equal or below 4.5 mmol/L,
- the patient has an eGFR of equal or above 60 mL/min/1.73 m², and
- the eGFR decrease is equal or below 30%, based on the relative eGFR change from baseline, then the dose is up-titrated to a dose of 40 mg as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 4.8 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the patient receives a dose of 10 mg of the compound according to formula (I), and
- the potassium level is equal or below 4.8 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the patient receives a dose of 10 mg of the compound according to formula (I), and
- the potassium level is equal or below 5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg. as follows:

| | |
|---|---|
| Step 1 | 10 mg to 20 mg |
| Step 2 | 20 mg to 40 mg. |

In one embodiment, the patient receives a dose of 10 mg of the compound according to formula (I), and
- the potassium level is equal or below 5.5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the patient receives a dose of 10 mg of the compound according to formula (I), and
- the potassium level is equal or below 5.5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg. as follows:

| | |
|---|---|
| Step 1 | 10 mg to 20 mg |
| Step 2 | 20 mg to 40 mg. |

In one embodiment, the patient receives a dose of 20 mg of the compound according to formula (I), and
- the potassium level is equal or below 4.5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the patient receives a dose of 20 mg of the compound according to formula (I), and
- the potassium level is equal or below 4.8 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the patient receives a dose of 20 mg of the compound according to formula (I), and
- the potassium level is equal or below 5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the patient receives a dose of 20 mg of the compound according to formula (I), and
- the potassium level is equal or below 5.5 mmol/L,
- the patient has an eGFR of equal or above 60 mL/min/1.73 m², and
- the eGFR decrease is equal or below 30%, based on the relative eGFR change from baseline,
then the dose is up-titrated to a dose of 40 mg.

In one embodiment, the dose the patient is receiving is down-titrated.

In one embodiment, the dose the patient is receiving is down-titrated depending on the potassium level.

In one embodiment, the dose the patient is receiving is down-titrated, if the potassium level is equal or above 5.5, 6, 6.5, 7, 7.5 or 8 mmol/L. In one embodiment, the dose the patient is receiving is down-titrated, if the potassium level is equal or above 5.5 mmol/L. In one embodiment, the dose the patient is receiving is down-titrated, if the potassium level is equal or above 6 mmol/L. In one embodiment, the dose the patient is receiving is down-titrated, if the potassium level is equal or above 6.5 mmol/L. In one embodiment, the dose the patient is receiving is down-titrated, if the potassium level is equal or above 7 mmol/L. In one embodiment, the dose the patient is receiving is down-titrated, if the potassium level is equal or above 7.5 mmol/L. In one embodiment, the dose the patient is receiving is down-titrated, if the potassium level is equal or above 8 mmol/L.

In one embodiment, the dose the patient is receiving is down-titrated, if the potassium level is equal or above 5.5 to 8 mmol/L. In one embodiment, the dose the patient is receiving is down-titrated, if the potassium level is equal or above 5.5 to 7.5 mmol/L. In one embodiment, the dose the patient is receiving is down-titrated, if the potassium level is equal or above 5.5 to 7 mmol/L. In one embodiment, the dose the patient is receiving is down-titrated, if the potassium level is equal or above 5.5 to 6.5 mmol/L. In one embodiment, the dose the patient is receiving is down-titrated, if the potassium level is equal or above 5.5 to 6 mmol/L.

In one embodiment, the patient receives a higher dose of the compound according to formula (I), and
- the potassium level is equal or below 7 mmol/L,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
then the dose is down-titrated to the next lower dose. In this embodiment, the next lower dose can be selected from 10 mg and 20 mg; and the higher dose can be selected from 20 mg and 40 mg.

In one embodiment, the patient receives a dose of the compound according to formula (I) in an amount of 40 mg, and the potassium level is equal or below 7 mmol/L, then the dose is down-titrated to 20 mg. In one embodiment, the patient receives a dose of the compound according to formula (I) in an amount of 40 mg, and the potassium level is equal or below 6.5 mmol/L, then the dose is down-titrated to 20 mg. In one embodiment, the patient receives a dose of the compound according to formula (I) in an amount of 40 mg, and the potassium level is equal or below 6 mmol/L, then the dose is down-titrated to 20 mg. In one embodiment, the patient receives a dose of the compound according to formula (I) in an amount of 40 mg, and the potassium level is equal or below 5.5 mmol/L, then the dose is down-titrated to 20 mg.

In one embodiment, the patient receives a dose of the compound according to formula (I) in an amount of 40 mg, and the potassium level is equal or below 7 mmol/L, then the dose is down-titrated to 10 mg. In one embodiment, the patient receives a dose of the compound according to formula (I) in an amount of 40 mg, and the potassium level is equal or below 6.5 mmol/L, then the dose is down-titrated to 10 mg. In one embodiment, the patient receives a dose of the compound according to formula (I) in an amount of 40 mg, and the potassium level is equal or below 6 mmol/L, then the dose is down-titrated to 10 mg. In one embodiment, the patient receives a dose of the compound according to formula (I) in an amount of 40 mg, and the potassium level is equal or below 5.5 mmol/L, then the dose is down-titrated to 10 mg.

In one embodiment, the patient receives a dose of the compound according to formula (I) in an amount of 20 mg, and the potassium level is equal or below 7 mmol/L, then the dose is down-titrated to 10 mg. In one embodiment, the patient receives a dose of the compound according to formula (I) in an amount of 20 mg, and the potassium level is equal or below 6.5 mmol/L, then the dose is down-titrated to 10 mg. In one embodiment, the patient receives a dose of the compound according to formula (I) in an amount of 20 mg, and the potassium level is equal or below 6 mmol/L, then the dose is down-titrated to 10 mg. In one embodiment, the patient receives a dose of the compound according to formula (I) in an amount of 20 mg, and the potassium level is equal or below 5.5 mmol/L, then the dose is down-titrated to 10 mg.

In one embodiment, the patient receives a dose of the compound according to formula (I) in an amount of 40 mg, and the potassium level is equal or below 7 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, and the potassium level is equal or below 6.5 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, and the potassium level is equal or below 6 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, and the potassium level is equal or below 5.5 mmol/L, then the dose is down-titrated to 10 mg as follows:

| | |
|---|---|
| Step 1 | from 40 mg to 20 mg |
| Step 2 | from 20 mg to 10 mg. |

In one embodiment, the dose the patient is receiving is down-titrated depending eGFR decrease.

In one embodiment, the dose the patient is receiving is down-titrated depending eGFR decrease, based on the relative eGFR change from baseline.

In one embodiment, the dose the patient is receiving is down-titrated, if the eGFR decrease is selected from equal or above 20, 25, 30, 35, 40, 45, 50, 55, and 57 %, based on the relative eGFR change from baseline. In one embodiment, the dose the patient is receiving is down-titrated, if the eGFR decrease is equal or above 25 %, based on the relative eGFR change from baseline. In one embodiment, the dose the patient is receiving is down-titrated, if the eGFR decrease is equal or above 30 %, based on the relative eGFR change from baseline. In one embodiment, the dose the patient is receiving is down-titrated, if the eGFR decrease is equal or above 40 %, based on the relative eGFR change from baseline. In one embodiment, the dose the patient is receiving is down-titrated, if the eGFR decrease is equal or above 50 %, based on the relative eGFR change from baseline. In one embodiment, the dose the patient is receiving is down-titrated, if the eGFR decrease is equal or above 57 %, based on the relative eGFR change from baseline.

In one embodiment, the dose the patient is receiving is down-titrated, if the eGFR decrease is equal or above 25 % to equal or below 57 %, based on the relative eGFR change from baseline. In one embodiment, the dose the patient is receiving is down-titrated, if the eGFR decrease is equal or above 25 % to equal or below 55 %, based on the relative eGFR change from baseline. In one embodiment, the dose the patient is receiving is down-titrated, if the eGFR decrease is equal or above 25 % to equal or below 50 %, based on the relative eGFR change from baseline. In one embodiment, the dose the patient is receiving is down-titrated, if the eGFR decrease is equal or above 25 % to equal or below 45 %, based on the relative eGFR change from baseline. In one embodiment, the dose the patient is receiving is down-titrated, if the eGFR decrease is equal or above 25 % to equal or below 40 %, based on the relative eGFR change from baseline.

In one embodiment, the dose the patient is receiving is down-titrated, if the eGFR decrease is equal or above 25 % to equal or below 35 %, based on the relative eGFR change from baseline. In one embodiment, the dose the patient is receiving is down-titrated, if the eGFR decrease is equal or above 25 % to equal or below 30 %, based on the relative eGFR change from baseline.

In one embodiment, the dose the patient is receiving is down-titrated, if the eGFR decrease is equal or above 30 % to equal or below 57 %, based on the relative eGFR change from baseline. In one embodiment, the dose the patient is receiving is down-titrated, if the eGFR decrease is equal or above 30 % to equal or below 55 %, based on the relative eGFR change from baseline. In one embodiment, the dose the patient is receiving is down-titrated, if the eGFR decrease is equal or above 30 % to equal or below 50 %, based on the relative eGFR change from baseline. In one embodiment, the dose the patient is receiving is down-titrated, if the eGFR decrease is equal or above 30 % to equal or below 45 %, based on the relative eGFR change from baseline. In one embodiment, the dose the patient is receiving is down-titrated, if the eGFR decrease is equal or above 30 % to equal or below 40 %, based on the relative eGFR change from baseline. In one embodiment, the dose the patient is receiving is down-titrated, if the eGFR decrease is equal or above 30 % to equal or below 35 %, based on the relative eGFR change from baseline. In one embodiment, the dose the patient is receiving is down-titrated, if the eGFR decrease is equal or above 35 % to equal or below 57 %, based on the relative eGFR change from baseline. In one embodiment, the dose the patient is receiving is down-titrated, if the eGFR decrease is equal or above 35 % to equal or below 55 %, based on the relative eGFR change from baseline. In one embodiment, the dose the patient is receiving is down-titrated, if the eGFR decrease is equal or above 35 % to equal or below 50 %, based on the relative eGFR change from baseline. In one embodiment, the dose the patient is receiving is down-titrated, if the eGFR decrease is equal or above 35 % to equal or below 45 %, based on the relative eGFR change from baseline. In one embodiment, the dose the patient is receiving is down-titrated, if the eGFR decrease is equal or above 35 % to equal or below 40 %, based on the relative eGFR change from baseline. In one embodiment, the dose the patient is receiving is down-titrated, if the eGFR decrease is equal or above 40 % to equal or below 57 %, based on the relative eGFR change from baseline. In one embodiment, the dose the patient is receiving is down-titrated, if the eGFR decrease is equal or above 40 % to equal or below 55 %, based on the relative eGFR change from baseline. In one embodiment, the dose the patient is receiving is down-titrated, if the eGFR decrease is equal or above 40 % to equal or below 50 %, based on the relative eGFR change from baseline. In one embodiment, the dose the patient is receiving is down-titrated, if the eGFR decrease is equal or above 40 % to equal or below 45 %, based on the relative eGFR change from baseline. In one embodiment, the dose the patient is receiving is down-titrated, if the eGFR decrease is equal or above 45 % to equal or below 57 %, based on the relative eGFR change from baseline. In one embodiment, the dose the patient is receiving is down-titrated, if the eGFR decrease is equal or above 45 % to equal or below 55 %, based on the relative eGFR change from baseline. In one embodiment, the dose the patient is receiving is down-titrated, if the eGFR decrease is equal or above 45 % to equal or below 50 %, based on the relative eGFR change from baseline. In one embodiment, the dose the patient is receiving is down-titrated, if the eGFR decrease is equal or above 50 % to equal or below 57 %, based on the relative eGFR change from baseline. In one embodiment, the dose the patient is receiving is down-titrated, if the eGFR decrease is equal or above 50 % to equal or below 55 %, based on the relative eGFR change from baseline. In one embodiment, the dose the patient is receiving is down-titrated, if the eGFR decrease is equal or above 55 % to equal or below 57 %, based on the relative eGFR change from baseline.

In one embodiment, the dose is adjusted based on the potassium level and/or the eGFR decrease.

In one embodiment, the patient receives a higher dose of the compound according to formula (I), and
- the potassium level is equal or below 7 mmol/L,
- the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a dose of the compound according to formula (I) in an amount of 40 mg, the eGFR decrease is equal or above 30 %, based on the relative eGFR change from baseline, and the potassium level is equal or below 7 mmol/L, then the dose is down-titrated to 20 mg. In one embodiment, the patient receives a dose of the compound according to formula (I) in an amount of 40 mg, the eGFR decrease is equal or above 30 %, based on the relative eGFR change from baseline, and the potassium level is equal or below 6.5 mmol/L, then the dose is down-titrated to 20 mg. In one embodiment, the patient receives a dose of the compound according to formula (I) in an amount of 40 mg, the eGFR decrease is equal or above 30 %, based on the relative eGFR change from baseline, and the potassium level is equal or below 6 mmol/L, then the dose is down-titrated to 20 mg. In one embodiment, the patient receives a dose of the compound according to formula (I) in an amount of 40 mg, the eGFR decrease is equal or above 30 %, based on the relative eGFR change from baseline, and the potassium level is equal or below 5.5 mmol/L, then the dose is down-titrated to 20 mg.

In one embodiment, the patient receives a dose of the compound according to formula (I) in an amount of 40 mg, the eGFR decrease is equal or above 30 %, based on the relative eGFR change from baseline, and the potassium level is equal or below 7 mmol/L, then the dose is down-titrated to 10 mg. In one embodiment, the patient receives a dose of the compound according to formula (I) in an amount of 40 mg, the eGFR decrease is equal or above 30 %, based on the relative eGFR change from baseline, and the potassium level is equal or below 6.5 mmol/L, then the dose is down-titrated to 10 mg. In one embodiment, the patient receives a dose of the compound according to formula (I) in an amount of 40 mg, the eGFR decrease is equal or above 30 %, based on the relative eGFR change from baseline, and the potassium level is equal or below 6 mmol/L, then the dose is down-titrated to 10 mg. In one embodiment, the patient receives a dose of the compound according to formula (I) in an amount of 40 mg, the eGFR decrease is equal or above 30 %, based on the relative eGFR change from baseline, and the potassium level is equal or below 5.5 mmol/L, then the dose is down-titrated to 10 mg.

In one embodiment, the patient receives a dose of the compound according to formula (I) in an amount of 20 mg, the eGFR decrease is equal or above 30 %, based on the relative eGFR change from baseline, and the potassium level is equal or below 7 mmol/L, then the dose is down-titrated to 10 mg. In one embodiment, the patient receives a dose of the compound according to formula (I) in an amount of 20 mg, the eGFR decrease is equal or above 30 %, based on the relative eGFR change from baseline, and the potassium level is equal or below 6.5 mmol/L, then the dose is down-titrated to 10 mg. In one embodiment, the patient receives a dose of the compound according to formula (I) in an amount of 20 mg, the eGFR decrease is equal or above 30 %, based on the relative eGFR change from baseline, and the potassium level is equal or below 6 mmol/L, then the dose is down-titrated to 10 mg. In one embodiment, the patient receives a dose of the compound according to formula (I) in an amount of 20 mg, the eGFR decrease is equal or above 30 %, based on the relative eGFR change from baseline, and the potassium level is equal or below 5.5 mmol/L, then the dose is down-titrated to 10 mg.

In one embodiment, the patient receives a dose of the compound according to formula (I) in an amount of 40 mg, the eGFR decrease is equal or above 30 %, based on the relative eGFR change from baseline, and the potassium level is equal or below 7 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, the eGFR decrease is equal or above 30 %, based on the relative eGFR change from baseline, and the potassium level is equal or below 6.5 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, the eGFR decrease is equal or above 30 %, based on the relative eGFR change from baseline, and the potassium level is equal or below 6 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, the eGFR decrease is equal or above 30 %, based on the relative eGFR change from baseline, and the potassium level is equal or below 5.5 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, the eGFR decrease is equal or above 40 %, based on the relative eGFR change from baseline, and the potassium level is equal or below 7 mmol/L, then the dose is down-titrated to 20 mg. In one embodiment, the patient receives a dose of the compound according to formula (I) in an amount of 40 mg, the eGFR decrease is equal or above 40 %, based on the relative eGFR change from baseline, and the potassium level is equal or below 6.5 mmol/L, then the dose is down-titrated to 20 mg. In one embodiment, the patient receives a dose of the compound according to formula (I) in an amount of 40 mg, the eGFR decrease is equal or above 40 %, based on the relative eGFR change from baseline, and the potassium level is equal or below 6 mmol/L, then the dose is down-titrated to 20 mg. In one embodiment, the patient receives a dose of the compound according to formula (I) in an amount of 40 mg, the eGFR decrease is equal or above 40 %, based on the relative eGFR change from baseline, and the potassium level is equal or below 5.5 mmol/L, then the dose is down-titrated to 20 mg.

In one embodiment, the patient receives a dose of the compound according to formula (I) in an amount of 40 mg, the eGFR decrease is equal or above 40 %, based on the relative eGFR change from baseline, and the potassium level is equal or below 7 mmol/L, then the dose is down-titrated to 10 mg. In one embodiment, the patient receives a dose of the compound according to formula (I) in an amount of 40 mg, the eGFR decrease is equal or above 40 %, based on the relative eGFR change from baseline, and the potassium level is equal or below 6.5 mmol/L, then the dose is down-titrated to 10 mg. In one embodiment, the patient receives a dose of the compound according to formula (I) in an amount of 40 mg, the eGFR decrease is equal or above 40 %, based on the relative eGFR change from baseline, and the potassium level is equal or below 6 mmol/L, then the dose is down-titrated to 10 mg. In one embodiment, the patient receives a dose of the compound according to formula (I) in an amount of 40 mg, the eGFR decrease is equal or above 40 %, based on the relative eGFR change from baseline, and the potassium level is equal or below 5.5 mmol/L, then the dose is down-titrated to 10 mg.

In one embodiment, the patient receives a dose of the compound according to formula (I) in an amount of 20 mg, the eGFR decrease is equal or above 40 %, based on the relative eGFR change from baseline, and the potassium level is equal or below 7 mmol/L, then the dose is down-titrated to 10 mg. In one embodiment, the patient receives a dose of the compound according to formula (I) in an amount of 20 mg, the eGFR decrease is equal or above 40 %, based on the relative eGFR change from baseline, and the potassium level is equal or below 6.5 mmol/L, then the dose is down-titrated to 10 mg. In one embodiment, the patient receives a dose of the compound according to formula (I) in an amount of 20 mg, the eGFR decrease is equal or above 40 %, based on the relative eGFR change from baseline, and the potassium level is equal or below 6 mmol/L, then the dose is down-titrated to 10 mg. In one embodiment, the patient receives a dose of the compound according to formula (I) in an amount of 20 mg, the eGFR decrease is equal or above 40 %, based on the relative eGFR change from baseline, and the potassium level is equal or below 5.5 mmol/L, then the dose is down-titrated to 10 mg.

In one embodiment, the patient receives a dose of the compound according to formula (I) in an amount of 40 mg, the eGFR decrease is equal or above 40 %, based on the relative eGFR change from baseline, and the potassium level is equal or below 7 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, the eGFR decrease is equal or above 40 %, based on the relative eGFR change from baseline, and the potassium level is equal or below 6.5 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, the eGFR decrease is equal or above 40 %, based on the relative eGFR change from baseline, and the potassium level is equal or below 6 mmol/L, then the dose is down-titrated to 10 mg as follows:

| | |
|---|---|
| Step 1 | from 40 mg to 20 mg |
| Step 2 | from 20 mg to 10 mg. |

In one embodiment, the patient receives a dose of the compound according to formula (I) in an amount of 40 mg, the eGFR decrease is equal or above 40 %, based on the relative eGFR change from baseline, and the potassium level is equal or below 5.5 mmol/L, then the dose is down-titrated to 10 mg as follows:

| | |
|---|---|
| Step 1 | from 40 mg to 20 mg |
| Step 2 | from 20 mg to 10 mg. |

In one embodiment, the dose is adjusted based on the potassium level and/or the eGFR.

In one embodiment, the patient receives a higher dose of the compound according to formula (I), and
- the potassium level is equal or below 7 mmol/L,
- the patient has an eGFR of equal or above 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m2, and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m2, and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m2, and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 30 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 30 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 30 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or above 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or above 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or above 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or above 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 7 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6.5 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 6 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 40 %, based on the relative eGFR change from baseline,
then the dose is down-titrated to a lower dose stepwise. In this embodiment, the lower dose can be selected from 10 mg and 20 mg; and the higher dose can be selected from 20 mg and 40 mg.

In one embodiment, the patient receives a higher dose of the compound according to formula (I), and
- the potassium level is equal or below 5.5 mmol/L,
- the patient has an eGFR of equal or below 60 mL/min/1.73 m², and the eGFR decrease is equal or above 40 %, based on the relative eGFR change from baseline,
then the dose is down-titrated to the next lower dose. In this embodiment, the next lower dose can be selected from 10 mg and 20 mg; and the higher dose can be selected from 20 mg and 40 mg.

In one embodiment, the patient receives a dose of 40 mg of the compound according to formula (I), and
- the potassium level is equal or above 5.5 mmol/L,
- the patient has an eGFR of equal or above 60 mL/min/1.73 m²
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or above 60 mL/min/1.73 m²
then the dose is down-titrated to a dose of 10 mg as follows:

| | |
|---|---|
| Step 1 | 40 mg to 20 mg |
| Step 2 | 20 mg to 10 mg. |

In one embodiment, the patient receives a dose of 40 mg of the compound according to formula (I), and
- the potassium level is equal or above 5.5 mmol/L,
- the patient has an eGFR of equal or below 60 mL/min/1.73 m²
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or below 60 mL/min/1.73 m²
then the dose is down-titrated to a dose of 10 mg as follows:

| | |
|---|---|
| Step 1 | 40 mg to 20 mg |
| Step 2 | 20 mg to 10 mg. |

In one embodiment, the patient receives a dose of 40 mg of the compound according to formula (I), and
- the potassium level is equal or above 5.5 mmol/L,
- the patient has an eGFR of equal or above 60 mL/min/1.73 m²
then the dose is down-titrated to a dose of 20 mg.

In one embodiment, the patient receives a dose of 40 mg of the compound according to formula (I), and
- the potassium level is equal or above 5.5 mmol/L,
- the patient has an eGFR of equal or below 60 mL/min/1.73 m²
then the dose is down-titrated to a dose of 20 mg.

In one embodiment, the patient receives a dose of 20 mg of the compound according to formula (I), and
- the potassium level is equal or above 5.5 mmol/L,
- the patient has an eGFR of equal or above 60 mL/min/1.73 m²
then the dose is down-titrated to a dose of 10 mg.

In one embodiment, the patient receives a dose of 20 mg of the compound according to formula (I), and
- the potassium level is equal or above 5.5 mmol/L,
- the patient has an eGFR of equal or below 60 mL/min/1.73 m²
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or above 60 mL/min/1.73 m²,
- the eGFR decrease is more than 30%, based on the relative eGFR change from baseline,
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or above 60 mL/min/1.73 m²,
- the eGFR decrease is more than 30%, based on the relative eGFR change from baseline,
then the dose is down-titrated to a dose of 10 mg as follows:

| | |
|---|---|
| Step 1 | 40 mg to 20 mg |
| Step 2 | 20 mg to 10 mg. |

In one embodiment, the patient receives a dose of 40 mg of the compound according to formula (I), and
- the potassium level is equal or above 5.5 mmol/L,
- the patient has an eGFR of equal or below 60 mL/min/1.73 m²,
- the eGFR decrease is more than 30%, based on the relative eGFR change from baseline,
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or below 60 mL/min/1.73 m²,
- the eGFR decrease is more than 30%, based on the relative eGFR change from baseline,
then the dose is down-titrated to a dose of 10 mg as follows:

| | |
|---|---|
| Step 1 | 40 mg to 20 mg |
| Step 2 | 20 mg to 10 mg. |

In one embodiment, the patient receives a dose of 40 mg of the compound according to formula (I), and
- the potassium level is equal or above 5.5 mmol/L,
- the patient has an eGFR of equal or above 60 mL/min/1.73 m²,
- the eGFR decrease is more than 30%, based on the relative eGFR change from baseline,
then the dose is down-titrated to a dose of 20 mg.

In one embodiment, the patient receives a dose of 40 mg of the compound according to formula (I), and
- the potassium level is equal or above 5.5 mmol/L,
- the patient has an eGFR of equal or below 60 mL/min/1.73 m²,
- the eGFR decrease is more than 30%, based on the relative eGFR change from baseline,
then the dose is down-titrated to a dose of 20 mg.

In one embodiment, the patient receives a dose of 20 mg of the compound according to formula (I), and
- the potassium level is equal or above 5.5 mmol/L,
- the patient has an eGFR of equal or above 60 mL/min/1.73 m²,
- the eGFR decrease is more than 30%, based on the relative eGFR change from baseline,
then the dose is down-titrated to a dose of 10 mg.

In one embodiment, the patient receives a dose of 20 mg of the compound according to formula (I), and
- the potassium level is equal or above 5.5 mmol/L,
- the patient has an eGFR of equal or below 60 mL/min/1.73 m²,
- the eGFR decrease is more than 30%, based on the relative eGFR change from baseline,
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or above 60 mL/min/1.73 m²,
- the eGFR decrease is more than 40%, based on the relative eGFR change from baseline,
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or above 60 mL/min/1.73 m²,
- the eGFR decrease is more than 40%, based on the relative eGFR change from baseline,
then the dose is down-titrated to a dose of 10 mg as follows:

| | |
|---|---|
| Step 1 | 40 mg to 20 mg |
| Step 2 | 20 mg to 10 mg. |

In one embodiment, the patient receives a dose of 40 mg of the compound according to formula (I), and
- the potassium level is equal or above 5.5 mmol/L,
- the patient has an eGFR of equal or below 60 mL/min/1.73 m²,
- the eGFR decrease is more than 40%, based on the relative eGFR change from baseline,
then the dose is down-titrated to a dose of 10 mg.

In one embodiment, the patient receives a dose of 40 mg of the compound according to formula (I), and
- the potassium level is equal or above 5.5 mmol/L,
- the patient has an eGFR of equal or below 60 mL/min/1.73 m²,
- the eGFR decrease is more than 40%, based on the relative eGFR change from baseline,
then the dose is down-titrated to a dose of 10 mg as follows:

| | |
|---|---|
| Step 1 | 40 mg to 20 mg |
| Step 2 | 20 mg to 10 mg. |

In one embodiment, the patient receives a dose of 40 mg of the compound according to formula (I), and
- the potassium level is equal or above 5.5 mmol/L,
- the patient has an eGFR of equal or above 60 mL/min/1.73 m²,
- the eGFR decrease is more than 40%, based on the relative eGFR change from baseline,
then the dose is down-titrated to a dose of 20 mg.

In one embodiment, the patient receives a dose of 40 mg of the compound according to formula (I), and
- the potassium level is equal or above 5.5 mmol/L,
- the patient has an eGFR of equal or below 60 mL/min/1.73 m²,
- the eGFR decrease is more than 40%, based on the relative eGFR change from baseline,
then the dose is down-titrated to a dose of 20 mg.

In one embodiment, the patient receives a dose of 20 mg of the compound according to formula (I), and
- the potassium level is equal or above 5.5 mmol/L,
- the patient has an eGFR of equal or above 60 mL/min/1.73 m²,
- the eGFR decrease is more than 40%, based on the relative eGFR change from baseline,
then the dose is down-titrated to a dose of 10 mg.

In one embodiment, the patient receives a dose of 20 mg of the compound according to formula (I), and
- the potassium level is equal or above 5.5 mmol/L,
- the patient has an eGFR of equal or below 60 mL/min/1.73 m²,
- the eGFR decrease is more than 40%, based on the relative eGFR change from baseline,
then the dose is down-titrated to a dose of 10 mg.

In one embodiment, the patient receives a dose of the compound according to formula (I) in an amount of between 0,5 to 80 mg, the eGFR decrease is equal or above 30 %, based on the relative eGFR change from baseline, then the dose is down-titrated. The dose can be down-titrated directly or stepwise.

In one embodiment, the patient receives a dose of the compound according to formula (I) in an amount of between 0,5 to 80 mg, the eGFR decrease is equal or above 25 % to equal or below 40 %, based on the relative eGFR change from baseline, then the dose is down-titrated. The dose can be down-titrated directly or stepwise.

In one embodiment, the patient receives a dose of the compound according to formula (I) in an amount of between 0,5 to 80 mg, the eGFR decrease is equal or above 40 %, based on the relative eGFR change from baseline, then the dose is down-titrated. The dose can be down-titrated directly or stepwise.

In one embodiment, the patient receives a dose of the compound according to formula (I) in an amount of between 0,5 to 80 mg, the eGFR decrease is equal or above 30 %, based on the relative eGFR change from baseline, then the treatment is interrupted.

In one embodiment, the patient receives a dose of the compound according to formula (I) in an amount of between 0,5 to 80 mg, the eGFR decrease is equal or above 25 % to equal or below 40 %, based on the relative eGFR change from baseline, then the treatment is interrupted.

In one embodiment, the patient receives a dose of the compound according to formula (I) in an amount of between 0,5 to 80 mg, the eGFR decrease is equal or above 40 %, based on the relative eGFR change from baseline, then the treatment is interrupted.

In one embodiment, the patient receives a dose of the compound according to formula (I) in an amount of 20 mg, 30 mg or 40 mg, the eGFR decrease is equal or above 30 %, based on the relative eGFR change from baseline, then the dose is down-titrated. The dose can be down-titrated directly or stepwise to 10 mg, 20 mg or 30 mg.

In one embodiment, the patient receives a dose of the compound according to formula (I) in an amount of 20 mg, 30 mg or 40 mg, the eGFR decrease is equal or above 25 % to equal or below 40 %, based on the relative eGFR change from baseline, then the dose is down-titrated. The dose can be down-titrated directly or stepwise to 10 mg, 20 mg or 30 mg.

In one embodiment, the patient receives a dose of the compound according to formula (I) in an amount of 20 mg, 30 mg or 40 mg, the eGFR decrease is equal or above 40 %, based on the relative eGFR change from baseline, then the dose is down-titrated. The dose can be down-titrated directly or stepwise to 10 mg, 20 mg or 30 mg.

In one embodiment, the patient receives a dose of the compound according to formula (I) in an amount of 20 mg, 30 mg or 40 mg, the eGFR decrease is equal or above 30 %, based on the relative eGFR change from baseline, then the treatment is interrupted.

In one embodiment, the patient receives a dose of the compound according to formula (I) in an amount of 20 mg, 30 mg or 40 mg, the eGFR decrease is equal or above 25 % to equal or below 40 %, based on the relative eGFR change from baseline, then the treatment is interrupted.

In one embodiment, the patient receives a dose of the compound according to formula (I) in an amount of 20 mg, 30 mg or 40 mg, the eGFR decrease is equal or above 40 %, based on the relative eGFR change from baseline, then the treatment is interrupted.

In one embodiment, the patient receives a dose of the compound according to formula (I) in an amount of between 0,5 to 80 mg, the eGFR decrease is equal or above 25 % to equal or below 40 %, based on the relative eGFR change from baseline, and receives at least one co-medication, then the dose is down-titrated. The dose can be down-titrated directly or stepwise. Comedications are described above in detail.

In one embodiment, the patient receives a dose of the compound according to formula (I) in an amount of between 0,5 to 80 mg, the eGFR decrease is equal or above 40 %, based on the relative eGFR change from baseline, and receives at least one co-medication, then the dose is down-titrated. The dose can be down-titrated directly or stepwise. Comedications are described above in detail.

In one embodiment, the patient receives a dose of the compound according to formula (I) in an amount of between 0.5 to 80 mg, the eGFR decrease is equal or above 30 %, based on the relative eGFR change from baseline, and receives at least one comedication, then the treatment is interrupted. Comedications are described above in detail. In one embodiment thereof, the comedication is selected from loop diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), glucagon-like peptide-1 (GLP-1) agonists, CYP3A4 inducers, CYP3A4 inhibitors and mixtures thereof.

In one embodiment, an amount of the compound according to formula (I)the patient receives an amount of the compound according to formula (I) in an amount of between 0.5 to 80 mg, the eGFR decrease is equal or above 25 % to equal or below 40 %, based on the relative eGFR change from baseline, and receives at least one comedication, then the treatment is interrupted. In one embodiment thereof, the comedication is selected from loop diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), glucagon-like peptide-1 (GLP-1) agonists, CYP3A4 inducers, CYP3A4 inhibitors and mixtures thereof.

In one embodiment, an amount of the compound according to formula (I)the patient receives an amount of the compound according to formula (I) in an amount of between 0.5 to 80 mg, the eGFR decrease is equal or above 40 %, based on the relative eGFR change from baseline, and receives at least one comedication, then the treatment is interrupted. In one embodiment thereof, the comedication is selected from loop diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), glucagon-like peptide-1 (GLP-1) agonists, CYP3A4 inducers, CYP3A4 inhibitors and mixtures thereof.

In the following further aspects of the disclosure are described. The embodiments disclosed above also apply to these embodiments.

In the following further embodiments of dose managements or adjustments are described:
In one embodiment, at least one sample of the patient is taken and the dose the patient is receiving as follows:
- FIRST SAMPLE:
   ∘ potassium level is <5 mmol/L: Continue the current dose level (maintain dose the patient is receiving) or increase to the next higher dose
   ∘ potassium level is ≥5 to <5.5 mmol/L: Continue the current dose level (maintain dose the patient is receiving)
   ∘ potassium level is ≥5.5 to <6 mmol/L: Down-titrate to the next lower dose
   ∘ potassium level is ≥6 mmol/L: Interrupt treatment.

The dose the patient is receiving can be selected from 10, 20, 30 and 40 mg. In one embodiment the dose the patient is receiving is 10 mg. In one embodiment, the dose the patient is receiving is 20 mg. In one embodiment the dose the patient is receiving is 30 mg. In one embodiment the dose the patient is receiving is 40 mg. The "higher dose" can be selected from 10, 20, 30, and 40 mg. In one embodiment, the "higher dose" is 20 mg. In one embodiment, the "higher dose" is 30 mg. In one embodiment, the "higher dose" is 40 mg. The "lower dose" can be selected from 10, 20, 30, and 40 mg. In one embodiment, the "lower dose" is 20 mg. In one embodiment, the "lower dose" is 30 mg. In one embodiment, the "lower dose" is 40 mg.

In one embodiment, at least two samples of the patient are taken and the dose the patient is receiving as follows:
- FIRST SAMPLE:
   ∘ potassium level is <5 mmol/L: Increase to the next higher dose
   ∘ potassium level is ≥5 to <5.5 mmol/L: Continue the current dose level (maintain dose the patient is receiving)
   ∘ potassium level is ≥5.5 to <6 mmol/L: Down-titrate to the next lower dose
   ∘ potassium level is ≥6 mmol/L: Interrupt treatment
- SECOND SAMPLE:
   ∘ potassium level is <5.5 mmol/L: Continue current dose
   ∘ potassium level is ≥5.5 mmol/L: Down-titrate to the next lower dose if possible, or interrupt study intervention and recheck potassium level

The dose the patient is receiving can be selected from 10, 20, 30 and 40 mg. In one embodiment the dose the patient is receiving is 10 mg. In one embodiment, the dose the patient is receiving is 20 mg. In one embodiment the dose the patient is receiving is 30 mg. In one embodiment the dose the patient is receiving is 40 mg. The "higher dose" can be selected from 10, 20, 30, and 40 mg. In one embodiment, the "higher dose" is 20 mg. In one embodiment, the "higher dose" is 30 mg. In one embodiment, the "higher dose" is 40 mg. The "lower dose" can be selected from 10, 20, 30, and 40 mg. In one embodiment, the "lower dose" is 20 mg. In one embodiment, the "lower dose" is 30 mg. In one embodiment, the "lower dose" is 40 mg.

In one embodiment, at least two samples of the patient are taken and the dose the patient is receiving as follows:
- FIRST SAMPLE:
   ∘ potassium level is <5 mmol/L: Increase to the next higher dose
   ∘ potassium level is ≥5 to <5.5 mmol/L: Continue the current dose level (maintain dose the patient is receiving)
   ∘ potassium level is ≥5.5 to <6 mmol/L: Down-titrate to the next lower dose
   ∘ potassium level is ≥6 mmol/L: Interrupt treatment

- SECOND SAMPLE:
   ∘ potassium level is <5.5 mmol/L: Restart
   ∘ potassium level is ≥5.5 mmol/L: Continue to withhold intervention, further monitoring of potassium level.

The dose the patient is receiving can be selected from 10, 20, 30 and 40 mg. In one embodiment the dose the patient is receiving is 10 mg. In one embodiment, the dose the patient is receiving is 20 mg. In one embodiment the dose the patient is receiving is 30 mg. In one embodiment the dose the patient is receiving is 40 mg. The "higher dose" can be selected from 10, 20, 30, and 40 mg. In one embodiment, the "higher dose" is 20 mg. In one embodiment, the "higher dose" is 30 mg. In one embodiment, the "higher dose" is 40 mg. The "lower dose" can be selected from 10, 20, 30, and 40 mg. In one embodiment, the "lower dose" is 20 mg. In one embodiment, the "lower dose" is 30 mg. In one embodiment, the "lower dose" is 40 mg. The dose with which the treatment is restarted can be selected from 10 mg, 20 mg and 40 mg. In one embodiment, the dose with which the treatment is restarted is 10 mg. In one embodiment, the dose with which the treatment is restarted is 20 mg. In one embodiment, the dose with which the treatment is restarted is 30 mg. In one embodiment, the dose with which the treatment is restarted is 40 mg.

In one embodiment, at least two samples of the patient are taken and the dose the patient is receiving as follows:
- FIRST SAMPLE:
   ∘ potassium level is <5 mmol/L: Increase to the next higher dose
   ∘ potassium level is ≥5 to <5.5 mmol/L: Continue the current dose level (maintain dose the patient is receiving)
   ∘ potassium level is ≥5.5 to <6 mmol/L: Down-titrate to the next lower dose
   ∘ potassium level is ≥6 mmol/L: Interrupt treatment
- SECOND SAMPLE:
   ∘ potassium level is <5.5 mmol/L: Restart
   ∘ potassium level is ≥5.5 mmol/L: Continue to withhold intervention, further monitoring of potassium level. Restart, if potassium level is <5.0 mmol/L

The dose the patient is receiving can be selected from 10, 20, 30 and 40 mg. In one embodiment the dose the patient is receiving is 10 mg. In one embodiment, the dose the patient is receiving is 20 mg. In one embodiment the dose the patient is receiving is 30 mg. In one embodiment the dose the patient is receiving is 40 mg. The "higher dose" can be selected from 10, 20, 30, and 40 mg. In one embodiment, the "higher dose" is 20 mg. In one embodiment, the "higher dose" is 30 mg. In one embodiment, the "higher dose" is 40 mg. The "lower dose" can be selected from 10, 20, 30, and 40 mg. In one embodiment, the "lower dose" is 20 mg. In one embodiment, the "lower dose" is 30 mg. In one embodiment, the "lower dose" is 40 mg. In one embodiment, the "lower dose" is 40 mg. The dose with which the treatment is restarted can be selected from 10 mg, 20 mg and 40 mg. In one embodiment, the dose with which the treatment is restarted is 10 mg. In one embodiment, the dose with which the treatment is restarted is 20 mg. In one embodiment, the dose with which the treatment is restarted is 30 mg. In one embodiment, the dose with which the treatment is restarted is 40 mg.

In the following, further embodiments of dose adjustment based on the eGFR decrease are disclosed:
In case the eGFR (mL/min/1.73 m²) decrease is between ≥25% and <40%, potential reversible cause can be checked, e.g.
   - Concomitant medications known to affect renal function (e.g. NSAIDs, antibiotics),
   - Adverse event (e.g. urinary infection, urinary retention, dehydration), and/or
   - Further potential reversible causes if considered clinically appropriate.
In case the eGFR (mL/min/1.73 m²) decrease is ≥40%, potential reversible cause can be checked, as mentioned above, e.g.
   - Concomitant medications known to affect renal function (e.g. non-steroidal anti-inflammatory drug (NSAIDs), antibiotics),
   - Adverse event (e.g. urinary infection, urinary retention, dehydration), and/or
   - Further potential reversible causes if considered clinically appropriate.
In case the eGFR (mL/min/1.73 m²) decrease is ≥40%, the dose the patient is receiving can be adjusted or the treatment can be interrupted. In case the eGFR (mL/min/1.73 m²) decrease is ≥40%, the dose the patient is receiving can be adjusted or the treatment can be interrupted and the following can be checked, e.g.:
   - Further monitor eGFR/creatinine
   - If eGFR/creatinine has reached acceptable levels (to be determined for the individual participant), restart study intervention at the next lower dose level (or dose level 1 if the participant was already on this dose), and/or
   - Re-test at central laboratory after 4 weeks to confirm eGFR decrease of ≥50% or ≥57%.

In one embodiment, the patient
- receives a dose selected from 10, 20, 30, and 40 mg, and
- eGFR (mL/min/1.73 m²) decrease is ≥40%,
then the dose is down-titrated.

In one embodiment, the dose is adjusted as follows:
Step (1):
   - the patient receives a dose selected from 10, 20, 30, and 40 mg, and
   - eGFR (mL/min/1.73 m²) decrease is ≥40%,
Step (2):
   - down-titrate.

Many embodiments of down-titration, doses, restart and interruption are described in detail above and can be applied to this embodiment.

In one embodiment, the dose is adjusted as follows:
Step (1):
   - the patient receives a dose selected from 10, 20, 30, and 40 mg, and
   - eGFR (mL/min/1.73 m²) decrease is ≥40%,
Step (2):
   - down-titrate as follows:
      ∘ monitor eGFR/creatinine;
      ∘ If eGFR/creatinine has reached acceptable levels (to be determined for the individual), and/or
      ∘ Re-test to confirm eGFR decrease of ≥50% or ≥57%.

Many embodiments of down-titration, doses, restart and interruption are described in detail above and can be applied to this embodiment.

In one embodiment, the dose is adjusted as follows:
Step (1):
   - the patient receives a dose selected from 10, 20, 30, and 40 mg, and
   - eGFR (mL/min/1.73 m²) decrease is ≥40%,
Step (2):
   - interrupted as follows:
      ∘ monitor eGFR/creatinine;
      ∘ If eGFR/creatinine has reached acceptable levels (to be determined for the individual), re-start study intervention at the next lower dose (or lowest dose level, if the participant was already on this dose), and/or
      ∘ Re-test to confirm eGFR decrease of ≥50% or ≥57%.

Many embodiments of down-titration, doses, restart and interruption are described in detail above and can be applied to this embodiment.

In one embodiment, the dose is adjusted as follows:
Step (1):
   - the patient receives a dose selected from 10, 20, 30, and 40 mg, and
   - eGFR (mL/min/1.73 m²) decrease is ≥40%,
Step (2):
   - down-titrated or interrupted as follows:
      ∘ monitor eGFR/creatinine;
      ∘ If eGFR/creatinine has reached acceptable levels (to be determined for the individual), re-start study intervention at the next lower dose (or lowest dose level, if the participant was already on this dose), and/or
      ∘ Re-test to confirm eGFR decrease of ≥50% or ≥57%.

Many embodiments of down-titration, doses, restart and interruption are described in detail above and can be applied to this embodiment.

The dose can be adapted based on the type of heart failure, the left ventricular ejection fraction (LVEF), the Urine Albumin to Creatinine Ratio (UACR), the sodium level, the urea level, the N-terminal prohormone of brain natriuretic peptide (NT-proBNP) level, KDIGO stage, chloride level, uric acid, hematologic biomarkers, cardiac biomarkers and/or the medicaments the patient receives. Embodiments of these examples are described in detail above and further below as well as in the examples below.

In one embodiment, the amount of the compound according to formula (I) can be adjusted based on the comedication.

In one embodiment, the amount of the compound according to formula (I) is adjusted based on the comedication with a CYP3A4 inducer. CYP3A4 inducers are disclosed in detail including specific examples above. The amount of the compound according to formula (I) can be based on the amount and/or type of the CYP3A4 inducer. Adjustments, including down-titration, up-titration, continuation of the dose or interruption of treatment are disclosed in detail and in many embodiments above.

In one embodiment, the patient receives
- a CYP3A4 inducer and
- the compound according to formula (I),
wherein The amount of the compound according to formula (I) is adjusted by up-titration. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of between 0.5 mg to below 80 mg,
wherein The amount of the compound according to formula (I) is adjusted by up-titration to a dose of between 5 to 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 5 to below 80 mg,
wherein The amount of the compound according to formula (I) is adjusted by up-titration to a dose of between above 5 to 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount selected from 5 mg, 10 mg, 20 mg, 30 mg, 40 mg and 60 mg,
wherein the dose is adjusted by up-titration to a dose selected from 10 mg, 20 mg, 30 mg, 40 mg, 60 mg, and 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount selected from 5 mg, 10 mg, 20 mg, and 30 mg,
wherein the dose is adjusted by up-titration to a dose selected from 10 mg, 20 mg, 30 mg and 40 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 5 mg,
wherein the dose is adjusted by up-titration to a dose selected from 10 mg, 20 mg, 30 mg, 40mg, 60 mg, and 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 5 mg,
wherein the dose is adjusted by up-titration to a dose selected from 10 mg, 20 mg, 30 mg and 40 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 10 mg,
wherein the dose is adjusted by up-titration to a dose selected from 20 mg, 30 mg, 40mg, 60 mg, and 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 10 mg,
wherein the dose is adjusted by up-titration to a dose selected from 20 mg, 30 mg and 40 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 20 mg,
wherein the dose is adjusted by up-titration to a dose selected from 30 mg, 40mg, 60 mg, and 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 20 mg,
wherein the dose is adjusted by up-titration to a dose selected from 30 mg and 40 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 40 mg,
wherein the dose is adjusted by up-titration to a dose selected from 60 mg, and 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 60 mg,
wherein the dose is adjusted by up-titration to a dose of 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

În one embodiment, the patient receives
- a CYP3A4 inducer and
- the compound according to formula (I),
wherein The amount of the compound according to formula (I) is adjusted by down-titration. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of between 5 mg to 80 mg,
wherein The amount of the compound according to formula (I) is adjusted by down-titration to a dose of between 0.5 to below 80 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount selected from 10 mg, 20 mg, 30 mg, 40 mg, 60 mg and 80 mg,
wherein the dose is adjusted by down-titration to a dose selected from 5 mg, 10 mg, 20 mg, 30 mg, 40 mg, and 60 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount selected from 10 mg, 20 mg, 30 mg and 40 mg,
wherein the dose is adjusted by down-titration to a dose selected from 5 mg, 10 mg, 20 mg, and 30 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 10 mg,
wherein the dose is adjusted by down-titration to a dose of 5mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 20 mg,
wherein the dose is adjusted by down-titration to a dose selected from 5 mg, and 10 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 40 mg,
wherein the dose is adjusted by down-titration to a dose selected from 5 mg, 10 mg, 20 mg and 30 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 60 mg,
wherein the dose is adjusted by down-titration to a dose of 5 mg, 10 mg, 20 mg, 30 mg and 40 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, the patient receives
- a CYP3A4 inducer and
- the compound according to formula (I) in an amount of 80 mg,
wherein the dose is adjusted by down-titration to a dose selected from 5 mg, 10 mg, 20 mg, 30 mg, 40 mg, and 60 mg. In one embodiment thereof, the CYP3A4 inducer is a strong CYP3A4 inducer. In one embodiment thereof, the CYP3A4 inducer is a moderate CYP3A4 inducer. In one embodiment thereof, the CYP3A4 inducer is a weak CYP3A4 inducer. In one embodiment thereof, the CYP3A4 inducer is a CYP3A4 inducer of unspecified potency.

In one embodiment, The amount of the compound according to formula (I) is adjusted based on the comedication with a CYP3A4 inhibitor. CYP3A4 inhibitors are disclosed in detail including specific examples above. The amount of the compound according to formula (I) can be based on the amount and/or type of the CYP3A4 inhibitor. Adjustments, including down-titration, up-titration, continuation of the dose or interruption of treatment are disclosed in detail and in many embodiments above.

In one embodiment, the patient receives
- a CYP3A4 inhibitor and
- the compound according to formula (I),
wherein The amount of the compound according to formula (I) is adjusted by up-titration. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of between 0.5 mg to below 80 mg,
wherein The amount of the compound according to formula (I) is adjusted by up-titration to a dose of between 5 to 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 5 to below 80 mg,
wherein The amount of the compound according to formula (I) is adjusted by up-titration to a dose of between above 5 to 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount selected from 5 mg, 10 mg, 20 mg, 30 mg, 40 mg and 60 mg,
wherein the dose is adjusted by up-titration to a dose selected from 10 mg, 20 mg, 30 mg, 40 mg, 60 mg, and 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount selected from 5 mg, 10 mg, 20 mg, and 30 mg,
wherein the dose is adjusted by up-titration to a dose selected from 10 mg, 20 mg, 30 mg and 40 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 5 mg,
wherein the dose is adjusted by up-titration to a dose selected from 10 mg, 20 mg, 30 mg, 40mg, 60 mg, and 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 5 mg,
wherein the dose is adjusted by up-titration to a dose selected from 10 mg, 20 mg, 30 mg and 40 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 10 mg,
wherein the dose is adjusted by up-titration to a dose selected from 20 mg, 30 mg, 40mg, 60 mg, and 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 10 mg,
wherein the dose is adjusted by up-titration to a dose selected from 20 mg, 30 mg and 40 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 20 mg,
wherein the dose is adjusted by up-titration to a dose selected from 30 mg, 40mg, 60 mg, and 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 20 mg,
wherein the dose is adjusted by up-titration to a dose selected from 30 mg and 40 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 40 mg,
wherein the dose is adjusted by up-titration to a dose selected from 60 mg, and 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 60 mg,
wherein the dose is adjusted by up-titration to a dose of 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

În one embodiment, the patient receives
- a CYP3A4 inhibitor and
- the compound according to formula (I),
wherein The amount of the compound according to formula (I) is adjusted by down-titration. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of between 5 mg to 80 mg,
wherein The amount of the compound according to formula (I) is adjusted by down-titration to a dose of between 0.5 to below 80 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount selected from 10 mg, 20 mg, 30 mg, 40 mg, 60 mg and 80 mg,
wherein the dose is adjusted by down-titration to a dose selected from 5 mg, 10 mg, 20 mg, 30 mg, 40 mg, and 60 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount selected from 10 mg, 20 mg, 30 mg and 40 mg,
wherein the dose is adjusted by down-titration to a dose selected from 5 mg, 10 mg, 20 mg, and 30 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 10 mg,
wherein the dose is adjusted by down-titration to a dose of 5mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 20 mg,
wherein the dose is adjusted by down-titration to a dose selected from 5 mg, and 10 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 40 mg,
wherein the dose is adjusted by down-titration to a dose selected from 5 mg, 10 mg, 20 mg and 30 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 60 mg,
wherein the dose is adjusted by down-titration to a dose of 5 mg, 10 mg, 20 mg, 30 mg and 40 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

In one embodiment, the patient receives
- a CYP3A4 inhibitor and
- the compound according to formula (I) in an amount of 80 mg,
wherein the dose is adjusted by down-titration to a dose selected from 5 mg, 10 mg, 20 mg, 30 mg, 40 mg, and 60 mg. In one embodiment thereof, the CYP3A4 inhibitor is a strong CYP3A4 inhibitor. In one embodiment thereof, the CYP3A4 inhibitor is a moderate CYP3A4 inhibitor. In one embodiment thereof, the CYP3A4 inhibitor is a weak CYP3A4 inhibitor. In one embodiment thereof, the CYP3A4 inhibitor is a CYP3A4 inhibitor of unspecified potency.

The dose the patient receives can be adjusted based on the patient data. The patient data can comprise
- data indicating the disease the patient is suffering from and/or what stage of the disease the patient is in and/or what symptoms the patient is suffering from and/or how severe one or more symptoms are, and/or
- information about the medical history of the patient, including information about pre-existing diseases, hospitalizations, and/or genetic background, and/or
- information about patient's medications, including medical intervention parameters such as regular medication, occasional medication, and/or other previous or current medical interventions and/or any adverse reactions to medications, and/or
- demographic data, including age, sex, ethnicity, and/or other/further personal information about the patient,
- results from physical examinations on the patient, and/or laboratory tests, including body size, body weight, body mass index, fat percentage of the body, muscle percentage of the body, water content of the body, resting heart rate, heart rate variability, sugar concentration in urine, body temperature, thoracic impedance, systolic and/or diastolic arterial peripheral blood pressure, glomerular filtration rate, estimated glomerular filtration rate, urine albumin-to-creatinine ratio, creatinine clearance, blood sugar, blood oxygen saturation, erythrocyte count, hemoglobin content, leukocyte count, platelet count, inflammation values, blood lipids including low-density lipoprotein cholesterol and/or high-density lipoprotein cholesterol (HDL), ions including Na+, K+ and/or corrected calcium, and/or
- information about the patient's condition obtained from the patient himself/herself.

Embodiments of these examples are described in detail above.

A further aspect of the disclosure is a method of reducing the risk of cardiovascular death in a patient, comprising administering to the patient a therapeutically effective amount of the compound (I) 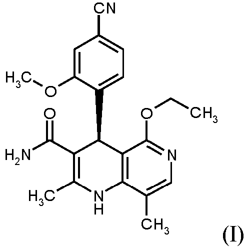 or a hydrate, solvate, pharmaceutically acceptable salt thereof, or a polymorph thereof.

A further aspect of the disclosure is a method of improving symptoms of heart failure in a patient, comprising administering to the patient a therapeutically effective amount of the compound (I) 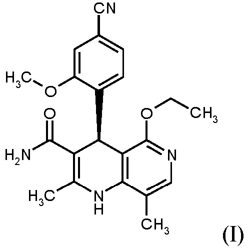 or a hydrate, solvate, pharmaceutically acceptable salt thereof, or a polymorph thereof.

A further aspect of the disclosure is a method of preventing worsening of symptoms of heart failure in a patient, comprising administering to the patient a therapeutically effective amount of the compound (I) 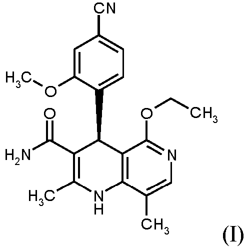 or a hydrate, solvate, pharmaceutically acceptable salt thereof, or a polymorph thereof.

A further aspect of the disclosure is a method of reducing the risk of hospitalization for heart failure in a patient, comprising administering to the patient a therapeutically effective amount of the compound (I) 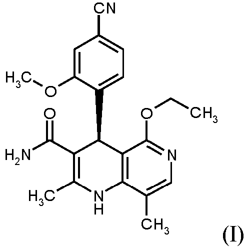 or a hydrate, solvate, pharmaceutically acceptable salt thereof, or a polymorph thereof.

A further aspect of the disclosure is a method for treating or preventing symptomatic heart failure with left ventricular ejection fraction (LVEF) of equal or above 40%, in a patient, comprising administering to the patient a therapeutically effective amount of the compound (I) 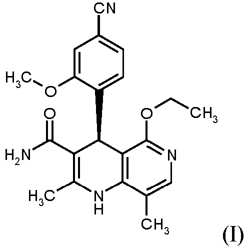 or a hydrate, solvate, pharmaceutically acceptable salt thereof, or a polymorph thereof.

A further aspect of the disclosure is a method of reducing the risk of cardiovascular death in patients with heart failure with left ventricular ejection fraction (LVEF) of equal or above 40%, in a patient, comprising administering to the patient a therapeutically effective amount of the compound (I) 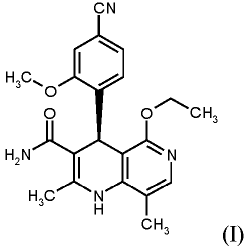 or a hydrate, solvate, pharmaceutically acceptable salt thereof, or a polymorph thereof.

A further aspect of the disclosure is a method of reducing the risk of hospitalization for heart failure in patients with heart failure with left ventricular ejection fraction (LVEF) of equal or above 40%, in a patient, comprising administering to the patient a therapeutically effective amount of the compound (I) 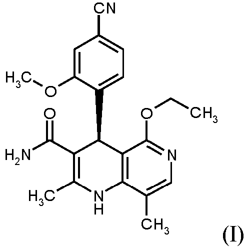 or a hydrate, solvate, pharmaceutically acceptable salt thereof, or a polymorph thereof.

A further aspect of the disclosure is a method of reducing the risk urgent heart failure visits in patients with heart failure with left ventricular ejection fraction (LVEF) of ≥ 40%, in a patient, comprising administering to the patient a therapeutically effective amount of the compound (I) 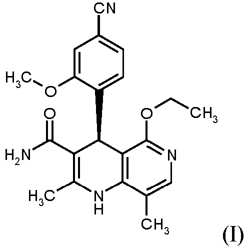 or a hydrate, solvate, pharmaceutically acceptable salt thereof, or a polymorph thereof.

The disclosure also refers to a pharmaceutical composition comprising the compound according to formula (I) 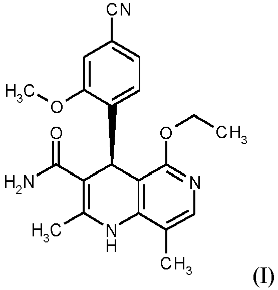 or a hydrate, solvate, pharmaceutically acceptable salt thereof, or a polymorph thereof that can be used in the methods of treatment as described in detail above. The disclosure also refers to a pharmaceutical composition comprising a therapeutically effective amount of the compound (I) 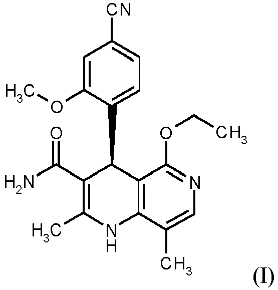 or a hydrate, solvate, pharmaceutically acceptable salt thereof, or a polymorph thereof that can be used in the methods of treatment as described in detail above.

Pharmaceutical compositions are known to the person skilled in the art. These can be liquid or solid formulations for example without limitation tablets, coated tablets, capsules, pills, powders, granules, elixirs, tinctures, solution, suspensions, syrups, solid and liquid aerosols and emulsions. Typical pharmaceutical formulation, but not limited to, are tablets and capsules. These can be used in an intermediate release form or a retard form. Such pharmaceutical formulations are known by the skilled person.

In one embodiment the pharmaceutical composition is a capsule. In one embodiment thereof, the capsule is an intermediate release capsule. In one embodiment thereof, the capsule is a retard formulation. In one embodiment the pharmaceutical composition is a tablet. In one embodiment thereof, the tablet is a intermediate release tablet. In one embodiment thereof, the tablet is a retard formulation.

In another embodiment, the compound according to formula (I) can be tableted with conventional tablet bases such as lactose, sucrose and corn starch in combination with binders such as acacia, corn starch or gelatine, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials can be present as coatings or to otherwise modify the physical form

The disclosure further relates to medicaments which comprise at least one compound according to formula (I), typically together with one or more inert, non-toxic, pharmaceutically suitable excipients, and to the use thereof for the aforementioned purposes.

### Figures:

| | | |
|---|---|---|
| Figure 1 | Schematic overview: Example 2 designed to evaluate the efficacy and safety of finerenone in patients with HF and LVEF ≥40%, with or without diabetes, and across a broad range of renal function | |
| Figure 2 | Example 2 trial, participants with HFmref or HFpEF Cardo-Renal-Metabolic-Overlap | |
| Figure 3 | Eligibility Flowchart | |
| Figure 4 | Cardio-Kidney-Metabolic Overlap FINE-HEART (Example 9). | |
| Figure 5 | Recency of Worsening HF Events Before Randomization in Example 2 (FINEARTS-HF): During worsening HF event:n=749 (12%) | |
| | 7 days or less: n=470 (8%) | |
| | >7days to 3 months: n=2028 (34%) | |
| | >3 months: n=936 (16%) | |
| | No worsening HF event: n=1818 (30%) | |
| | A total of 1219 patients (20%) had a worsening HF event within 7 days before randomization, with 749 (12%) patients were enrolled during the index event. Of those enrolled during a worsening HF event, 652 (87%) were enrolled during a HF hospitalization and 97 (13%) were enrolled during an outpatient HF visit. 2028 (34%) patients had a worsening HF event between 7 days and 3 months before randomization, 936 (16%) patients had had a worsening HF event more than 3 months before randomization, and 1818 (30%) patients had never had a prior worsening HF event (Figure 5). There were notable baseline differences between patients based on the recency of a HF event before randomization. Patients randomized within 7 days of a worsening HF event had higher NYHA functional class, lower KCCQ-TSS, higher baseline NT-proBNP levels, and lower baseline eGFR. The use of SGLT2 inhibitors and ARNIs was also notably higher in those within ≤3 months of a worsening HF event compared with the residual randomized population. | |
| Figure 6 | Graphical Abstract: Baseline Characteristics of Example 2 (FINEARTS-HF) | |
| | Participants: | |
| | Between September 14^{th}, 2020 and January 10^{th}, 2023, we screened 7463 patients from 653 sites across 37 countries for enrollment. The primary reason (90%) for screen failure was not meeting ≥1 inclusion or exclusion criterion; 53% had sub-threshold natriuretic peptide levels, 9% had a serum potassium level >5.0 mEq/L, and 28% did not meet other inclusion or exclusion criteria. Overall, 6016 randomizations to receive finerenone or placebo occurred. Two of these randomizations were identified in connection to the same patient, and both entries were excluded. In addition, thirteen patients from a single site were excluded from the primary analysis due to significant Good Clinical Practice (GCP) violations. In total, 6001 patients were validly randomized and included in subsequent analyses. | |
| Figure 7 | †Graphical display of the distributions of KDIGO kidney risk among Example 2 participants with available eGFR and UACR data (n = 5797) at baseline. ‡Graphical display of the distributions of KDIGO kidney risk among weighted NHANES (2015 to March 2020) sample of US adults (weighted n = 5,189,186) with HF, age ≥40 years, eGFR ≥25 mL/min/1.73 m2 , and serum potassium ≥5 mmol/L. §Sankey diagram showing reclassification of KDIGO kidney risk after incorporation of eGFR and UACR, compared with eGFR stages alone. eGFR, estimated glomerular filtration rate; HF, heart failure; KDIGO, Kidney Disease Improving Global Outcomes; NHANES, National Health and Nutrition Examination Surveys; UACR, urine albumin-to-creatinine ratio) | |
| Figure 8 | Distribution of KDIGO kidney risk among Example 2 participants by diabetes status: In Example 2 participants without diabetes, 40.7%, 30.4%, 18.4%, and 10.5% were classified as KDIGO low, moderate, high, and very high risk, respectively. In Example 2 participants with diabetes, 26.3%, 27.2%, 22.9%, and 23.6% were classified as KDIGO low, moderate, high, and very high risk, respectively. | |
| Figure 9 | Example 13, Patient Flow | |
| Figure 10 | Figure 1. Panel a) Cumulative incidence plot for the primary endpoint of total cardiovascular deaths and heart failure events. Panel b) Cumulative incidence plot for total heart failure events. Panel c) Kaplan-Meier plot for death from cardiovascular causes. Panel d) Kaplan-Meier plot for first occurrence of cardiovascular death or heart failure event. | |
| Figure 11 | Forest plot of primary treatment effect for prespecified subgroups. | |
| Figure 12 | Forest plot for prespecified subgroups for time to first cardiovascular death or heart failure event. | |
| Figure 13 | Systematic Search of the Literature to Identify Eligible Trials for Inclusion in FINE-HEART; Example 14 | |
| Figure 14 | KDIGO Risk Distribution at Baseline in FINE-HEART; Example 14 | |
| Figure 15 | Baseline Cardio-Kidney-Metabolic Overlap in FINE-HEART; Example 14 | |
| Figure 16 | | (a) Key Efficacy Outcomes in Each Individual Trial |
| | | (b) Efficacy Outcomes |
| | | (c) Cumulative Incidence of Key Efficacy Endpoints |
| | | (d) Subgroup Forest Plot for Primary Endpoint (CV Death) |
| | Example 14 | |
| Figure 17 | Kaplan Meier curves illustrating the cumulative incidence of cardiovascular death or hospitalisation for heart failure, heart failure hospitalisation, cardiovascular death and all-cause death. Nelson-Aalen curves showing the cumulative incidence of total (first and repeat) heart failure hospitalisations and composite of total heart failure hospitalisations and cardiovascular death excluding RALES as time to events was not available; Example 15 | |
| Figure 18 | Event rates in RALES, EMPHASIS-HF, TOPCAT and FINEARTS-HF for each outcome by randomised therapy; Example 15 | |
| Figure 19 | Event rates in HFrEF and HFmrEF/HFpEF trials combined and for all trials for each outcome by randomised therapy; Example 15 | |
| Figure 20 | Effect of mineralocorticoid receptor antagonist treatment on the pre-specified efficacy outcomes in each of the trials; Example 15 | |
| Figure 21 | Effect estimates from the individual patient level meta-analysis of mineralocorticoid receptor antagonists and of cardiovascular death or hospitalisation for heart failure, heart failure hospitalisation, cardiovascular death, total (first and repeat) heart failure hospitalisations and composite of total heart failure hospitalisations and cardiovascular death, and all-cause death. FINEARTS-HF includes urgent visits for worsening heart failure as a hospitalisation equivalent. Estimates from the models in all 4 trials and split by HFrEF and HFmrEF/HFpEF trials with treatment by trial interaction p values are displayed.: Example 15 | |
| Figure 22 | Two stage meta-analysis of cardiovascular death or heart failure hospitalisations, heart failure hospitalisations, cardiovascular death and all cause deaths | |
| Figure 23 | Effect of MRAs in each of the trials on pre specified efficacy outcomes including and excluding undetermined deaths from the definition of cardiovascular death; Example 15 | |
| Figure 24 | Total heart failure hospitalisations and total heart failure hospitalisation and cardiovascular deaths analysed in semi-parametric proportional rates model excluding RALES and including EMPHASIS-HF, TOPCAT and FINEARTS-HF; Example 15 | |
| Figure 25 | Sensitivity analysis of the effect of MRAs in each of the trials on pre specified efficacy outcomes using all patients included in RALES, EMPHASIS-HF, FINEARTS-HF and only patients randomised in the Americas in TOPCAT; Exxample 15 | |
| Figure 26 | Effect of mineralocorticoid receptor antagonist treatment on the composite of cardiovascular death or hospitalisation for heart failure (time-to-first event analysis) in key subgroups: Panel A) combined RALES and EMPAHASIS-HF trials and Panel B) combined TOPCAT and FINEARTS-HF trials. FINEARTS-HF includes urgent visits for worsening heart failure as a hospitalisation equivalent; Example 15 | |
| Figure 27 | Interaction between left ventricular ejection fraction and the effect of randomised treatment on the composite of cardiovascular death or hospitalisation for heart failure (left ventricular ejection fraction modelled as a restricted cubic spline). The p-values are p-values for interaction between left ventricular ejection fraction and effect of mineralocorticoid receptor antagonist treatment in the trials including patients with heart failure and reduced ejection fraction (HFrEF) and in the trials including patients with heart failure and mildly reduced and preserved ejection fraction (HFmrEF/HFpEF). FINEARTS-HF includes urgent visits for worsening heart failure as a hospitalisation equivalent; Example 15 | |
| Figure 28 | Subgroup analysis of the outcome of cardiovascular death or first hospitalisation for heart failure in all four trials; Example 15 | |
| Figure 29 | Effect of mineralocorticoid receptor antagonist treatment on additional pre-specified safety outcomes in each trial; Example 15 | |
| Figure 30 | Table 9 Safety outcomes in HFrEF and HFmrEF/HFpEF trials combined and all 4 trials combined; Example 15 | |
| Figure 31 | Effect of mineralocorticoid receptor antagonist treatment on the pre-specified safety outcomes in each trial eGFR<45; Example 15 | |
| Figure 32 | Effect of mineralocorticoid receptor antagonist treatment on the pre-specified safety outcomes in each trial eGFR≥45; Example 15 | |
| Figure 33 | Effect of mineralocorticoid receptor antagonist treatment on the pre-specified safety outcomes in each trial TOPCAT Americas; Example 15 | |

### Experimental part

### Abbreviations:

| Abbreviations | |
|---|---|
| AE | adverse event |
| ± or +/- | plus / minus |
| ASCVD | atherosclerotic cardiovascular disease |
| BP | blood pressure |
| BUN | Blood urea nitrogen |
| CAD | coronary artery disease |
| CHF | chronic heart failure |
| CHF | chronic heart failure |
| CKD | Chronic Kidney Disease |
| CKD | chronic kidney disease |
| CKD-EPI | Chronic Kidney Disease Epidemiology Collaboration |
| CKM | cardio-kidney-metabolic |
| CONUT | controlling nutritional status |
| COPD | Chronic obstructive pulmonary disease |
| d | day |
| D | Day |
| DBP | diastolic blood pressure |
| dL | decilitre |
| DM | diabetes mellitus |
| ECG | electrocardiogram |
| eGFR | estimated glomerular filtration rate |
| EOS | end-of-study |
| EQ 5D 5L | EuroQoL Group 5 dimension 5-level questionnaire |
| GNRI | geriatric nutritional risk index |
| HF | Heart failure |
| HFmrEF | heart failure with mid-range ejection fraction |
| HFpEF | Heart failure with preserved ejection fraction |
| HFrEF | heart failure with reduced ejection fraction |
| HHF | Hospitalization for Heart failure |
| hs-TnT | high-sensitivity troponin-t |
| IxRS i | interactive voice / web response system |
| K/K+ | potassium |
| KCCQ | Kansas City Cardiomyopathy Questionnaire |
| KDIGO | The Kidney Disease: Improving Global Outcomes |
| kPa | kilopascals |
| L | Liter |
| M | Month |
| MAGGIC | Meta-Analysis Global Group in Chronic (MAGGIC) Heart Failure Risk Score |
| med. | medication |
| mEq | mmol |
| mg | Miligramm |
| MI | myocardial infarction |
| mL | Mililiter |
| mmHg | millimeters of mercury |
| mmol | Milimol |
| mol | Mol |
| MR | Mineralocorticoid Receptor |
| MRA | Mineralocorticoid Receptor Antagonist |
| ng | Nanogram |
| NT-proBNP | N-terminal prohormone of brain natriuretic peptide |
| NYHA | New York Heart Association |
| O | on-site |
| OD | Once daily |
| OSA | obstructive sleep apnea |
| PAD | peripheral artery diseas |
| PD | premature discontinuation |
| pg | picogramm |
| PGIC | Patient Global Impression of Change |
| PGIS | PGI of Severity |
| PNI | prognostic nutritional index |
| PT | post-treatment |
| RAAS | renin-angiotensin-aldosterone system |
| SBP | systolic blood pressure |
| Serum [K+] | serum potassium level |
| SGLT2 | sodium-glucose-transporter-2 |
| SoA | schedule of activities |
| T2D | type 2 DM |
| tel. | telephone |
| TTA | Transient ischemic attack |
| TTA | transient ischemic attack |
| WCHF | worsening chronic heart failure |
| WCHF | worsening chronic heart failure |
| WHO | World Health Organization |
| yr | year |

### Example 1 - Measurements of Finerenone Polymorph

### Lattice Constants of Compound of the Formula (I) in Crystalline Form of Polymorph I

Polymorph I
Crystal system orthorhombic
Space group P2(1)2(1)2(1)
Molecules per unit
cell 4
Length of axis a [Å] 7.8610(3)
Length of axis b [Å] 11.7797(6)
Length of axis c [Å] 20.1792(8)
α [°] 90
β [°] 90
γ [°] 90
Calculated density at
100 K [g cm-3] 1.345
Measuring parameters of the x-ray diffractometry for the measurement of compound of the formula (I) in crystalline form of polymorph I

| | |
|---|---|
| Data set name | 2429-08a r2 |
| Scan axis | 2Theta-Omega |
| Start position [°2Th.] | 2.0000 |
| End position [°2Th.] | 37.9900 |
| Type of divergence screen | Fixed |
| Size of divergence screen [°] | 1.0000 |
| Measurement temperature [° C.] | 25 |
| Anode material | Cu |
| K-Alpha1 [Å] | 1.54060 |
| Generator setting | 35 mA, 45 kV |
| Diffractometer type | Transmission diffractometer |
| Goniometer radius [mm] | 240.00 |
| Focus-div. screen gap [mm] | 91.00 |
| Primary beam monochromator | Yes |
| Sample rotation | Yes |

Peak maximum
[2 Theta]
Polymorph I
8.5
11.4
11.9
13.4
14.1
14.8
15.0
15.4
16.0
17.2
18.5
19.0
19.8
20.5
20.8
22.1
22.7
23.0
23.1
23.6
23.9
24.6
24.9
25.2
25.6
26.0
26.5
27.1
27.3
28.3
28.5
28.8
29.6
30.1
30.6
31.5
31.9
32.4
32.9
33.1
33.4
33.7
34.5
34.7
35.0
35.8
36.2
36.5
37.2
37.4

### Measuring Conditions for the IR and Raman Spectroscopy for the Measurement of the Compound of the Formula (I) in Crystalline Form of Polymorph I:

### IR:

| | |
|---|---|
| Instrument | Perkin Elmer Spectrum One |
| Number of scans | 32 |
| Resolution | 4 cm⁻¹ |
| Technique | Diamond ATR unit |

### Raman:

| | |
|---|---|
| Instrument | Bruker Raman RFS 100/S |
| Number of scans | 64 |
| Resolution | 2 - 4 cm⁻¹ |
| Laser Power | 350 mW |
| Laser wavelength | 1064 nm |

### Band maximum [cm⁻¹]

| 1R-ATR | | Raman | |
|---|---|---|---|
| Polymorph I | | Polymorph I | |
| | 3475 | | 3074 |
| | 3416 | | 2997 |
| | 3366 | | 2970 |
| | 3074 | | 2941 |
| | 2992 | | 2920 |
| | 2952 | | 2836 |
| | 2835 | | 2231 |
| | 2230 | | 1659 |
| | 1681 | | 1641 |
| | 1658 | | 1623 |
| | 1606 | | 1601 |
| | 1572 | | 1577 |
| | 1485 | | 1487 |
| | 1464 | | 1443 |
| | 1454 | | 1383 |
| | 1431 | | 1362 |
| | 1420 | | 1327 |
| | 1407 | | 1303 |
| | 1381 | | 1267 |
| | 1355 | | 1230 |
| | 1341 | | 1191 |
| | 1325 | | 1161 |
| | 1303 | | 1123 |
| | 1285 | | 1093 |
| | 1267 | | 1032 |
| | 1255 | | 991 |
| | 1229 | | 883 |
| | 1222 | | 827 |
| | 1161 | | 810 |
| | 1136 | | 759 |
| | 1097 | | 734 |
| | 1031 | | 708 |
| | 991 | | 671 |
| | 976 | | 613 |
| | 967 | | 528 |
| | 924 | | 505 |
| | 909 | | 471 |
| | 875 | | 442 |
| | 847 | | 346 |
| | 827 | | 320 |
| | 810 | | 297 |
| | 776 | | 186 |
| | 758 | | 155 |
| | 746 | | 114 |
| | 733 | | |
| | 723 | | |
| | 706 | | |
| | 697 | | |
| | 670 | | |

### Example 1b - Alternative assays for measuring levels of NT-proBNP [ng/L]

Levels of NT-proBNP, the N-terminal fragment of pro-natriuretic peptide, type B, can be determined using, for example, immunological assays such as the Roche Cobas Elecsys proBNPII or the Siemens Immulite. Both assays are immunological in vitro diagnostic tests for the quantitative determination of NT-proBNP in human plasma or serum and typically use monoclonal (or polyclonal) antibodies against NT-proBNP to capture and detect the analyte NT-proBNP. The absolute concentration of NT-proBNP in FINEARTS-HF samples was determined using either of the two assays. To account for assay-to-assay differences in the absolute NT-proBNP values between Roche and Siemens, samples analyzed on Siemens Immulite were adjusted (after analyzing matched samples on both instruments) to Roche Cobas NT-proBNP as primary read-out resulting in lower overall NT-proBNP values compared to Siemens". Throughout the specification and claims, NT-proBNP values determined by the above described Siemens Immulite assay are identified by "¹⁾" following the NT-proBNP value. NT-proBNP values determined by the above described Roche Cobas assay are identified by "²⁾" following the NT-proBNP value.

### Example 2 - Study Design

### Study Objectives and Endpoints

Example 2 is a global, multicenter, placebo-controlled, event-driven randomized clinical trial investigating the long-term efficacy and safety of the non-steroidal MRA finerenone in symptomatic patients with HF with LVEF ≥40%. The trial was designed collaboratively by members of the steering committee and the sponsor. The trial has been registered on ClinicalTrials.gov (NCT04435626) and EudraCT (2020-000306-29).

### Study design

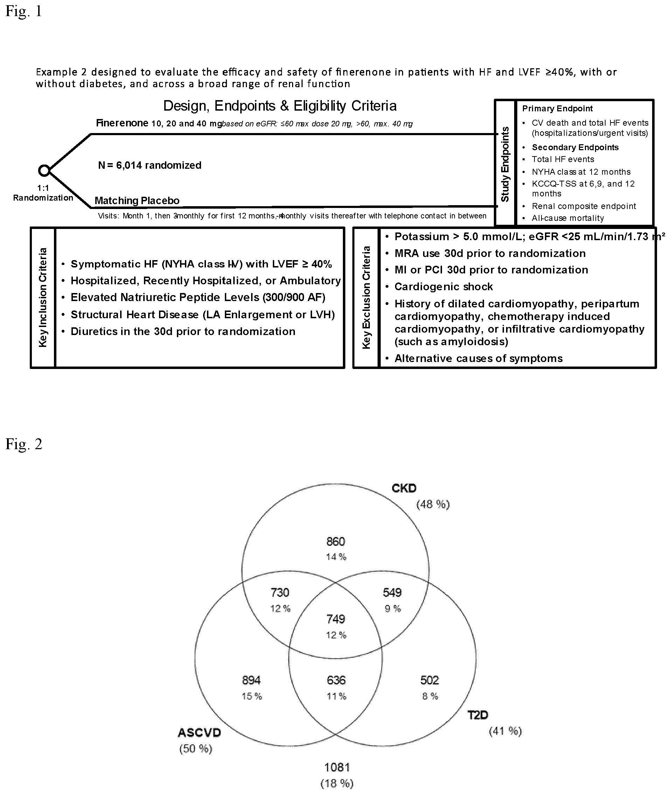

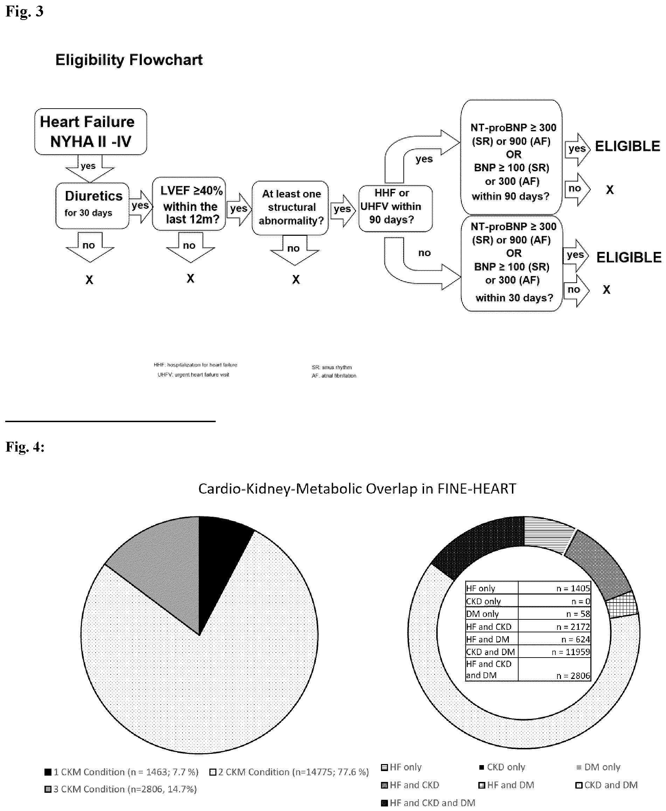

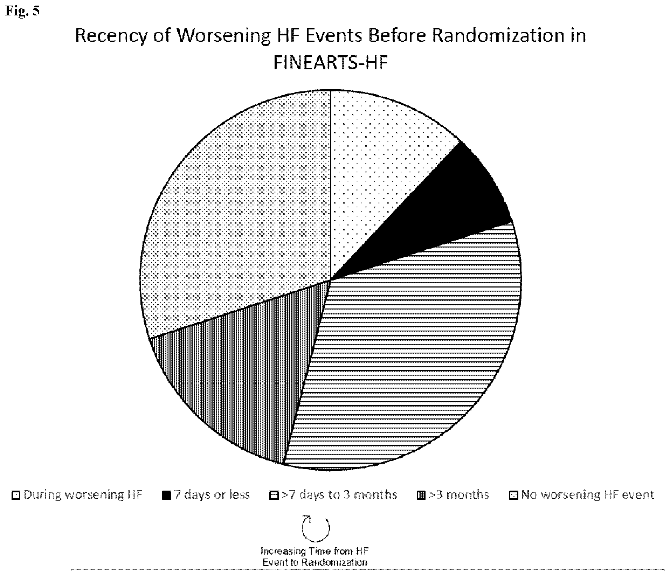

The Design, Endpoints and Eligibility criteria are shown in Figure 1. In brief, FINEARTS-HF enrolled adults (≥40 years) with symptomatic HF (New York Heart Association class II-IV) with diuretic requirement at least 30 days prior to randomization. LVEF ≥40% and evidence of structural heart disease (left atrial enlargement or left ventricular hypertrophy) were required and could be measured locally by any modality within 12 months of screening. Left atrial diameter ≥3.8cm, left atrial area ≥20cm2, or left atrial volume index >30 mL/m2 qualified as left atrial enlargement. Left ventricular mass index ≥115 g/m2 (if male) or ≥95 g/m2 (if female) or septal or posterior wall thickness≥1.1cm qualified as left ventricular hypertrophy. Enrollment of patients with prior LVEF<40% with subsequent improvement to ≥40% (HFimpEF) was permitted provided that ongoing HF symptoms were present. The subgroup of patients with LVEF ≥60% was capped at a maximum of 20% of participants in the trial. All patients were required to have elevated natriuretic peptide levels, with eligibility thresholds adjusted based on the timing of recent HF events and the presence of atrial fibrillation. For patients in sinus rhythm, NT-proBNP ≥300 pg/mL (or BNP≥100 pg/mL) were required, measured within 30 days (in those without a recent worsening HF event) or within 90 days (in those with a recent worsening HF event). Qualifying levels of NT-proBNP or BNP were tripled if a patient was in atrial fibrillation/flutter at screening. A worsening HF event could include either a hospitalization for HF or an urgent visit for HF. An eligibility flowchart is presented in Figure 3.

### Schedule of Activities (SoA)

The schedule of activities (SoA) is displayed for the study as a whole in Table 1 ('Main SoA') and for participants who prematurely discontinue the study, minimal assessments will need to be performed as outlined in Table 2 (`Premature Discontinuation SoA').

**Table 1 ('Main SoA') - Study assessments and procedures by study period and visit are presented**

| Table 1 Main SoA | | | | | | | | | | | | | | |
|---|---|---|---|---|---|---|---|---|---|---|---|---|---|---|
| Visit Number / Name | Screeni ng^{a} | Baseline ^{a} 1 | 2 | 3 | 4 | 5 | 6 | 7, 9, 11, 13, 15, 17, 19, 21 etc.^{b} | 8, 12, 16, 20 etc. | 10, 14, 18 etc. | Up-titration, re-start and safety check ^{c} | PD Visit ^{d} | EO S Visi t ^{e} | PT Visit ^{f} |
| Day (D) / Month (M) | | D1 | M 1 | M 3 | M 6 | M 9 | M 12 | M14 and every 4 months (i.e. M18, M22, M26, M30, M34, M38, M42 etc.) | M16 and every 8 months (i.e. M24 , M32, M40 etc.) | M20 and every 8 months (i.e. M28 , M36 etc.) | | | | |
| Visit window (days) | | | ±3 | ±3 | ±6 | ±6 | ±6 | ±7 | ±7 | ±7 | ±7 | | | +5 |
| On-site (O)/Tel. contact ( ) | O | O | O | O | O | O | O | | O | O | O | O | O | |

| Initiation procedures | | | | | | | | | | | | | | |
|---|---|---|---|---|---|---|---|---|---|---|---|---|---|---|
| Informed consent | X | | | | | | | | | | | | | |
| Demographic data | X | | | | | | | | | | | | | |
| Substance use (alcohol & tobacco) | | X | | | | | | | | | | | | |
| Medical history | X | | | | | | | | | | | | | |
| NYHA class assessment | X | X | X | X | X | X | X | X | X | X | | X | X | X |
| Prior and concomitant medication | X | X | X | X | X | X | X | X | X | X | X | X | X | X |
| In- and exclusion criteria | X | X | | | | | | | | | | | | |

| Clinical procedures/ assessments | | | | | | | | | | | | | | |
|---|---|---|---|---|---|---|---|---|---|---|---|---|---|---|
| Weight | X | X | X | X | X | X | X | | X | X | X | X | X | |
| Height | X | | | | | | | | | | | | | |
| Waist and hip circumference | X | | | | | | | | | | | | | |
| Vital signs ^{g} | X | X | X | X | X | X | X | | X | X | X | X | X | |
| 12-lead ECG (local) | | X | | | | | | | | | | | | |
| AE and endpoint assessment (renal endpoints require additional confirmed creatinine measurement) | | X | X | X | X | X | X | X | X | X | X | X | X | X |

| Study intervention | | | | | | | | | | | | | | |
|---|---|---|---|---|---|---|---|---|---|---|---|---|---|---|
| Randomization (IxRS) | | X | | | | | | | | | | | | |
| Dispense study intervention | | X | X | X | X | X | X | | X | X | X^{r} | | | |

| Table 1 Main SoA | | | | | | | | | | | | | | |
|---|---|---|---|---|---|---|---|---|---|---|---|---|---|---|
| Visit Number / Name | Screeni ng ^{a} | Baseline ^{a} 1 | 2 | 3 | 4 | 5 | 6 | 7, 9, 11, 13, 15, 17, 19, 21 etc.^{b} | 8, 12, 16, 20 etc. | 10, 14, 18 etc. | Up-titration, re-start and safety check ^{c} | PD Visit ^{d} | EO S Visi t ^{e} | PT Visit ^{f} |
| Day (D) / Month (M) | | D1 | M 1 | M 3 | M 6 | M 9 | M 12 | M14 and every 4 months (i.e. M18, M22, M26, M30, M34, M38, M42 etc.) | M16 and every 8 months (i.e. M24 , M32, M40 etc.) | M20 and every 8 months (i.e. M28 , M36 etc.) | | | | |
| Visit window (days) | | - | ±3 | ±3 | ±6 | ±6 | ±6 | ±7 | ±7 | ±7 | ±7 | | | +5 |
| On-site (O)/Tel. contact ( ) | O | O | O | O | O | O | O | | O | O | O | O | O | |
| Provide and review the study contact card | | X | | | | | X | | | X | | | | |
| Administration of study intervention at study site | | X | | X | | | | | | | | | | |
| Administration of study intervention before the visit | | | X | | X | X | X | | X | X | | | | |
| Study intervention accountability | | | X | X | X | X | X | | X | X | X | X | X | |

| Local/central laboratory | | | | | | | | | | | | | | |
|---|---|---|---|---|---|---|---|---|---|---|---|---|---|---|
| Local laboratory ^{h} (potassium and creatinine ⁱ) | X^{j} | X^{j, m} | X^{k} | X^{k} | X^{k} | X^{k} | X^{k} | | X^{k} | X^{k} | X^{k} | X^{k} | X^{k} | |
| Pregnancy test | X^{L} | X^{L, m} | | | | | | | | | | | | |
| Central laboratory including urinalysis | | X^{m} | X | X | X^{s} | X | X | | X | X | | X | X | |
| Biomarkers NT-proBNP and hs-TnT | | X ^{m} | | X | | | X | | | | | | | |
| Exploratory biomarkers | | X^{m} | | X | | | X | | | | | | X | |
| Pharmacokinetics | | | | Xⁿ | | | X^{o} | | | X^{o} | | | | |

| Other study procedures | | | | | | | | | | | | | | |
|---|---|---|---|---|---|---|---|---|---|---|---|---|---|---|
| KCCQ ^{q} | | X | | | X | X | X | | X | | | X | X | |
| EQ-5D-5L ^{q} | | X | | | X | X | X | | X | | | X | X | |
| PGIC (applicable to selected sites only) ^{q} | | | | | X | X | X | | | | | | | |
| PGIS (applicable to selected sites only) ^{q} | | X | | | X | X | X | | | | | | | |
| *Please note that footnotes to both SoAs can be found below Table 2.* | | | | | | | | | | | | | | |

| Table 1 Main SoA | | | | | | | | | | | | | | |
|---|---|---|---|---|---|---|---|---|---|---|---|---|---|---|
| Visit Number / Name | Screeni ng^{a} | Baseline ^{a} 1 | 2 | 3 | 4 | 5 | 6 | 7, 9, 11, 13, 15, 17, 19, 21 etc.^{b} | 8, 12, 16, 20 etc. | 10, 14, 18 etc. | Up-titration, re-start and safety check ^{c} | PD Visit ^{d} | EO S Visi t ^{e} | PT Visit ^{f} |
| Day (D) / Month (M) | | D1 | M 1 | M 3 | M 6 | M 9 | M 12 | M14 and every 4 months (i.e. M18, M22, M26, M30, M34, M38, M42 etc.) | M16 and every 8 months (i.e. M24 , M32, M40 etc.) | M20 and every 8 months (i.e. M28 , M36 etc.) | | | | |
| Visit window (days) | | - | ±3 | ±3 | ±6 | ±6 | ±6 | ±7 | ±7 | ±7 | ±7 | | | +5 |
| On-site (O)/Tel. contact ( ) | O | O | O | O | O | O | O | | O | O | O | O | O | |

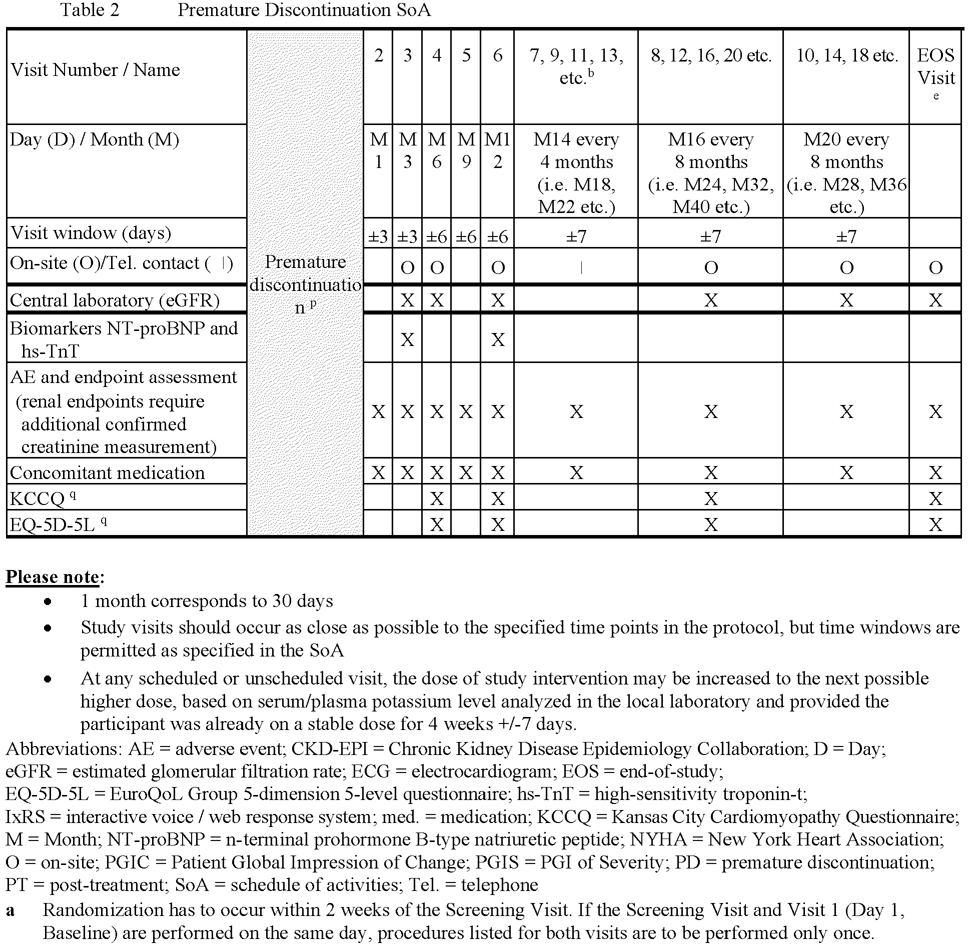

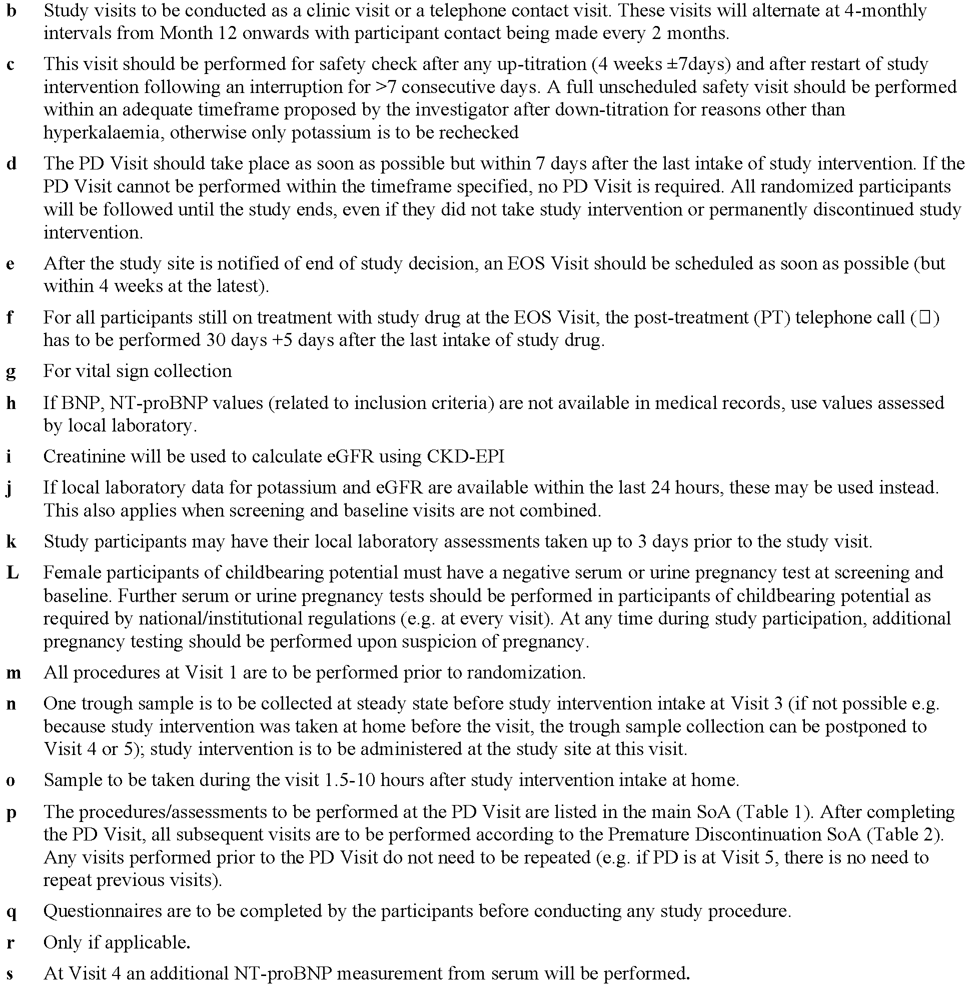

### Eligibilitys

Eligibility Criteria. Final inclusion and exclusion criteria are summarized below. Table 3, table 4 and table 5 depict summaries of some selected aspects of the study:

**Table 3: Key inclusion and key exclusion criteria**

| **Key Inclusion Criteria** | **Key Exclusion Criteria** |
|---|---|
| - Symptomatic HF (NYHA class II-V) with LVEF ≥ 40% | - Potassium > 5.0 mmol/L; eGFR <25 mL/min/1.73 m² |
| - Hospitalized, Recently Hospitalized, or Ambulatory | - MRA use 30d prior to randomization |
| | - MI or PCI 30d prior to randomization |
| - Elevated Natriuretic Peptide Levels (300/900 AF) | - Cardiogenic shock |
| | - History of dilated cardiomyopathy, peripartum |
| - Structural Heart Disease (LA Enlargement or LVH) | cardiomyopathy, chemotherapy induced cardiomyopathy, or infiltrative cardiomyopathy (such as amyloidosis) |
| - Diuretics in the 30d prior to randomization | |
| | - Alternative causes of symptoms |

**Table 4: Study Endpoints**

| **Primary Endpoint** | **Secondary Endpoints** |
|---|---|
| CV death and total HF events (hospitalizations/urgent visits) | - Total HF events |
| | - NYHA class at 12 months |
| | - KCCQ-TSS at 6,9, and 12 months |
| | - Renal composite endpoint |
| | - All-cause mortality |

**Table 5: Main inclusion and main exclusion criteria - Example 2 includes patients with LVEF ≥40% and eGFR ≥25 ml/min/1.73 m², with or without diabetes mellitus DM**

| **Main inclusion criteria** | **Main exclusion criteria** |
|---|---|
| - **Aged ≥40 years** | - **eGFR <25** ml/min/1.73 m² |
| - **HF diagnosis with NYHA class II-IV** | - **Serum [K+] >5.0** mmol/1 |
| - **LVEF ≥40%** | - **MI** or any event that could have **reduced the EF** within 90 days prior to randomisation |
| - **eGFR ≥25** ml/min/1.73 m² | |
| - **Serum [K+]** ≤**5.0** mmol/1 | |
| - **In patients with sinus rhythm: NT-proBNP ≥300pg/ml or BNP ≥100 pg/ml** | - **Systolic blood pressure (SBP)** ≥**160** mmHg |
| - **In patients with AF: NT-proBNP ≥900 pg/ml or BNP ≥300pg/ml** | |

### Detailed Example 2

### (1) Study Population

Patients with a diagnosis of HF, NYHA class II-IV, and documented LVEF of ≥40%.

In the following, an overview of the inclusion and exclusion criteria as well as the patient population is summarized.

### a. Inclusion Criteria

Participants are eligible to be included in the study only if all of the following criteria apply:
Main inclusion criteria were:
- Aged ≥40 years
- HF diagnosis with NYHA class II-IV
- LVEF≥40%
- eGFR ≥25 ml/min/1.73 m²
- Serum [K+] ≤5.0 mmol/l
- In patients with sinus rhythm: [K+] NT-proBNP ≥300 pg/ml, or BNP ≥100 pg/ml

### Age

- Participant must be aged 40 years and older, at the time of signing the informed consent.

### Type of Participant and Disease Characteristics:

- Diagnosis of heart failure with NYHA class II-IV, ambulatory or hospitalized primarily for heart failure (if a hospitalized patient cannot be randomized as an in-patient, randomization as soon as possible after discharge is encouraged)
- On diuretic treatment for at least 30 days prior to randomization
- Documented LVEF of ≥40% measured by any modality within the last 12 months, at the latest at screening; if several values are available, the most recent one shall be reported. If LVEF was not measured in the past 12 months, a new measurement may be done at screening
- Structural heart abnormalities based on any local imaging measurement within the last 12 months, latest at screening, defined by at least 1 of the following findings:
- LAD ≥3.8cm, LAA ≥20cm², LAVI >30 mL/m², LVMI ≥115 g/m2 (♂) / 95 g/m2 (♀), septal thickness or posterior wall thickness ≥1.1 cm
- NT-proBNP ≥300 pg/mL (BNP ≥100 pg/mL) in sinus rhythm and patient does not have an ongoing diagnosis of paroxysmal atrial fibrillation or NT-proBNP ≥900 pg/mL (BNP ≥300 pg/mL) in atrial fibrillation (or if atrial fibrillation status is unknown or if patient has an ongoing diagnosis of paroxysmal atrial fibrillation) for participants obtained at the following time:
- Within 90 days prior to randomization if patient had been hospitalized for HF requiring initiation or change in HF therapy or if patient had an urgent visit for HF requiring intravenous (IV) diuretic therapy, both within 90 days prior to randomization
   OR
- Within 30 days prior to randomization if patient has not been hospitalized for HF nor had an urgent HF visit within the past 90 days.

### Sex

### Male or female.

Women of childbearing potential can only be included in the study if a pregnancy test is negative at screening and baseline and if they agree to use adequate contraception which is consistent with local regulations regarding the methods for contraception for those participating in clinical trials.

### Informed Consent

Capable of giving signed informed consent which includes compliance with the requirements and restrictions listed in the informed consent form (ICF) and in this protocol.

### b. Exclusion Criteria

Main exclusion criteria were:
- eGFR <25 ml/min/1.73 m²
- Serum [K+] >5.0 mmol/l
- MI or any event that could have reduced the EF within 90 days prior to randomization
- SBP ≥160 mmHg

Participants are excluded from the study if any of the following criteria apply:

### Medical Conditions

- eGFR <25 mL/min/1.73 m² at either screening or randomization visit. NOTE: one reassessment of eGFR is allowed at the screening and randomization visit, respectively
- Serum/plasma potassium >5.0 mmol/L at either screening or randomization visit. NOTE: one reassessment of potassium is allowed at the screening and randomization visit, respectively
- Acute inflammatory heart disease, e.g. acute myocarditis, within 90 days prior to randomization
- Myocardial infarction or any event which could have reduced the ejection fraction within 90 days prior to randomization
- Coronary artery bypass graft surgery in the 90 days prior to randomization
- Percutaneous coronary intervention in the 30 days prior to randomization
- Stroke or transient ischemic cerebral attack within 90 days prior to randomization
- Probable alternative cause of participants' HF symptoms that in the opinion of the investigator primarily accounts for patient's dyspnea such as significant pulmonary disease, anemia or obesity.
Specifically, patients with the below are excluded:
- Severe pulmonary disease requiring home oxygen, or chronic oral steroid therapy
- History of primary pulmonary arterial hypertension
- Hemoglobin <10 g/dl
- Valvular heart disease considered by the investigator to be clinically significant
- Body mass index (BMI) >50 kg/m2 at screening
- Systolic blood pressure (SBP) ≥160 mmHg if not on treatment with ≥3 blood pressure lowering medications or ≥180 mmHg irrespective of treatments on 2 consecutive measurements at least 2-minute apart, at screening or at randomization
- Life-threatening or uncontrolled arrhythmias at screening and/or randomization including but not limited to sustained ventricular tachycardia and atrial fibrillation, or atrial flutter with resting ventricular rate >110 bpm
- Symptomatic hypotension with mean systolic blood pressure <90 mmHg at screening or at randomization
- Any primary cause of HF scheduled for surgery, e.g. valve disease such as severe aortic stenosis or severe mitral regurgitation by the time of screening or randomization
- History of periparium cardiomyopathy, chemotherapy induced cardiomyopathy, viral myocarditis, right heart failure in absence of left-sided structural disease, pericardial constriction, genetic hypertrophic cardiomyopathy, or infiltrative cardiomyopathy including amyloidosis
- Presence of left ventricular assist device by the time of screening or randomization
- History of hyperkalemia or acute renal failure during MRA treatment for >7 consecutive days, leading to permanent discontinuation of the MRA treatment
- Pregnant or nursing (lactating) women, where pregnancy is defined as the state of a female after conception and until the termination of gestation, confirmed by a positive human chorionic gonadotrophin urine or serum test
- Known hypersensitivity to the study intervention (active substance or excipients)
- Hepatic insufficiency classified as Child-Pugh C at screening or randomization
- Addison's disease.

### Prior/Concomitant Therapy

- Requirement of any IV vasodilating drug (e.g. nitrates, nitroprusside), any IV natriuretic peptide (e.g. nesiritide, carperitide), any IV positive inotropic agents, or mechanical support (intra-aortic balloon pump, endotracheal intubation, mechanical ventilation, or any ventricular assist device) within 24 hours prior to randomization
- Participants who require treatment with more than one ACEI, ARB or angiotensin-receptor neprilysin inhibitor (ARNI), or two simultaneously at randomization
- Continuous (at least 90 days) treatment with an MRA (e.g. spironolactone, eplerenone, canrenone, esaxerenone) within 12 months prior to screening. Last intake at least 30 days before randomization. Treatment with MRA should not be interrupted with the purpose of enrollment into the study
- Concomitant treatment with any renin inhibitor or potassium-sparing diuretic that cannot be stopped prior to randomization and for the duration of the treatment period
- Concomitant systemic therapy with potent cytochrome P450 isoenzyme 3A4 (CYP3A4) inhibitors (e.g. itraconazole, ritonavir, indinavir, cobicistat, clarithromycin) or moderate or potent CYP3A4 inducers, that cannot be discontinued 7 days prior to randomization and for the duration of the treatment period.

### Other Exclusions

- Any other condition or therapy, which would make the participant unsuitable for this study and will not allow participation for the full planned study period (e.g. active malignancy or other condition limiting life expectancy to less than 12 months)
- Previous assignment to treatment during this study
- Participation in another interventional clinical study (e.g. Phase 1 to 3 clinical studies) or treatment with another investigational medicinal product within 30 days prior to randomization
- Close affiliation with the investigational site; e.g. a close relative of the investigator, dependent person (e.g. employee or student of the investigational site)
- Known current alcohol and/or illicit drug abuse that may interfere with the participant's safety and/or compliance at the discretion of the investigator
- Participant is in custody by order of an authority or a court of law.

In brief, Example 2 enrolled adults (≥40 years) with symptomatic HF (New York Heart Association class II-IV) with diuretic requirement at least 30 days prior to randomization. LVEF ≥40% and evidence of structural heart disease (left atrial enlargement or left ventricular hypertrophy) were required and could be measured locally by any modality within 12 months of screening. Previous LVEF that was <40% that has since improved to ≥40% was permitted. The subgroup of LVEF ≥60% was capped at a maximum of 20% of participants in the trial. All patients were required to have elevated natriuretic peptide levels, with differential recency of laboratory measurement and thresholds based on rhythm status and recent worsening HF event. In patients in sinus rhythm, NT-proBNP ≥300 pg/mL (or BNP≥100 pg/mL) were required measured within 30 days (in those without a recent worsening HF event) or within 90 days (in those with a recent worsening HF event). Qualifying levels of NT-proBNP or BNP were tripled if a patient was in atrial fibrillation/flutter at screening.

Patients could be enrolled irrespective of clinical care setting (whether hospitalized, recently hospitalized, or ambulatory). The proportion of patients without a recent worsening HF event within 3 months of randomization was capped globally at approximately 50% of the original planned sample size.

Estimated glomerular filtration rate <25 ml/min/1.73 m² or serum/plasma potassium >5.0 mmol/l at screening or randomization were key exclusion criteria. Recent treatment with an MRA within 12 months of screening was not permitted, and discontinuation of an MRA for the purposes of study enrollment was discouraged.

All participants signed informed consents for participation and the study protocol was approved by the ethics committees and institutional review boards for each participating site. The trial started on September 14, 2020 and has validly randomized over 6000 participants across 634 sites in 37 countries. The study is being conducted in accordance with Good Clinical Practice and the Declaration of Helsinki.

### Study Design.

Following a screening period of up to 2 weeks, eligible participants were randomized in a 1:1 ratio to either finerenone or matching placebo. The randomization was stratified by country/region and LVEF (<60%, ≥60%). Starting dose was selected based on baseline eGFR. Participants with an eGFR ≤60 mL/min/1.73 m² started 10 mg once daily with a maximum maintenance dose of 20 mg once daily, whereas participants with an eGFR >60 mL/min/1.73 m² started 20 mg once daily with a maximum maintenance dose of 40 mg once daily (Table 3).

In this ongoing study, patients are followed with 2 scheduled visits within the first 3 months of randomization, and then every 3 months until 1 year. After 1 year, telephone follow-up will alternate with on-site visits every 2 months until the end of the trial.

After the completion of the randomized phase, any participant still taking the study drug will enter a post-treatment follow-up period for 30 days.

### Dose Titration and Monitoring of Safety and Tolerability.

The study aimed to encourage reaching the maximum maintenance dose and sustaining it as long as safe and tolerated. Blood measurements of potassium and creatinine were performed serially at study visits to capture safety events. Up-titration is expected to occur any time after 4 weeks based on serum/plasma potassium level and if eGFR decrease does not exceed ≥30%. Down-titration may occur at any time if potassium ≥5.5mEq/L and temporarily interrupted if already at the lowest dose.

### Study Objectives.

The primary objective is to demonstrate the superiority of finerenone to placebo in reducing the rate of the composite of cardiovascular death and total (first and recurrent) HF events (HF hospitalization or urgent HF visit). The secondary objectives will be to determine the superiority of finerenone to placebo for each secondary endpoint: total HF events; improvement in NYHA class from baseline to Month 12; change from baseline to Month 6, 9 and 12 in total symptom score of the Kansas City Cardiomyopathy Questionnaire (KCCQ); time to first occurrence of composite renal endpoint (defined as sustained decrease in eGFR ≥50% relative to baseline over at least 4 weeks, or sustained eGFR decline <15ml/min/1.73m2, or initiation of dialysis or renal transplantation); time to all-cause mortality; and the safety and tolerability of finerenone.

### Statistical Considerations.

The primary analysis will evaluate the total occurrences of the composite of cardiovascular death, hospitalization for HF, and urgent HF events. It will be performed in the full analysis set (FAS) of all patients that have been randomized and have no major good clinical practice violations. The primary analysis of the primary composite endpoint will be based on a Lin, Wei, Yang and Ying (LWYY) model (Lin et al. 2000, https://doi.org/10.1111/1467-9868.00259). This is equivalent to the stratified Andersen-Gill model with use of robust standard errors (sandwich estimator) to account for correlations of event times within a participant. The model will include treatment group as a fixed effect and including geographic region and baseline LVEF (<60%, ≥60%) as stratification factors. A small adjustment will be made to the nominal significance level at the final analysis to take into account the interim analysis. A point estimate of the rate ratio together with a 95% CI will be presented. Events and follow-up time after discontinuation of treatment will be included in the analysis. Non-CV death is assumed to be a censoring event, since the treatment is not assumed to have an effect on these events and interest lies in the treatment effect on composite events while patients are alive.

If a participant experiences an HF event and subsequently dies for a cardiovascular reason, this will be considered as two separate events for the primary analysis unless the subject dies on the same calendar day as the HF event (both events would still be considered for the analyses of the separate components). If a subject is hospitalized for HF shortly after an urgent HF visit, this will be considered as two separate events for the primary analysis unless they occur on the same calendar day.

The primary analysis method has been investigated with extensive simulation studies and it has been confirmed that it keeps the alpha level and has good operating characteristics across a range of plausible scenarios, see also Fritsch et al (2023, https://doi.org/10.1080/19466315.2021.1945488).

The trial is designed to show an effect on the primary endpoint with a power of 90% at a two-sided alpha of 5%. The sample size determination is based on a simulation study assuming a joint frailty model in order to account for the correlation between HF events and cardiovascular death, and to model participant heterogeneity with respect to baseline intensities/hazards (Rogers, et al. 2016, https://doi.ors/10.1002/sim.6853). The placebo rate parameter of the Poisson process and the hazard rate of the exponential distribution were first chosen as 0.014 HHFs/month per participant and 0.004 CV deaths/month per participant, respectively. These values lead to an observed annualized placebo rate of first composite events of 9.0 (events/100 participant-years) and an observed annualized placebo rate of CV death of 3.5. These observed rates are similar to rates observed in the literature, i.e. an annualized rate of first composite event of 9.1 was observed in the CHARM-Preserved trial, 8.9 was observed in PARAGON-HF, and 8.5 in the BNP stratum of the TOPCAT trial. Regarding CV death, an annualized placebo rate of 3.9 per 100 participant-years was observed in CHARM-Preserved, 3.1 was observed in PARAGON-HF, and 3.9 in the TOPCAT BNP stratum. Since Example 2 is planned to recruit more participants with a very recent hospitalization than in previous trials, which would be at a higher risk of events, the rate parameters were subsequently increased by 25% for CV death leading to a rate of 0.005125 CV deaths/month per participant. For HF events, the rate was increased by 30% to 0.0182 HF events/month per participant to also account for the inclusion of urgent HF visits. As treatment effects, a hazard ratio for CV death of 0.8 and a rate ratio for heart failure events of 0.75 are assumed.

It was originally anticipated that 5,500 participants will be randomized. A total of approximately 2,375 total (first and recurrent) primary composite events are targeted. Due to blinded event rates being lower than those assumed in the sample size calculation, the planned number of randomized subjects was increased to approximately 6,000.

The secondary hypotheses will be formally tested, and statistical inferences will be made only if the primary hypothesis is rejected. Secondary endpoints will be tested sequentially: total HF events, NYHA class and KCCQ (tested using the Bonferroni-Holm procedure), composite renal endpoint. If the primary hypothesis is rejected, then all-cause mortality will be tested at a full two-sided significance level of 5% outside this hierarchy,

Total HF events will be analyzed using an LWYY model in a similar fashion to the primary endpoint. The percentage of participants with improvement in NYHA class from Baseline to Month 12, will be analyzed with a logistic regression model including factors for treatment group and stratification levels. Patients that died or are lost to follow-up at month 12 will be considered to have not improved. The absolute change from baseline including measurements up to Month 12 of the TSS of the KCCQ, will be analyzed by a repeated measures mixed model including the factors treatment group, baseline, visit, baseline-by-visit interaction, and factors for the stratification levels. The comparison assumes a common treatment effect across Month 6, 9 and 12. All observed values will be included into the analysis regardless of treatment discontinuation. The composite renal endpoint and all-cause mortality will be analyzed using a stratified log-rank test for testing and a stratified Cox proportional hazards model for obtaining a point estimate with 95% confidence interval. The Cox proportional hazards model will be stratified according to the stratification factors and include treatment group as a fixed effect.

### Interim Analyses.

One non-binding interim analysis for futility was planned when approximately 30% of the required total number of primary endpoint events were observed. If the observed rate ratio on the primary endpoint was above 0.95, the trial was planned to be stopped for futility. In addition, one formal interim analysis for efficacy was planned when approximately 2/3 of the required total number of primary endpoint events were observed. If the interim analysis showed clear and consistent benefit in the finerenone treatment group (primary efficacy endpoint with two-sided p-value<0.0027 and cardiovascular death component with nominal two-sided p-value<0.05), the Data Monitoring Committee may have recommended early study termination. A group sequential design with a single interim analysis when 2/3 of the information is available with a stopping rule of two-sided p <0.00270 would require a small adjustment to the alpha level at the final analysis to maintain the overall significance level at 0.05. For an information fraction of 2/3, the adjusted alpha level of 0.04967 applies. If the study is not stopped early for success a p-value of p<0.04967 is therefore required at the final analysis to achieve formal statistical significance.

### DISCUSSION

Example 2 will be a broadly inclusive trial of patients with HF with mildly reduced or preserved ejection fraction that is positioned to shed light on several key areas of uncertainty. While MR blockade in this target population has a strong theoretical basis, the clinical evidence to date has not been definitive. In Example 2, finerenone will be tested against a placebo comparator as steroidal MRAs currently lack any specific regulatory labeling or class I recommendation for use in patients with HF with mildly reduced or preserved ejection fraction, and thus are not considered a part of global standard of care. The design of the trial has been informed by learnings from previous large-scale outcomes trials of various pharmacotherapies. Example 2 is large (with over 6,000 patients enrolled) and targets a cohort at high-risk for disease progression, and thus is well-powered to definitively establish whether MRAs have a role in this patient population. The primary endpoint of the trial will reflect a broader look at "worsening HF" by including cardiovascular death and total (not just first) HF events. Finally, dosing of finerenone has been optimized and guided by a phase 2 program, in particular the ARTS-HF trial (Filippatos et al, 2016, https://doi.org/10.1093/eurheartj/ehw132), in which treatment effects on natriuretic peptide levels appeared to be dose dependent. Example 2 will uniquely test a 40mg target maintenance dose in people with an eGFR >60 mL/min/1.73 m², which is not currently available in the treatment of CKD.

The trial is also enriched to examine safety and efficacy of finerenone in a number of key subgroups. The trial will have the highest percentage of hospitalized or recently hospitalized patients of any contemporary HFmrEF/HFpEF outcomes trial. As global guidelines now support in-hospital and early post-discharge optimization of guideline directed medical therapies, Example 2 will directly inform this implementation strategy in the care of high-risk patients with HFmrEF/HFpEF. As most previous trials of steroidal MRAs have enrolled patients in stable ambulatory care, the safety of MRA initiation in this high-risk window of a worsening HF episode is less certain. A previous trial examined the early safety of high-dose spironolactone (administered at 100mg daily) in 360 patients with acute HF, however MRA therapy was not continued post-discharge in this trial.23 Furthermore, Example 2 is adequately represented across the range of ejection fraction such that the therapeutic effects of MRA in patients with LVEF ≥60% will be clarified.

The study adds data to a better understanding of the role of finerenone across the spectrum of cardio-renal-metabolic health. To date, large-scale outcomes trials of finerenone in CKD have been restricted to those with concomitant diabetes. Example 2 will be the first outcomes trial to test the efficacy and safety of finerenone in patients without diabetes and across a broad range of renal function (down to 25mL/min/1.73m2) and will complement data from the FIND-CKD trial (ClinicalTrials.gov NCT05047263), a dedicated trial of finerenone in non-diabetic CKD on change in total eGFR slope. Steroidal MRA use in indicated populations of HF is particularly low in patients with comorbid CKD,24 so Example 2 affirming safety of finerenone in this range of kidney function will add confidence to its use at this high-risk intersection. Indeed, a participant-level pooled analysis of Example 2, FIDELIO-DKD, and FIGARO-DKD is prespecified and will help map finerenone's therapeutic effects across cardio-renal-metabolic disease states.

The Example 2 trial will determine the efficacy and safety of the non-steroidal MRA finerenone in a broadly inclusive population of hospitalized and ambulatory patients with mildly reduced or preserved ejection fraction. Example 2 has the potential to inform the safe application and implementation of finerenone in this target population.

### (2) Further statistical analyses

In addition to the main analysis of the primary and secondary endpoints described above sensitivity and supportive analyses will be performed. Unless otherwise specified, all analyses will be performed in the full analysis set.

Plots and summaries of the mean cumulative function for the primary endpoint (Nelsen-Aalen estimate) will be presented by treatment group. The cumulative incidence functions for time to CV death and time to non-CV death will also be calculated using Aalen-Johansen estimates.

As supportive analysis, a time to first composite of HF event or CV death analysis will be performed using a stratified Cox proportional hazard regression analysis. A plot of Aalen-Johansen estimates of the cumulative incidence function will be provided.

As a sensitivity analysis, the number of primary composite events will also be analyzed using a negative binomial regression model including stratification factors and treatment group as covariates and log follow-up time as an offset parameter. A further sensitivity analysis will include baseline LVEF as continuous covariate into the primary analysis model instead of the stratification factor LVEF (<60%, ≥60%).

As further sensitivity analysis, plots and summaries of the mean cumulative function for the primary endpoint will be derived based on a competing-risk approach (Ghosh and Lin, 2000, https://doi.org/10.1111/j.0006-341X.2000.00554.x)
A separate estimate of the treatment effect for CV death as one of the components of the primary endpoint will be obtained. The main cause-specific treatment effect estimate for CV death will be derived from a stratified Cox proportional hazards model for time to CV death and the main p-value from a stratified log-rank test.

A supportive analysis of the primary endpoint will exclude urgent HF visits and consider only CV deaths and HHFs as events. Also, an additional analysis of the primary endpoint will restrict CV deaths to HF-related events and thus will consider HF events and CV deaths due to HF. These analyses will both be performed for total (first and recurrent) events and for first events only.

A total-time approach considering times from randomization to the onset of first, second, third composite event using a Wei, Lin, and Weissfeld (WLW, 1989, https://doi.org/10.1080/01621459.1989.10478873) model will be applied. This model enables analysis of the cumulative effect on the primary endpoint from randomization (i.e. the effect on second event includes the effect on the first, and the effect on third event includes the effects on the first and second). In addition, a conditional gap-time model according to Prentice, Williams and Peterson (PWP,1981, https://doi.org/10.1093/biomet/68.2.373 ) will be applied to obtain HR estimates with 95% CIs for the time from first to second and from second to third event (note that this gives a non-randomized comparison). Both models will employ robust standard errors and include the stratification factors and treatment group as fixed effects.

An "on-treatment" analysis will be performed, including only events occurring up to 30 days after treatment discontinuation and within 5 months after the last visit with complete information on all components of the composite primary endpoint. A 5-month time window is used as visits are 4-monthly and in order to allow for late attendance by an additional 1 month. The on-treatment analysis will be done in the safety analysis set including participants in the full analysis set that have been treated.

The primary analysis for the primary endpoint will also be repeated for the "Total HF events and all-cause mortality" endpoint.

The primary analysis will also be repeated where patients are included with only up to a maximum of 4 composite events, to examine the impact of patients with a large number of events.

An additional analysis of the primary endpoint will include a time-dependent covariate for SGLT-2 inhibitor use.

As a sensitivity analysis for the total HF events endpoint, a joint frailty model with constant baseline hazard for CV death and constant baseline intensity for HF events will be fitted including effects for treatment group, pooled region for stratified analyses and baseline LVEF (<60%, ≥60%). A gamma frailty distribution will be assumed. This model gives a treatment effect on total HF events which is adjusted for a potential treatment effect on CV death.

As supportive analysis for the total HF events endpoint, stratified Cox proportional hazard regression analyses will be performed for the following endpoints and plots of Aalen-Johansen estimates of the cumulative incidence functions will be provided:
- Time to first HF event
- Time to first HHF
- Time to first urgent HF visit

The additional analyses of the secondary time-to-first event endpoints will include an "on treatment analysis" as described above for the primary endpoint.

The proportional hazards assumption will be investigated by including a time-treatment interaction into the model and plotting smoothed Schoenfeld residuals. For the renal endpoint, a time-to-first event analysis will be done separately for each of the components.

A supportive analysis of the KCCQ TSS will apply a worst-case imputation for death which means that if a patient dies, a worst score of 0 for the TSS will be imputed for all subsequent visits after the patient's death. Treatment effects at Month 6, 9 and 12 will also be investigated individually by adding a treatment-by-visit interaction into the model.

A responder analysis for the KCCQ TSS will also be performed, defining patients with an increase of ≥5 points from baseline to Month 12 (or, for those with a baseline score of >95, a score of >95 at Month 12 without decline from baseline) as a responder. All observed values will be included irrespective of any permanent treatment discontinuation. In case of missing data, a patient's last available post-baseline score prior to Month 12 will be used unless the patient died before Month 12 in which case they will be imputed as a non-responder.

Responder status will be analysed using a logistic regression model including treatment, baseline TSS and stratification factors as covariates; the odds ratio and associated 95% CI will be reported. This analysis will be repeated for cut-offs of ≥10 points increase from baseline to Month 12 (or maintaining a score of >90 from baseline to Month 12 without decrease from baseline) and ≥20 points increase (or maintaining a score of >80 without decrease from baseline). These cut-offs correspond to small (≥5), moderate (≥10) and large (≥20) clinically meaningful improvements (Spertus et al, 2005, https://doi.org/10.1016/j.ahj.2004.12.010). A further analysis will define those responders who do not experience a ≥5 points decrease from baseline (or, for those with a baseline score of <5, a score of ≥5 at Month 12). This is equivalent to not experiencing a small deterioration. Te number and percentage of patients who are responders or non-responders per each of the above criteria will be presented at Months 6, 9 and 12.

A second responder analysis for the KCCQ TSS will use the thresholds derived from the anchor-based analyses with the Patient Global Impression of Severity (PGIS) and Patient Global Impression of Change (PGIC) for a clinically meaningful within-patient change in KCCQ TSS at month 6, 9 and 12, respectively, which are performed separately on blinded data. The specific thresholds will be derived separately for each timepoint. and hence may differ for each timepoint.

### (3) Study interventions

Study intervention is defined as any investigational intervention(s), marketed product(s), placebo, or medical device(s) intended to be administered to a study participant according to the study protocol.

### (4) Safety

Safety assessments will include adverse events (AEs), serious adverse events (SAEs), vital signs including heart rate and blood pressure assessment. Safety laboratory tests will include blood chemistry, hematology and urinalysis.

All safety assessments will be performed in the safety analysis set.

The following safety procedures and variables will be assessed during the study:
- SAEs and AEs leading to discontinuation of treatment with study intervention
- Change in body weight from baseline
- Change in serum potassium from baseline
- Number of participants with hyperkalemia (serum potassium > 5.5 mmol/L)
- Number of participants with severe hyperkalemia (serum potassium > 6.0 mmol/L)
- Number of participants with hospitalization for hyperkalemia
- Number of participants permanently discontinuing study intervention due to hyperkalemia
- Change in vital signs (heart rate, SBP and DBP) from baseline
- Change in renal function measured by eGFR (CKD-EPI formula) change from baseline
- Number of participants with hospitalization for worsening of renal function
- Number of participants permanently discontinuing study intervention due to worsening
- of renal function
- Changes in laboratory values from baseline.

AEs that occurred or worsened after the first dose of study drug and up to 3 days after the last dose of study drug will be considered as treatment-emergent AEs (TEAEs).

An overall summary of all AEs and treatment emergent AEs (TEAEs) will be generated by treatment group.

The number and percentage of patients with TEAEs, post-treatment AEs occurring more than 3 days after last intake of study intervention, treatment-emergent SAEs, treatment-emergent study intervention-related AEs, treatment-emergent study intervention-related SAEs, TEAEs causing premature and permanent discontinuation of study intervention, treatment-emergent non-serious AEs, TEAEs by maximum intensity, drug-related TEAEs by maximum intensity will be summarized by treatment group using Medical Dictionary for Regulatory Activity (MedDRA) terms grouped by Primary System Organ Class and Preferred Term.

The number of patients with treatment-emergent (until 3 days after last study intervention administration) abnormal laboratory values above or below the normal range will be tabulated by the laboratory parameter and treatment group.

Summary statistics including changes from baseline will be calculated by treatment group and visit for all quantitative laboratory parameters, i.e. for hematology, clinical chemistry and urinalysis. Geometric statistics and ratios to baseline will be presented for urinary albumin-tocreatinine ratio (UACR), creatinine, and NT-proBNP, instead of arithmetic statistics with changes from baseline. For eGFR the relative change will be displayed in addition to the absolute change from baseline.

Summary statistics for serum potassium, eGFR, and serum creatinine will also be repeated by treatment group and visit separately for each level of the stratification factors (region and LVEF).

The following special safety parameters will be further assessed by displaying the number and percentage of patients with safety events as described below by treatment group, visit, and for any time on treatment (including unscheduled assessments) and until 3 days after last study intervention administration. This will also be performed by stratification factors. The summaries will be provided for the number and percentage of patients with:
- Absolute value of serum potassium >5.0 mmol/L, >5.5 mmol/L and >6 mmol/L
- Relative decrease from baseline in eGFR of ≥25%, ≥30%, ≥40%, ≥50% and ≥57%
- Absolute value of eGFR <30 mL/min/1.73 m²
- Increase from baseline in serum creatinine >0.3 mg/dL and >0.5 mg/dL.

### (5) Dosage

Eligible participants will receive study intervention at the doses illustrated in Table 6 (Table 6: Dosage of Study Intervention for Administration, dispensed as outlined in the SoA (see above Table 1 - schedule of activities). The dose of finerenone will depend on the eGFR value at the Baseline Visit (determined by the local laboratory):
1. Participants with an eGFR ≤60 mL/min/1.73m² will start with 10 mg (dose level 1) and have a maintenance dose of 20 mg (dose level 2). Dose level 1 is the minimum dose and dose level 2 is the maximum permitted dose in this group of patients
2. Participants with an eGFR >60 mL/min/1.73m² will start with 20 mg (dose level 2) and have a maintenance dose of 40 mg (dose level 3). Dose level 1 is the minimum dose and dose level 3 is the maximum permitted dose in this group of patients.

The dose can be up-titrated once the participant has been on a stable dose for 4 weeks (±7 days), either at the next regular visit or at an Up-titration Visit (see Table 6a: Dosage of Study Intervention for Administration). Participants who do not tolerate their starting dose of 20 mg may be down-titrated at any point during the study, including between-scheduled visits if required for safety reasons. These participants may be up-titrated again based on the rules provided in Table 6a or tables 6b, or 6c. If the participant is already at the minimum dose, the study intervention can be interrupted at the investigator's discretion as detailed in Section 6.6, based on blood potassium levels and renal function which will be monitored throughout the study.

**Table 6a: Dosage of Study Intervention for Administration**

| **eGFR value at the Baseline Visit, based on local laboratory results:** | **eGFR 25 to ≤60 mL/min/1.73m** | **eGFR >60 mL/min/1.73m²** |
|---|---|---|
| **Participant randomized to group:** | **Finerenone Placebo** | **Finerenone Placebo** |
| | 10 mg finerenone OD Placebo OD **(Dose Level 1)** | 20 mg finerenone OD Placebo OD **(Dose Level 2)** |
| **Starting** dose: | | |
| | 20 mg finerenone OD Placebo OD **(Dose Level 2)** | |
| **Maintenance** dose: | | 40 mg finerenone OD Placebo OD **(Dose Level 3)** |
| **Minimum** dose after down-titration: | 10 mg finerenone OD Placebo OD 20 mg finerenone OD Placebo OD | 10 mg finerenone OD Placebo OD 40 mg finerenone OD Placebo OD |
| **Maximum** dose after up-titration: | | |
| **Study intervention intake** | One tablet of study intervention OD, preferably in the morning at approximately the same time each day. | |
| | Note: Study intervention will be administered at home, except on the day of the first PK visit when the tablet will be taken at the study site | |
| **Missed intake** | • If discovered within 16 hours after the scheduled time, the participant should take one tablet of study intervention as soon as possible | |
| | • If discovered >16 hours after the scheduled time, this will be considered to be a 'missed' dose and the participant should wait and take the next tablet of study intervention at the usual (scheduled) time. | |
| **Up-titration of dose** | | |
| | Finerenone | |
| | • Up-titrate study intervention to the next possible higher dose based on serum/plasma potassium level | |
| | • eGFR decrease is <30% compared to last scheduled visit. | |
| | Placebo | |
| | • Sham-titrate. | |
| **Down-titration of dose** | | |
| | • If potassium ≥5.5, down-titrate to the next lower dose level in a step-wise manner (dose level 2 to 1, or dose level 3 to 2) | |
| | • If at dose level 1, interrupt study intervention treatment; study intervention should be re-introduced at dose level 1 as soon as the investigator considers it to be medically justified without compromising safety | |
| | • If in the opinion of the investigator, the participant cannot tolerate the maximum dose level of study intervention, the study intervention dose may be reduced to the next lower dose level. | |

**Table 6b: Management and Dosing Adjustments in Response to Changes in Potassium and Renal Function**

| **Serum / plasma potassium (K⁺ mmol/L)** | | | **Action to be taken** |
|---|---|---|---|
| **First sample:** | | | |
| | **<5.0** | | Increase to the next higher dose level (or continue at maximum permitted dose level) |
| | **≥5.0 to <5.5** | | Continue the current dose level |
| | **≥5.5 to <6.0** | | Down-titrate to the next lower dose if possible; if patient is already on dose level 1, interrupt study intervention. K⁺ should be re-checked within 72 h of initial K⁺ result awareness |
| | **≥6.0** | | Interrupt study intervention and K⁺ should be re-checked within 72 h of initial K⁺ result awareness |

| Second and subsequent sample: | | | |
|---|---|---|---|
| **Option a** | | **<5.5** | Continue current dose |
| | | **≥5.5** | Down-titrate to the next lower dose if possible, or interrupt study intervention and recheck K+ |
| **Option b** | | **<5.5** | Restart at dose level 1 |
| | | **≥5.5** | Continue to withhold study intervention, further monitoring of K⁺. Restart at dose level 1 **ONLY** if K⁺ is <5.0 mmol/L |

The following aspects have also to be taken into consideration:
- If the participant is already on dose level 1 no further decrease is possible after interruption, the same dose level should be restarted once serum/plasma potassium falls below 5.5 mmol/L. Serum/plasma potassium is to be measured at a safety visit 4 weeks ± 7 days after re-starting treatment or dose adjustment.
- If the participant is on dose level 1 of study intervention, but hyperkalemia recurs soon after a previous event of hyperkalemia leading to interruption of study intervention, and there is no explanation for the recurring hyperkalemia event other than intake of study intervention, premature and permanent discontinuation of study intervention is recommended.
- In case of hyperkalemia, it is at the investigator's discretion to take measures, including treatment and monitoring in accordance with local practice standards.

**Table 6c: Management and Dosing Adjustments in Response to Changes in Potassium and Renal Function**

| **eGFR (mL/min/1.73 m²) at any time after randomization** | **Action to be taken** | |
|---|---|---|
| Decrease ≥25% and <40% from baseline | • Check for potential reversible causes: | |
| | | • Concomitant medications known to affect renal function (e.g. NSAIDs, antibiotics) |
| | | • Adverse event (e.g. urinary infection, urinary retention, dehydration) |
| | • Address potential reversible causes if considered clinically appropriate | |
| Decrease ≥40% from baseline | • Check for potential reversible causes and address, as above. | |
| | • At the investigator's discretion, study drug can be down-titrated or interrupted as follows: | |
| | | • Further monitor eGFR/creatinine |
| | | • If eGFR/creatinine has reached acceptable levels (to be determined for the individual participant), please re-start study intervention at the next lower dose level (or dose level 1 if the participant was already on this dose). |
| | • Re-test at central laboratory after 4 weeks to confirm eGFR decrease of ≥50% or ≥57%* | |

| | | |
|---|---|---|
| Abbreviations: eGFR = estimated glomerular filtration rate; NSAID = non-steroidal anti-inflammatory drug; * Decrease based on central laboratory data. | | |

### (6) Outcomes:

**Table 7: The primary outcome of Example 2 is the number of CV deaths and HF events up to month 42**

| Primary outcome | | Secondary outcomes | | |
|---|---|---|---|---|
| Number of CV deaths and HF events up to month 42 | | Time to total HF events (first and recurrent) | Time to first occurrence of the composite kidney endpoint: | |
| HF events are: | | | | • Sustained decrease in eGFR ≥50% relative to baseline for ≥4 weeks |
| | • First and recurrent events | Improvement in NYHA class from baseline to Month 12 | | |
| | • HHF or urgent care visit for HF | | | • Sustained eGFR decline <15 ml/min/1.73 m² |
| | | Change in KCCQ-TSS (from baseline to months 6, 9 and 12) | | |
| | | | | • Initiation of dialysis or kidney transplantation |
| | | | Time to death from any cause | |

The following exploratory endpoints are planned to be analyzed:
- Time to first CV hospitalization
- Total number of CV hospitalizations
- Time to first all-cause hospitalization
- Total number of all-cause hospitalizations
- Time to first occurrence of the following composite endpoint: CV death or non-fatalCV event (i.e. non-fatal myocardial infarction, non-fatal stroke, or HHF)
- Time to first occurrence of the following composite endpoint: sustained decrease in eGFR ≥ 57% relative to baseline over at least 4 weeks, or sustained eGFR decline < 15 ml/min/1.73m2 or initiation of dialysis or renal transplantation
- Change in eGFR from baseline
- Mean rate of change in eGFR slope and its subcomponents acute and chronic slope
- Change in UACR from baseline
- Days alive and out of hospital (DAOH)
- Time to new onset of atrial fibrillation
- Change in health-related quality of life summary scores from baseline measured by KCCQ and EuroQol Group 5-dimension 5-level questionnaire (EQ-5D-5L), Patient Global Impression of Change (PGIC) and Severity (PGIS)

Further endpoints can be:
- Clinical endpoints of interest
- Laboratory-based endpoints of interest
- Breakdown of endpoints

### CLINICAL ENDPOINTS OF INTEREST

In addition to the efficacy and safety variables described herein, the effect of finerenone on the following endpoints will be explored. These events that are imbalanced between arms may be analyzed as time-to-event to better understand the time course of event accrual.
- Post-randomization signs and symptoms of HF and an expanded composite outcome of CV death, worsening HF event as defined herein or signs and symptoms of HF
- Cardio-renal composite endpoint which includes components of the primary endpoint and components of the prespecified secondary renal endpoint.
- Renal composite endpoint and cardiorenal composite endpoints analyzed using win statistics (hierarchies to be defined before analysis)
- Achievement of post-randomization blood pressure control in those with blood pressure above established thresholds at baseline, including those with apparent treatment resistant hypertension
- Post-randomization changes in number of blood pressure lowering therapies
- New initiation, discontinuation, or dose changes of diuretics
- New initiation, discontinuation, or dose changes of ACE inhibitor / ARB / ARNI
- New initiation or discontinuation of SGLT2 inhibitors
- New initiation or discontinuation of potassium lowering therapies
- New initiation or discontinuation of potassium supplementation
- Post-randomization open-label use and dosing of mineralocorticoid receptor antagonists (including spironolactone, eplerenone, potassium canrenoate, finerenone)
- First and total investigator-reported fatal or non-fatal myocardial infarction
- First and total investigator-reported fatal or non-fatal stroke
- Cardiac ischemic events including myocardial infarction, unstable angina, unplanned coronary revascularization, and stroke (individually and as a composite)
- Major adverse cardiac events defined either as i) a composite of CV death, non-fatal myocardial infarction, or non-fatal stroke or ii) a composite of death from atherosclerotic CV disease, non-fatal myocardial infarction, or non-fatal stroke
- Proportion of patients with clinically meaningful deterioration (5 point or greater worsening), and small (≥5 point), moderate (≥10 point) and large (≥15 point) improvement in KCCQ-TSS is prespecified herein. Similar responder analyses will be performed for KCCQ-CSS, OSS, PL, QoL and Social Limitations Scores.
- Analysis of individual domains and questions of the KCCQ
- In addition to assessing improvement in NYHA class as prespecified herein, changes in NYHA class will be analyzed on an ordinal basis
- Proportion of patients who become NYHA functional class I post-randomization
- Analysis of days alive and out of hospital/days lost due to death or hospitalization adjusted for quality of life using KCCQ-OSS, EQ-5D-5L, and NYHA
- Outcomes related to new onset AF/AFL and total number of episodes of AF/AFL
- Ventricular arrhythmias, defibrillator discharges, resuscitated cardiac arrest, or sudden death
- Post-randomization HF hospitalizations based on treatment complexity (e.g., standard intravenous diuretics, other intravenous HF therapies, mechanical fluid removal, invasive or non-invasive ventilation, mechanical circulatory support) and treatment course (e.g., intensive care unit requirement, length of stay)

### LABORATORY-BASED ENDPOINTS OF INTEREST

The effect of finerenone will be evaluated for all laboratory measures of interest using each as a continuous variable. The following laboratory-based endpoints will be assessed in addition to the analyses described herein:

### eGFR

∘ The composite renal endpoint as a secondary endpoint is defined as sustained decrease in eGFR ≥50% relative to baseline over at least 4 weeks, or sustained eGFR decline <15ml/min/1.73m2 or initiation of dialysis or renal transplantation. Herein it is prespecifies analyses of sustained declines in eGFR declines reaching ≥50% and ≥57% thresholds from baseline. Lower thresholds (≥30% and ≥40%) for sustained decreases in eGFR will be considered. In addition, a blanking period will be included to account for acute, expected eGFR changes.
∘ Focused examination of the "eGFR dip", the acute changes in eGFR at 1-month and 3-months after randomization
∘ Recalculation of eGFR based on variable calculators (including the 2021 CKD-EPI Equation)
∘ Changes in KDIGO risk categories and transitions in CKD stages post-randomization UACR
∘ New-onset macroalbuminuria (among those with normoalbuminuria or microalbuminuria at baseline) or microalbuminuria (among those with normoalbuminuria at baseline)
∘ Regression to normoalbuminuria or microalbuminuria (among those with macroalbuminuria at baseline)

### Potassium

∘ New onset hypokalemia (<3.5 mmol/L)
∘ Assessment of mortality and cardiovascular events for any given potassium level in both arms

### Sodium

∘ Development of hypo- and hyper- natremia during follow up and resolution of hypo- and hyper-natremia during follow-up. Effect of treatment on sodium as a continuous variable will additionally be examined, as will the association between baseline levels (categories, continuous) and clinical outcomes.

### Chloride

∘ Development of hypo- and hyper- chloremia during follow up and resolution of hypo- and hyper-chloremia during follow-up. Effect of treatment on chloride as a continuous variable will additionally be examined, as will the association between baseline levels (categories, continuous) and clinical outcomes.

### Uric acid

∘ Change in uric acid level with finerenone treatment and incidence of gout. Association between uric acid level at baseline (categories, continuous) and clinical outcomes.

### Hematologic biomarkers

∘ Change in hemoglobin and hematocrit with finerenone treatment and incidence of anemia/resolution of anemia. Association between hemoglobin and hematocrit levels at baseline (categories, continuous), and anemia, and clinical outcomes.
∘ Change in neutrophil/lymphocyte ratio with finerenone treatment. Association between neutrophil/lymphocyte ratio at baseline (categories, continuous) and clinical outcomes.

### Cardiac biomarkers

∘ Changes in NT-proBNP at 3 months and 12 months and proportion of patients with NT-proBNP levels <1,000 pg/mL post-randomization
∘ Changes in high-sensitivity troponin at 3 months and 12 months and proportion of patients with levels in the normal reference range post-randomization

### Renin and aldosterone levels

∘ Change in renin and aldosterone levels and aldosterone renin ratio with finerenone treatment
∘ Association between renin and aldosterone levels and aldosterone renin ratio at baseline and clinical outcomes
∘ Assessment of proportion of patients with evidence of primary hyperaldosteronism based on renin and aldosterone levels
   Other biomarkers will be analyzed at baseline, month 3, month 12, and during the end of study visit. Finerenone's treatment effects on these biomarkers over time and the association between these biomarkers at baseline (categories, continuous) will be assessed.
∘ Biomarkers of fibrosis including but not limited to N-terminal propeptide of collagen I and III, tissue inhibitor of matrix metalloproteinase 1, carboxyl-terminal telopeptide of collagen type I, and soluble ST2
∘ Growth/differentiation factor-15
∘ Cancer antigen 125
∘ High sensitivity C-reactive protein
   In addition to conventional cardiac biomarkers (e.g. natriuretic peptides and troponins), FINEARTS-HF will prospectively collect exploratory biomarkers that relate to its unique mode of action. These biomarker profiles may help to further distinguish finerenone's mechanistic effects compared with those of conventional MR antagonists.

### Breakdown of Endpoints

- Mode of death (including sudden death, worsening HF death, other CV death, non-CV deaths)
- Reasons for hospitalization (non-CV hospitalization, HF-related hospitalization, and other CV hospitalizations)
- Breakdown of worsening HF events (including urgent visits / Emergency Department stays / oral loop diuretic escalation)
- The primary endpoint of CV death and total HF events and the endpoint of CV death alone will be considered with and without incorporation of unknown / undetermined deaths. In a prespecified exploratory analysis, we will apply a probabilistic model (predetermined prior to database lock) to better distinguish unknown deaths as either CV or non-CV in etiology. This probabilistic model will be built based on known clinical factors that differentially predict adjudicated known cases of CV vs. non-CV deaths.

### (7) Baseline Characteristis

In total 6014 participiants were recruited. The baseline characteristic of these patients are shown in table 8, table 9 and table 10 below.

**Table 8a: All Example 2 Participants (n=6014)**

| | | | | |
|---|---|---|---|---|
| Age (years) | | 72±10 | Heart Rate (beats/min) | 71±12 |
| Female Sex | | 45% | Systolic Blood Pressure (mmHg) | 129±15 |
| BMI (kg/m2) | | 30±6 | NT-proBNP (ng/L) (median) | 1502 ¹⁾ |
| | | | | 1041 ²⁾ |
| Race | | | eGFR (mL/min/1.73m²) | 62±20 |
| | Asian | 17% | eGFR >=60 | 52% |
| | Black | 2% | Prior HF Hospitalization | 60% |
| | Other | 3% | History of LVEF <=40% | 5% |
| | White | 79% | History of Diabetes | 41% |
| Asia | | 16% | History of Atrial Flutter/Fibrillation | 55% |
| Eastern Europe | | 44% | History of Hypertension | 89% |
| Latin America | | 11% | History of Myocardial Infarction | 26% |
| North America | | 8% | History of Stroke | 14% |
| Western Europe, Oceania and Others | | 21% | Loop Diuretic | 98% |
| NYHA class | | | Beta-blocker | 85% |
| | II | 69% | Ace Inhibitor (ACEi) | 36% |
| | III | 30% | Angiotensin Receptor Blocker (ARB) | 44% |
| | IV | 0.7% | Angiotensin Receptor-Neprilysin Inhibitor (ARNI) | 9% |
| KCCQ-TSS | | 67±24 | Calcium Channel Blockers | 33% |
| LVEF (%) | | 53±8 | Sodium-glucose Cotransporter-2 Inhibitor (SGLT-2i) | 14% |

The baseline mean LVEF was 53 ± 8% (range, 34 to 84%); 36% of participants had a LVEF <50% and 64% had a LVEF ≥50%. The median NT-proBNP was 1500 [527, 2962] ¹⁾ and 1041 [449-1946] ²⁾ pg/ml. 1219 (20%) patients were enrolled during or within 7 days of a worsening HF event, and 3247 (54%) patients were enrolled within 3 months of a worsening HF event. Compared with prior large-scale HFmrEF/HFpEF trials, FINEARTS-HF participants were more likely to have recent (within 6 months) HF hospitalization, higher NT-proBNP levels, and greater symptoms and functional limitations. Further, concomitant medications included a larger percentage of sodium-glucose co-transporter 2 inhibitors and angiotensin receptor-neprilysin inhibitors than previous trials.

Definition of ¹⁾ and ²⁾ in Example 1b.

**Table 8b: Further Example 2 Participants - Baselinecharacteristics**

| | | p-value |
|---|---|---|
| LVEF (%) | Mean value +/- | |
| KCCQ-TSS | Mean value +/- | |
| eGFR (mL/min/1.73m²) | Mean value +/- | |
| NT-proBNP (ng/L) (median) | Mean value +/- | |
| Baseline UACR mg/g | Mean value +/- | |
| Baseline UACR category, mg/g | | |
| <30 | In% | |
| 30-299 | In% | |
| ≥300 | In% | |
| Potassium level (mmol/L) | Mean value +/- | |
| Baseline heart rate, beats/min | Mean value +/- | |
| Baseline systolic blood pressure, mmHg | Mean value +/- | |

**Table 9a: Baseline Characteristics by Ejection Fraction (The values may be rounded.)**

| | | LVEF <50 (n=2179) | LVEF ≥50 to <60 (n=2659) | LVEF ≥60 (n=1145) | P-value |
|---|---|---|---|---|---|
| Age | | 70±10 | 73±9 | 74±9 | <0.001 |
| Female Sex | | 31% | 51% | 59% | <0.001 |
| LVEF (%) | | 44±3 | 54±3 | 64±5 | <0.001 |
| NYHA class | | | | | 0.31 |
| | II | 69% | 68% | 71% | |
| | III | 31% | 31% | 28% | |
| | IV | 0.6% | 0.9% | 0.5% | |
| KCCQ-TSS | | 69±24 | 66±24 | 66±24 | <0.001 |
| eGFR (mL/min/1.73m²) | | 65±20 | 61±19 | 60±19 | <0.001 |
| NT-proBNP (ng/L) (median) | | 1661¹⁾ and 1139²⁾ | 1470¹⁾ and 1008²⁾ | 1305¹⁾ and 941²⁾ | <0.001 |
| History of Atrial Flutter/Fibrillation | | 50% | 59% | 55% | <0.001 |
| History of Diabetes | | 40% | 41% | 41% | 0.72 |
| Prior HF Hospitalization | | 67% | 59% | 51% | <0.001 |
| History of LVEF <=40% | | 9% | 3% | 1% | <0.001 |

| | | | | | |
|---|---|---|---|---|---|
| Definition of ¹⁾ and ²⁾ in Example 1b. | | | | | |

**Table 9b: Baseline Characteristics by Ejection Fraction (The values may be rounded.)**

| | | **Baseline LVEF Group** | | | |
|---|---|---|---|---|---|
| **Characteristic** | | **<50% (n=2172)** | **51-59% (n=2674)** | **≥60% (n=1147)** | ***P* value** |
| Age, y | | 69.6 ± 10.1 | 73.3 ± 9.1 | 73.5 ± 9.2 | <0.001 |
| Women | | 679 (31.3%) | 1367 (51.1%) | 679 (59.2%) | <0.001 |
| Race | | | | | <0.001 |
| | Asian | 432 (20.0%) | 359 (13.5%) | 205 (17.9%) | |
| | Black | 23 (1.1 %) | 36 (1.3 %) | 29 (2.5 %) | |
| | Other | 50 (2.3 %) | 88 (3.3 %) | 27 (2.4 %) | |
| | White | 1659 (76.7%) | 2185 (81.9%) | 883 (77.2%) | |
| Geographic region | | | | | <0.001 |
| | Asia | 429 (19.8%) | 355 (13.3%) | 199 (17.3%) | |
| | Eastern Europe | 1007 (46.4%) | 1140 (42.6%) | 503 (43.9%) | |
| | Latin America | 255 (11.7%) | 281 (10.5%) | 105 (9.2 %) | |
| | North America | 125 (5.8 %) | 218 (8.2 %) | 123 (10.7%) | |
| | Western Europe, Oceania, Others | 356 (16.4%) | 680 (25.4%) | 217 (18.9%) | |
| History of diabetes | | 859 (39.5%) | 1091 (40.8%) | 470 (41.0%) | 0.61 |
| History of myocardial infarction | | 195 (9.0 %) | 67 (2.5 %) | 9 (0.8 %) | <0.001 |
| History of LVEF <40% | | 803 (37.0%) | 569 (21.3%) | 163 (14.2%) | <0.001 |
| Smoking status | | | | | <0.001 |
| | Current | 235 (10.8%) | 188 (7.0 %) | 84 (7.3 %) | |
| | Former | 713 (32.8%) | 748 (28.0%) | 331 (28.9%) | |
| | Never | 1224 (56.4%) | 1738 (65.0%) | 732 (63.8%) | |
| Baseline body mass index, kg/m² | | 29.3 ± 5.9 | 30.3 ± 6.2 | 30.4 ± 6.2 | <0.001 |
| Body mass index groups, kg/m² | | | | | <0.001 |
| | <18.5 (underweight) | 509 (23.5%) | 515 (19.3%) | 217 (19.0%) | |
| | 18.5-<25 (normal weight) | 755 (34.8%) | 873 (32.8%) | 360 (31.4%) | |
| | 25-<30 (overweight) | 532 (24.5%) | 697 (26.2%) | 314 (27.4%) | |
| | 30-<35 (class I obesity) | 30 (1.4 %) | 21 (0.8 %) | 14 (1.2 %) | |
| | ≥35 (class II-III obesity) | 344 (15.9%) | 558 (20.9%) | 240 (21.0%) | |
| Any prior HF hospitalization | | 1449 (66.7%) | 1582 (59.2%) | 581 (50.7%) | <0.001 |
| Prior HF hospitalization (≤6 months) | | 1143 (52.6%) | 1246 (46.6%) | 433 (37.8%) | <0.001 |
| Prior HF hospitalization (≤1 year) | | 1216 (56.0%) | 1339 (50.1%) | 467 (40.7%) | <0.001 |
| Recency of worsening HF event* | | | | | <0.001 |
| | ≤7 days | 483 (22.2%) | 549 (20.5%) | 187 (16.3%) | |
| | >7 days-≤3 months | 792 (36.5%) | 941 (35.2%) | 288 (25.1%) | |
| | >3 months or no worsening HF event | 897 (41.3%) | 1184 (44.3%) | 672 (58.6%) | |
| KCCQ - Total Symptom Score | | 69.2 ± 23.9 | 65.9 ± 23.8 | 65.5 ± 23.9 | <0.001 |
| NYHA functional class at baseline | | | | | 0.13 |
| | II | 1499 (69.0%) | 1827 (68.3%) | 815 (71.1%) | |
| | III | 661 (30.4%) | 824 (30.8%) | 325 (28.4%) | |
| | IV | 12 (0.6 %) | 23 (0.9 %) | 6 (0.5 %) | |
| Baseline LVEF, % | | 44.4 ± 2.8 | 54.2 ± 2.9 | 64.0 ± 4.6 | <0.001 |
| Baseline NT-proBNP, pg/mL | | 1661 [587, 3390]¹⁾ and 1139 [506-2205]²⁾ | 1472 [490, 2870]¹⁾ and 1008 [426-1880] ²⁾ | 1301 [502, 2636] ¹⁾ and 941 [406-1776] ²⁾ | <0.001 |
| Baseline ECG AF | | 1401 (64.5%) | 1575 (58.9%) | 726 (63.3%) | <0.001 |
| Baseline heart rate, beats/min | | 72.1 ± 11.8 | 71.5 ± 11.7 | 70.4 ± 11.8 | <0.001 |
| Baseline heart rate (AF), beats/min | | 77.4 ± 12.4 | 76.2 ± 12.3 | 75.5 ± 12.8 | 0.024 |
| Baseline heart rate (non-AF), beats/min | | 69.1 ± 10.4 | 68.1 ± 10.1 | 67.4 ± 10.1 | <0.001 |
| Baseline systolic blood pressure, mmHg | | 127.5 ± 14.9 | 130.2 ± 15.5 | 131.2 ± 15.3 | <0.001 |
| Baseline eGFR, mL/min/1.73 m² | | 64.8 ± 20.1 | 61.0 ± 19.3 | 59.6 ± 19.4 | <0.001 |
| Baseline eGFR ≥60 mL/min/1.73 m² | | 1243 (57.2%) | 1330 (49.7%) | 537 (46.8%) | <0.001 |
| Baseline Potassium (mmol/L) | | 4.42 ± 0.49 | 4.35 ± 0.47 | 4.34 ± 0.44 | < 0.001 |
| Baseline UACR category, mg/g | | | | | 0.98 |
| | <30 | 619 (29.4%) | 753 (29.4%) | 338 (30.2%) | |
| | 30-299 | 1274 (60.5%) | 1559 (60.8%) | 674 (60.2%) | |
| | ≥300 | 213 (10.1%) | 253 (9.9 %) | 108 (9.6 %) | |
| Baseline pharmacotherapy | | | | | |
| | β-blocker | 1918 (88.3%) | 2243 (83.9%) | 926 (80.7%) | <0.001 |
| | ACEi | 870 (40.1%) | 890 (33.3%) | 394 (34.4%) | <0.001 |
| | ARB | 957 (44.1%) | 1160 (43.4%) | 495 (43.2%) | 0.85 |
| | ARNI | 340 (15.7%) | 144 (5.4 %) | 26 (2.3 %) | <0.001 |
| | SGLT2i | 335 (15.4%) | 364 (13.6%) | 113 (9.9 %) | <0.001 |
| | Loop diuretic | 1965 (90.5%) | 2304 (86.2%) | 941 (82.0%) | <0.001 |
| | CCB | 516 (23.8%) | 945 (35.3%) | 502 (43.8%) | <0.001 |

| | | | | | |
|---|---|---|---|---|---|
| Definition of ¹⁾ and ²⁾ in Example 1b. | | | | | |

Values are n (%), mean ± SD, or median [IQR]. P value reflects global comparison across subgroups defined by the recency of HF event before randomization. *: Measured relative to randomization.
**: Baseline UACR unavailable in 204 participants, hence percentages are expressed as the number of participants out of 5797. Abbreviations: ACEi = angiotensin-converting enzyme inhibitor; AF = atrial fibrillation; ARB = angiotensin receptor blocker; ARNI = angiotensin receptor-neprilysin inhibitor; CCB = calcium channel blocker; ECG = electrocardiogram; eGFR = estimated glomerular filtration rate; HF = heart failure; KCCQ = Kansas City Cardiomyopathy Questionnaire; LVEF = left ventricular ejection fraction; NT-proBNP = N-terminal prohormone of B-type natriuretic peptide; NYHA = New York Heart Association; SGLT2i = sodium-glucose co-transporter 2 inhibitor; SR = sinus rhythm; UACR = urinary albumin-to-creatinine ratio.

**Table 9c: Baseline Characteristics by LVEF Group: A total of 2172 (36%) patients had LVEF ≥40 to <50%, 2674 (45%) patients had LVEF ≥50% to <60%, and 1147 (19%) patients had LVEF ≥60%. Participants with a higher LVEF were more likely to be women compared with men. Those with lower LVEF were more likely to have younger age, had a prior myocardial infarction, a prior history of LVEF <40%, a prior HF hospitalization, elevated NT-proBNP levels, and be treated with β-blockers, SGLT2 inhibitors, or an ARNI.**

| | | **Baseline LVEF Group** | | | |
|---|---|---|---|---|---|
| **Characteristic** | | **≥40 to <50% (n=2172)** | **≥50 to <60% (n=2674)** | **≥60% (n=1147)** | **P value** |
| Age, y | | 69.6 ± 10.1 | 73.3 ± 9.1 | 73.5 ± 9.2 | <0.001 |
| Women | | 679 (31.3%) | 1367 (51.1%) | 679 (59.2%) | <0.001 |

| Race | | | | | <0.001 |
|---|---|---|---|---|---|
| | Asian | 432 (20.0%) | 359 (13.5%) | 205 (17.9%) | |
| | Black | 23 (1.1 %) | 36 (1.3 %) | 29 (2.5 %) | |
| | Other | 50 (2.3 %) | 88 (3.3 %) | 27 (2.4 %) | |
| | White | 1659 (76.7%) | 2185 (81.9%) | 883 (77.2%) | |
| Geographic region | | | | | <0.001 |
| | Asia | 429 (19.8%) | 355 (13.3%) | 199 (17.3%) | |
| | Eastern Europe | 1007 (46.4%) | 1140 (42.6%) | 503 (43.9%) | |
| | Latin America | 255 (11.7%) | 281 (10.5%) | 105 (9.2 %) | |
| | North America | 125 (5.8%) | 218 (8.2 %) | 123 (10.7%) | |
| | Western Europe, Oceania, Others | 356 (16.4%) | 680 (25.4%) | 217 (18.9%) | |
| History of chronic obstructive pulmonary disease | | 265 (12.2%) | 346 (12.9%) | 158 (13.8%) | 0.42 |
| History of type 2 diabetes | | 866 (39.9%) | 1096 (41.0%) | 472 (41.2%) | 0.67 |
| History of hypertension | | 1858 (85.5%) | 2411 (90.2%) | 1046 (91.2%) | <0.001 |
| History of myocardial infarction | | 804 (37.0%) | 569 (21.3%) | 163 (14.2%) | <0.001 |
| History of stroke | | 308 (14.2%) | 372 (13.9%) | 146 (12.7%) | 0.50 |
| History of LVEF <40% | | 195 (9.0 %) | 67 (2.5 %) | 9 (0.8 %) | <0.001 |
| Smoking status | | | | | <0.001 |
| | Current | 235 (10.8%) | 188 (7.0%) | 84 (7.3 %) | |
| | Former | 713 (32.8%) | 748 (28.0%) | 331 (28.9%) | |
| | Never | 1224 (56.4%) | 1738 (65.0%) | 732 (63.8%) | |
| Baseline body mass index, kg/m² | | 29.3 ± 5.9 | 30.3 ± 6.2 | 30.4 ± 6.2 | <0.001 |
| Body mass index groups, kg/m² | | | | | <0.001 |
| | <18.5 (underweight) | 30 (1.4 %) | 21 (0.8 %) | 14 (1.2 %) | |
| | 18.5-<25 (normal weight) | 509 (23.5%) | 515 (19.3%) | 217 (19.0%) | |
| | 25-<30 (overweight) | 755 (34.8%) | 874 (32.8%) | 360 (31.4%) | |
| | 30-<35 (class 1 obesity) | 532 (24.5%) | 697 (26.2%) | 314 (27.4%) | |
| | ≥35 (class II-III obesity) | 344 (15.9%) | 558 (20.9%) | 240 (21.0%) | |
| Any prior HF hospitalization | | 1449 (66.7%) | 1582 (59.2%) | 583 (50.8%) | <0.001 |
| Prior HF hospitalization (≤6 months) | | 1143 (52.6%) | 1247 (46.6%) | 435 (37.9%) | <0.001 |
| Prior HF hospitalization (≤1 year) | | 1216 (56.0%) | 1339 (50.1%) | 469 (40.9%) | <0.001 |
| Recency of index HF event* | | | | | <0.001 |
| | ≤7 days | 483 (22.2%) | 549 (20.5%) | 187 (16.3%) | |
| | >7 days to ≤3 months | 792 (36.5%) | 943 (35.3%) | 290 (25.3%) | |
| | >3 months or no index HF event | 897 (41.3%) | 1182 (44.2%) | 670 (58.4%) | |
| KCCQ - Total Symptom Score | | 69.2 ± 23.9 | 65.9 ± 23.8 | 65.5 ± 23.9 | <0.001 |
| NYHA functional class at baseline | | | | | 0.31 |
| | II | 1499 (69.0%) | 1827 (68.3%) | 815 (71.1%) | |
| | III | 661 (30.4%) | 824 (30.8%) | 325 (28.4%) | |
| | IV | 12 (0.6 %) | 23 (0.9 %) | 6 (0.5 %) | |
| Baseline LVEF, % | | 44.4 ± 2.8 | 54.2 ± 2.9 | 64.0 ± 4.6 | <0.001 |
| Baseline hemoglobin A1c, % | | 6.5 ± 1.2 | 6.4 ± 1.2 | 6.4 ± 1.2 | 0.014 |
| Baseline NT-proBNP, pg/mL | | 1661 [587, 3390] ¹⁾ and 1139 [506-2205] ²⁾ | 1472 [490, 2870] ¹⁾ and 1008 [426-1880]²⁾ | 1301 [502, 2636] ¹⁾ and 941 [406-1776] ²⁾ | <0.001 |
| Baseline ECG AF | | 771 (35.5%) | 1099 (41.1%) | 421 (36.7%) | <0.001 |
| Baseline heart rate, beats/min | | 72.1 ± 11.8 | 71.5 ± 11.7 | 70.4 ± 11.8 | <0.001 |
| Baseline heart rate (AF), beats/min | | 77.4 ± 12.4 | 76.2 ± 12.3 | 75.5 ± 12.8 | 0.024 |
| Baseline heart rate (non-AF), beats/min | | 69.1 ± 10.4 | 68.1 ± 10.1 | 67.4 ± 10.1 | <0.001 |
| Baseline systolic blood pressure, mmHg | | 127.5 ± 14.9 | 130.2 ± 15.5 | 131.2 ± 15.3 | <0.001 |
| Baseline eGFR, mL/min/1.73 m² | | 64.8 ± 20.1 | 61.0 ± 19.3 | 59.6 ± 19.4 | <0.001 |
| Baseline eGFR ≤60 mL/min/1.73 m² | | 929 (42.8%) | 1345 (50.3%) | 610 (53.2%) | <0.001 |
| Baseline Potassium, mmol/L | | 4.4 ± 0.5 | 4.4 ± 0.5 | 4.3 ± 0.4 | <0.001 |
| Baseline UACR, mg/g | | 18 [7, 68] | 19 [7, 67] | 19 [7, 62] | 0.82 |
| Baseline UACR category, mg/g** | | | | | 0.98 |
| | <30 | 1274 (60.5%) | 1559 (60/8%) | 674 (60.2%) | |
| | 30 to <300 | 619 (29.4%) | 753 (29.4%) | 338 (30.2%) | |
| | ≥300 | 213 (10.1%) | 253 (9.9 %) | 108 (9.6 %) | |
| Baseline pharmacotherapy | | | | | |
| | β-blocker | 1919 (88.4%) | 2243 (83.9%) | 927 (80.8%) | <0.001 |
| | ACEi | 870 (40.1%) | 889 (33.2%) | 392 (34.2%) | <0.001 |
| | ARB | 955 (44.0%) | 1159 (43.3%) | 492 (42.9%) | 0.82 |
| | ARNI | 339 (15.6%) | 143 (5.3%) | 27 (2.4%) | <0.001 |
| | SGLT2i | 335 (15.4%) | 366 (13.7%) | 113 (9.9%) | <0.001 |
| | Loop diuretic | 1973 (90.8%) | 2318 (86.7%) | 943 (82.2%) | <0.001 |
| | CCB | 516 (23.8%) | 946 (35.4%) | 504 (43.9%) | <0.001 |

| | | | | | |
|---|---|---|---|---|---|
| Definition of ¹⁾ and ²⁾ in Example 1b. | | | | | |

Values are n (%), mean ± SD, or median [IQR]. *P* value reflects global comparison across subgroups defined by the recency of HF event before randomization.
*: Measured relative to randomization.
**: Baseline UACR unavailable in 204 participants, hence percentages are expressed as the number of participants out of 5797.
Abbreviations: ACEi = angiotensin-converting enzyme inhibitor; ARB = angiotensin receptor blocker; ARNI = angiotensin receptor-neprilysin inhibitor; CCB = calcium channel blocker; ECG = electrocardiogram; eGFR = estimated glomerular filtration rate; HF = heart failure; KCCQ = Kansas City Cardiomyopathy Questionnaire; LVEF = left ventricular ejection fraction; NT-proBNP = N-terminal prohormone of B-type natriuretic peptide; NYHA = New York Heart Association; SGLT2i = sodium-glucose co-transporter 2 inhibitor; UACR = urinary albumin-to-creatinine ratio

**Table 10a: Baseline Characteristics in Patients Recently Hospitalized**

| | HHF <= 7 Days Before Randomization (n=1220) | HHF between > 7 days and <= 3 Months Before Randomization (n=2033) | HHF >3 Months Before Randomization or no Prior HHF (n=2761) | P-value |
|---|---|---|---|---|
| Age | 72±10 | 71±10 | 72±9 | <0.001 |
| Female Sex | 48% | 46% | 44% | 0.11 |
| LVEF (%) | 52±8 | 52±7 | 54±8 | <0.001 |
| NYHA class | | | | <0.001 |
| II | 51% | 72% | 75% | |
| III | 47% | 28% | 25% | |
| IV | 1.9% | 0.6% | 0.2% | |
| KCCQ-TSS | 53±24 | 70±23 | 71±22 | <0.001 |
| eGFR (mL/min/1.73m²) | 60±20 | 63±20 | 62±19 | <0.001 |
| NT-proBNP (ng/L) (median) | 1790 ¹⁾ and 1168²⁾ | 1691 ¹⁾ and 1119²⁾ | 1322 ¹⁾ and 952²⁾ | <0.001 |
| History of Atrial Flutter/Fibrillation | 61% | 55% | 53% | <0.001 |
| History of Diabetes | 42% | 41% | 40% | 0.50 |
| Prior HF Hospitalization | 88% | 83% | 32% | <0.001 |

| | | | | |
|---|---|---|---|---|
| Definition of ¹⁾ and ²⁾ in Example 1b. | | | | |

**Table 10b: Baseline Characteristics by Recency of Worsening HF Event Before Randomization: Patients had a mean age of 72 +/- 10 years; 46% were women, and 79% were White. 69% of patients were NYHA class II and 31% NYHA class III or IV. 41% had a history of diabetes and 26% had a history of myocardial infarction. The mean eGFR was 62 mL/min/1.73 m2, and 48% had a baseline eGFR less than 60 mL/min/1.73 m2. Overall, 60% of patients had a prior HF hospitalization. The baseline LVEF was 53 +/- 8% and 271 (4.5%) patients had a history of LVEF <40%. The median baseline NT-proBNP in the overall randomized trial population was 1500 [527, 2962] ¹⁾ and 1041 [449-1946] ²⁾ pg/ml. Among patients with known AFF, the median NT-proBNP was 2619 [1687, 4347] ¹⁾ and 1714 [1152-2807] ²⁾ pg/ml; the median NT-proBNP was 731 [360, 1882] ¹⁾ and 588 [313-1255] ²⁾ pg/ml in patients without AFF. The mean Kansas City Cardiomyopathy Questionnaire (KCCQ) total symptom score (TSS) was 67 +/- 24. The majority of patients (61%) had a urinary-albumin-to-creatinine ratio (UACR) <30 mg/g, 30% had UACR between 30 and 300 mg/g, and 10% had UACR >300 mg/g. Concomitant medications at baseline included beta-blockers (85%), angiotensin-converting enzyme inhibitors (ACEi) or angiotensin receptor blockers (ARB) (79%), an angiotensin receptor-neprilysin inhibitor (ARNI) (9%), sodium-glucose co-transporter 2 (SGLT2) inhibitors (14%), and loop diuretics (87%). Definition of ¹⁾ and ²⁾ in Example 1b.**

| | | | **Recency of Worsening HF Event** | | | |
|---|---|---|---|---|---|---|
| **Characteristic** | | **All FINEARTS-HF Participants (n=6001)** | **≤7 days** | **>7 days to ≤3 months** | **>3 months or no event** | ***P* value*** |
| | | | **(n=1219)** | **(n=2028)** | **(n=2754)** | |
| Age, y | | 72.0 ± 9.6 | 72.2 ± 9.7 | 71.3 ± 10.3 | 72.4 ± 9.1 | <0.001 |
| Women | | 2731 (45.5%) | 583 (47.8%) | 936 (46.2%) | 1212 (44.0%) | 0.06 |
| Race | | | | | | <0.001 |
| | Asian | 996 (16.6%) | 74 (6.1 %) | 507 (25.1%) | 415 (15.1%) | |
| | Black | 88 (1.5 %) | 7 (0.6 %) | 34 (1.7 %) | 47 (1.7 %) | |
| | Other | 165 (2.8 %) | 29 (2.4 %) | 70 (3.5 %) | 66 (2.4 %) | |
| | White | 4735 (79.1%) | 1106 (91.0%) | 1412 (69.8%) | 2217 (80.8%) | |
| Geographic region | | | | | | <0.001 |
| | Asia | 983 (16.4%) | 74(6.1 %) | 505 (25.0%) | 404 (14.7%) | |
| | Eastern Europe | 2650 (44.2%) | 759 (62.3%) | 665 (32.8%) | 1226 (44.5%) | |
| | Latin America | 641 (10.7%) | 122 (10.0%) | 297 (14.7%) | 222 (8.0 %) | |
| | North America | 471 (7.8 %) | 16 (1.3 %) | 120 (5.9%) | 335 (12.2%) | |
| | Western Europe, Oceania, Others | 1256 (20.9%) | 248 (20.3%) | 441 (21.7%) | 567 (20.6%) | |
| History of chronic obstructive pulmonary disease | | 770 (12.8%) | 181 (14.8%) | 251 (12.4%) | 338 (12.3%) | 0.06 |
| History of type 2 diabetes | | 2438 (40.6%) | 510 (41.8%) | 829 (40.9%) | 1099 (39.9%) | 0.50 |
| History of hypertension | | 5323 (88.7%) | 1117 (91.6%) | 1761 (86.8%) | 2445 (88.8%) | <0.001 |
| History of myocardial infarction | | 1539 (25.6%) | 269 (22.1%) | 435 (21.4%) | 835 (30.3%) | <0.001 |
| History of stroke | | 827 (13.8%) | 169 (13.9%) | 301 (14.8%) | 357 (13.0%) | 0.16 |
| History of LVEF <40% | | 271 (4.5 %) | 36 (3.0 %) | 96 (4.7 %) | 139 (5.0 %) | 0.012 |
| Smoking status | | | | | | 0.020 |
| | Current | 509 (8.5 %) | 104 (8.5 %) | 176 (8.7 %) | 229 (8.3 %) | |
| | Former | 1793 (29.9%) | 343 (28.1%) | 567 (28.0%) | 883 (32.1%) | |
| | Never | 3699 (61.6%) | 772 (63.3%) | 1285 (63.4%) | 1642 (59.6%) | |
| Baseline body mass index, kg/m² | | 29.9 ± 6.1 | 30.5 ± 6.2 | 29.4 ± 6.3 | 30.1 ± 5.9 | <0.001 |
| Body mass index groups, kg/m² | | | | | | <0.001 |
| | <18.5 (underweight) | 65 (1.1 %) | 13 (1.1 %) | 37 (1.8 %) | 15 (0.5 %) | |
| | 18.5-<25 (normal weight) | 1241 (20.7%) | 222 (18.3%) | 502 (24.8%) | 517 (18.8%) | |
| | 25-<30 (overweight) | 1990 (33.2%) | 397 (32.6%) | 646 (31.9%) | 947 (34.5%) | |
| | 30-<35 (class I obesity) | 1546 (25.8%) | 319 (26.2%) | 489 (24.1%) | 738 (26.9%) | |
| | ≥35 (class II-III obesity) | 1146 (19.1%) | 265 (21.8%) | 351 (17.3%) | 530 (19.3%) | |
| Any prior HF hospitalization | | 3618 (60.3%) | 1065 (87.4%) | 1685 (83.1%) | 868 (31.5%) | <0.001 |
| Prior HF hospitalization (≤6 months) | | 2827 (47.1%) | 1024 (84.0%) | 1623 (80.0%) | 180 (6.5%) | <0.001 |
| Prior HF hospitalization (≤1 year) | | 3027 (50.4%) | 1034 (84.8%) | 1644 (81.1%) | 349 (12.7%) | <0.001 |
| KCCQ - Total Symptom Score | | 67.0 ± 23.9 | 52.9 ± 23.9 | 70.2 ± 23.3 | 71.0 ± 22.0 | <0.001 |
| NYHA functional class at baseline | | | | | | <0.001 |
| | II | 4145 (69.1%) | 618 (50.7%) | 1455 (71.8%) | 2072 (75.2%) | |
| | III | 1814 (30.2%) | 578 (47.4%) | 559 (27.6%) | 677 (24.6%) | |
| | IV | 41 (0.7%) | 23 (1.9 %) | 13 (0.6 %) | 5 (0.2 %) | |
| Baseline LVEF, % | | 52.6 ± 7.8 | 51.7 ± 7.7 | 51.8 ± 7.3 | 53.5 ± 8.1 | <0.001 |
| Pooled LVEF groups, % | | | | | | <0.001 |
| | ≥40 to <50 | 2172 (36.2%) | 483 (39.6%) | 792 (39.2%) | 897 (32.6%) | |
| | ≥50 to <60 | 2674 (44.6%) | 549 (45.0%) | 943 (46.6%) | 1182 (43.0%) | |
| | ≥60 | 1147 (19.1%) | 187 (15.3%) | 290 (14.3%) | 670 (24.4%) | |
| Baseline hemoglobin Alc, % | | 6.4 ± 1.2 | 6.4 ± 1.2 | 6.4 ± 1.2 | 6.4 ± 1.2 | 0.13 |
| Baseline NT-proBNP, pg/mL | | 1500 [527, 2962] ¹⁾ and 1041 [449-1946] ²⁾ | 1775 [538, 3846] ¹⁾ and 1168 [474-2451] ²⁾ | 1674 [541, 3292] ¹⁾ and 1119 [473-2113] ²⁾ | 1324 [515, 2542] ¹⁾ and 952 [426-1718]²⁾ | <0.001 |
| Baseline ECG AF | | 2293 (38.2%) | 534 (43.8%) | 783 (38.6%) | 976 (35.4%) | <0.001 |
| Baseline heart rate, beats/min | | 71.5 ± 11.8 | 72.5 ± 11.5 | 72.0 ± 12.2 | 70.6 ± 11.6 | <0.001 |
| Baseline systolic blood pressure, mmHg | | 129.4 ± 15.3 | 127.1 ± 13.9 | 128.6 ± 15.9 | 131.0 ± 15.3 | <0.001 |
| Baseline eGFR, mL/min/1.73 m² | | 62.1 ± 19.7 | 60.2 ± 20.0 | 63.3 ± 20.2 | 62.1 ± 19.2 | <0.001 |
| Baseline eGFR <60 mL/min/1.73 m² | | 2888 (48.1%) | 637 (52.3%) | 918 (45.3%) | 1333 (48.4%) | <0.001 |
| Baseline Potassium, mmol/L | | 4.4 ± 0.5 | 4.4 ± 0.5 | 4.4 ± 0.5 | 4.4 ± 0.4 | 0.06 |
| Baseline UACR, mg/g | | 18 [7, 67] | 19 [7, 73] | 19 [7, 72] | 18 [7, 581 | 0.05 |
| Baseline UACR category, mg/g** | | | | | | 0.30 |
| | <30 | 3511 (60.6%) | 711 (60.7%) | 1152 (59.1%) | 1648 (61.5%) | |
| | 30 to <300 | 1712 (29.5%) | 347 (29.6%) | 582 (29.9%) | 783 (29.2%) | |
| | ≥300 | 574 (9.9 %) | 113 (9.6%) | 214 (11.0%) | 247 (9.2 %) | |

| Baseline pharmacotherapy | | | | | | |
|---|---|---|---|---|---|---|
| | β-blocker | 5096 (84.9%) | 1017 (83.4%) | 1701 (83.9%) | 2378 (86.3%) | 0.016 |
| | ACEi | 2154 (35.9%) | 474 (38.9%) | 638 (31.5%) | 1042 (37.8%) | <0.001 |
| | ARB | 2610 (43.5%) | 486 (39.9%) | 948 (46.7%) | 1176 (42.7%) | <0.001 |
| | ARNI | 509 (8.5 %) | 76 (6.2%) | 251 (12.4%) | 182 (6.6%) | <0.001 |
| | SGLT2i | 816 (13.6%) | 188 (15.4%) | 370 (18.2%) | 258 (9.4%) | <0.001 |
| | Loop diuretic | 5240 (87.3%) | 1075 (88.2%) | 1880 (92.7%) | 2285 (83.0%) | <0.001 |
| | CCB | 1970 (32.8%) | 427 (35.0%) | 615 (30.3%) | 928 (33.7%) | 0.009 |

| | | | | | | |
|---|---|---|---|---|---|---|
| Definition of ¹⁾ and ²⁾ in Example 1b. | | | | | | |

Values are n (%), mean ± SD, or median [IQR].
*: *P* value reflects global comparison across subgroups defined by recency of HF event before randomization.
**: Baseline UACR unavailable in 204 participants, hence percentages are expressed as the number of participants out of 5797.
Abbreviations: ACEi = angiotensin-converting enzyme inhibitor; ARB = angiotensin receptor blocker; ARNI = angiotensin receptor-neprilysin inhibitor; CCB = calcium channel blocker; ECG = electrocardiogram; eGFR = estimated glomerular filtration rate; HF = heart failure; KCCQ = Kansas City Cardiomyopathy Questionnaire; LVEF = left ventricular ejection fraction; NT-proBNP = N-terminal prohormone of B-type natriuretic peptide; NYHA = New York Heart Association; SGLT2i = sodium-glucose co-transporter 2 inhibitor; UACR = urinary albumin-to-creatinine ratio

### (8) Further subgroup analyses

Exploratory subgroup analysis will be done for the primary and secondary efficacy variables.

The subgroup analyses will include subgroups based on e.g. the stratification factors. The list of key subgroups (in addition to the stratification factors) and other subgroups analyzed is specified below, e.g.:
- Subgroups based on stratification factors
- Subgroups via demography and other baseline characteristics
- Subgroups by baseline characteristics
- Subgroups by medical history
- SPECIAL POPULATIONS (e.g. Recent Worsening HF Event, Diabetes Status)

Analysis will include descriptive statistics, graphical display of estimated treatment effects with 95% confidence intervals (CIs) in a forest plot and a statistical test for interaction.

The stratified analyses mentioned in the sections above will combine countries/regions into pooled regions as follows:
- Western Europe and Oceania: Australia, Austria, Denmark, Germany, Israel, Netherlands, New Zealand, United Kingdom
- Southwestern Europe: Italy, Portugal, Spain
- Central Europe: Czechia, Hungary, Poland, Slovakia
- Southeastern Europe: Bulgaria, Greece, Romania, Turkey
- Northeastern Europe: Finland, Latvia, Lithuania, Russia, Ukraine
- Asia: China, Hong Kong, India, Japan, Malaysia, South Korea, Taiwan
- North America: Canada, United States of America
- Latin America: Argentina, Brazil, Colombia, Mexico

### Subgroups based on stratification factors

- Pooled region for subgroup analysis (Western Europe, Oceania and Others; Eastern Europe; Asia; North America; Latin America); the subgroup Western Europe, Oceania and Others combines the groups Western Europe and Oceania and Southwestern Europe from the pooled region for stratified analyses. The subgroup Eastern Europe combines the groups Central Europe, Southeastern Europe, and Northeastern Europe from the pooled region for stratified analyses.
- LVEF (<60%, ≥60%)

### Subgroups

- Baseline serum potassium value (≤ 4.5, > 4.5 mmol/L)
- eGFR category at baseline (eGFR <60, ≥60 mL/min/1.73 m²)
- Atrial fibrillation at baseline electrocardiogram (ECG) (present, absent)
- Diabetes Mellitus at baseline (present, absent)
- Index HF event (very recent (≤ 7 days before randomization), recent (> 7 days - ≤ 3
- months), > 3 months or no index HF event)
- Race (white, black, Asian, other)
- Sex (male, female)
- Age (≤ median vs. > median)
- Baseline body mass index (BMI) (< 30 vs. ≤30 kg/m²)
- Systolic blood pressure (SBP) at baseline (≤ median vs. > median)
- Angiotensin-converting enzyme inhibitor (ACEI), angiotensin receptor blocker (ARB) or angiotensin receptor-neprilysin inhibitor (ARNI) use at baseline (yes, no)
- Treatment with sodium-glucose transport proteins-2 inhibitor (SGLT-2i) (yes, no)
- NYHA functional class at baseline (II, III/IV)
- Baseline NT-proBNP (≤ median vs. > median)
- Baseline UACR (<30 vs. ≤30 mg/g)

### Subgroups via demography and other baseline characteristics

Demography includes age (continuous and categorized by 40-<65, 65-<75, 75-<85, ≥85), sex, race, ethnicity, pooled region (for stratified analysis and for subgroup analysis), body weight (continuous and categorized by <60, 60-<90, ≥90 kg), body height, BMI (continuous and categorized by <30 vs. ≤30 as well as by <18.5, 18.5 to <25, 25 to <30, 30 to <35, ≥35 kg/m²), hip and waist circumference and waist-hip ratio, smoking history (never, former, current smoker) and alcohol consumption.

### Subgroups by other baseline characteristics:

- LVEF (continuous and categorized by <60% vs. ≥60% as well as <50, 50-<60, ≥60%)
- NYHA Class (II, III, IV)
- index HF event (randomized during/at HF event, very recent (≤ 7 days from randomization), recent (>7 days - ≤ 3 months), >3 months, no index; as well as ≤ 7 days from randomization, >7 days - ≤ 3 months, >3 months or no index event)
- type of (latest) index HF event with respective timing (hospitalization for heart failure, urgent HF visit, no index event)
- serum potassium (continuous and categorized by ≤4.5 mmol/L vs. >4.5 mmol/L)
- eGFR (calculated by CKD-EPI formula, Japanese formula adjustment made for subjects recruited in Japan; continuous and categorized by <60 vs. ≥60 mL/min/1.73 m² as well as <45, 45 to <60, 60 to <90, ≥90 mL/min/1.73 m²),
- serum creatinine (continuous)
- SBP (continuous and categorized by 90-<130, 130-< 160, ≥160 mmHg)
- diastolic blood pressure [DBP] (continuous)
- heart rate (continuous)
- NT-proBNP (continuous)
- UACR (continuous and categorized by <30 vs. ≤30 as well as <30, 30-<300, ≥300 mg/g)
- history of LVEF<40% (yes [improved], no). For subjects with a history of LVEF <40%, prior LVEF values will be summarized
- Atrial fibrillation at baseline per ECG
- Diabetes Mellitus at baseline
- ACEI, ARB or ARNI use at baseline
- SGLT-2i use at baseline.

### Subgroups by medical history:

Medical history will be coded using the Medical Dictionary for Regulatory Activities (MedDRA). Medical history will be presented for each MedDRA Primary System Organ Class (SOC) and Preferred Term (PT) by treatment group. Additional medical history terms by the following Standard MedDRA Queries (SMQs), Project-specific Bayer MedDRA Queries (PBMQs), Bayer MedDRA Labeling Groupings (MLGs) or selected PTs will also be presented:
- Hyperlipidemia (MLG)
- Hypertension (MLG)
- Type 2 diabetes mellitus (PT)
- Atrial fibrillation/flutter (PBMQ)
- Ischemic Stroke/Transitory Ischemic Attack (TIA) (PBMQ)
- Myocardial Infarction (MLG)
- Coronary Artery Disease (PBMQ)
- Peripheral Arterial Occlusive Disease (PBMQ)
- Cardiac Failure (MLG)
- Coronary Artery Bypass Graft (CABG) (PBMQ)
- Chronic Obstructive Pulmonary Disease (COPD) (PT)
- Percutaneous coronary intervention (PCI) (PBMQ)
- COVID-19 (SMQ narrow)
- Liver cirrhosis (PT)
- Sleep Apnea Syndrome (PT)
- Chronic Kidney Disease (CKD) (PT)

### Subgroups by concomitant therapy:

Concomitant medications will be coded using the World Health Organization Drug Dictionary (WHO-DD). The number of subjects who took at least one concomitant medication, the number of subjects who took at least one medication that started before administration of study drug and the number of subjects who took at least one concomitant medication that started after start of study drug and the number of subjects who took at least one medication ongoing at baseline (i.e. starting before or on the day of randomization and ending at least one day after the day of randomization) drug will be presented by treatment group and overall using anatomical therapeutic chemical (ATC) classes and subclasses. These tables will be repeated summarizing the number of subjects with medications in the Standard drug groups of interest:
- ACEIs and ARBs
- ARNI
- Beta-blockers
- Loop diuretics
- Thiazide diuretics
- Digoxin
- Nitrates
- Potassium supplements
- Potassium lowering agents (including binders)
- Alpha blocking agents
- Calcium channel blockers
- Centrally acting antihypertensives
- Strong, unclassified, moderate, weak cytochrome P450 isoenzyme 3A4 (CYP3A4)
- inhibitors and CYP3A4 inducers
- Aspirin
- Statins
- History of prior MRA use (yes, no)
- Organic anion transporting polypeptides (OATP) substrates

### Anti-diabetic drugs

- Insulin and analogues
- SGLT-2 inhibitors
- Other anti-diabetic drugs (Dipeptidyl Peptidase 4 inhibitors or Glucagon-like peptide-1 agonists or Biguanides or Sulfonylureas or Alpha glucosidase inhibitors or Metiglinides or Thiazolidinediones.)

### Special Populations

### Recent Worsening HF Event:

Example 2 is specifically enriched to examine the population of patients with recent worsening HF. Depending on the analysis, subpopulations of interest may be considered including randomization at the time of a hospitalization for HF or urgent HF visit, within 7 days of worsening HF, and within 3 months of worsening HF.
- Among those who are hospitalized at the time of randomization, 30-, 60-, and 90-day readmissions (all-cause, HF-related, and CV-related) will be examined
- Early changes in diuretic use and dosing
- Early trajectory of health status and quality of life
- Early safety and tolerability with particular attention to blood pressure and renal function changes
- No prior trial of HFmrEF/HFpEF have specifically targeted those with recent worsening heart failure event, a strategy of enrichment that has been employed in HFrEF¹. In Example 2, the proportion of patients without recent worsening HF event in the 3 months prior to enrollment were prospectively planned to account for approximately half of the trial population. Indeed, 54% of all participants in Example 2 have had a worsening heart failure within 3 months of enrollment. This resulted in a somewhat higher risk population than seen in prior trials, as evidenced by the highest natriuretic peptide levels and highest symptom burden (based on the lowest baseline KCCQ-total symptom score). Participants with recent worsening heart failure events are anticipated to face relatively higher event rates. In addition, 12% of Example 2 participants were enrolled during an episode of worsening HF. Recent data have suggested that rapid implementation of guideline-directed medical therapies including MRAs appears safe and highly effective after a hospitalization for HF. FINEARTS-HF will add placebo-controlled prospective data informing the safety and efficacy of in-hospital initiation of non-steroidal MRAs.
- Recency of HF Events Prior to Randomization in Example 2: The Example 2 trial population can be segmented according to timing from most recent HF event (HF hospitalization or urgent HF visit) prior to trial enrollment: 749 (12%) [during index HF event], 470 (8%) [equal or below 7 days], 2024 (34%) [above 7 days to 3 months], 937 (16 %) [above 3 months], 1821 (30 %) [no index HF event].

### Diabetes Status:

As finerenone was previously studied in patients with chronic kidney disease exclusively with comorbid diabetes, Example 2 will provide the largest examination to date regarding the use of finerenone in patients without diabetes.
- Treatment effects on primary and secondary endpoints will be assessed by glycemic categories (no diabetes, prediabetes, and T2D), across HbA1c as a continuous measure, and across various background anti-hyperglycemic therapies including insulin
- In the T2D subgroup, new anti-hyperglycemic therapy initiation and changes in insulin dose (in those on insulin at baseline) will be examined
- In the T2D subgroup, changes in hemoglobin A1c will be examined
- In the T2D subgroup, assessment of the primary and secondary endpoints will be performed by background anti-hyperglycemic therapies including focused examination of patients on various combinations of therapies
- In the non-T2D subgroup, new diagnosis of diabetes will be examined
- Focused assessment of the renal endpoints as described herein will be performed separately in patients with and without diabetes
- Focused assessment of safety and tolerability as described herein will be performed separately in patients with and without diabetes

### Albuminuria:

Example 2 targeted a high-risk population at the intersection of cardio-kidney-metabolism. Despite an average eGFR >60mL/min/1.73m2, almost 40% of participants had evidence moderately or severely increased levels of albuminuria.

### Further subgroups:

The following subgroups of interest will be explored to examine event rates and for consistency of efficacy and safety of finerenone. All subgroups will be identified based on randomization or pre-randomization data unless otherwise specified. For each subgroup, we will assess the treatment effect and interaction with treatment for the primary endpoint and each of the secondary endpoints, including components of the primary endpoint. For laboratory variables, the association between baseline levels and outcomes will be evaluated, along with the effect of finerenone on these variables.
- Potassium subgroups are specified above according to the following cutpoints (≤4.5, >4.5 mmol/L). Additional subgroups defined by median, quantiles, and other standard definitions of hypo-, hyper- and normo-kalemia will be assessed. Potassium will additionally be examined as a continuous function.
- Sodium subgroups defined by the median, quantiles, and standard definitions of hypo-, hyper- and normo-natremia. Sodium will additionally be examined as a continuous function.
- Chloride subgroups defined by the median, quantiles, and standard definitions of hypo-, hyper- and normo-chloremia. Chloride will additionally be examined as a continuous function.
- Hemoglobin and hematocrit subgroups defined by the median, quantiles, and standard definitions of anemia. Hemoglobin and hematocrit will additionally be examined as a continuous function.
- eGFR subgroups are specified above according to the following cutpoints <60, ≥60 mL/min/1.73 m². eGFR categories will additionally be evaluated according the full KDIGO risk categories and treatment effects will be examined across eGFR as a continuous function
- Focused examination of Stage 4 CKD (if eGFR was less than 30mL/min/1.73m2 at randomization or at any post-randomization measurement)
- UACR subgroups are specified above according to the following cutpoints (<30 vs. ≤30 mg/g). UACR will additionally as a continuous function.
- BUN and BUN/creatinine ratio subgroups defined by the median and quantiles.
- BUN and BUN/Cr ratio will additionally be examined as a continuous function.
- Patients suspected to have primary hyperaldosteronism based on renin and aldosterone levels
- BMI subgroups are specified above according to the following cutpoints (<30kg/m²≥30kg/m²). BMI categories will additionally be evaluated according to the full WHO classification and treatment effects will be examined across BMI as a continuous function. Similar analyses will be used for other recognized anthropometric measures e.g., waist-to-height ratio, waist-hip ratio, body roundness index, examining these as categorical variables using recognized cut points, medians, quantiles, and as continuous variables.
- Subgroups defined by standard nutritional indices e.g., the geriatric nutritional risk index (GNRI), prognostic nutritional index (PNI), and controlling nutritional status (CONUT), examining these as categorical variables using recognized cut points, medians, quantiles, and as continuous variables.
- Age subgroups are specified above according to the following cutpoints (median age). Specific evaluation of older age categories will be considered and treatment effects will be examined across age as a continuous function
- Race subgroups are specified above. Focused examination of patients who are Black, Asian, and Native American
- Systolic blood pressure subgroups are specified above according to the following cutpoints (median age). SBP and DBP will be assessed at the median, quantiles, and various global definitions of hypertension (based on both SBP and DBP). Treatment effects will be examined across SBP and DBP as a continuous function
- Patients with apparent treatment resistant hypertension
- Atrial fibrillation on baseline ECG is a prespecified subgroup above . Atrial fibrillation or atrial flutter on baseline ECG will be further examined. In addition, a previous history of atrial fibrillation/flutter will be assessed, together with the overlap between medical history and baseline ECG and derived history of persistent/permanent AF versus paroxysmal AF.
- Patients with improved/recovered LVEF (those who had LVEF ≤40% at any time prior to randomization)
- LVEF is a stratification variable at the following cutpoint (<60%, ≥60%). Different cutpoints (≤ 49%, 50 to 59%, ≥60%) will also be applied to examine those with HF with mildly reduced ejection fraction. Additional LVEF subgroups to limit digit preference will be considered and treatment effects will be examined across LVEF as a continuous function. In addition, the two-way interaction between sex and LVEF will be examined.
- Other anthropometric indices e.g., waist-to-hip ratio, waist-to-height ratio using quantiles and recognized cutpoints
- Time from prior HF hospitalization as a continuous measure
- Time from index HF diagnosis
- Patients with COPD
- Patients with OSA
- Patients with cirrhosis
- Patients with any atherosclerotic cardiovascular disease
- Patients with history of stroke/TIA
- Patients with history of coronary artery disease / prior MI
- Patients with history of coronary artery bypass graft surgery
- Patients with peripheral artery disease
- Patients with metabolic syndrome (using standard definitions)
- Subgroups based on baseline use and dosing of diuretics
- Patients with multimorbidity and frailty
- Patients with cardio-renal-metabolic overlap (intersection of atherosclerotic cardiovascular disease, CKD, and diabetes)
- Total baseline medications will be categorized as ("non-polypharmacy": <5 medications; "polypharmacy": 5 to 9 medications; and "hyperpolypharmacy": ≥10 medications) and also assessed as a continuous function
- Number of blood pressure lowering therapies at baseline
- Patients with baseline risk as determined by the MAGGIC and other HF risk scores
- Patients with baseline risk as determined by the Kidney Failure Risk Equation
- Subgroups based on baseline evidence of congestion and congestion scores
- Regional subgroups based on socioeconomic differences based on the GINI coefficient
- Subgroups based on KCCQ-TSS and other KCCQ domains at baseline.
- Subgroups based on EQ-5D-5L VAS and index scores at baseline.
- Time from diagnosis of heart failure, analyzed as both a continuous and a categorical variable
- Time from HF hospitalization continuously and focused examination for those never hospitalized
- NT-proBNP and high-sensitivity troponin will be examined at median, quantiles, and as continuous measures
- History of any prior MRA use prior to randomization
- Background HF therapies including focused examination of patients on various combinations of therapies (including the Heart Failure Collaboratory score)
- Patients on or off β-blockers at baseline
- Patients on or off potassium supplements at baseline
- Patients on or off potassium lowering therapies at baseline
- Patients with phenotypic characteristics of amyloid cardiomyopathy, based on validated clinical models/scores

**The trial according to Example 2 was performed. The following examples describe e.g. outcomes and subgroups:**

### Example 3 - Finerenone in Heart Failure with Mildly Reduced or Preserved Ejection Fraction:

### Design and Baseline Characteristics of the Example 2 Trial

Over 6000 patients (mean age 72 ± 10 years; 45% women, 41% type 2 diabetes, LVEF 53 ± 8%) from 635 sites across 37 countries were randomized. 12% were enrolled in hospital, 8% were enrolled within 7 days of a HF hospitalization or urgent HF visit, 34% were enrolled between 8 and 90 days of a HF hospitalization or urgent HF visit, and 46% had no recent HF hospitalization or urgent HF visit.19% had an LVEF≥60%. Comorbidities were common and 54% of patients had a history of atrial fibrillation/flutter. Notable differences in baseline characteristics are discernible among different enrollment subgroups in the Example 2 study and comparative trials. For example, in Example 2, patients enrolled during a hospitalization or within seven days of a worsening HF episode, constituting 48% females and a mean LVEF of 51.7±7.7, represented a comparatively higher risk group. This subgroup demonstrated a higher prevalence of atrial fibrillation both historically (60%) and at baseline (45%), a higher median N-terminal prohormone of brain natriuretic peptide (NT-proBNP) level (1797 ¹⁾ and 1168 ²⁾), and greater hypertension incidence (91%) than other subgroups. In a broader comparison, the Example 2 cohort exhibits more severe health status than those in the EMPEROR Preserved and DELIVER trials. This is suggested by a lower proportion of patients in the NYHA class II (69% in Example 2 versus 82% and 75% in EMPEROR Preserved and DELIVER respectively), and higher median NT-proBNP levels (1509') and 1041 ²⁾) in Example 2 compared to 974 in EMPEROR Preserved and 1011 in t DELIVER trials).
Definition of ¹⁾ and ²⁾ in Example 1b.

Example 2 has enrolled a broad global population of patients with HFmrEF/HFpEF. Over half were enrolled during or within 90 days of an episode of worsening HF. We will present the design and baseline data from Example 2 in the context of other contemporary trials in similar patients.

### Example 4 - Finerenone in Heart Failure with Mildly Reduced or Preserved Ejection Fraction:

### Design and Baseline Characteristics of the Example 2 Trial

Over 6000 patients (mean age 72 +/- 10 yrs; 45% women, 41% T2D, LVEF 53 ± 8%; Table 11: Baseline Characteristics of Example 2, Example 2 subgroups, and other recent trials in HFmrEF or HFpEF) from 635 sites across 37 countries were randomized. 12% were enrolled in hospital, 8% were enrolled within 7 days of a HF hospitalization or urgent HF visit, 34% were enrolled between 8 and 90 days of a HF hospitalization or urgent HF visit, and 46% had no recent HF hospitalization or urgent HF visit. 19% had an LVEF≥60%. Comorbidities were common and 54% of patients had a history of atrial fibrillation/flutter at enrollment. Baseline characteristics differed substantially by enrollment subgroup (Table: Baseline Characteristics of Example 2, Example 2 subgroups, and other recent trials in HFmrEF or HFpEF). Participants in Example 2 represent a higher risk cohort with worse functional class and higher natriuretic peptides than in prior trials of HFmrEF/HFpEF (Table 11: Baseline Characteristics of Example 2, Example 2 subgroups, and other recent trials in HFmrEF or HFpEF).

Example 2 has enrolled a broad global population of patients with HHFmrEF/HFpEF. Over half were enrolled during or within 90 days of an episode of worsening HF. We present the design and baseline data from Example 2 in the context of other contemporary trials in similar patients:

**Table 11: Baseline Characteristics of Example 2, Example 2 subgroups, and other recent trials in HFmrEF or HFpEF**

| | | **Example 2 Subgroups based on Setting/Timing of Enrollment** | | | | |
|---|---|---|---|---|---|---|
| **Characteristic** | **Example 2 Overall (N=6,016)** | **Enrolled in Hospital or within 7 days of worsening HF episode (N=1,226)** | **Enrolled between 8 and 90 days after worsening HF episode (N=2,031)** | **No recent worsening HF episode (N=2,759)** | **EMPERO R Preserved (N=5,988)** | **DELIVER (N=6,263)** |
| **Age (mean ± SD)** | 72 ± 10 | 72 ± 10 | 71± 10 | 72± 9 | 72±9 | 72±10 |
| **Female** | 45% | 48% | 46% | 44% | 45% | 44% |
| **LVEF (mean ± SD)** | 52.5 ± 7.8 | 51.7 ± 7.7 | 51.8 ± 7.4 | 53.5 ± 8.0 | 54 ± 9 | 54 ± 9 |
| **eGFR (mean ± SD)** | 62.7 ± 19.9 | 61.1 ± 20.0 | 64.0 ± 20.8 | 62.5 ± 19.1 | 61 ± 20 | 62 ± 19 |
| **NT-proBNP (median)** | 1509 ¹⁾ and 1041 ²⁾ | 1797 ¹⁾ and 1168²⁾ | 1690 ¹⁾ and 1119²⁾ | 1331¹⁾ and 952²⁾ | 974 | 1011 |
| **NYHA II** | 69% | 51% | 72% | 75% | 82% | 75% |
| **History of AF** | 54% | 60% | 54% | 52% | 52% | 56% |
| **AF at baseline** | 39% | 45% | 39% | 36% | 35% | 42% |
| **History of Hypertension** | 88% | 91% | 86% | 88% | 90% | 89% |
| **Type 2 diabetes mellitus** | 41% | 42% | 41% | 40% | 49% | 45% |
| **History of Myocardial infarction** | 26% | 22% | 21% | 30% | 29% | 26% |
| **MEDICATION S** | | | | | | |
| **Diuretics** | 99% | 99% | 99% | 100% | 86% | 98% |
| **Beta-blockers** | 86% | 86% | 84% | 86% | 86% | 76% |
| **Angiotensin Receptor Blockers** | 45% | 43% | 47% | 43% | 39% | 34% |
| **ACE-inhibitors** | 38% | 43% | 34% | 39% | 40% | 33% |
| **ARNI** | 8.6% | 6.3% | 13% | 7% | 2% | 4% |
| **Calcium channel blockers** | 33% | 37% | 31% | 32% | 31% | 31% |
| **SGLT-2 inhibitors** | 13% | 14% | 18% | 9% | - | - |

| | | | | | | |
|---|---|---|---|---|---|---|
| Definition of ¹⁾ and ²⁾ in Example 1b. | | | | | | |

### Example 5 - Pain and anxiety in heart failure - findings from Example 2

Usually, assessment of health status in heart failure is focused on typical symptoms such as dyspnoea and fatigue. However, patients may suffer other physical and psychological consequences of heart failure and associated comorbidities, which can be evaluated using generic health-related quality-of-life instruments such as the EuroQol 5-Dimension questionnaire (EQ-5D-5L). We used the EQ-5D-5L to obtain a broader assessment of health status in patients with heart failure with mildly reduced or preserved ejection fraction (HFmrEF/HFpEF) enrolled in the Example 2.

The 5 dimensions of the descriptive section of the EQ-5D-5L include mobility ("walking around"), self-care ("washing or dressing self'), usual activities ("e.g., work, study, housework, family or leisure activities"), pain/discomfort ("pain or discomfort"), and anxiety/depression ("anxious or depressed"). Each dimension is divided into 5 levels (1-5) i.e., no problem, slight problem, moderate problem, severe problem, and extreme problem (cannot do activity/extreme symptom).

Among 6,001 participants in Example 2, 5778 (99.6%) completed all EQ-5D-5L questions (Figure). Only around a third of patients reported no problem with mobility and carrying out usual activities (and 38% and 33% of patients, respectively, had moderate or greater problems in these dimensions). Overall, only 39% of patients were free of pain/discomfort and half did not report anxiety or depression (although 26% and 19%, respectively, had moderate or greater problems in these dimensions).

Most patients with HFmrEF/HFpEF experience some difficulty related to mobility, undertaking usual activities, and even self-care. More surprisingly, many patients report pain or discomfort, the basis of which is unclear and requires further investigation. Approximately half of patients experienced some anxiety or depression, and this was at least moderately severe in one in five participants; the management of this dimension may be an important unmet need in heart failure. Generic health-related quality-of-life instruments may provide additional and complementary information to that obtained from disease-specific instruments such as KCCQ. Results are summarized in Table 12.

**Table 12 - Distribution of answers to each EQ-5D-5L question at baseline. EQ-5D-5L = EuroQol 5-Dimension 5-Level questionnaire.**

| | No Problem [%] | Slight Problem [%] | Moderate Problem [%] | Severe Problem [%] | Unable to do [%] |
|---|---|---|---|---|---|
| Mobility | 32.7 | 29.1 | 26.8 | 10.6 | 0.8 |
| Self-care | 65 | 20.4 | 11.5 | Less than 5 | Less than 5 |
| Usual activities | 35.6 | 31.9 | 23.6 | 6.9 | 3.1 |
| Pain/discomfort | 39.3 | 35.2 | 19.7 | 5.2 | 2 |
| Anxiety/depression | 51.4 | 29.5 | 14.3 | Less than 5 | Less than 5 |

### Example 6 - Generalizability of the Example 2 Trial to the US Population across the Spectrum of Kidney Risk

To assess generalizability, we conducted serial cross-sectional analyses of US adults from the National Health and Nutrition Examination Surveys (2015-2016, 2017-2018, 2019-March 2020). Samples were weighted to be nationally representative of the US population. We identified adults with HF (age≥40 years with HF, eGFR≥25 mL/min/1.73 m², and potassium≤5.0 mmol/L) in NHANES. In both the enrolled Example 2 and the NHANES HF sample, we examined the distribution of KDIGO risk categories.

Among 6,001 patients enrolled in Example 2, 5,810 (97%) had available eGFR and UACR data. Median baseline eGFR was 61 (IQR 47-77) mL/min/1.73m² and 2,789 (48%) had an eGFR<60 mL/min/1.73m². Median baseline UACR was 18 (IQR 7-67) with 2,279 (39%) with UACR≥30mg/g. In Example 2, 35%, 29%, 20%, and 16% of participants were classified as KDIGO low, moderately increased, high, and very high risk, respectively. Among patients with diabetes, 26%, 27%, 23%, and 24% were KDIGO low, moderately increased, high, and very high risk, respectively. Participants in higher KDIGO risk categories were more likely be older, women, Asian, more functionally impaired with greater symptom burden, were more likely to have a previous HF hospitalization and had higher prevalence of diabetes, and had markedly higher natriuretic peptide levels (Table 13- Baseline Clinical Profiles of Example 2 Trial Participants with Available eGFR and UACR Data). We estimated 5,189,186 (4,333,114 to 6,045,257) individuals with HF in the US of whom 48%, 32%, 12%, and 9% would be classified as KDIGO low, moderately increased, high, and very high risk, respectively (Table 14 - KDIGO Risk Categories - US HF Population in NHANES - (Weighted n=5,189,186 from 2015-2020)).

Example 2 has enrolled a global population of patients with HF with mildly reduced or preserved ejection fraction with a broad spectrum of kidney risk well-represented. Distribution of kidney risk is highly overlapping and generalizable to many patients with HF in the US.

**Table 13 - Baseline Clinical Profiles of Example 2 Trial Participants with Available eGFR and UACR Data**

| Baseline Clinical Profiles of Example 2 Trial Participants with Available eGFR and UACR Data (n=5,810) | | | | | |
|---|---|---|---|---|---|
| | KDIGO Risk Categories | | | | |
| | Low | Moderately Increased | High | Very High | |
| | n=2035 | n=1688 | n=1172 | n=915 | P-value |
| Age | 68.2 ± 9.7 | 72.2±8.9 | 75.0±9.0 | 75.3±8.8 | <0.001 |
| Women | 835 (41.0%) | 801 (47.5%) | 573 (48.9%) | 421 (46.0%) | <0.001 |
| Body Mass Index (kg/m²) | 29.8±5.9 | 30.2±6.1 | 29.6±6.4 | 30.1±6.3 | 0.82 |

| *Race* | | | | | <0.001 |
|---|---|---|---|---|---|
| White | 1629 (80.3%) | 1344 (79.8%) | 904 (77.5%) | 680 (74.4%) | |
| Asian | 318 (15.7%) | 277 (16.4%) | 206 (17.7%) | 190 (20.8%) | |
| Black | 25 (1.2%) | 27 (1.6 %) | 18(1.5 %) | 11 (1.2%) | |
| Other | 56 (2.8 %) | 37 (2.2 %) | 38 (3.3 %) | 33 (3.6 %) | |

| *Region* | | | | | <0.001 |
|---|---|---|---|---|---|
| Asia | 314 (15.4%) | 272 (16.1%) | 206 (17.6%) | 188 (20.5%) | |
| Eastern Europe | 1084 (53.3%) | 784 (46.4%) | 444 (37.9%) | 271 (29.6%) | |
| Latin America | 218 (10.7%) | 164 (9.7 %) | 149 (12.7%) | 97 (10.6%) | |
| North America | 112(5.5 %) | 127 (7.5 %) | 101 (8.6 %) | 95 (10.4%) | |
| Western Europe, Oceania and Others | 307 (15.1%) | 341 (20.2%) | 272 (23.2%) | 264 (28.9%) | |

| *NYHA Class* | | | | | <0.001 |
|---|---|---|---|---|---|
| NYHA Class II | 1507 (74.1%) | 1175 (69.6%) | 790 (67.4%) | 551 (60.2%) | |
| NYHA Class III | 514 (25.3%) | 506 (30.0%) | 372 (31.7%) | 356 (38.9%) | |
| NYHA Class IV | 13 (0.6 %) | 7 (0.4 %) | 10 (0.9 %) | 8 (0.9 %) | |
| KCCQ Total Symptom Score | 70.0±22.4 | 67.4±23.5 | 64.8±24.6 | 62.2±26.0 | <0.001 |
| Heart Rate (bpm) | 70.6±11.0 | 71.8±11.7 | 72.4±12.4 | 71.8±12.6 | <0.001 |
| Systolic Blood Pressure (mmHg) | 128.4±14.4 | 129.7±15.3 | 129.2±15.7 | 131.4±16.7 | <0.001 |
| LVEF (%) | 51.9±7.7 | 52.7±7.9 | 52.9±8.1 | 53.1±7.9 | <0.001 |
| NT-proBNP (pg/mL) | 822 [351, 1873] ¹⁾ | 1500 [577, 2788] ¹⁾ | 2076 [924, 3743] ¹⁾ | 2716 [1224, 5729] ¹⁾ | <0.001 |
| | 630 [302, 1279] ²⁾ | 1047 [478, 1826] ²⁾ | 1412 [682, 2439] ²⁾ | 1789 [849, 3551]²⁾ | |
| Prior HF Hospitalization | 1170 (57.5%) | 999 (59.2%) | 723 (61.7%) | 617 (67.4%) | <0.001 |
| History of LVEF <40% | 86 (4.2 %) | 74 (4.4 %) | 53 (4.5 %) | 49 (5.4 %) | 0.21 |
| History of Diabetes | 622 (30.6%) | 641 (38.0%) | 538 (45.9%) | 554 (60.5%) | <0.001 |
| History of Atrial Flutter/Fibrillation | 928 (45.6%) | 960 (56.9%) | 748 (63.8%) | 553 (60.4%) | <0.001 |
| History of Hypertension | 1742 (85.6%) | 1500 (88.9%) | 1059 (90.4%) | 847 (92.6%) | <0.001 |
| History of Myocardial Infarction | 575 (28.3%) | 408 (24.2%) | 298 (25.4%) | 213 (23.3%) | 0.005 |
| Loop diuretic | 1997 (98.1%) | 1654 (98.0%) | 1158 (98.8%) | 896 (97.9%) | 0.75 |
| Beta blocker | 1732 (85.1%) | 1437 (85.1%) | 1004 (85.7%) | 758 (82.8%) | 0.27 |
| ACEi | 839 (41.2%) | 595 (35.2%) | 380 (32.4%) | 261 (28.5%) | <0.001 |
| ARB | 820 (40.3%) | 764 (45.3%) | 557 (47.5%) | 408 (44.6%) | 0.002 |
| ARNI | 180 (8.8 %) | 138 (8.2 %) | 103 (8.8 %) | 75 (8.2 %) | 0.68 |
| SGLT2i | 203 (10.0%) | 213 (12.6%) | 185 (15.8%) | 183 (20.0%) | <0.001 |

| | | | | | |
|---|---|---|---|---|---|
| Definition of ¹⁾ and ²⁾ in Example 1b. | | | | | |

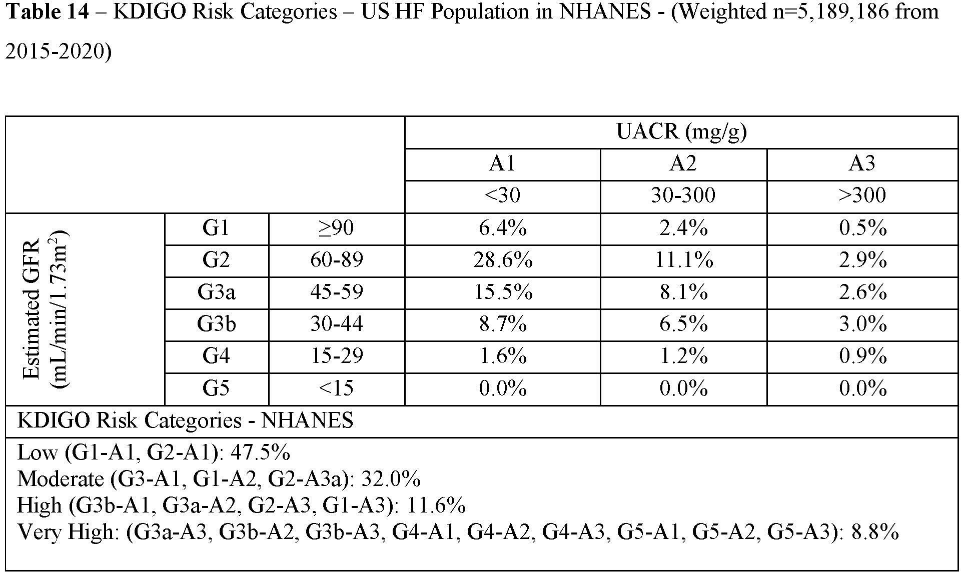

### Example 7 - Characterization of Primary Aldosteronism Pathophysiology in Example 2

Primary aldosteronism (PA) is a highly prevalent, underrecognized condition in patients with hypertension. PA, and its characteristic pathophysiology, is highly prevalent. PA pathophysiology has adverse consequences. The spectrum of PA pathophysiology is a targetable phenotype.

Identifying PA pathophysiology in Example 2: In HFpEF, where hypertension is a highly prevalent risk factor, it is hypothesize that chronic activation of the MR and resulting cardiac, vascular, and renal fibrosis may be driven by unrecognized PA in a meaningful proportion of patients. Thus, identifying underlying PA and its hallmark pathophysiology of renin-independent aldosterone production may help to a) ascertain a critical subgroup at elevated risk of adverse outcomes, b) identify those enriched for a clinical response to MR blockade with finerenone, and c) allow for evaluation of treatment effect based on the impact of finerenone on renin suppression, an emerging biomarker of adequate MR blockade for efficacy in PA. AIM: Identify prevalence, prognostic significance, and treatment effect of PA pathophysiology at baseline.

### Baseline biochemical assessment:

a. Measurement of plasma renin activity (if available, based on sample collection protocols) and/or plasma renin concentration at baseline
b. Measurement of plasma aldosterone on the day of renin measurement at baseline
c. Measurement of serum potassium and urine potassium & sodium (if available) on the day of renin measurement at baseline
d. Analytical approach - *in overall study population and in subset meeting criteria for apparent treatment resistant hypertension*
   i. Designation based on baseline renin into suppressed vs. unsuppressed renin subgroups
   ii. Assessment of outcomes and treatment effect by baseline renin group
   iii. Assessment of outcomes and treatment effect by aldosterone, stratified by baseline renin group, and by aldosterone-to-renin ratio
   iv. Sensitivity analyses restricting population to:
      1. Patients on ACE/ARB (expected to raise renin, so suppression on these medications increases probability of PA pathophysiology)
      2. Patients not on Beta Blocker (expected to lower renin, so suppression on beta blockade is less specific for PA pathophysiology)

AIM: Evaluate impact of reversing PA pathophysiology by inducing a rise in renin with MR antagonist on outcomes and treatment effect.

### On-treatment biochemical assessment:

a. Measurement of plasma renin activity (if available, based on sample collection protocols) and/or plasma renin concentration on treatment
b. Measurement of serum potassium and urine potassium & sodium (if available) on the day of renin measurement on treatment
c. Analytical approach

i. Assessment of outcomes and treatment effect by change in renin
   1. Continuous relationship of change in renin to change in BP
   2. Assessment of outcomes and treatment effect comparing categorical change from suppressed to unsuppressed renin vs. persistently suppressed renin on treatment.

### Example 8 - Cardio-Renal-Metabolic Overlap and Cardiorenal Risk in the Example 2

Patients with heart failure with mildly reduced or preserved ejection fraction (HFmrEF/HFpEF) commonly have cardio-renal-metabolic (CRM) comorbidities. The implications of these intersections on markers of risk and health status in this population are less well studied.

We first defined the clinical overlap in CRM conditions (atherosclerotic cardiovascular disease [ASCVD], type 2 diabetes [T2D], and chronic kidney disease [CKD; defined as eGFR<60mL/min/1.73m²). We then examined distributions of markers of cardiorenal risk (UACR, NT-proBNP) and health status (KCCQ-total symptom score; range 0-100 with 100 reflecting best health status) across the CRM spectrum. Among 6,001 participants in Example 2, 18%, 38%, 32%, and 12% had 0, 1, 2, or 3 CRM conditions (Figure 2 - Example 2 trial, participants with HFmref or HFpEF Cardo-Renal-Metabolic-Overlap). Median UACR was 12 [5-34] mg/g in those with 0 CRM conditions and 51 [14-248] mg/g in those with 3 CRM conditions (P<0.001). Median NT-proBNP was 1,352 [472-2,500] ¹⁾ pg/mL in those with 0 CRM conditions and 1,822 [654-3,983] ¹⁾ pg/mL in those with 3 CRM conditions (P<0.001). Mean KCCQ-TSS was 68.7 ± 23.1 in those with 0 CRM conditions and 63.8 ± 25.5 in those with 3 CRM conditions (P<0.001); see Table 15 - Markers of Cardiorenal Risk and Heath Status by Presence of Cardio-Renal-Metabolic Conditions). Four out of 5 patients with HFmrEF/HFpEF have at least 1 coexisting CRM condition. Patients with greater CRM overlap have higher markers of cardiorenal progression and worse health status and symptom burden. These data support prioritizing treatment optimization and care approaches in patients with HF and high CRM overlap.
Definition of ¹⁾ and ²⁾ in Example 1b.

### Example 9 - An Integrated Pooled Analysis of Finerenone in >19,000 Participants across 3 Phase III Trials of HF & CKD (FINE-HEART)

Background: The non-steroidal mineralocorticoid receptor antagonist (MRA), finerenone, has been independently studied in prospective randomized trials of patients with type 2 diabetes and chronic kidney disease (CKD) and separately in patients with heart failure (HF) with mildly reduced or preserved ejection fraction. However, none of these trials were individually powered to evaluated treatment effects on cardiovascular mortality or efficacy in key subgroups subpopulations such as those with overlapping cardio-kidney-metabolic (CKM) intersection. In light of the strong epidemiological overlap and shared mechanistic drivers of the enrolled populations, a pooled assessment summarizing the totality of evidence from these cardio-kidney trials was prespecified.

Methods: We conducted a protocol prespecified participant-level pooled analysis of 3 phase III global, multicenter, double-blind, placebo-controlled randomized clinical trials of Finerenone (FINE-Heart).The FIDELITY program (encompassing the FIDELIO-DKD and FIGARO-DKD trials) enrolled participants with type 2 diabetes and CKD with albuminuria (UACR≥30mg/g) on maximally tolerated doses of renin-angiotensin system inhibitors across 48 countries. FINEARTS-HF enrolled participants with symptomatic HF, LVEF ≥40%, elevated natriuretic peptides, and evidence of structural heart disease across 37 countries. Baseline characteristics were summarized across the pooled population including the prevalence of key CKM comorbidities (HF, diabetes, and CKD eGFR<60mL/min/1.73m2 or UACR≥30mg/g]) and their overlap. The primary endpoint for FINE-HEART was cardiovascular death, centrally adjudicated by independent clinical endpoints committees in each of the trials. Primary analyses of FINE-HEART have been predefined in a dedicated Statistical Analysis Plan.

Results: Overall, FINE-HEART comprised 19,027 participants from these three trials (mean age 67±10 years; 35% women). See Table 16. Participants were at high kidney risk for CKD progression with either reduced eGFR (30% with eGFR <45 and 26% with eGFR 45 to 60 mL/min/1.73m²) and/or albuminuria (31% with "A2" UACR 30-300mg/g and 49% with "A3" UACR>300mg/g). Prevalence of CKM comorbidities was high with history of HF (37%), diabetes (81%), and CKD in 89% resulting in 8% with 1 CKM condition, 78% having 2 CKM conditions, and 15% with all 3 CKM conditions (HF, diabetes, and CKD). See **Figure 4****:** shows the Cardio-Kidney-Metabolic Overlap in FINE-HEART. Overall, 1,691 (9%) of participants were co-treated with an SGLT2i. Over median follow-up of 3 years in the FIDELITY program, there were 686 cardiovascular deaths (1.7 per 100py). While follow-up is still underway in the event-driven FINEARTS-HF trial, under the assumptions of sample size determination, approximately 535 cardiovascular deaths are expected, resulting in a total of 1,221 expected cardiovascular deaths in FINE-HEART.

Conclusions: FINE-HEART will have increased precision to robustly assess the safety and efficacy of the non-steroidal MRA finerenone on cardiovascular death and other cardio-kidney outcomes, and will be enriched for participants with high burden of CKM multimorbidity.

**Table 16: Baseline Characteristics of FINE-HEART (n = 19027)**

| | |
|---|---|
| **Age** | 67 +/- 10 |
| **Women** | 35 % |
| **BMG (kg/m²)** | 31 +/-6 |
| **Systolic blood pressure (BP) (mmHg)** | 134 +/- 15 |
| **eGFR (mL/min/1.73m²)** | 59 +/- 21 |
| Below 25 | 1 % |
| 25 to below 45 | 29 % |
| 45 to below 60 | 26 % |
| Equal or above 60 | 44 % |
| **UACR (mg/g)** | 640 +/- 913 |
| A1: below 30 | 20 % |
| A2: 30 to below 300 | 31 % |
| A3: equal or above 300 | 49% |
| **Hemoglobin A1c (%)** | 7.3 +/- 1.4 |
| **History of HF** | 37 % |
| **History of Diabetes** | 81 % |
| **History of AF/AFL** | 25 % |
| **Diuretic** | 63 % |
| **ACEi** | 38 % |
| **ARB** | 56 % |
| **SGLT2i** | 9 % |

### Example 10 - Generalizability of the FINEARTS-HF Trial to the US Population Across the Spectrum of Kidney Risk

Example 2 evaluated the efficacy and safety of finerenone in patients with heart failure (HF) and left ventricular ejection fraction ≥40%, with or without diabetes, and across a broad range of kidney function **(****Figure 1**). We examined the distribution of kidney risk represented in the Example 2 trial and compared it with that in the US HF population.

Serial cross-sectional analyses of adults from the National Health and Nutrition Examination Surveys (NHANES; 2015-2016, 2017-2018, and 2019-2020) identified a US sample. Samples were weighted to derive a nationally representative cohort of adults with HF (aged ≥40 years with HF, estimated glomerular filtration rate (eGFR) ≥25 mL/min/1.73 m², and potassium ≤5.0 mmol/L). Kidney risk was analyzed per Kidney Disease Improving Global Outcomes (KDIGO) risk categories, defined by eGFR and urine albumin-to-creatinine ratio (UACR).

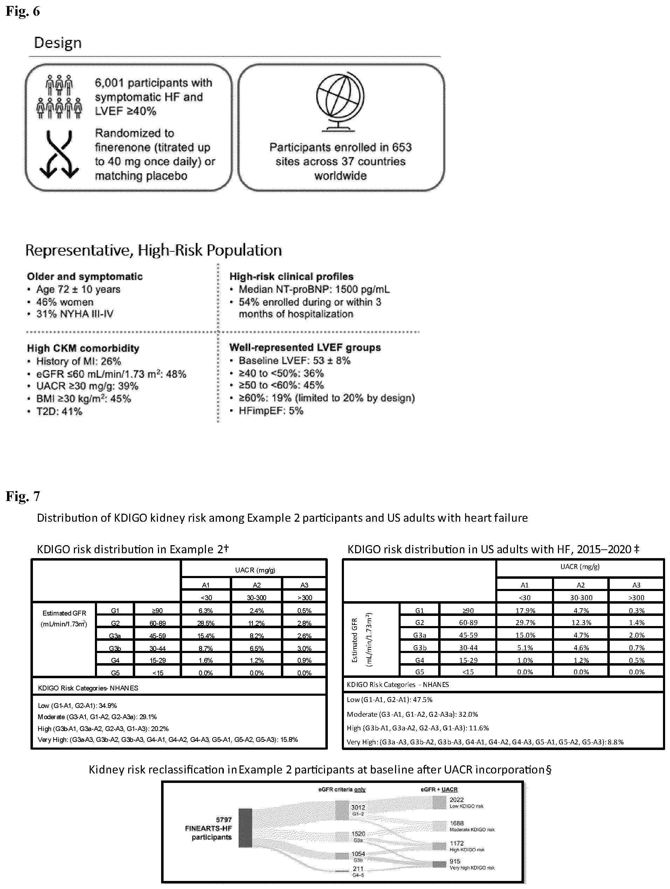

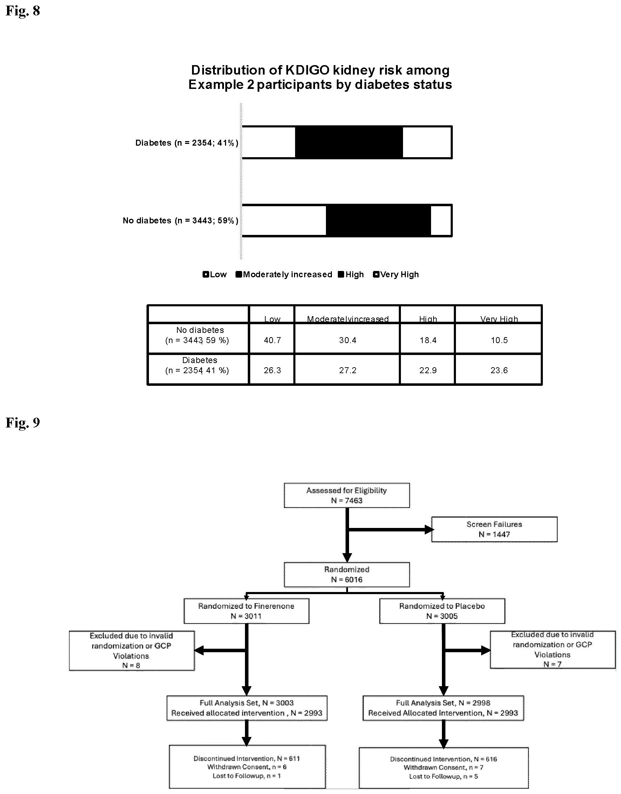

Among 6001 randomized participants, 5797 (97%) could be assigned to a KDIGO category. Median baseline eGFR (mL/min/1.73 m² [interquartile range {IQR}]) was 61 (47-77); 2785 (48%) had an eGFR < 60 mL/min/1.73 m². Median baseline UACR (mg/g [IQR]) was 18 (7-67); 2279 (39%) had a UACR ≥30 mg/g. In Example 2, 34.6%, 29.1%, 20.2%, and 15.8% of participants were classified as KDIGO low, moderate, high, and very high risk, respectively. Using NHANES data, we estimated that of 5 189 186 US individuals with HF (95% confidence interval 4 333 114 to 6 045 257), 47.6%, 32.0%, 11.6%, and 8.8% would be classified as KDIGO low, moderate, high, and very high risk, respectively (Figure 7; †Graphical display of the distributions of KDIGO kidney risk among Example 2 participants with available eGFR and UACR data (n = 5797) at baseline. ‡Graphical display of the distributions of KDIGO kidney risk among weighted NHANES (2015 to March 2020) sample of US adults (weighted n = 5,189,186) with HF, age ≥40 years, eGFR ≥25 mL/min/1.73 m², and serum potassium ≥5 mmol/L. §Sankey diagram showing reclassification of KDIGO kidney risk after incorporation of eGFR and UACR, compared with eGFR stages alone. eGFR, estimated glomerular filtration rate; HF, heart failure; KDIGO, Kidney Disease Improving Global Outcomes; NHANES, National Health and Nutrition Examination Surveys; UACR, urine albumin-to-creatinine ratio); p<0.001 for comparison with KDIGO risk distribution in Example 2. In Example 2 participants without diabetes, 40.7%, 30.4%, 18.4%, and 10.5% were classified as KDIGO low, moderate, high, and very high risk, respectively (Figure 8). In Example 2 participants with diabetes, 26.3%, 27.2%, 22.9%, and 23.6% were classified as KDIGO low, moderate, high, and very high risk, respectively (Figure 8).

Incorporating UACR reclassified kidney risk in approximately one in three Example 2 participants and US adults with HF. This emphasizes the importance of albuminuria as part of a comprehensive risk assessment in the HF population.

### Example 11 - Tablets comprising finerenone (4S) - 4- (4-cyano-2-methoxyphenyl) -5-ethoxy-2,8-dimethyl-1,4-dihydro-1,6-naphthyridine-3-carboxamide of the formula (I)

A granulate solution of the compound of formula (I) in crystalline form in micronized form, hypromellose 5 cP, sodium lauryl sulfate in purified water was prepared. Microcrystalline cellulose, lactose monohydrate and croscarmellose sodium were mixed in a container or a fluidized bed granulator (premix). The premix and the granulate solution were granulated in the fluid-bed granulator. The lubricant magnesium stearate was added after the granules were dried and sieved. A ready-to-press mixture was thus produced. The ready-to-press mixture was pressed into tablets using a rotary tablet press. A homogeneous coating suspension was made from hypromellose, talc, titanium dioxide, yellow iron oxide, red iron oxide and purified water. The coating suspension was sprayed onto the tablets in a suitable coating device.

**Table 17: Tablets (number 1 to 7) obtained by the process described above**

| Composition | | Tablets | | | | | | |
|---|---|---|---|---|---|---|---|---|
| | | 1 [mg] | 2 [mg] | 3 [mg] | 4 [mg] | 5 [mg] | 6 [mg] | 7 [mg] |
| | Finerenone, micronized | 1.25 | 2.50 | 5.00 | 7.50 | 10.00 | 15.00 | 20.00 |
| Excipients | Cellulose microcrystalline | 73.80 | 72.50 | 69.90 | 67.30 | 64.70 | 62.00 | 59.30 |
| | Crosscarmellose sodium | 4.50 | 4.50 | 4.50 | 4.50 | 4.50 | 4.50 | 4.50 |
| | Hypromellose 5 cP | 4.50 | 4.50 | 4.50 | 4.50 | 4.50 | 4.50 | 4.50 |
| | Lactose monohydrate | 45.00 | 45.00 | 45.00 | 45.00 | 45.00 | 42.50 | 40.00 |
| | Magnesium stearate | 0.90 | 0.90 | 0.90 | 0.90 | 0.90 | 0.90 | 0.90 |
| | Sodium laurilsulfate | 0.05 | 0.10 | 0.20 | 0.30 | 0.40 | 0.60 | 0.80 |
| | Weight (uncoated tablet) | 130.00 | 130.00 | 130.00 | 130.00 | 130.00 | 130.00 | 130.00 |
| Film-coating | Hypromellose 5 cP | 3.0336 | 3.0336 | 3.0336 | 3.0336 | 3.0336 | 3.0336 | 3.0336 |
| | Titanium dioxide | 2.3196 | 2.3196 | 2.3196 | 2.3196 | 2.3196 | 2.3196 | 2.3196 |
| | Talcum | 0.6072 | 0.6072 | 0.6072 | 0.6072 | 0.6072 | 0.6072 | 0.6072 |
| | Iron oxide yellow | 0.0324 | 0.0324 | 0.0324 | 0.0324 | 0.0324 | 0.0324 | 0.0324 |
| | Iron oxide red | 0.0072 | 0.0072 | 0.0072 | 0.0072 | 0.0072 | 0.0072 | 0.0072 |
| | Weight (film-coating) | 6.0000 | 6.0000 | 6.0000 | 6.0000 | 6.0000 | 6.0000 | 6.0000 |
| | Weight (coated tablet) | 136.00 | 136.00 | 136.00 | 136.00 | 136.00 | 136.00 | 136.00 |

### Example 12 - Tablets comprising finerenone (4S) - 4- (4-cyano-2-methoxyphenyl) -5-ethoxy-2,8-dimethyl-1,4-dihydro-1,6-naphthyridine-3-carboxamide of the formula (I)

A granulate suspension of the compound of formula (I) in crystalline form in micronized form, hypromellose , sodium lauryl sulfate in purified water was prepared. Microcrystalline cellulose, lactose monohydrate and croscarmellose sodium were mixed in a container or a fluidized bed granulator (premix). The premix and the granulate solution were granulated in the fluid-bed granulator. The lubricant magnesium stearate was added after the granules were dried and sieved. A ready-to-press mixture was thus produced. The ready-to-press mixture was compressed into tablets using a rotary tablet press. A homogeneous coating suspension was made from hypromellose, talc, titanium dioxide, yellow iron oxide, red iron oxide and/or black iron oxide and purified water. The coating suspension was sprayed onto the tablets in a suitable coating device. The composition of the tablets obtained by the process described are listed in table 18.

**Table 18: Tablets (8 to 12 obtained) obtained by the process described above**

| **Composition** | | **Tablets** | | | | |
|---|---|---|---|---|---|---|
| | | **8 [mg]** | **9 [mg]** | **10 [mg]** | **11 [mg]** | **12 [mg]** |
| | Finerenone micronized | 40 | 5 | 10 | 20 | 40 |
| Excipients | Cellulose microcrystalline | 110 | 69.9 | 64.7 | 59.3 | 110 |
| | Croscarmellose sodium | 15 | 4.5 | 4.5 | 4.5 | 15 |
| | Hypromellose 5 cP | 7 | 4.5 | 4.5 | 4.5 | 7 |
| | Lactose monohydrate | 25 | 45 | 45 | 40 | 25 |
| | Magnesium stearate | 1.4 | 0.9 | 0.9 | 0.9 | 1.4 |
| | Sodium laurilsulfate | 1.6 | 0.2 | 0.4 | 0.8 | 1.6 |
| | Purified water in bulk | q.s. | q.s. | q.s. | q.s. | q.s. |
| | Weight (uncoated tablet) | 200 | 130 | 130 | 130 | 200 |
| Film coating | Hypromellose 5 cP | 3.5392 | 3 | 3 | 3 | 3.5 |
| | Talc | 0.7084 | 0.6 | 0.6 | 0.6 | 0.7 |
| | Titanium dioxide | 2.7062 | 2.36 | 2.28 | 1.92 | 2.222 |
| | Ferric oxide yellow | 0.0378 | - | - | 0.48 | 0.473 |
| | Ferric oxide red | 0.0084 | - | 0.12 | - | 0.105 |
| | Ferric oxide black | - | 0.04 | - | - | - |
| | Purified water in bulk | q.s. | q.s. | q.s. | q.s. | q.s. |
| | Weight (film coating) | 7 | 6 | 6 | 6 | 7 |
| Weight (coated tablet) | | 207 | 136 | 136 | 136 | 207 |

As film coatings, commercially available film coatings can be used for the tablets disclosed in tables 17 and 18 above. Examples are Opadry^{®} film coatings such as Opadry^{®} 02A275000 light gray, Opadry^{®} 02A240005 light pink or Opadry^{®} 02A220009 light yellow.

### Example 13 - Finerenone in Heart Failure with Mildly Reduced or Preserved Ejection Fraction

Background: Steroidal mineralocorticoid receptor antagonists reduce morbidity and mortality in patients with heart failure with reduced ejection fraction but their efficacy in those with heart failure with mildly reduced or preserved ejection has not been established. We tested the efficacy and safety of the non-steroidal MRA finerenone in patients with heart failure and mildly reduced or preserved ejection fraction.

Methods: We randomly assigned 6,001 patients with heart failure and a left ventricular ejection fraction ≥40% to finerenone (up to 40 mg once daily) or matching placebo. The primary outcome was a composite of total worsening heart failure events and cardiovascular death.

Results: Over a median of 32 months, there were 1083 primary events in 624 patients in the finerenone group and 1283 primary events in 719 patients in the placebo group (rate ratio, 0.84; 95% confidence interval [CI], 0.74 to 0.95; P = 0.007). There were 842 total worsening heart failure events in the finerenone group and 1024 total worsening heart failure events in the placebo group (rate ratio, 0.82; 95% confidence interval [CI], 0.71 to 0.94, p = 0.006). The incidence of death from cardiovascular causes was 8.1% and 8.7%, respectively (hazard ratio, 0.93; 95% CI, 0.78 to 1.11). Finerenone increased the risk of hyperkalemia and reduced the risk of hypokalemia.

Conclusions: Finerenone reduced the composite of total worsening heart failure events and cardiovascular death in patients with heart failure and mildly reduced or preserved ejection fraction. (NCT04435626).

### In Detail:

### METHODS

### Detailed Inclusion and Exclusion Criteria

### Study Population: Patients with a diagnosis of HF, NYHA class II-IV, and documented LVEF of ≥40%. Inclusion Criteria

Participants are eligible to be included in the study only if all of the following criteria apply:

### Age

- Participant must be aged 40 years and older, at the time of signing the informed consent.

### Type of Participant and Disease Characteristics:

- Diagnosis of heart failure with NYHA class II-IV, ambulatory or hospitalized primarily for heart failure (if a hospitalized patient cannot be randomized as an in-patient, randomization as soon as possible after discharge is encouraged)
- On diuretic treatment for at least 30 days prior to randomization
- Documented LVEF of ≥40% measured by any modality within the last 12 months, at the latest at screening; if several values are available, the most recent one shall be reported. If LVEF was not measured in the past 12 months, a new measurement may be done at screening
- Structural heart abnormalities based on any local imaging measurement within the last 12 months, latest at screening, defined by at least 1 of the following findings:
   ∘ LAD ≥3.8cm, LAA ≥20cm², LAVI >30 mL/m², LVMI ≥115 g/m² (♂) / 95 g/m² (♀), septal thickness or posterior wall thickness ≥1.1 cm
- NT-proBNP ≥300 pg/mL (BNP ≥100 pg/mL) in sinus rhythm and patient does not have an ongoing diagnosis of paroxysmal atrial fibrillation or NT-proBNP ≥900 pg/mL (BNP ≥300 pg/mL) in atrial fibrillation (or if atrial fibrillation status is unknown or if patient has an ongoing diagnosis of paroxysmal atrial fibrillation) for participants obtained at the following time:
   - Within 90 days prior to randomization if patient had been hospitalized for HF requiring initiation or change in HF therapy or if patient had an urgent visit for HF requiring intravenous (IV) diuretic therapy, both within 90 days prior to randomization
      OR
   - Within 30 days prior to randomization if patient has not been hospitalized for HF nor had an urgent HF visit within the past 90 days.

### Sex

- Male or female. Women of childbearing potential can only be included in the study if a pregnancy test is negative at screening and baseline and if they agree to use adequate contraception which is consistent with local regulations regarding the methods for contraception for those participating in clinical trials.

### Informed Consent

- Capable of giving signed informed consent which includes compliance with the requirements and restrictions listed in the informed consent form (ICF) and in this protocol.

### Exclusion Criteria

Participants are excluded from the study if any of the following criteria apply:

### Medical Conditions

- eGFR <25 mL/min/1.73 m² at either screening or randomization visit.
   NOTE: one reassessment of eGFR is allowed at the screening and randomization visit, respectively
- Serum/plasma potassium >5.0 mmol/L at either screening or randomization visit. NOTE: one reassessment of potassium is allowed at the screening and randomization visit, respectively
- Acute inflammatory heart disease, e.g. acute myocarditis, within 90 days prior to randomization
- Myocardial infarction or any event which could have reduced the ejection fraction within 90 days prior to randomization
- Coronary artery bypass graft surgery in the 90 days prior to randomization
- Percutaneous coronary intervention in the 30 days prior to randomization
- Stroke or transient ischemic cerebral attack within 90 days prior to randomization
- Probable alternative cause of participants' HF symptoms that in the opinion of the investigator primarily accounts for patient's dyspnea such as significant pulmonary disease, anemia or obesity. Specifically, patients with the below are excluded:
   - Severe pulmonary disease requiring home oxygen, or chronic oral steroid therapy
   - History of primary pulmonary arterial hypertension
   - Hemoglobin < 10 g/dl
   - Valvular heart disease considered by the investigator to be clinically significant
   - Body mass index (BMI) >50 kg/m² at screening
- Systolic blood pressure (SBP) ≥160 mmHg if not on treatment with ≥3 blood pressure lowering medications or ≥180 mmHg irrespective of treatments on 2 consecutive measurements at least 2-minute apart, at screening or at randomization
- Life-threatening or uncontrolled arrhythmias at screening and/or randomization including but not limited to sustained ventricular tachycardia and atrial fibrillation, or atrial flutter with resting ventricular rate >110 bpm
- Symptomatic hypotension with mean systolic blood pressure <90 mmHg at screening or at randomization
- Any primary cause of HF scheduled for surgery, e.g. valve disease such as severe aortic stenosis or severe mitral regurgitation by the time of screening or randomization
- History of periparium cardiomyopathy, chemotherapy induced cardiomyopathy, viral myocarditis, right heart failure in absence of left-sided structural disease, pericardial constriction, genetic hypertrophic cardiomyopathy, or infiltrative cardiomyopathy including amyloidosis
- Presence of left ventricular assist device by the time of screening or randomization
- History of hyperkalemia or acute renal failure during MRA treatment for >7 consecutive days, leading to permanent discontinuation of the MRA treatment
- Pregnant or nursing (lactating) women, where pregnancy is defined as the state of a female after conception and until the termination of gestation, confirmed by a positive human chorionic gonadotrophin urine or serum test
- Known hypersensitivity to the study intervention (active substance or excipients)
- Hepatic insufficiency classified as Child-Pugh C at screening or randomization
- Addison's disease.

### Prior/Concomitant Therapy

- Requirement of any IV vasodilating drug (e.g. nitrates, nitroprusside), any IV natriuretic peptide (e.g. nesiritide, carperitide), any IV positive inotropic agents, or mechanical support (intra-aortic balloon pump, endotracheal intubation, mechanical ventilation, or any ventricular assist device) within 24 hours prior to randomization
- Participants who require treatment with more than one ACEI, ARB or angiotensin-receptor neprilysin inhibitor (ARNI), or two simultaneously at randomization
- Continuous (at least 90 days) treatment with an MRA (e.g. spironolactone, eplerenone, canrenone, esaxerenone) within 12 months prior to screening. Last intake at least 30 days before randomization. Treatment with MRA should not be interrupted with the purpose of enrollment into the study
- Concomitant treatment with any renin inhibitor or potassium-sparing diuretic that cannot be stopped prior to randomization and for the duration of the treatment period
- Concomitant systemic therapy with potent cytochrome P450 isoenzyme 3A4 (CYP3A4) inhibitors (e.g. itraconazole, ritonavir, indinavir, cobicistat, clarithromycin) or moderate or potent CYP3A4 inducers, that cannot be discontinued 7 days prior to randomization and for the duration of the treatment period.

### Other Exclusions

- Any other condition or therapy, which would make the participant unsuitable for this study and will not allow participation for the full planned study period (e.g. active malignancy or other condition limiting life expectancy to less than 12 months)
- Previous assignment to treatment during this study
- Participation in another interventional clinical study (e.g. Phase 1 to 3 clinical studies) or treatment with another investigational medicinal product within 30 days prior to randomization
- Close affiliation with the investigational site; e.g. a close relative of the investigator, dependent person (e.g. employee or student of the investigational site)
- Known current alcohol and/or illicit drug abuse that may interfere with the participant's safety and/or compliance at the discretion of the investigator
- Participant is in custody by order of an authority or a court of law.

### Trial Design and Oversight

FINEARTS-HF was an international, multicenter, parallel-group, event-driven, randomized, double-blind trial in patients with chronic heart failure and left ventricular ejection fraction of 40% or greater, comparing the effect of finerenone, titrated to 20mg (if baseline eGFR ≤60 mL/min/1.73 m²) or 40mg once daily (if baseline eGFR>60 mL/min/1.73 m²) versus placebo, in addition to usual therapy. Details regarding the design of the trial have been e.g. above.

### Trial Participants

Eligibility requirements included stabilized heart failure in either inpatients or outpatients who were at least 40 years of age, had a left ventricular ejection fraction of 40% or more (including those with prior left ventricular ejection fraction less than 40%), evidence of structural heart disease and elevation of natriuretic peptides. Detailed inclusion and exclusion criteria have been described above. The patient flow is shown in **figure 9****.**

### Trial Procedures and Outcomes

All patients provided written informed consent. Patients who met the inclusion and exclusion criteria were randomly assigned to receive finerenone (at a starting dose of 10mg titrated to 20mg once daily in patients with a baseline eGFR ≤ 60 ml/min/1.73m2, or a starting dose of 20mg titrated to 40mg once daily in those with a baseline eGFR > 60 ml/min/1.73m2) or matching placebo. The primary outcome was a composite of cardiovascular death and total worsening heart failure events (defined as either an unplanned hospitalization for heart failure or an urgent heart failure visit). Secondary objectives were to determine whether finerenone was superior to placebo in reducing the total number of worsening heart failure events; in the Kansas City Cardiomyopathy Questionnaire (KCCQ) total symptom score (TSS) at months 6, 9, 12 (scores range from 0 to 100, with higher scores indicating fewer symptoms and physical limitations) and improving NYHA class at month 12; in reducing a composite kidney outcome (defined as a sustained 50% or greater decline in eGFR, a sustained decline in eGFR to < 15 ml/min/1.73m2 or initiation of chronic dialysis or kidney transplantation); and in reducing all-cause mortality. All primary events were adjudicated by an independent clinical events committee blinded to treatment assignment based on prespecified criteria. Adverse events and serious adverse events were collected and reported as both treatment-emergent (occurring in patients who received at least one dose of study drug and within 3 days of permanent discontinuation) and throughout the study.

### Statistical Analysis

The primary analysis compared the total rate of cardiovascular death and worsening heart failure events (either heart failure hospitalization or urgent heart failure visits) in an intention-to-treat approach using the semiparametric proportional rates method of Lin et al. (Lin DY, Wei LJ, Yang I, Ying Z. Semiparametric regression for the mean and rate functions of recurrent events. J R Stat Soc Series B Stat Methodol 2000;62:711-730) stratified according to geographic region and baseline LVEF (<60%, ≥60%). Significance for the primary outcome was set at two-sided p < 0.0497 based on an adjustment for the interim analysis.

It was determined that 2375 total (first and recurrent) primary composite events would be required to provide 90% power to detect an overall 19% lower event rate in the finerenone group. We estimated that the target number of events would be obtained by enrolling approximately 5,500 subjects over 24 months with a minimum follow-up of 18 months. Due to blinded event rates being lower than those assumed in the sample size calculation, the planned number of randomized subjects was increased to approximately 6000 in July, 2022. The target number of primary composite events was not changed.

The secondary hypotheses were tested hierarchically as follows based on the rejection of the primary null hypothesis: total HF events; KCCQ total symptom score improvement and NYHA class improvement (tested simultaneously using the Bonferroni-Holm procedure); and the composite kidney endpoint. All-cause mortality was tested outside this hierarchy, if the primary null hypothesis was rejected, at a nominal two-sided significance level of 5%.

One non-binding interim analysis for futility was performed when approximately 30% of the required total number of primary endpoint events were observed, and one formal interim analysis for efficacy was performed when approximately two-thirds of the required total number of primary endpoint events were observed. Following both pre-specified interim analyses, the Data Monitoring Committee recommended continuing the trial unchanged.

### Results

### Enrollment, Randomization, and follow-up

Between 14 September 2020 and 10 January 2023, we screened 7,463 patients from 654 sites across 37 countries (Figure S 1, Supplemental Appendix). Overall, 6,016 patients were randomized to receive finerenone or placebo. One patient was randomized twice by mistake, and both entries were excluded, and 13 patients from a single site were excluded from the primary analysis due to significant Good Clinical Practice violations. A total of 6,001 patients were therefore validly randomized and included in the primary analysis. At the end of the trial (June 14, 2024), 13 patients had withdrawn consent and 6 were otherwise lost to follow-up. The median duration of follow-up was 32 months in each group.

The characteristics of the patients at baseline were balanced between the two treatment groups (see Table 19).

**Table 19. Baseline Characteristics of Randomized Participants**

| | **Finerenone (N** = **3003)** | **Placebo (N** = **2998)** |
|---|---|---|
| Age | 71.9 ± 9.6 | 72.0 ± 9.7 |
| Women | 1355 (45.1%) | 1377 (45.9%) |

| Race | | |
|---|---|---|
| Asian | 497 (16.6%) | 499 (16.6%) |
| Black | 49 (1.6%) | 39 (1.3 %) |
| Other | 91 (3.0%) | 91 (3.0 %) |
| White | 2366 (78.8%) | 2369 (79.0%) |
| | | |

| Region | | |
|---|---|---|
| Asia | 493 (16.4%) | 490 (16.3%) |
| Eastern Europe | 1329 (44.3%) | 1321 (44.1%) |
| Latin America | 322 (10.7%) | 319 (10.6%) |
| North America | 235 (7.8 %) | 236 (7.9 %) |
| Western Europe, Oceania and Others | 624 (20.8%) | 632 (21.1%) |
| | | |
| Any prior HF Hospitalization | 1797 (59.8%) | 1822 (60.8%) |

| Recency of Heart Failure Event | | |
|---|---|---|
| <= 7 days from randomization | 609 (20.3%) | 610 (20.3%) |
| >7 days - <= 3 months | 1030 (34.3%) | 998 (33.3%) |
| >3 months or no index HF event | 1364 (45.4%) | 1390 (46.4%) |
| | | |
| Systolic Blood Pressure (mmHg) | 129.5 ± 15.3 | 129.3 ± 15.3 |
| Body Mass Index (kg/m2) | 29.9 ± 6.1 | 30.0 ± 6.1 |
| Creatinine (mg/dL) | 1.1 ± 0.3 | 1.1 ± 0.4 |
| eGFR (mL/min/1.73m2) | 61.9 ± 19.4 | 62.3 ± 20.0 |
| eGFR < 60 mL/min/1.73m² | 1451 (48.3%) | 1437 (47.9%) |
| UACR (mg/g) | 18 [7 , 67 ] | 19 [7 , 66 ] |
| potassium (mmol/L) | 4.4 ± 0.5 | 4.4 ± 0.5 |
| LVEF mean (%) | 52.6 ± 7.8 | 52.5 ± 7.8 |
| *LVEF < 50%* | 1093 (36%) | 1079 (36%) |
| *LVEF* ≥ *50% and* < *60%* | 1329 (44%) | 1345 (49%) |
| *LVEF* ≥ *60%* | 575 (19%) | 572 (19%) |
| NT-pro BNP (pg/mL) (median, IQR) | 1052 [467 , 1937 ] | 1028 [433 , 1963 ] |

| NYHA class | | |
|---|---|---|
| Missing | 1 (<0.1 %) | 0 (0.0 %) |
| NYHA Class II | 2081 (69.3%) | 2065 (68.9%) |
| NYHA Class III | 903 (30.1%) | 910 (30.4%) |
| NYHA Class IV | 18 (0.6 %) | 23 (0.8 %) |
| | | |

| Medical History | | |
|---|---|---|
| Hypertension | 2640 (87.9%) | 2685 (89.6%) |
| Diabetes Mellitus | 1217 (40.5%) | 1222 (40.8%) |
| Atrial Fibrillation on ECG at baseline | 1165 (38.8%) | 1128 (37.6%) |
| Stroke | 355 (11.8%) | 353 (11.8%) |
| Myocardial Infarction | 784 (26.1%) | 757 (25.3%) |
| | | |

| Medication Use | | |
|---|---|---|
| Beta-blocker | 2541 (84.6%) | 2554 (85.2%) |
| ACEI | 1083 (36.1%) | 1072 (35.8%) |
| ARB (w/o ARNI) | 1047 (34.9%) | 1055 (35.2%) |
| ARNI | 256 (8.5 %) | 257 (8.6 %) |
| Calcium channel blocker | 958 (31.9%) | 1010 (33.7%) |
| SGLT-2 inhib. | 393 (13.1%) | 424 (14.1%) |
| Loop diuretic | 2618 (87.2%) | 2621 (87.4%) |

Patients had a mean LVEF of 53 ± 8%; 36% had LVEF < 50%, 45% had LVEF between 50% and 59%, and 19% had LVEF ≥ 60%. The majority were New York Heart Association functional class II (69%), and 1219 (20%) patients were enrolled during or within 7 days of a worsening HF event. Baseline medications included beta blockers (85%), ACE inhibitors or ARBs (71%), ARNIs (9%) and SGLT-2 inhibitors (14%). Additional baseline details have been previously published.

### Outcomes

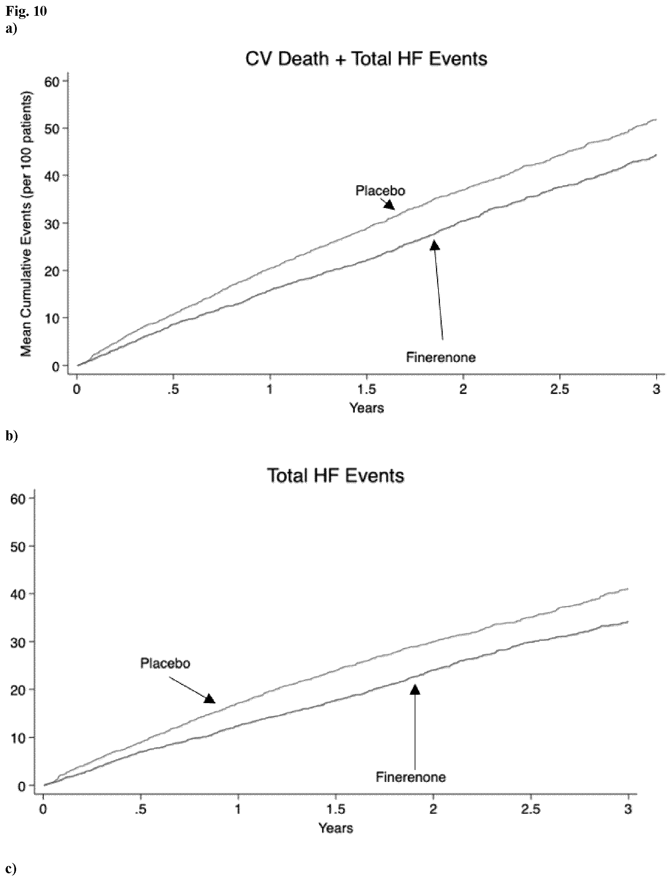

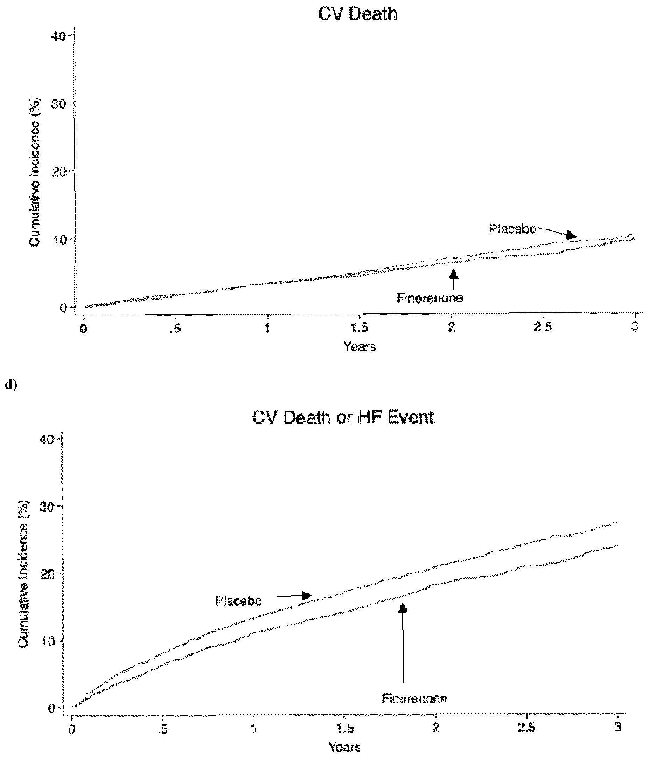

There were 1083 primary events (842 heart failure events and 242 deaths from cardiovascular causes) in 624 patients in the finerenone group and 1283 primary events (1024 heart failure events and 260 deaths for cardiovascular causes) in 719 patients in the placebo group (rate ratio, 0.84; 95% confidence interval [CI], 0.74 to 0.95; P = 0.007) (Table 20 and Fig. 10).

**Table 20. Primary and Secondary Outcomes.***

| **Outcome** | | **Finerenone (N = 3003)** | **Placebo (N = 2998)** | **Ratio or Difference (95% CI)** |
|---|---|---|---|---|
| **Primary composite outcome and components** | | | | |
| Total worsening heart failure events and death from cardiovascular causes | | | | RR, 0.84 (0.74-0.95), p = 0.007 |
| | Total no. of events** | 1083 | 1283 | |
| | Rate per 100 patient-yr | 14.9 | 17.7 | |
| Total no. of worsening heart failure events** | | 842 | 1024 | RR, 0.82 (0.71-0.94), p = 0.006 |
| Death from cardiovascular causes - no. (%) | | 242(8.1%) | 260(8.7%) | HR, 0.93 (0.78-1.11) |

| **Secondary outcomes** | | | | |
|---|---|---|---|---|
| Change in KCCQ clinical summary score at 6, 9 and 12 mo (least squares mean change) | | +8.0 ± 0.3 | +6.4 ± 0.3 | +1.6 (0.8-2.3), p < 0.001 |
| Improvement in NYHA class from baseline to 12 mo - no./total no. (%) | | 557/3002 (18.6%) | 553/2998 (18.4%) | OR, 1.01 (0.88-1.15) |
| Kidney composite outcome - no of events (%)§ | | 75 (2.5%) | 55 (1.8%) | HR, 1.33 (0.94-1.89) |
| Death from any cause - no of events (%) | | 491 (16.4%) | 522 (17.4%) | HR, 0.93 (0.83-1.06) |
| First worsening heart failure event or death from cardiovascular causes | | 624(20.8%) | 719(24.0%) | HR, 0.84 (0.76-0.94) |

| | | | | |
|---|---|---|---|---|
| * HR denotes hazard ratio, OR odds ratio, and RR rate ratio. ** one patient in each group was reported as having a heart failure event on the same day as a cardiovascular death and was counted as only one composite event in the primary analysis § Kidney composite outcome was defined as a sustained 50% or greater decline in eGFR, a sustained decline in eGFR to < 15 ml/min/1.73m² or initiation of chronic dialysis or kidney transplant | | | | |

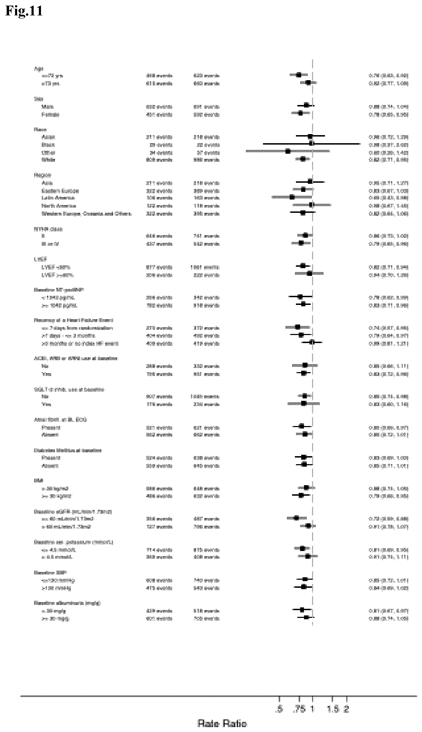

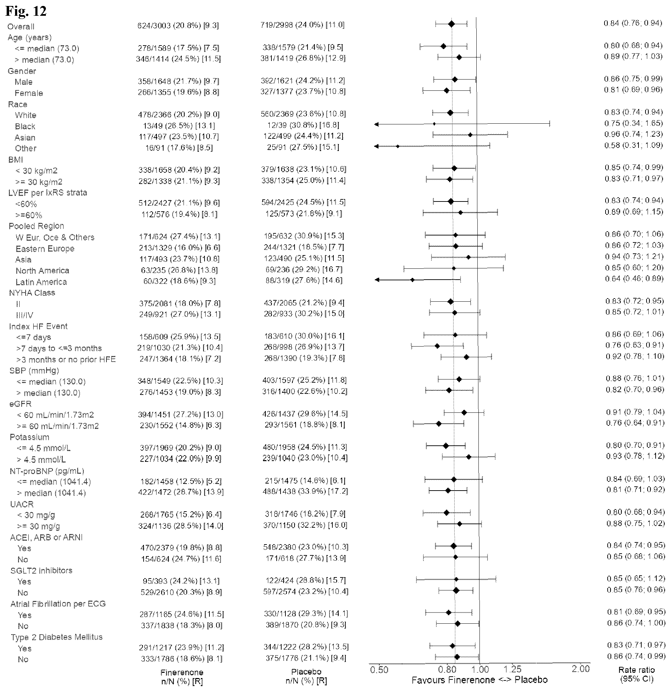

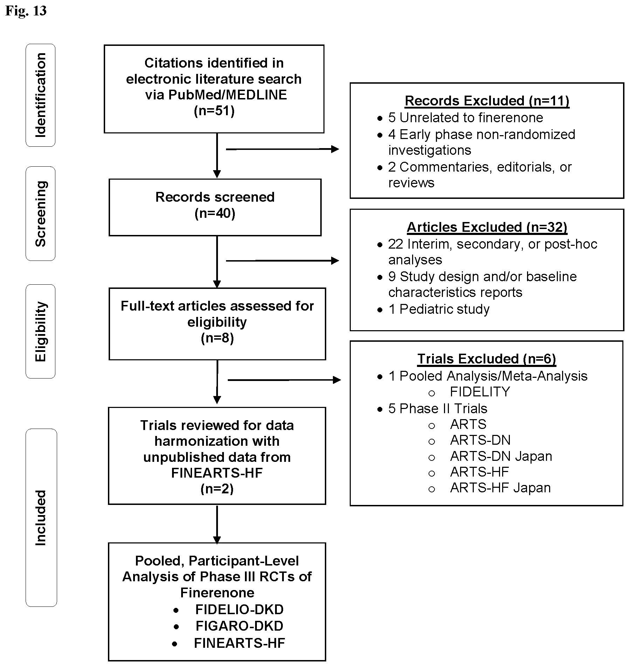

There were 842 and 1024 total heart failure events respectively in the finerenone and placebo groups (rate ratio, 0.82; 95% confidence interval [CI], 0.71 to 0.94, p = 0.006). The first occurrence of cardiovascular death or heart failure event was also reduced in patients receiving finerenone compared with placebo (HR=0.84, 95% CI 0.76-0.94). The incidence of death from cardiovascular causes was 8.1% and 8.7%, respectively (hazard ratio, 0.93; 95% CI, 0.78 to 1.11). The primary outcome was consistent in all 17 prespecified subgroups (Figure 11 and Figure 12), including based on ejection fraction or baseline use of SGLT2 inhibitors.

At months 6, 9 and 12, there was a mean change from baseline in the KCCQ total symptoms score of 8.0 points in the finerenone group and 6.4 points in the placebo group (between-group difference 1.6 points; 95% CI, 0.8 to 2.3, p < 0.001). NYHA class at 12 months improved in 557 patients (18.6%) in the finerenone group compared with 553 patients (18.4%) in the placebo group (odds ratio for improvement, 1.01; 95% CI, 0.88 to 1.15). The kidney composite outcome occurred in 75 patients (2.5%) in the finerenone group and 55 patients (1.8%) in the placebo group (hazard ratio, 1.33; 95% CI, 0.94 to 1.89). Death from any cause occurred in 491 patients (16.4%) in the finerenone group and 522 patients (17.4%) in the placebo group (hazard ratio, 0.93; 95% CI, 0.83 to 1.06).

Among those who received study drug, 611 patients (20.4%) in the finerenone group and 616 patients (20.6%) in the placebo group discontinued the trial drug for reasons other than death. At the end of study visit, among the patients who were continuing therapy, 68.4% in finerenone group were taking the individualized target dose, as compared with 78.4% in the placebo group. Mean daily dose of finerenone was 16mg and 32mg for patients with baseline eGFR ≤60 ml/min/1.73 m² and > 60 ml/min/1.73 m², respectively, with placebo patients receiving 17mg and 34mg of study drug, respectively.

Serious adverse events were similar between treatment groups (Table 21, Table 22).

**Table 21. Treatment Emergent Safety Outcomes *.**

| **Event** | **Finerenone (N = 2993)** | **Placebo (N = 2993)** |
|---|---|---|
| Any Serious adverse event | 1157 (38.7%) | 1213 (40.5%) |
| Serum creatinine ≥3.0 mg/dl | 57 (2.0 %) | 34 (1.2 %) |
| Serum potassium | | |
| >5.5 mmol/liter | 413 (14.3%) | 199 (6.9 %) |
| >6.0 mmol/liter | 86 (3.0%) | 41 (1.4 %) |
| < 3.5 mmol/liter | 127 (4.4 %) | 281 (9.7 %) |
| Investigator-Reported Hyperkalemia | 289 (9.7 %) | 125 (4.2 %) |
| Hyperkalemia leading to Hospitalization | 16 (0.5 %) | 6 (0.2 %) |
| Hyperkalemia leading to Death | 0 (0%) | 0 (0%) |
| Systolic blood pressure <100 mm Hg | 538 (18.5%) | 361 (12.4%) |

| | | |
|---|---|---|
| *Treatment emergent defined as all safety outcomes that occurred in patients who received at least one dose of study drug and up until 3 days following permanent discontinuation. All safety events occurring any time during the trial period are shown in Table. All safety analyses were restricted to the 5986 patients who received at least one dose of study drug. Counts of patients with abnormal creatinine, potassium, and SBP values were further restricted to patients with at least one treatment-emergent assessment (n=5785, 5787, and 5815 respectively) | | |

**Table 22. All adverse events during randomized treatment through the end of study**

| | **Event** | | **Finerenone (N = 2993)** | **Placebo (N = 2993)** |
|---|---|---|---|---|
| Any Serious adverse event | | | 1359 (45.4%) | 1378 (46.0%) |
| | Serum creatinine ≥3.0 mg/dl | | 77 (2.6%) | 45 (1.5%) |
| Serum potassium | | | | |
| | >5.5 mmol/liter | | 426 (14.6%) | 207 (7.1 %) |
| | | >6.0 mmol/liter | 90 (3.1 %) | 44 (1.5%) |
| < 3.5 mmol/liter | | | 145 (5.0%) | 299 (10.3%) |
| | Investigator-Reported Hyperkalemia | | 311 (10.4%) | 136 (4.5 %) |
| Hyperkalemia leading to Hospitalization | | | 24 (0.8%) | 7 (0.2 %) |
| Hyperkalemia leading to Death | | | 0 (0%) | 0 (0%) |
| Systolic blood pressure <100 mm Hg | | | 556 (19.0%) | 374 (12.7%) |

| | | | | |
|---|---|---|---|---|
| * Defined as all safety events that occurred in patients who received at least one dose of study drug and up until end of study data collection. All safety analyses were restricted to the 5986 patients who received at least one dose of study drug. Counts of patients with abnormal creatinine, potassium, and SBP values were further restricted to patients with at least one post-baseline assessment (n=5849, 5836, and 5869 respectively) | | | | |

At months 6, 9 and 12, there was a mean change from baseline in the KCCQ total symptoms score of 8.0 points in the finerenone group and 6.4 points in the placebo group (between-group difference 1.6 points; 95% CI, 0.8 to 2.3, p < 0.001). NYHA class at 12 months improved in 557 patients (18.6%) in the finerenone group compared with 553 patients (18.4%) in the placebo group (odds ratio for improvement, 1.01; 95% CI, 0.88 to 1.15). The kidney composite outcome occurred in 75 patients (2.5%) in the finerenone group and 55 patients (1.8%) in the placebo group (hazard ratio, 1.33; 95% CI, 0.94 to 1.89). Death from any cause occurred in 491 patients (16.4%) in the finerenone group and 522 patients (17.4%) in the placebo group (hazard ratio, 0.93; 95% CI, 0.83 to 1.06).

Among those who received study drug, 611 patients (20.4%) in the finerenone group and 616 patients (20.6%) in the placebo group discontinued the trial drug for reasons other than death. At the end of study visit, among the patients who were continuing therapy, 68.4% in finerenone group were taking the individualized target dose, as compared with 78.4% in the placebo group. Mean daily dose of finerenone was 16mg and 32mg for patients with baseline eGFR ≤60 ml/min/1.73 m2 and > 60 ml/min/1.73 m2, respectively, with placebo patients receiving 17mg and 34mg of study drug, respectively.

Serious adverse events were similar between treatment groups (Table 21, Table 22). Patients in the finerenone group were more likely to have increases in creatinine and potassium levels than those in the placebo group, although potassium values greater than 6.0 mmol/L were rare as were hyperkalemia episodes leading to hospitalization and there were no reported fatal episodes of hyperkalemia. Rates of decreased potassium levels (hypokalemia) by laboratory assessment or investigator report were less frequent with finerenone. The mean systolic blood pressure at 6 months was 3.4 mm Hg (95% CI, 2.6 to 4.2) lower in the finerenone group than in the placebo group. The most frequent adverse events are summarized in Table 23.

### Table 23. Serious Adverse Events, Adverse Events Leading to Discontinuation, and Other Adverse Events of Interest

**Table 23.1: Number of subjects with a treatment-emergent* SAE (>=0.5% in Finerenone group), by preferred term**

| | Finerenone | Placebo |
|---|---|---|
| | n=2993 | n=2993 |
| Pneumonia | 87 (2.9%) | 110 (3.7%) |
| Atrial fibrillation | 77 (2.6%) | 74 (2.5%) |
| COVID-19 | 69 (2.3%) | 71 (2.4%) |
| Acute kidney injury | 54 (1.8%) | 28 (0.9%) |
| Death | 29 (1.0%) | 52 (1.7%) |
| Angina unstable | 38 (1.3 %) | 36 (1.2%) |
| Anaemia | 34 (1.1%) | 31 (1.0%) |
| COVID-19 pneumonia | 28 (0.9%) | 30 (1.0%) |
| Urinary tract infection | 29 (1.0%) | 26 (0.9%) |
| Syncope | 25 (0.8%) | 22 (0.7%) |
| Chest pain | 23 (0.8%) | 22 (0.7%) |
| Chronic obstructive pulmonary disease | 18 (0.6%) | 27 (0.9%) |
| Angina pectoris | 17 (0.6%) | 26 (0.9%) |
| Cellulitis | 17 (0.6%) | 24 (0.8%) |
| Sepsis | 25 (0.8%) | 16 (0.5%) |
| Coronary artery disease | 20 (0.7%) | 17 (0.6%) |
| Cardiac failure | 18 (0.6%) | 18 (0.6%) |
| Sudden death | 16 (0.5%) | 17 (0.6%) |
| Femur fracture | 15 (0.5%) | 17 (0.6%) |
| Fall | 15 (0.5%) | 14 (0.5%) |
| Gastrointestinal haemorrhage | 17 (0.6%) | 8 (0.3 %) |
| Hyperkalaemia | 19 (0.6%) | 6 (0.2 %) |

| | | |
|---|---|---|
| * Treatment emergent SAE is defined as any event occurring after a patient has received one dose of study drug and within 3 days of permanent discontinuation | | |

**Table 23.2: Number of subjects with AE of death or leading to death (>=0.2% in Finerenone group), by preferred term**

| | Finerenone | Placebo |
|---|---|---|
| | n=2993 | n=2993 |
| Death | 70 (2.3 %) | 97 (3.2%) |
| Pneumonia | 27 (0.9 %) | 23 (0.8%) |
| Sudden death | 20 (0.7 %) | 26 (0.9%) |
| Sudden cardiac death | 18 (0.6 %) | 19 (0.6%) |
| Sepsis | 19 (0.6 %) | 7 (0.2 %) |
| Septic shock | 12 (0.4 %) | 12 (0.4%) |
| COVID-19 | 7 (0.2 %) | 17 (0.6%) |
| Cardiac arrest | 12 (0.4 %) | 12 (0.4%) |
| COVID-19 pneumonia | 9 (0.3 %) | 10 (0.3%) |
| Cardiac failure | 6 (0.2 %) | 12 (0.4%) |
| Respiratory failure | 7 (0.2 %) | 4 (0.1%) |
| Lung neoplasm malignant | 7 (0.2 %) | 3 (0.1%) |

**Table 23.3: Number of subjects with treatment-emergent AE leading to treatment discontinuation (>=0.1 % in Finerenone group), by preferred term**

| | Finerenone | Placebo |
|---|---|---|
| | n=2993 | n=2993 |
| Hyperkalaemia | 12 (0.4%) | 5 (0.2 %) |
| Renal impairment | 5 (0.2 %) | 4 (0.1%) |
| Diarrhoea | 4 (0.1 %) | 4 (0.1%) |
| Nausea | 3 (0.1 %) | 3 (0.1%) |
| Asthenia | 5 (0.2 %) | 0 (0.0 %) |
| Dyspnoea | 3 (0.1 %) | 1 (0.0 %) |
| Hypotension | 3 (0.1 %) | 0 (0.0 %) |

### Discussion

In this randomized, placebo-controlled trial in patients with heart failure and a mildly reduced or preserved ejection fraction, finerenone resulted in a lower risk of the primary composite outcome of cardiovascular death and total worsening heart failure events, compared with placebo. Each of the components of this composite outcome was less common in the finerenone group than in the placebo group, and results were consistent in all pre-specified subgroups. Finerenone reduced both total and first worsening heart failure events, and modestly improved patient-reported health status as measured by the Kansas City Cardiomyopathy Questionnaire total symptom score. Overall serious adverse events were similar between treatment groups, while those receiving finerenone experienced more hyperkalemia, as evidenced by laboratory measurements or investigator-reported events, and more elevation in serum creatinine, but hypokalemia was less frequent with finerenone.

TOPCAT was the only other outcomes trial to test mineralocorticoid antagonism in patients with heart failure and mildly reduced or preserved ejection fraction. In that trial, the steroidal MRA spironolactone did not reduce the primary endpoint although post hoc analyses revealed differential event rates, study drug use, and treatment effects according to geographic region. Thus, the results of FINEARTS-HF with the nonsteroidal MRA finerenone provide the first definitive evidence that an MRA is beneficial in these patients and will inform clinicians treating these patients and potentially future guidelines.

While we did not observe any heterogeneity in the treatment effect for the primary outcome in any of the pre-specified subgroups, suggestions of greater benefit in patients with a more recent heart failure event were consistent with findings in other trials. Importantly, there was no evidence of heterogeneity in relation to background use of a sodium-glucose co-transporter 2 inhibitor, the only treatment with a strong guideline recommendation in this population, although this recommendation was not introduced until late in the conduct of the FINEARTS-HF trial. Finerenone treatment also led to a modest improvement in a patient-reported outcome (KCCQ-Total Symptom Score) which was of a similar magnitude to that noted with other agents in other outcomes trials in heart failure (e.g. PARGAGON, DELIVER, EMPEROR) . However, finerenone did not improve NYHA functional class, a physician-reported assessment of functional status. This may reflect the relative insensitivity of the four-category NYHA classification compared to the KCCQ-Total Symptom Score and this discordance has been noted in other trials.

Finerenone did not reduce the occurrence of the secondary composite kidney outcome in this population at low risk for kidney disease progression with low levels of albuminuria and low total number of kidney events. This finding is in contrast to FIDELITY, conducted in T2D patients with CKD and albuminuria at high risk of kidney disease progression, where finerenone reduced a similar kidney endpoint. However, the response in eGFR after initiation of a MRA may be different between patients with heart failure compared to patients with diabetic kidney disease. Other inhibitors of the renin-angiotensin-aldosterone system have failed to show kidney benefits in patients with heart failure but demonstrated a nephroprotective effect in patients with chronic kidney disease and type 2 diabetes . With respect to safety, in line with previous trials with MRAs, hyperkalemia, whether assessed as a measured laboratory value or as an investigator-reported adverse event, was more common in the finerenone group than in the placebo group, although only 0.5% of patients in the finerenone group, and 0.2% of patients in the placebo group had hyperkalemia leading to hospitalization and no patients experienced hyperkalemia leading to death.

Among patients with heart failure and a mildly reduced or preserved ejection fraction, finerenone reduced the risk of the primary composite outcome of cardiovascular death and total heart failure events, reduced total heart failure events, and improved patient-reported health status. These findings were consistent across prespecified subgroups. Hyperkalemia was more common, and hypokalemia less common, in those receiving finerenone. These data support the use of finerenone in patients with heart failure with mildly reduced or preserved ejection fraction.

### Example 14 - FINE-HEART: An Integrated Pooled Analysis of Finerenone across Three Phase III Trials of Heart Failure and Chronic Kidney Disease with Type 2 Diabetes

Cardiovascular-kidney-metabolic syndrome (CKM) is a health disorder that connects cardiovascular diseases, chronic kidney disease, and diabetes. The non-steroidal mineralocorticoid receptor antagonist, finerenone, has been studied in 3 prospective randomized trials of patients with CKM. However, none of these trials were individually powered to evaluate treatment effects on mortality outcomes.

Objective: To summarize the efficacy and safety of finerenone on cardiovascular, kidney, and mortality outcomes in all completed phase III clinical trials conducted to date.

### Participant-level pooled analysis of 3 phase III global, multicenter, double-blind, placebo-controlled randomized clinical trials

Chronic kidney disease and type 2 diabetes (FIDELIO-DKD and FIGARO-DKD) and heart failure with mildly reduced or preserved ejection fraction (FINEARTS-HF)

The primary endpoint was cardiovascular death (excluding undetermined deaths). A prespecified sensitivity analysis considered deaths of undetermined causes to be cardiovascular deaths. Secondary endpoints included all cause death, all-cause hospitalization, HF hospitalization, and a composite kidney outcome (50% or greater sustained decline in estimated glomerular filtration rate, kidney failure, or death from kidney causes).

The 3 trials included 18,991 participants (mean age 67±10 years; 35% women). During 2.9 years median follow-up, cardiovascular death occurred in 421 (4.4%) assigned to finerenone and 471 (5.0%) assigned to placebo (HR 0.89; 95% CI 0.78-1.01; P=0.076). Consistent findings were observed in the prespecified sensitivity analysis including both cardiovascular deaths and undetermined deaths (HR 0.88; 95% CI 0.79-0.98; P=0.025). Death from any cause occurred in 1,042 (11.0%) participants in the finerenone arm and 1,136 (12.0%) in the placebo arm (HR 0.91; 95% CI 0.84-0.99; P=0.027). Finerenone further reduced the risk of HF hospitalization (HR 0.83; 95% CI 0.75-0.92; P<0.001) and the composite kidney outcome (HR 0.80; 95% CI 0.72-0.90; P<0.001). Treatment effects on cardiovascular death were generally consistent across the 16 subgroups examined. Serious adverse events were lower with finerenone than placebo (34.6% vs. 36.6%), but hyperkalemia was increased with finerenone.

Conclusions and Relevance: While this pooled analysis failed to demonstrate significant reductions in cardiovascular death, finerenone was associated with significantly lower deaths of any cause, cardiovascular events, and kidney outcomes. The totality of the evidence thus supports the disease-modifying potential of finerenone in broad, high-risk patient populations encompassing cardiovascular, kidney, and metabolic diseases. ClinicalTrials.gov Trial Registration: NCT04435626; NCT02540993; NCT02545049. PROSPERO Registration of Pooled Analysis: CRD42024570467.

The efficacy and safety of the non-steroidal mineralocorticoid receptor antagonist finerenone across a broad range of people with cardio-kidney-metabolic conditions: We conducted a pooled, participant-level analysis of all 3 phase III clinical trials of finerenone completed to date. While finerenone did not significantly reduce the primary endpoint of cardiovascular death, this result was sensitive to the definition of cardiovascular death and all-cause death was significantly lower with finerenone as were cardiovascular and kidney events. The totality of evidence supports the addition of finerenone as a foundational pillar of care in broad, high-risk patient populations encompassing cardiovascular, kidney, and metabolic diseases.

### In Detail:

### Methods

Search Strategy and Trial Selection. We conducted a participant-level pooled analysis of 2 trials of CKD and type 2 diabetes (FIDELIO-DKD [Finerenone in Reducing Kidney Failure and Disease Progression in Diabetic Kidney Disease; NCT02540993] and FIGARO-DKD [Finerenone in Reducing Cardiovascular Mortality and Morbidity in Diabetic Kidney Disease; NCT02545049]) and a trial of patients with HF (FINEARTS-HF [FINerenone trial to investigate Efficacy and sAfety superioR to placebo in paTientS with Heart Failure; NCT04435626]) that included patients with and without diabetes. The designs18-20 and primary results15-17 of each of the 3 trials have been published. Key design elements of each of the trials are summarized in Table 24.

**Table 24 - Key Study Design Features**

| | **FINEARTS-HF** | | **FIDELIO-DKD and FIGARO-DKD** | |
|---|---|---|---|---|
| **Validly Randomized** | 6,001 | | 12,990 | |
| **Countries** | 37 | | 48 | |
| **Patient population** | HF with mildly reduced or preserved ejection fraction | | CKD and T2D | |
| **Inclusion criteria** | | • Adults (≥40 years) | | • Adults (≥18 years old) |
| | | • Symptomatic HF | | • T2D |
| | | • LVEF ≥40% | | • UACR ≥ 30 mg/g |
| | | • Elevation natriuretic peptides | | • Maximally tolerated renin-angiotensin system inhibitors |
| | | • Structural heart disease | | |
| | | • Recent diuretic use | | |
| **Exclusion criteria** | Potassium ≤5.0 mmol/L | | Potassium ≤4.8 mmol/L | |
| **Dosage and titration** | eGFR ≤60: 10 up to 20 mg | | eGFR <60: 10 up to 20 mg | |
| | eGFR >60: 20 up to 40 mg (potentially down to 10 mg) | | eGFR ≥60: 20 mg (potentially down to 10 mg) | |
| **Study duration** | 2.6 years | | 2.6 years (FIDELIO-DKD) | |
| | | | 3.4 years (FIGARO-DKD) | |

| | | | | |
|---|---|---|---|---|
| Abbreviations: CKD = chronic kidney disease; eGFR = estimated glomerular filtration rate; HF = heart failure; T2D = type 2 diabetes; UACR = urine albumin-to-creatinine ratio | | | | |

We further conducted a systematic review of the literature using PubMed and MEDLINE to ensure that other relevant trials were not missed. Data from the FINEARTS-HF trial were unpublished at the time of analysis and included for this analysis.

Design of FIDELIO-DKD and FIGARO-DKD. In brief, both FIDELIO-DKD and FIGARO-DKD trials enrolled adults (≥18 years old) with type 2 diabetes and CKD across 48 countries. FIDELIO-DKD required urinary albumin-to-creatinine ratio (UACR) of 30 to <300mg/g, estimated glomerular filtration rate (eGFR) of 25 to <60mL/min/1.73m2, and a history of diabetic retinopathy or a UACR of 300 to 5,000mg/g and eGFR of 25 to <75mL/min/1.73m2. FIGARO-DKD required a UACR of 30 to <300mg/g and an eGFR of 25 to 90 mL/min/1.73m2 or a UACR of 300 to 5,000mg/g and an eGFR ≥60mL/min/1.73m2. Both trials required a serum potassium ≤4.8 mmol/L for enrollment. Renin-angiotensin system inhibitor use and dosing was optimized prior to screening during run-in phases (lasting 4 to 16 weeks) in both trials. Patients with symptomatic HF with reduced ejection fraction were excluded, but those with HF and higher EF were eligible.

Design of FINEARTS-HF. FINEARTS-HF enrolled adults (≥40 years) with symptomatic HF with mildly reduced or preserved ejection fraction across 37 countries. Key inclusion criteria included left ventricular ejection fraction ≥40%, elevated natriuretic peptides (adjusted based on atrial fibrillation status and clinical setting of screening), evidence of structural heart disease and recent diuretic use for at least 30 days. Patients were required to have an eGFR ≥25 mL/min/1.73m2 and a serum potassium level ≤5.0 mmol/L for enrollment. Participants could be enrolled regardless of clinical care setting (whether hospitalized, recently hospitalized, or ambulatory).

All participants were randomly allocated to finerenone or placebo with initial dosing determined based on kidney function. The initial dose was 10mg for patients with an eGFR <60mL/min/1.73m2, titrated up to a target dose of 20mg once daily as tolerated. Participants with an eGFR ≥60mL/min/1.73m2 were started at the target dose of 20mg once daily. In FINEARTS-HF, participants with an eGFR >60mL/min/1.73m2 were started on 20mg and could be titrated up to 40mg once daily as tolerated, while 20 mg was the target dose for patients with eGFR ≤ 60 ml/min/1.73m2. The trial protocols were approved by ethics committees or institutional review boards at all participating sites and all patients provided explicit written informed consent.

FINE-HEART Pooled Analysis Endpoints. Individual participant-level data were accessed and pooled with harmonized data elements for baseline characteristics and clinical outcomes (Table 25).

**Table 25 - Harmonization of Key Outcome Measures and Their Assessment Across Trials**

| **Data Element** | **Harmonization Strategy** | |
|---|---|---|
| **Primary Efficacy Endpoint of CV Death** | In the FIDELIO-DKD and FIGARO-DKD trials, deaths of undetermined causes were considered cardiovascular. In the FINEARTS-HF trial, deaths of undetermined causes were not considered cardiovascular. | |
| | For the pooled FINE-HEART analysis, the primary endpoint was CV death alone (excluding deaths of undetermined causes). Prespecified sensitivity analyses considered all undetermined deaths as cardiovascular in etiology. | |
| **Stratification Factors for Efficacy Models** | In the time to event analyses, stratification factors were individual trial and geographic region. The countries included in each geographic region varied by trial. | |
| | For the purposes of FINE-HEART, geographic region was harmonized to the following designations: | |
| | | • Western Europe, Oceania and Others: Australia, Austria, Belgium, Denmark, France, Germany, Ireland, Israel, Italy, Netherlands, New Zealand, Norway, Portugal, South Africa, Spain, Sweden, Switzerland, United Kingdom |
| | | • Eastern Europe: Bulgaria, Czechia, Finland, Greece, Hungary, Latvia, Lithuania, Poland, Romania, Russia, Slovakia, Turkey, Ukraine |
| | | • Asia: China, Hong Kong, India, Japan, Malaysia, Philippines, Singapore, South Korea, Taiwan, Thailand, Vietnam |
| | | • North America: Canada, Puerto Rico, United States of America |
| | | • Latin America: Argentina, Brazil, Chile, Colombia, Mexico |
| **Kidney Composite Endpoint** | The primary kidney composite endpoint specified in the FIDELITY program (which encompassed FIDELIO-DKD and FIGARO-DKD) was time to first onset of kidney failure, sustained ≥57% decrease in eGFR from baseline over ≥4 weeks, or death from kidney causes. The kidney composite endpoint (a secondary endpoint) in the FINEARTS-HF trial was time to first onset of kidney failure or sustained ≥50% decrease in eGFR from baseline over ≥4 weeks. Kidney outcomes were adjudicated by independent clinical events committees in each of the 3 trials. | |
| | In FINE-HEART, the kidney composite endpoint selected was time to first onset of kidney failure, sustained ≥50%decrease in eGFR from baseline over ≥4 weeks, or death from kidney causes. | |
| **Treatment Emergent Adverse Events** | The definition of treatment emergent safety events slightly differed across trials. Whereas FINEARTS-HF considered treatment emergent adverse events as any adverse event occurring in a patient who received at least one dose of study drug and up until 3 days of permanent discontinuation, FIDELIO-DKD and FIGARO-DKD considered the same period but excluded adverse events while a participant was on temporary interruption of study drug. | |
| | To remain conservative in our safety accounting, in FINE-HEART, we considered all adverse events occurring in any patient who has received at least one dose of study drug and within 3 days of permanent discontinuation, including during periods of temporary drug interruption. | |

All participants randomized in each of the 3 trials were considered for this pooled analysis with only patients with critical Good Clinical Practice violations excluded. All efficacy outcomes were analyzed in randomized patients under intention-to-treat principles, while all safety outcomes were analyzed in randomized patients who had taken at least one dose of the study drug. The primary endpoint (cardiovascular death) was assessed across key subgroups including age, sex, race, region, baseline body mass index, baseline systolic blood pressure, Kidney Disease: Improving Global Outcomes (KDIGO) risk, baseline serum potassium levels, baseline eGFR, baseline UACR, history of HF, history of diabetes, presence of CKD, degree of cardio-kidney-metabolic overlap, and baseline use of sodium-glucose-co-transporter-2 inhibitors (SGLT2i) or glucagon-like-peptide-1 receptor agonists (GLP-1RA).

The prespecified primary endpoint for FINE-HEART was time to cardiovascular death. The definition of the endpoint cardiovascular death differed slightly between the three trials and was harmonized for FINE-HEART as time to cardiovascular death (excluding undetermined deaths); Table 25. All deaths were adjudicated by independent clinical endpoint committees in each of the respective trials included in this pooled analysis. Other prespecified endpoints included a kidney composite endpoint (defined as a sustained decline in eGFR to ≥50% from baseline, sustained decrease in eGFR to <15 mL/min/1.73m2, end-stage kidney disease, and death due to kidney causes), HF hospitalization, composite of cardiovascular death or HF hospitalization, new-onset atrial fibrillation, major adverse cardiovascular events (a composite of non-fatal myocardial infarction, non-fatal stroke, HF hospitalization, or cardiovascular death), all-cause death, and hospitalization for any reason. The composite of all-cause death or all-cause hospitalization was defined post hoc to describe the total burden or morbidity and mortality. Select treatment-emergent adverse events related to hyperkalemia, acute kidney injury, hypotension, and gynecomastia were also reported in the pooled population.

Statistical Analysis. All primary and secondary endpoints were analyzed as time-to-first endpoints and analyzed using a stratified Cox proportional hazards model including the study intervention group as a fixed effect and stratified by geographic region and individual trial (Table 25). All treatment effect estimates are presented as hazard ratios (HR) with associated 95% confidence intervals (CI). Select primary and secondary endpoints were additionally graphically displayed using Kaplan Meier methods. A prespecified sensitivity analysis was conducted for the primary endpoint which considered deaths of undetermined causes to be cardiovascular deaths in all 3 trials. Treatment effects on cardiovascular death were assessed across all prespecified subgroups. Incidence rates of cardiovascular death as a function of baseline eGFR and log-transformed UACR were estimated separately for each treatment arm using Poisson regression models, allowing for potentially non-linear relationships using restricted cubic splines with 3 knots. The treatment effect of finerenone was then estimated as the ratio of these 2 group-specific estimates.

The statistical analysis plan for this pooled analysis was prespecified and the protocol was prospectively registered in the International Prospective Register of Systematic Reviews (PROSPERO CRD42024570467) before unblinding the FINEARTS-HF trial. All 3 trials were assessed as high quality with a low risk of bias prior to pooling (Table 26).

**Table 26 - Risk of Bias Assessment Using the Revised Tool to Assess Risk of Bias in Randomized Trials (RoB 2.0)**

| Study Name | Year | Randomization Bias | Intervention Deviation | Missing Outcome Data | Measurement of Outcome | Reporting of Outcome | Overall Risk |
|---|---|---|---|---|---|---|---|
| FIDELIO-DKD | 2020 | Low | Low | Low | Low | Low | Low |
| FIGARO-DKD | 2021 | Low | Low | Low | Low | Low | Low |
| FINEARTS | 2024 | Low | Low | Low | Low | Low | Low |

The primary analyses, interpretation of the data, and initial manuscript drafting were conducted. Statistical analyses were conducted using STATA version 18.

### Results

Overall, FINE-HEART comprised 18,991 participants from these 3 trials. Baseline clinical profiles and treatment patterns are summarized for the overall pooled population (Table 27.1) and by individual trial (Table 27.2).

**Table 27.1 - Baseline Characteristics**

| | Finerenone | Placebo |
|---|---|---|
| | n=9,501 | n=9,490 |
| Age | 67.0 ± 10.0 | 67.1 ± 10.2 |
| Female | 3390 (35.7%) | 3274 (34.5%) |

| *Race^{a}* | | |
|---|---|---|
| Asian | 1910 (20.1%) | 1946 (20.5%) |
| Black | 300 (3.2%) | 308 (3.2%) |
| Other | 476 (5.0%) | 447 (4.7%) |
| White | 6815 (71.7%) | 6789 (71.5%) |
| | | |

| *Region* | | |
|---|---|---|
| Asia | 1808 (19.0%) | 1815 (19.1%) |
| Eastern Europe | 3001 (31.6%) | 2941 (31.0%) |
| Latin America | 1041 (11.0%) | 1034 (10.9%) |
| North America | 1259 (13.3%) | 1261 (13.3%) |
| Western Europe, Oceania and Others | 2392 (25.2%) | 2439 (25.7%) |
| | | |
| Body Mass Index (kg/m²) | 30.9 ± 6.1 | 30.9 ± 6.0 |
| Systolic Blood Pressure (mmHg) | 134.5 ± 14.9 | 134.4 ± 15.0 |
| Potassium (mmol/L) | 4.4 ± 0.5 | 4.4 ± 0.5 |
| | | |
| eGFR (mL/min/1.73m²) | 58.9 ± 21.0 | 59.1 ± 21.3 |

| *eGFR Category* | | |
|---|---|---|
| < 25 mL/min/1.73m² | 100 (1.1%) | 94 (1.0 %) |
| 25 to < 45 mL/min/1.73m² | 2742 (28.9%) | 2782 (29.3%) |
| 45 to < 60 mL/min/1.73m² | 2513 (26.5%) | 2469 (26.0%) |
| ≥ 60 mL/min/1.73m² | 4145 (43.6%) | 4143 (43.7%) |
| | | |
| UACR (mg/g) | 283 [46-836] | 293 [47-855] |

| *Albuminuria Category* | | |
|---|---|---|
| A1 (< 30 mg/g) | 1885 (20.1%) | 1856 (19.8%) |
| A2 (30 to < 300 mg/g) | 2910 (31.0%) | 2883 (30.7%) |
| A3 (≥300 mg/g) | 4602 (49.0%) | 4646 (49.5%) |
| | | |
| Hemoglobin A1c (%) | 7.3 ± 1.4 | 7.3 ± 1.4 |
| AF on Electrocardiogram | 1449 (15.3%) | 1379 (14.5%) |
| History of HF^{b} | 3488 (36.7%) | 3520 (37.1%) |
| Baseline CKD^{c} | 7949 (83.7%) | 7929 (83.6%) |
| History of DM^{d} | 7715 (81.2%) | 7714 (81.3%) |
| | | |

| *Background Medication Use* | | |
|---|---|---|
| Diuretics | 6291 (66.2%) | 6340 (66.8%) |
| ACEi/ARB/ARNI | 8866 (93.3%) | 8860 (93.4%) |
| Aspirin | 4145 (43.6%) | 4171 (44.0%) |
| Statins | 6687 (70.4%) | 6750 (71.1%) |
| SGLT-2 Inhibitors | 829 (8.7%) | 861 (9.1%) |
| GLP-1 Receptor Agonists | 576 (6.1 %) | 534 (5.6%) |
| Potassium Lowering Therapies^{e} | 99 (1.0%) | 96 (1.0%) |

| | | |
|---|---|---|
| *Abbreviations:* ACEi = angiotensin converting enzyme inhibitors; AF = atrial fibrillation; ARB = angiotensin II receptor blocker; ARNI = angiotensin receptor neprilysin inhibitor; CKD = chronic kidney disease; DM = diabetes mellitus; eGFR = estimated glomerular filtration rate; GLP-1 = glucagon-like peptide-1 receptor agonist; SGLT-2 = sodium-glucose co-transporter-2; UACR = urine albumin-creatinine ratio ^{a}Represents self reported race. Participants choosing not disclose race or who self-identified as multiple races are included in the "Other" category for descriptive purposes. ^{b}HF includes all participants in FINEARTS-HF and those with investigator-reported history of HF in the primary CKD outcomes trials (FIDELIO-DKD, FIGARO-DKD) ^{c}CKD includes all participants in the primary CKD outcomes trials (FIDELIO-DKD, FIGARO-DKD) and participants in FINEARTS-HF with baseline eGFR<60mL/min/1.73m² ^{d}Diabetes includes all participants in the primary CKD outcomes trials (FIDELIO-DKD, FIGARO-DKD) and those with a history of diabetes in FINEARTS-HF ^{e} Includes patiromer, sodium polystyrene sulfonate, calcium polystyrene sulfonate | | |

**Table 27.2 - Baseline Characteristics by Trial**

| | **FIDELIO-DKD** | **FIGARO-DKD** | **FINEARTS-HF** |
|---|---|---|---|
| | **n=5,662** | **n=7,328** | **n=6,001** |
| Age | 65.6 ± 9.0 | 64.2 ± 9.8 | 72.0 ± 9.6 |
| Female | 1688 (29.8%) | 2244 (30.6%) | 2732 (45.5%) |

| *Race^{a}* | | | |
|---|---|---|---|
| Asian | 1430 (25.3%) | 1430 (19.5%) | 996 (16.6%) |
| Black | 262 (4.6%) | 258 (3.5 %) | 88 (1.5 %) |
| Other | 378 (6.7%) | 363 (5.0 %) | 182 (3.0 %) |
| White | 3592 (63.4%) | 5277 (72.0%) | 4735 (78.9%) |
| | | | |

| *Region* | | | |
|---|---|---|---|
| Asia | 1317 (23.3%) | 1323 (18.1%) | 983 (16.4%) |
| Eastern Europe | 1169 (20.6%) | 2123 (29.0%) | 2650 (44.2%) |
| Latin America | 593 (10.5%) | 841 (11.5%) | 641 (10.7%) |
| North America | 942 (16.6%) | 1107 (15.1%) | 471 (7.8 %) |
| Western Europe, Oceania and Others | 1641 (29.0%) | 1934 (26.4%) | 1256 (20.9%) |
| | | | |
| Body Mass Index (kg/m²) | 31.1 ± 6.0 | 31.4 ± 6.0 | 29.9 ± 6.1 |
| Systolic Blood Pressure (mmHg) | 138.0 ± 14.4 | 135.7 ± 14.0 | 129.4 ± 15.3 |
| Potassium (mmol/L) | 4.4 ± 0.5 | 4.3 ± 0.4 | 4.4 ± 0.5 |
| | | | |
| eGFR (mL/min/1.73m²) | 44.3 ± 12.6 | 67.8 ± 21.7 | 62.1 ± 19.7 |

| *eGFR Category* | | | |
|---|---|---|---|
| < 25 mL/min/1.73m² | 135 (2.4%) | 27 (0.4 %) | 32 (0.5 %) |
| 25 to < 45 mL/min/1.73m² | 2973 (52.5%) | 1251 (17.1%) | 1300 (21.7%) |
| 45 to < 60 mL/min/1.73m² | 1896 (33.5%) | 1530 (20.9%) | 1556 (25.9%) |
| ≥ 60 mL/min/1.73m² | 656 (11.6%) | 4519 (61.7%) | 3113 (51.9%) |
| | | | |
| Baseline UACR (mg/g) | 853 [446-1636] | 309 [108-741] | 18 [7-67] |

| *Albuminuria Category* | | | |
|---|---|---|---|
| A1 (< 30 mg/g) | 23 (0.4 %) | 207 (2.8%) | 3511 (60.6%) |
| A2 (30 to < 300 mg/g) | 682 (12.1%) | 3399 (46.4%) | 1712 (29.5%) |
| A3 (≥300 mg/g) | 4954 (87.5%) | 3720 (50.8%) | 574 (9.9 %) |
| | | | |
| Hemoglobin A1c (%) | 7.7 ± 1.3 | 7.7 ± 1.4 | 6.4 ± 1.2 |
| AF on Electrocardiogram | 233 (4.1 %) | 302 (4.1 %) | 2293 (38.2%) |
| History of HF^{b} | 436 (7.7%) | 571 (7.8%) | 6001 (100.0%) |
| Baseline CKD^{c} | 5662 (100.0%) | 7328 (100.0%) | 2888 (48.1%) |
| History of DM^{d} | 5662 (100.0%) | 7328 (100.0%) | 2439 (40.6%) |
| | | | |

| *Background Medication Use* | | | |
|---|---|---|---|
| Diuretics | 3209 (56.7%) | 3492 (47.7%) | 5930 (98.8%) |
| ACEi/ARB/ARNI | 5648 (99.8%) | 7319 (99.9%) | 4759 (79.3%) |
| Aspirin | 2787 (49.2%) | 3581 (48.9%) | 1948 (32.5%) |
| Statins | 4208 (74.3%) | 5179 (70.7%) | 4050 (67.5%) |
| SGLT-2 Inhibitors | 258 (4.6%) | 615 (8.4%) | 817 (13.6%) |
| GLP-1 Receptor Agonists | 393 (6.9 %) | 550 (7.5 %) | 167 (2.8%) |
| Potassium Lowering Therapies | 136 (2.4%) | 46 (0.6%) | 13 (0.2%) |

| | | | |
|---|---|---|---|
| *Abbreviations:* ACEi = angiotensin converting enzyme inhibitors; AF = atrial fibrillation; ARB = angiotensin II receptor blocker; ARNI = angiotensin receptor neprilysin inhibitor; CKD = chronic kidney disease; DM = diabetes mellitus; eGFR = estimated glomerular filtration rate; GLP-1 = glucagon-like peptide-1 receptor agonist; SGLT-2 = sodium-glucose co-transporter-2; UACR = urine albumin-creatinine ratio ^{a}Represents self reported race. Participants choosing not disclose race or who self-identified as multiple races are included in the "Other" category for descriptive purposes. ^{b}HF includes all participants in FINEARTS-HF and those with investigator-reported history of HF in the primary CKD outcomes trials (FIDELIO-DKD, FIGARO-DKD) ^{c}CKD includes all participants in the primary CKD outcomes trials (FIDELIO-DKD, FIGARO-DKD) and participants in FINEARTS-HF with baseline eGFR<60mL/min/1.73m² ^{d}Diabetes includes all participants in the primary CKD outcomes trials (FIDELIO-DKD, FIGARO-DKD) and those with a history of diabetes in FINEARTS-HF ^{e} Includes patiromer, sodium polystyrene sulfonate, calcium polystyrene | | | |

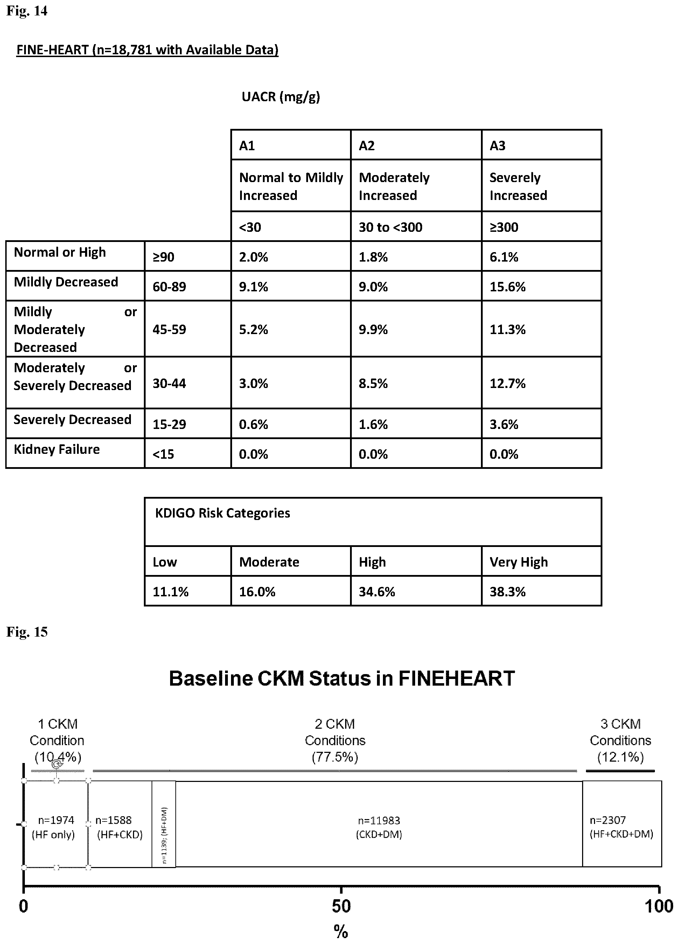

Mean age was 67±10 years, 35.1% were women, and participants were enrolled across all major geographic regions. Participants were at high risk for CKD progression (Figure 14) with either reduced eGFR (30.1% with eGFR <45 and 26% with eGFR 45 to 60 mL/min/1.73m2) and/or albuminuria (30.8% with "A2" UACR 30-299mg/g and 49.2% with"A3" UACR≥300mg/g). 2,307 (12.1%) participants had all CKM conditions (HF, CKD, and diabetes); Figure 15. At baseline, 1,690 (8.9%) participants were co-treated with an SGLT2i and 1,110 (5.8%) with a GLP-1RA. Baseline characteristics and concurrent medical management were well-balanced between treatment arms (Table 27.1).

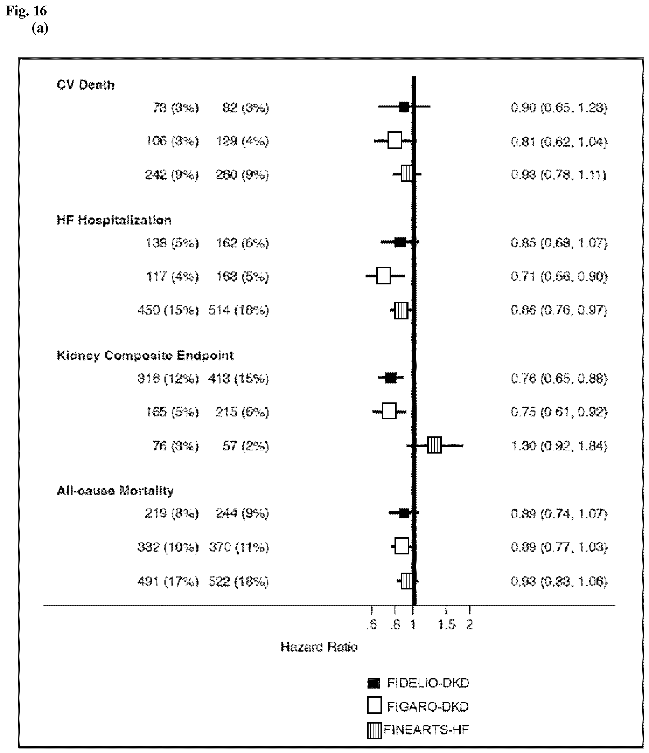

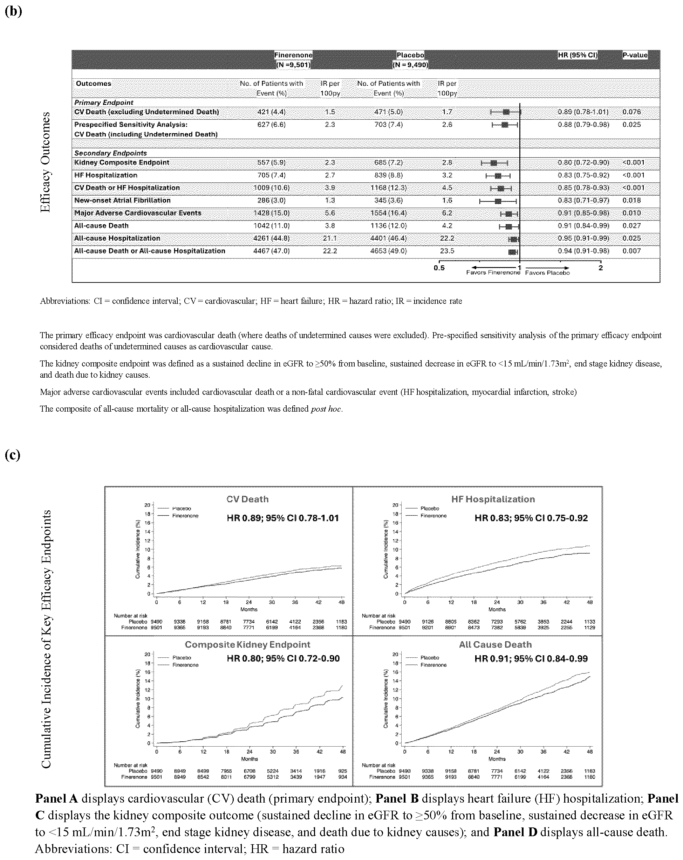

Median duration of follow-up was 2.6 years (FIDELIO-DKD), 3.4 years (FIGARO-DKD), and 2.6 years (FINEARTS-HF). Median follow-up of the pooled patient population was 2.9 years. Cardiovascular death occurred in 421 (4.4%) in the finerenone arm and 471 (5.0%) in the placebo arm (HR 0.89; 95% CI 0.78-1.01; P=0.076) with consistent findings in prespecified sensitivity analysis including both cardiovascular deaths and undetermined deaths (HR 0.88; 95% CI 0.79-0.98; P=0.025); Figure 16 (b). Effects on cardiovascular death were consistent across individual trials: FIDELIO-DKD (HR 0.89; 95% CI 0.65-1.22); FIGARO-DKD (HR 0.81; 95% CI 0.62-1.04); and FINEARTS-HF (HR 0.93; 95% CI 0.78-1.11); Pinteraction=0.68; Figure 16 (a). Deaths from any cause occurred in 1,042 (11.0%) participants in the finerenone arm and 1,136 (12.0%) in the placebo arm (HR 0.91; 95% CI 0.84-0.99; P=0.027); Figure 16(c).

Finerenone reduced the risk of the composite kidney endpoint by 20% (HR 0.80; 95% 0.72-0.90; P<0.001) with effects that appeared to be driven by FIDELIO-DKD and FIGARO-DKD (eFigure 16 (a)). Finerenone reduced the risk of hospitalizations due to HF alone (HR 0.83; 95% CI 0.75-0.92; P<0.001) and the composite of cardiovascular death or HF hospitalization (HR 0.85; 95% CI 0.78-0.93; P<0.001). Hospitalizations of any cause were also lower with finerenone compared with placebo (HR 0.95; 95% 0.91-0.99; P=0.025). Finerenone further reduced the risk of the composite of all-cause death or all-cause hospitalization (HR 0.94; 0.91-0.98; P=0.007).

Additional risk reductions were observed for the prevention of new-onset atrial fibrillation and major adverse cardiovascular events (Figure 16 (b)).

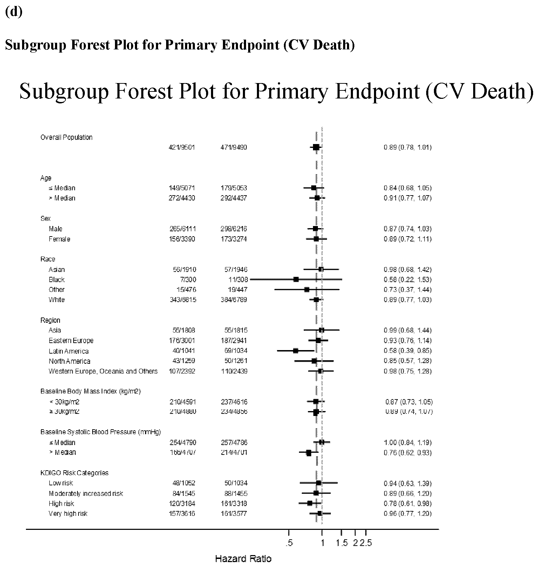

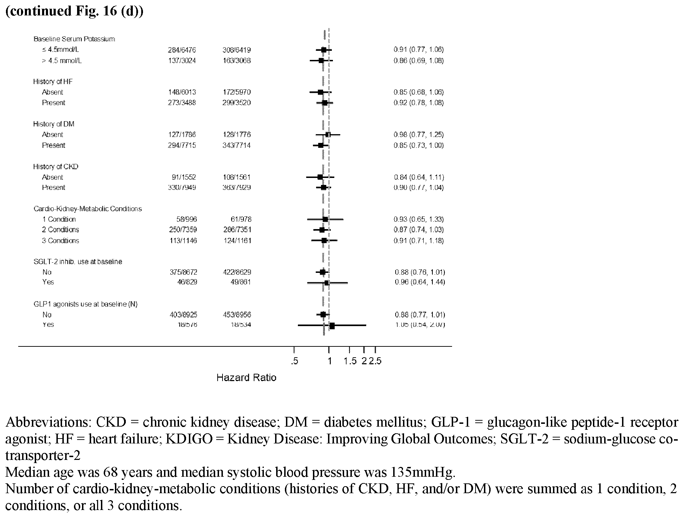

Treatment effects on cardiovascular death were generally consistent across the 16 subgroups examined (Figure 16 (d)). The efficacy of finerenone on cardiovascular death was consistent across the range of eGFR (Pinteraction=0.32) and UACR (Pinteraction=0.55). Treatment effects on cardiovascular death were also consistent across a range of CKM disease burden: 1 condition (HR 0.93; 95% CI 0.65-0.1.33); 2 conditions (HR 0.87; 95% CI 0.74-1.03); and 3 conditions (HR 0.91; 95% CI 0.71-1.18); Pinteraction=0.94.

Incidences of any serious adverse event were lower with finerenone than placebo (34.6% vs. 36.6%), although incidences of serious adverse events leading to drug discontinuation were slightly higher with finerenone (5.4% vs. 4.6%). Laboratory-defined hyperkalemia was increased, while laboratory-defined hypokalemia was decreased with finerenone. Incidences of investigator-reported hyperkalemia leading to permanent treatment discontinuation (1.3% vs. 0.5%) and hyperkalemia-related hospitalization (0.8% vs. 0.2%) were higher with finerenone. There were no deaths related to hyperkalemia and no between-group differences in incidences of acute kidney injury (Table 27.3).

**Table 27.3 - Safety Outcomes**

| | Finerenone | Placebo |
|---|---|---|
| | n=9,482 | n=9,467 |
| Any serious adverse event | 3283 (34.6%) | 3463 (36.6%) |
| Any adverse event leading to treatment discontinuation | 515 (5.4%) | 434 (4.6%) |
| | | |
| Any potassium >5.5 mmol/L^{a} | 1535 (16.5%) | 714 (7.7%) |
| Any potassium >6.0 mmol/L^{a} | 311 (3.3 %) | 133 (1.4%) |
| Any potassium <3.5 mmol/L^{a} | 448 (4.8%) | 938 (10.1%) |
| Hyperkalemia^{b} | 1216 (12.8%) | 586 (6.2%) |
| Hyperkalemia leading to treatment discontinuation^{b} | 123 (1.3%) | 43 (0.5%) |
| Hyperkalemia leading to hospitalization^{b} | 80 (0.8%) | 17 (0.2%) |
| Hyperkalemia leading to death^{b} | 0 (0.0 %) | 0 (0.0 %) |
| | | |
| Acute kidney injury | 345 (3.6 %) | 316 (3.3 %) |
| Acute kidney injury leading to treatment discontinuation | 15 (0.2%) | 12 (0.1%) |
| Acute kidney injury leading to hospitalization | 143 (1.5%) | 116 (1.2%) |
| | | |
| Systolic blood pressure<100mmHg | 1040 (11.1%) | 651 (7.0%) |
| Gynecomastia or breast hyperplasia | 16 (0.2%) | 19 (0.2%) |

| | | |
|---|---|---|
| ^{a} Based on central laboratory measurements of potassium levels ^{b} Based on investigator reported adverse events Treatment-emergent adverse events are defined as any adverse event occurring in any patient who has received at least one dose of study drug and within 3 days of permanent discontinuation. This safety table includes 1 patient who was randomized to placebo but who actually received finerenone. | | |

### Discussion:

This participant-level pooled analysis FINE-HEART represents the largest analysis of the efficacy and safety of the non-steroidal MRA finerenone across the CKM spectrum. While this pooled analysis failed to demonstrate a significant reduction in the primary endpoint of cardiovascular death, this result was sensitive to the definition of cardiovascular death based on the classification of deaths of undetermined causes. As such, we placed greater confidence in the endpoint of all-cause mortality, which was reduced with finerenone with nominal significance. This mortality signal with finerenone was further substantiated by clinically relevant benefits observed across abroad range of other cardio-kidney outcomes including kidney disease progression, HF hospitalizations, all-cause hospitalizations, new-onset atrial fibrillation, and major adverse cardiovascular events. Treatment effects were consistent across all tested clinical subgroups including those with multiple, intersecting CKM conditions. No new or unexpected safety signals were uncovered in this pooled analysis with a well-characterized modestly higher risks of hyperkalemia, but overall lower incidences of serious adverse events and no excess risk of acute kidney injury with finerenone. Taken together, these data affirm the potential of finerenone to prevent or delay morbidity and mortality in patients with CKM conditions while being safe and well tolerated.

Finerenone is approved for use in patients with CKD and type 2 diabetes with albuminuria. While several major multi-specialty guidelines strongly recommend finerenone to delay CKD progression and prevent HF events in people with CKD with type 2 diabetes.21-23 the latest KDIGO guideline24 has offered a class 2A recommendation potentially related to residual uncertainties regarding the mortality effects of finerenone in the FIDELIO-DKD and FIGARO-DKD trials. Furthermore, according to the guideline, its use is to be considered in patients already on standard-of-care therapies such as maximally tolerated renin-angiotensin system inhibitors and/or SGLT2 inhibitors.

These pooled data did not identify heterogeneity with finerenone's effects on cardiovascular death by background use of SGLT2 inhibitors or GLP-1 receptor agonists, although the statistical power of these subgroup findings was limited. This pooled analysis bolsters recent calls for the combination use of finerenone alongside these therapies as foundational "pillars" of care to maximize improvements in cardio-kidney outcomes.

Finerenone was shown to robustly reduce the kidney composite outcome by 20% in this pooled analysis, driven by benefits observed in FIDELIO-DKD and FIGARO-DKD. FINEARTS-HF enrolled a primary heart failure population with some coexisting kidney disease, but with relatively low levels of albuminuria; therefore, CKD progression over a relatively short period of follow-up was difficult to evaluate. It is reassuring that finerenone did not show an increase in reports of acute kidney injury, despite the high baseline kidney risk profile of the patient population and the varied clinical care settings of therapeutic initiation.

While each trial had broad eligibility criteria, some groups were understudied in individual trials. For instance, both FIGARO-DKD and FIDELIO-DKD exclusively enrolled participants with CKD and type 2 diabetes with albuminuria. FINEARTS-HF provides supportive and complementary evidence related to finerenone's therapeutic effects in previously understudied populations including those without diabetes (~60% of trial enrollment), those without CKD, and those without significant urinary albumin excretion (>60% of the trial with UACR<30mg/g). FINEARTS-HF exclusively enrolled patients with symptomatic HF across clinical care settings while FIDELIO-DKD and FIGARO-DKD specifically excluded patients with symptomatic HF with reduced ejection fraction. These pooled data demonstrate that finerenone's benefits extend to patients with different degrees of CKM multimorbidity and across broad patient profiles. The diverse spectrum of benefits on HF, arrhythmia, atherosclerotic risk, and kidney disease progression also underscores the systemic actions of finerenone in attenuating the adverse multi-organ effects of MR overactivation.5

Study Strengths and Limitations. A key strength of this pooled analysis was access to individual participant-level data from all phase III trials conducted to date with finerenone, which allowed harmonization of data elements related to baseline clinical characteristics, outcomes, and subgroups. Major efficacy outcomes were independently adjudicated and safety assessments were conducted with aligned definitions in a standardized fashion. It is noteworthy that the findings of this pooled data analysis were derived from randomized clinical trials with specific inclusion and exclusion criteria and thus may not be generalizable to all populations treated in clinical practice. Despite the large global population studied, enrollment of select groups such as Black patients remained limited. Certain data elements were not consistently available across trials to allow for pooling. For instance, urgent HF visits, which were included as a part of the FINEARTS-HF primary endpoint, were not collected in FIDELIO-DKD and FIGARO-DKD.

While this pooled analysis failed to demonstrate significant reductions in cardiovascular death, finerenone was associated with significantly lower deaths of any cause, cardiovascular events, and kidney outcomes, while being safe and well-tolerated. The totality of the evidence thus supports the disease-modifying potential of finerenone in broad, high-risk patient populations encompassing cardiovascular, kidney, and metabolic diseases.

### Example 15 - Metaanalysis - RALES / EMPHASIS-HF / TOPCAT / FINEARTS-HF

Mineralocorticoid receptor antagonists (MRAs) reduce hospitalisations and death in patients with heart failure and reduced ejection fraction (HFrEF) but the benefit in patients with HF and mildly reduced or preserved EF (HFmrEF/HFpEF) is unclear. We examined the effect of MRAs in four trials across the range of EF.

This is a pre-specified, individual patient-level meta-analysis including RALES (spironolactone) and EMPHASIS-HF (eplerenone), enrolling HFrEF patients, and TOPCAT (spironolactone) and FINEARTS-HF (finerenone), enrolling HFmrEF/HFpEF patients. We estimated the effect of MRAs on the outcomes of cardiovascular death or HF hospitalisation, components of this composite, total HF hospitalisations (with and without cardiovascular deaths) and all-cause death. An interaction between trials and treatment was tested to examine the heterogeneity of effect in these populations. The study is registered with PROSPERO, CRD42024541487.

In 13,846 patients, MRAs reduced the risk of cardiovascular death or HF hospitalisation (hazard ratio [HR] 0.77 [95%CI 0.72-0.83]). There was a statistically significant interaction by trials and treatment, p for interaction<0.001 due to the greater efficacy in HFrEF (HR 0.66 [95%CI 0.59-0.73]) compared to HFmrEF/HFpEF (HR 0.87 [0.79-0.95]). We observed significant reductions in HF hospitalisation in the HFrEF trials (HR 0.63 [0.56-0.72]) and the HFmrEF/HFpEF trials (HR 0.82 [0.74-0.91]). The same pattern was observed for total HF hospitalisations with or without cardiovascular death. Cardiovascular death was reduced in the HFrEF trials (HR 0.72 [0.63-0.82]) but not the HFmrEF/HFpEF trials (HR 0.92 [0.80-1.05]). With an MRA, the risk of hyperkalaemia was doubled compared to placebo, but the incidence of serious hyperkalaemia (potassium >6.0 mmol/L) was low (2.9% versus 1.4%); the risk of hypokalaemia (potassium <3.5 mmol/L) was halved (7% versus 14%).

We performed an individual patient-level meta-analysis of all four of the large prospective placebo-controlled trials of MRAs in heart failure. This analysis included nearly 14,000 patients and confirms the large benefit in patients with heart failure and reduced ejection fraction but also shows that MRAs reduce the risk of the composite of cardiovascular death or hospitalisation for heart failure in patients with heart failure and an ejection fraction ≥40%. The benefits were consistent across a broad range of subgroups.

Two pivotal clinical trials, RALES (Randomized Aldactone Evaluation Study) and EMPHASIS-HF (Eplerenone in Mild Patients Hospitalization and Survival Study in Heart Failure) demonstrated that the steroidal mineralocorticoid receptor antagonists (MRAs), spironolactone and eplerenone decrease the risk of death and hospitalisation in patients with heart failure and reduced ejection fraction (HFrEF). As a result, international guidelines make consistent and strong recommendations for spironolactone and eplerenone in patients with HFrEF. By contrast, the efficacy of these agents in heart failure with mildly reduced or preserved ejection (HFmrEF/HFpEF) is uncertain. Specifically, spironolactone did not improve the primary outcome of first heart failure hospitalization or cardiovascular death in the TOPCAT trial (Treatment of Preserved Cardiac Function Heart Failure with an Aldosterone Antagonist). However, a significant proportion of participants in that trial may not have had heart failure or taken randomised treatment, and post hoc analyses suggested possible benefit in those who did. Consequently, guideline recommendations for MRAs in heart failure with HFmrEF/HFpEF are weak or absent. The question of whether the non-steroidal MRA finerenone is efficacious in patients with HFmrEF/HFpEF was recently evaluated in the FINEARTS-HF trial (the finerenone trial to investigate efficacy and safety superior to placebo in patients with heart failure). Unlike spironolactone and eplerenone, finerenone is a non-steroidal MRA, a class with different physiochemical properties, and in FINEARTS-HF finerenone significantly reduced the risk of the primary composite outcome of total worsening heart failure events and cardiovascular death in patients with heart failure and an ejection fraction of 40% or above. We undertook a pre-specified individual patient-level meta-analysis of the four MRA trials to test the consistency of the effects of mineralocorticoid receptor antagonism across important clinical outcomes, including endpoints that no single trial was designed or powered to examine such as cardiovascular mortality, and across both MRA classes. Furthermore, we examined key safety outcomes and the efficacy and safety of treatment over a range of clinically important subpopulations including those at the highest end of the ejection fraction range and with kidney dysfunction.

### Methods

### Search strategy and selection criteria

Our pre-specified aim was to study the efficacy and safety of MRAs across the full range of ejection fraction in patients with heart failure and according to MRA class. We analysed the RALES trial with spironolactone in patients with HF with and left ventricular ejection fraction (LVEF) J 35%, EMPHASIS-HF with eplerenone in patients with HF and LVEF ≤ 35% (if LVEF >30 to 35%, a QRS duration of >130 msec on electrocardiography was required), TOPCAT with spironolactone in patients with HF and LVEF ≥ 45% and FINEARTS-HF with finerenone in patients with HF and LVEF ≥ 40%. Key information on the included trials and their inclusion and exclusion criteria along with the primary outcomes of each of the trials is given in the appendix (page 2). To ensure that we did not exclude any other important trials we conducted a systematic review of MEDLINE via PubMed of randomised trials of MRAs in patients with HF. Trials were included if they included at least 1000 patients with heart failure with an appropriately powered morbidity or mortality outcome. Data from the FINEARTS-HF trial were unpublished at the time of analysis, but were included for the current Example. Data were extracted, harmonized, and analysed.

### Outcomes and subgroups

The primary outcome of interest in this meta-analysis was a composite of time to first hospitalisation for HF or cardiovascular death. Secondary outcomes examined were time-to-first hospitalisation for heart failure, total (first and repeat) heart failure hospitalisations, total heart failure hospitalisation and cardiovascular death, cardiovascular death, and all-cause death. In each trial, outcomes were adjudicated by a blinded clinical endpoints committee. Cardiovascular death was analysed according to the original definition reported in each of the trials. Because the definition of cardiovascular death included undetermined deaths in RALES, EMPHASIS-HF and TOPCAT but not FINEARTS-HF, we conducted a sensitivity analysis with and without undetermined deaths counted as cardiovascular deaths. Unlike the older trials, FINEARTS-HF included urgent visits for worsening heart failure in the primary composite outcome as an equivalent to heart failure hospitalisation, reflecting changing clinical practice aimed at reducing admissions (and a practice which may have been more common during the COVID-19 pandemic). These events were defined as urgent, unscheduled ambulatory, or emergency room visits for the primary diagnosis of heart failure requiring intravenous diuretic or a vasoactive agent or mechanical or surgical intervention for heart failure. Intensification of an oral diuretic alone was not sufficient to meet this definition.

The effect of MRAs on the primary outcome was examined in key subgroups of interest, including age, sex, race, geographical region, body mass index (BMI), New York Heart Association (NYHA) class, LVEF, history of prior hospitalisation for heart failure, creatinine, estimated glomerular filtration rate, potassium, systolic blood pressure, history of diabetes, myocardial infarction, atrial fibrillation, stroke, and treatment with an ACE inhibitor or angiotensin receptor blocker, beta blocker, diuretic or a digitalis glycoside. A further subgroup of interest was the efficacy of steroidal (spironolactone and eplerenone) versus non-steroidal MRAs (finerenone).

Safety outcomes were examined in patients who received at least one dose of randomized treatment. The outcomes of interest included serum creatinine ≥2.5 and≥3 mg/dl, a >20% and >30% decline in eGFR, serum potassium <3.5 mmol/l, serum potassium >5.5 and >6 mmol/l and systolic blood pressure <90 and <100 mmHg.

### Statistical analysis

For all time-to-first event outcomes, point estimates from Cox proportional hazards models are presented as hazard ratios (HRs) with 95% CIs on an intention-to-treat basis. These models included a term for randomised treatment and were stratified by trial. Event rates for time to first events were calculated per 100 person-years and estimated using the normal approximation to the Poisson log-likelihood. For analysis of total events, rate ratios (RRs) with 95% CIs are presented from a negative binomial model as the time to subsequent hospitalisations was not available in RALES and the results were confirmed in a semi-parametric proportional rates model with a factor for randomised treatment and stratified by trial excluding RALES but including the other trials. Recurrent event rates were estimated from a Poisson model with robust standard errors. Between-trial heterogeneity of treatment effect was examined in the models described with an interaction term between trial and randomised therapy. Additionally, we tested treatment-by-trial heterogeneity of effect using Cochran's Q test and Higgins and Thompsons' I² from a two-stage meta-analysis using a random effects model. Subgroup analysis was performed in time-to-event Cox models with an interaction term between the subgroup of interest and randomised therapy in the model stratified by trial. For continuous variables, a restricted cubic spline was used with knots placed at points that resulted in the model with the lowest Akaike's information criterion value. An interaction term between the spline and randomised therapy was tested in the model and the interaction was represented graphically. The effect of randomised therapy on safety outcomes was assessed by calculating an odds ratio in a logistic regression model with a factor for treatment and trial and the interaction tested with a treatment by trial interaction term. A p-value below 0.05 was considered statistically significant.

The protocol and statistical analysis plan for the meta-analysis were pre-specified before the FINEARTS-HF trial database was locked and were pre-registered on PROSPERO (CRD42024541487). All trials were assessed as high quality but the risk of bias for TOPCAT with regards to the assessment of deviation from the intended intervention was high (appendix page 6). A sensitivity analysis only including the participants from the Americas was therefore undertaken to examine the impact of this potential bias. All participants provided written consent, and the study protocols were approved by the institutional review boards at all participating sites. All analyses were performed using Stata (version 18.0).

### Results

Overall, 13,846 patients were included in the four trials. Patients enrolled in RALES and EMPHASIS-HF were more often men and were less likely to have a history of hypertension, atrial fibrillation, or diabetes than those enrolled in TOPCAT and FINEARTS-HF (Table 28).

**Table 28 - Baseline characteristics of patients in each mineralocorticoid receptor antagonist trial and in the total population studied**

| | | **RALES** | **EMPHASIS-HF** | **TOPCAT** | **FINEARTS-HF** | **Total** |
|---|---|---|---|---|---|---|
| | | N=1,663 | N=2,737 | N=3,445 | N=6,001 | N=13,846 |
| Age (years) | | 65±11 | 68±7 | 68±9 | 72±9 | 69±9 |

| Sex | | | | | | |
|---|---|---|---|---|---|---|
| | Men | 1,217 (73) | 2,127 (78) | 1,670 (48) | 3,269 (54) | 8,283 (60) |
| | Women | 446 (27) | 610 (22) | 1,775 (52) | 2,732 (46) | 5,563 (40) |

| Race | | | | | | |
|---|---|---|---|---|---|---|
| | White | 1,440 (87) | 2,268 (83) | 3,062 (89) | 4,735 (79) | 11,505 (83) |
| | Black | 120 (7) | 67 (2) | 302 (9) | 88 (1) | 577 (4) |
| | Asian | 32 (2) | 316 (12) | 19 (1) | 996 (17) | 1,363 (10) |
| | Other | 71 (4) | 86 (3) | 62 (2) | 182 (3) | 401 (3) |

| Region | | | | | | |
|---|---|---|---|---|---|---|
| | North America | 114 (7) | 248 (9) | 1,477 (43) | 471 (8) | 2,310 (17) |
| | Latin America | 433 (26) | 98 (4) | 290 (8) | 641 (11) | 1,462 (11) |
| | Western Europe | 1,066 (64) | 1,005 (37) | 0 (0) | 1,204 (20) | 3,275 (24) |
| | Central and Eastern Europe | 0 (0) | 988 (36) | 1,678 (49) | 2,630 (44) | 5,296 (38) |
| | Asia-Pacific | 50 (3) | 398 (15) | 0 (0) | 1,055 (18) | 1,503 (11) |
| BMI (kg/m²) | | | 27.5±4.9 | 32.1±7.1 | 29.9±6.1 | 30.0±6.4 |
| | < 30 kg/m² | * | 1,983 (73) | 1,533 (45) | 3,296 (55) | 6,812 (56) |
| | ≥ 30 kg/m² | * | 739 (27) | 1,902 (55) | 2,692 (45) | 5,333 (44) |
| Systolic blood pressure (mmHg) | | 122±20 | 124±17 | 129±14 | 129±15 | 127±16 |
| Heart rate (beats/min) | | 81±14 | 72±13 | 69±10 | 71±11 | 72±12 |
| LVEF (%) | | 25±7 | 26±5 | 57±7 | 53±8 | 45±15 |
| NYHA class, N (%) | | | | | | |
| | I, II | 7(0) | 2,730 (100) | 2,303 (67) | 4,146 (69) | 9,186 (66) |
| | III, IV | 1,656 (100) | 3 (0) | 1,136 (33) | 1,854 (31) | 4,649 (34) |
| Prior HF hospitalization, N (%) | | * | 1,438 (53) | 2,489 (72) | 3,619 (60) | 7.546 (62) |
| NT-proBNP (pg/ml) | | ** | ** | 843.0 (463.0-1720.0) | 1041.4 (448.5-1945.9) | 1013.5 (449.6-1929.8) |
| eGFR (ml per min per 1.73 m²) | | 63±22 | 65±18 | 65±19 | 63±20 | 64±19 |
| eGFR (ml/min/1.73 m² ) | | | | | | |
| | <60 | 841 (51) | 1,092 (40) | 1,463 (42) | 2,844 (47) | 6.240 (45) |
| | >=60 | 817 (49) | 1,633 (60) | 1,980 (58) | 3,157 (53) | 7,587 (55) |
| Potassium (mmol/L) | | 4.2±0.4 | 4.3±0.4 | 4.3±0.4 | 4.4±0.5 | 4.3±0.5 |
| Diabetes, N (%) | | 369 (22) | 859 (31) | 1,118 (32) | 2,439 (41) | 4,785 (35) |
| Hypertension, N (%) | | 391 (24) | 1,819 (66) | 3,147 (91) | 5,325 (89) | 10,682 (77) |
| Atrial fibrillation, N (%) | | 183 (11) | 844 (31) | 1,214 (35) | 3,273 (55) | 5,514 (40) |
| Myocardial infarction, N (%) | | 472 (28) | 1,380 (50) | 893 (26) | 1,541 (26) | 4,286 (31) |
| Stroke, N (%) | | * | 262 (10) | 265 (8) | 708 (12) | 1,235 (10) |
| ACEI/ARB, N (%) | | 1,589 (96) | 2,558 (94) | 2,900 (84) | 4,246 (71) | 11,293 (82) |
| β-Blocker, N (%) | | 171 (10) | 2,374 (87) | 2,676 (78) | 5,095 (85) | 10,316 (75) |
| Diuretic, N (%) | | 1,502 (90) | 2,326 (85) | 2,817 (82) | 5,930 (99) | 12,575 (91) |
| Digitalis glycosides, N (%) | | 1,216 (73) | 740 (27) | 342 (10) | 471 (8) | 2,769 (20) |

| | | | | | | |
|---|---|---|---|---|---|---|
| NT-proBNP, N-terminal pro B type natriuretic peptide BMI, body mass index eGFR, estimated glomerular filtration rate ACEI, angiotensin-converting enzyme inhibitor ARB, angiotensin receptor blocker LVEF, left ventricular ejection fraction NYHA, New York Heart Association *missing in RALES ** missing in RALES and EMPHASIS-HF | | | | | | |

The estimated glomerular filtration rate at baseline was lowest in RALES and FINEARTS-HF. Baseline characteristics by randomised therapy are given below in Table 29 and were balanced between treatment groups.

**Table 29 - Baseline characteristics in the included trials by randomised therapy**

| | | **RALES spironolact one** | **RAL ES place bo** | **EMPHAS IS-HF eplerenon e** | **EMPHAS IS-HF placebo** | **TOPCAT spironolact one** | **TOPC AT placebo** | **FINEAR TS-HF finerenon e** | **FINEAR TS-HF placebo** |
|---|---|---|---|---|---|---|---|---|---|
| | | **N=822** | **N=84 1** | **N=1,364** | **N=1,373** | **N=1,722** | **N=1,72 3** | **N=3,003** | **N=2,998** |
| Age (years) | | 65±12 | 65±11 | 68±7 | 68±7 | 68±9 | 68±9 | 71±9 | 72±9 |

| Sex | | | | | | | | | |
|---|---|---|---|---|---|---|---|---|---|
| | Male | 603 (73) | 614 (73) | 1,055 (77) | 1,072 (78) | 834 (48) | 836 (49) | 1,648 (55) | 1,621 (54) |
| | Female | 219 (27) | 227 (27) | 309 (23) | 301 (22) | 888 (52) | 887(51) | 1,355 (45) | 1,377 (46) |

| Race | | | | | | | | | |
|---|---|---|---|---|---|---|---|---|---|
| | White | 712 (87) | 728 (87) | 1,127 (83) | 1,141 (83) | 1,525 (89) | 1,537 (89) | 2,366 (79) | 2,369 (79) |
| | Black | 56(7) | 64(8) | 37(3) | 30(2) | 153 (9) | 149 ( 9) | 49(2) | 39(1) |
| | Asian | 15(2) | 17(2) | 158 (12) | 158 (12) | 10(1) | 9(1) | 497 (17) | 499 (17) |
| | Other | 39(5) | 32(4) | 42(3) | 44(3) | 34(2) | 28(2) | 91 (3) | 91(3) |

| Region | | | | | | | | | |
|---|---|---|---|---|---|---|---|---|---|
| | North America | 56(7) | 58(7) | 126 (9) | 122 (9) | 738 (43) | 739 (43) | 235 ( 8) | 236 ( 8) |
| | Latin America | 216 (26) | 217 (26) | 47(3) | 51(4) | 148 ( 9) | 142 (8) | 322(11) | 319 (11) |
| | Western Europe | 526 (64) | 540 (64) | 500 (37) | 505 (37) | 0(0) | 0(0) | 599 (20) | 605 (20) |
| | Central Europe | 0(0) | 0(0) | 492 (36) | 496 (36) | 836 (49) | 842 (49) | 1,319 (44) | 1,311 (44) |
| | Asia-Pacific | 24(3) | 26(3) | 199(15) | 199 (14) | 0(0) | 0(0) | 528 (18) | 527 (18) |
| BMI (kg/m2) | | | | 27.5±4.9 | 27.5±4.9 | 32.1±7.1 | 32.1±7. 1 | 29.9±6.1 | 30.0±6.1 |
| | < 30 kg/m2 | | | 975 (72) | 1,008 (74) | 764 (44) | 769 (45) | 1,658 (55) | 1,638 (55) |
| | ≥ 30 kg/m2 | | | 383 (28) | 356 (26) | 956 (56) | 946 (55) | 1,338 (45) | 1,354 (45) |
| Systolic blood pressure (mmHg) | | 123±21 | 122±2 0 | 124±17 | 124±17 | 129±14 | 129±14 | 130±15 | 129±15 |
| Heart rate (beats/min) | | 81±14 | 81±15 | 72±12 | 72±13 | 69±11 | 69±10 | 72±12 | 71±12 |
| LVEF (%) | | 26±7 | 25±7 | 26±5 | 26±5 | 57±7 | 57±8 | 53±7 | 53±8 |

| NYHA, N (%) | | | | | | | | | |
|---|---|---|---|---|---|---|---|---|---|
| | I, II | 4(0) | 3(0) | 1,358 (100) | 1,372 (100) | 1,146 (67) | 1,157 (67) | 2,081 (69) | 2,065 (69) |
| | III, IV | 818 (100) | 838 (100) | 2(0) | 1(0) | 575 (33) | 561 (33) | 921 (31) | 933 (31) |
| Prior HF hospitalizatio n, N (%) | | | | 714 (52) | 724 (53) | 1,246 (72) | 1,243 (72) | 1,797 (60) | 1,822 (61) |
| NT-proBNP (pg/ml) | | | | | | 826.0 (460.0-1572.0) | 879.5 (465.5-2033.0) | 1052.5 (467.3-1937.0) | 1028.0 (4332-1963.1) |
| eGFR (ml per min per 1.73 m2) | | 63±22 | 62±21 | 66±18 | 65±18 | 65±19 | 65±18 | 63±19 | 63±20 |
| eGFR (ml/min/1.73 m2) < 60 | | | | | | | | | |
| | <60 | 419 (51) | 422 (50) | 531 (39) | 561 (41) | 747 (43) | 716 (42) | 1,432 (48) | 1,412 (47) |
| | >=60 | 401 (49) | 416 (50) | 829(61) | 804 (59) | 975 (57) | 1,005 (58) | 1,571 (52) | 1,586 (53) |
| Potassium (mmol/L) | | 4.2±0.5 | 4.2±0. 4 | 4.3±0.4 | 4.3±0.4 | 4.2±0.4 | 4.3±0.4 | 4.4±0.5 | 4.4±0.5 |
| Type 2 diabetes | | 174 (21) | 195 (23) | 459 (34) | 400 (29) | 565 (33) | 553 (32) | 1,217 (41) | 1,222 (41) |
| Hypertension, N (%) | | 193 (23) | 198 (24) | 910 (67) | 909 (66) | 1,567 (91) | 1,580 (92) | 2,640 (88) | 2,685 (90) |
| Atrial fibrillation, N (%) | | 98 (12) | 85 (10) | 409 (30) | 435 (32) | 611 (35) | 603 (35) | 1,648 (55) | 1,625 (54) |
| Myocardial infarction, N (%) | | 229 (28) | 243 (29) | 686 (50) | 694 (51) | 444 (26) | 449 (26) | 784 (26) | 757 (25) |
| Stroke, N (%) | | | | 136 (10) | 126(9) | 128 (7) | 137(8) | 355 (12) | 353 (12) |
| ACEI/ARB, N (%) | | 788 (96) | 801 (95) | 1,283 (95) | 1,275 (93) | 1,452 (84) | 1,448 (84) | 2,124 (71) | 2,122 (71) |
| β-Blocker, N (%) | | 85 (10) | 86 (10) | 1,181 (87) | 1,193(87) | 1,346 (78) | 1,330 (77) | 2,541 (85) | 2,554 (85) |
| Diuretic, N (%) | | 742 (90) | 760 (90) | 1,150 (85) | 1,176 (86) | 1,401 (81) | 1,416 (82) | 2,973 (99) | 2,957 (99) |
| Digitalis glycosides, N (%) | | 611 (74) | 605 (72) | 363 (27) | 377 (28) | 178 (10) | 164 (10) | 241 ( 8) | 230 ( 8) |

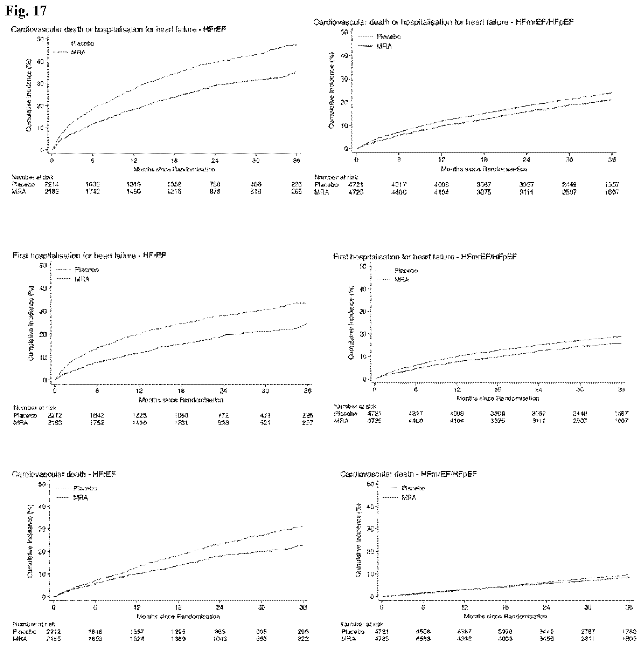

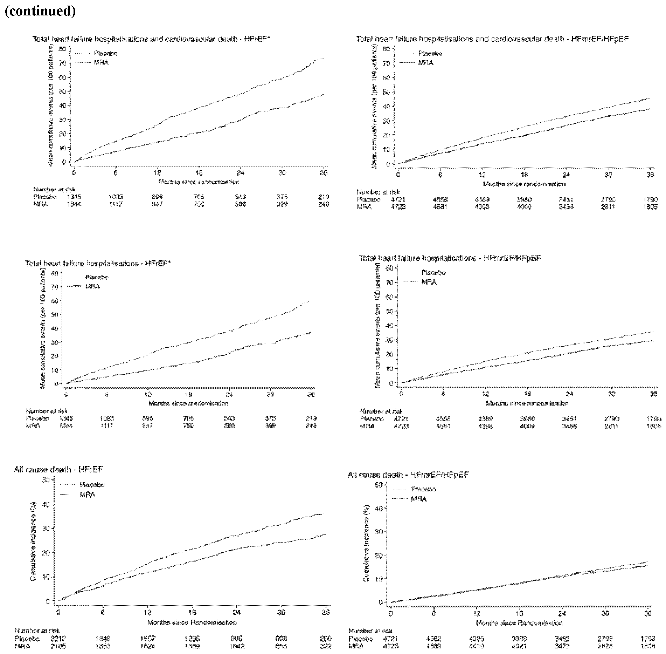

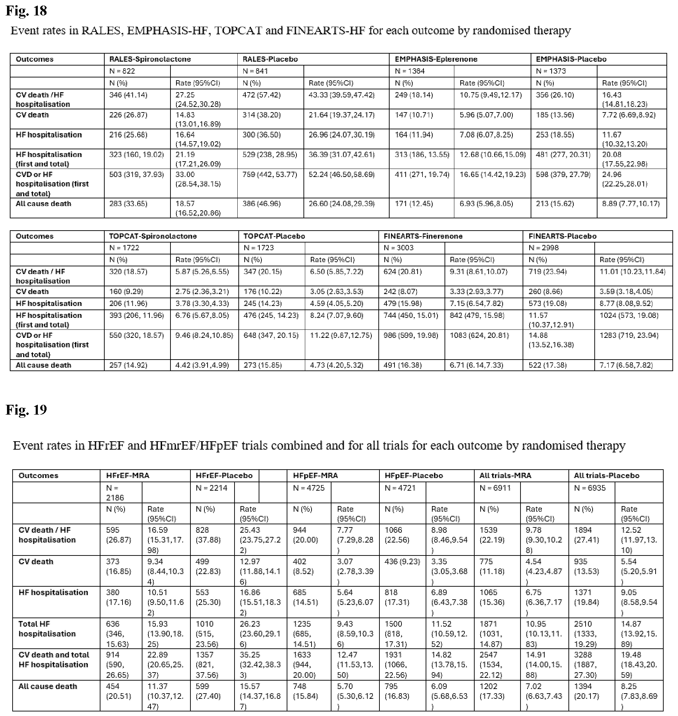

The rates of events in the placebo groups were higher in the trials including patients with HFrEF compared to those with HFmrEF/HFpEF (Figure 17, Figure 18, Figure 19).

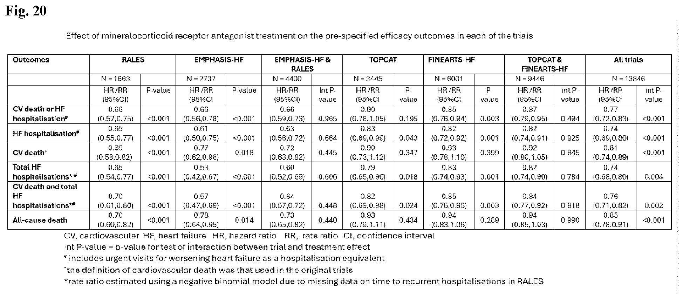

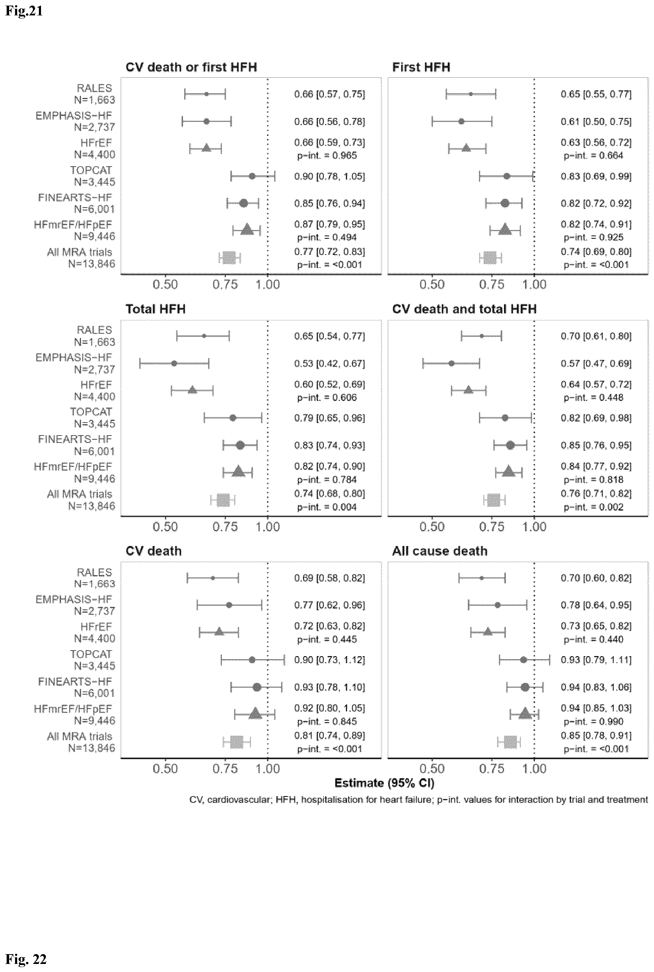

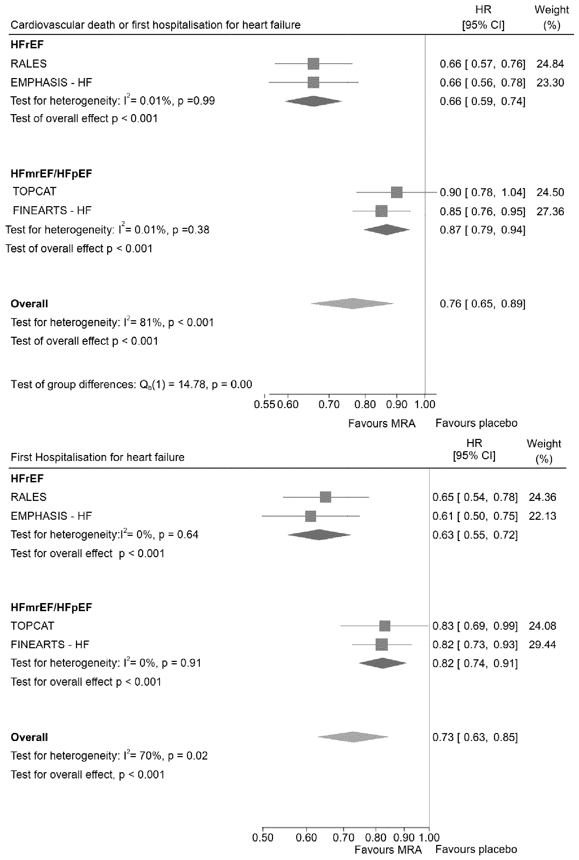

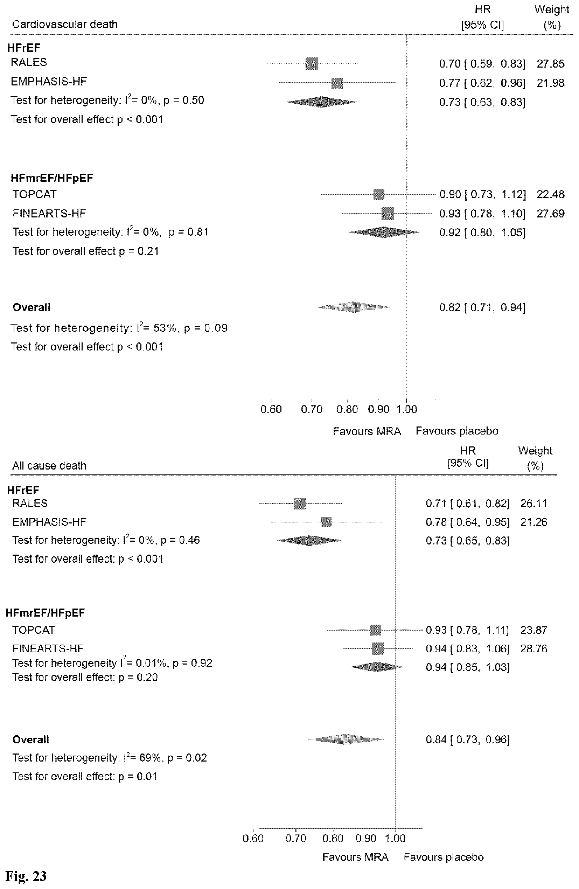

In the analysis of the time-to-first occurrence of cardiovascular death or HF hospitalisation, including all four trials, the hazard ratio (HR) for an MRA compared to placebo was 0.77 (95%CI 0.72-0.83) (Figure 20, Figure 21). However, there was a statistically significant interaction between trials and the effect of treatment (p for interaction <0.001, Figure 20) which was also observed in the two-stage meta-analysis (Cochrane's Q p<0.001, I² =83%), Figure 22. Inspection of the treatment estimates showed that the source of the interaction was the greater treatment efficacy in the HFrEF trials (pooled HFrEF trials HR 0.66 [95%CI 0.59-0.73]) compared to the HFmrEF/HFpEF trials (pooled HFmrEF/HFpEF trials HR 0.87 [95%CI 0.79-0.95]) (Figure 20, Figure 21). Among the HFrEF trials and the HFmrEF/HFpEF trials, as separate groups, there was no further heterogeneity (p for interaction HFrEF trials=0.97; HFmrEF/HFpEF trials=0.49).

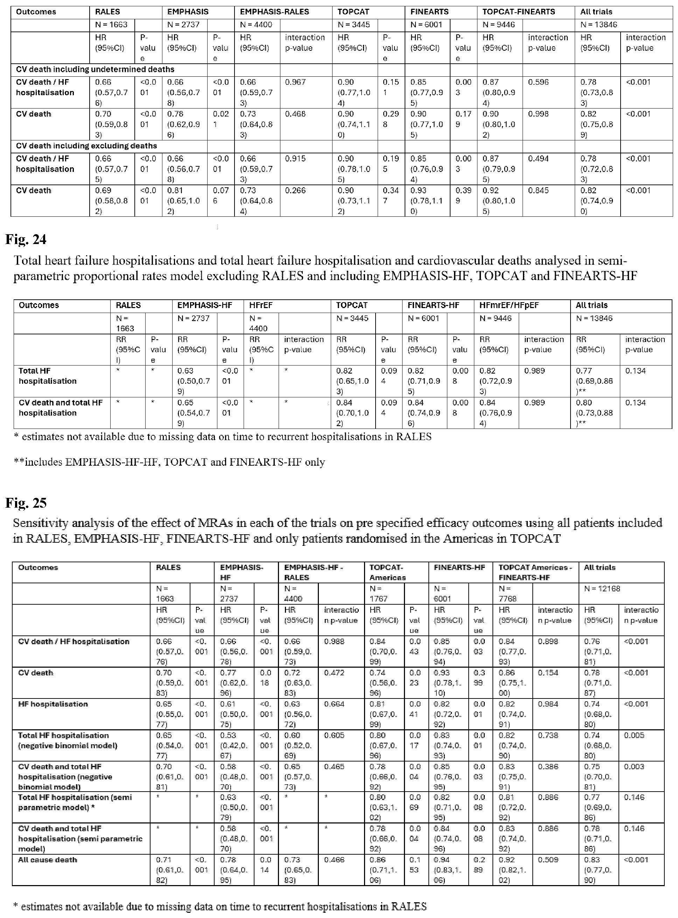

The same heterogeneity of treatment effect by trials was present for cardiovascular death, HF hospitalisation, and all-cause death (Figure 20, Figure 21). The HR for the effect of an MRA on cardiovascular death in the two HFrEF trials was 0.72 (95%CI 0.63-0.82) and in the two HFmrEF/HFpEF trials was 0.92 (95%CI 0.80-1.05). The p for interaction over all trials was <0.001 and the p for interaction in the HFrEF trials was 0.45 and in the HFmrEF/ HFpEF trials, it was 0.85 (Figure 20, Figure 21). For outcomes incorporating cardiovascular death, in sensitivity analyses including or excluding undetermined causes of death, the findings were similar (Figure 23).

The HR for the effect of an MRA on a first HF hospitalisation was 0.63 (95%CI 0.56-0.72) in the HFrEF trials and 0.82 (95%CI 0.74-0.91) in the HFmrEF/HFpEF trials (p for interaction<0.001) Figure 20, Figure 21. There was no further heterogeneity in these two sets of trials (p for interaction HFrEF trials=0.66; HFmrEF/HFpEF trials=0.93).

The rate ratio (RR) for the effect of an MRA on total HF hospitalisations was 0.60 (95%CI 0.52-0.69) in the HFrEF trials and 0.82 (95%CI 0.74-0.90) in the HFmrEF/HFpEF trials (p for interaction=0. 0.004) again with no interaction within the groups of trials (HFrEF trials = 0.61; HFmrEF/HFpEF trials=0.78) Figure 20. We observed similar results when total HF hospitalisations and cardiovascular deaths were examined: RR 0.64 95%CI 0.57-0.72 in the HFrEF trials and RR 0.84 95%CI 0.77-0.92 in the HFmrEF/HFpEF trials (p for interaction=0.001), with no further heterogeneity within the groups of trials (p for interaction HFrEF trials 0.45; HFmrEF/HFpEF trials=0.82) Figure 20. Results were similar using a semi-parametric proportional rates model (Figure 24).

The HR for the effect of an MRA on all-cause death in the HFrEF trials was 0.73 95%CI 0.65-0.82 and 0.94 95%CI 0.85-1.03 in the HFmrEF/HFpEF trials (p for interaction<0.001) with no further heterogeneity between the two groups of trials (p for interaction HFrEF trials=0.44; HFmrEF/HFpEF trials=0.99) Figure 20, Figure 21.

Given the interaction of the treatment effects by trial, the analyses of steroidal versus non-steroidal MRAs on outcomes were not conducted. A sensitivity analysis with a two-stage meta-analysis confirmed the results from the one-stage individual patient-level analysis (Figure 21). The results of the individual patient level meta-analysis were unchanged in a sensitivity analysis using only the patients randomised in the Americas in TOPCAT (Figure 25) with a hazard ratio estimate for cardiovascular death or HF hospitalisation of 0.84 (95%CI 0.77-0.93), heart failure hospitalisation of 0.82 (95%CI 0.74-0.91), and cardiovascular death of 0.86 (95%CI 0.75-1.00) in patients with HFmrEF/HFpEF.

### Subgroups

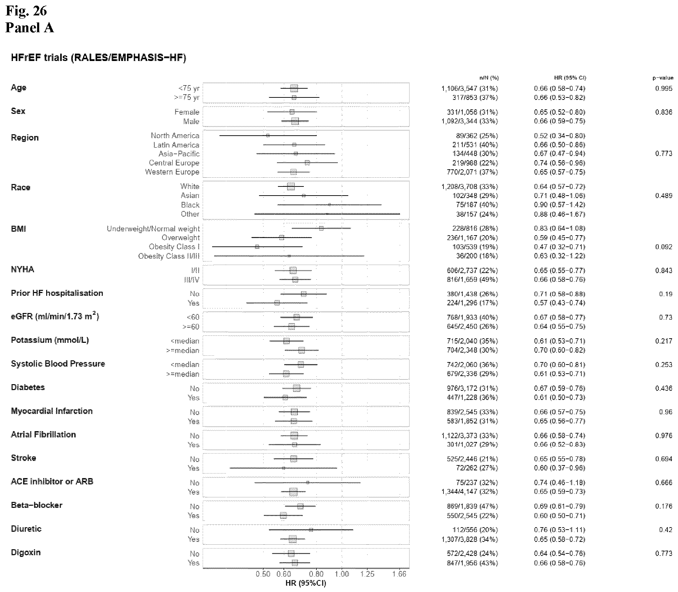

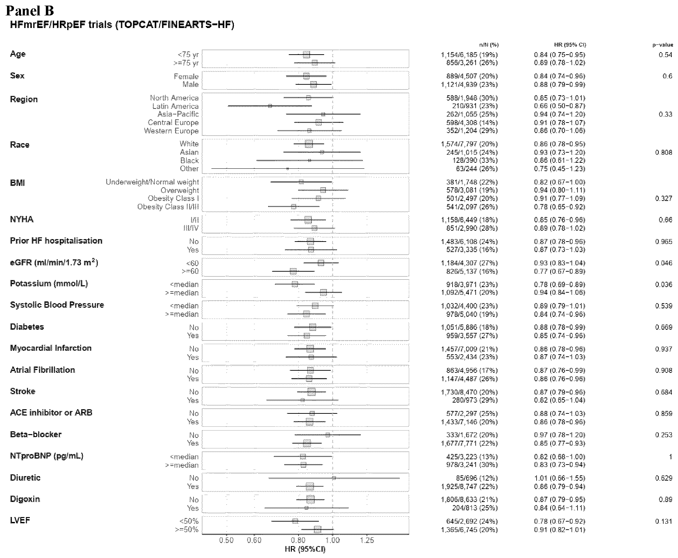

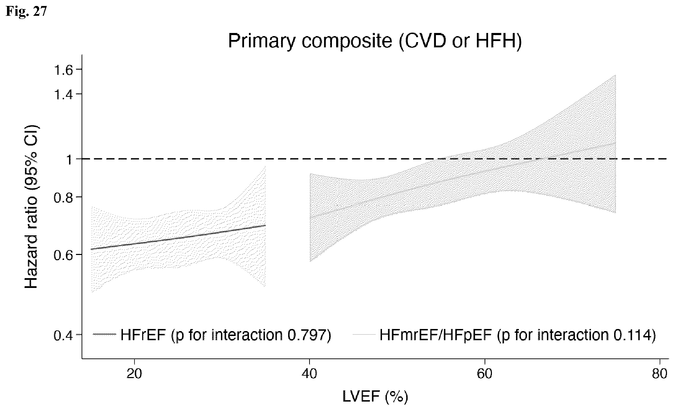

The effect of therapy was consistent across all subgroups in the HFrEF and HFmrEF/HFpEF trials (Figure 26). In a subgroup analysis of all four trials, there was an interaction between baseline potassium and treatment efficacy with greater efficacy in those with a lower potassium (Figure 24). The interaction with ejection fraction as a categorical variable was similar to the interaction between the trials. When the interaction between therapy and ejection fraction was modelled as a continuous variable within the HFrEF and HFmrEF/HFpEF trials separately, there was no treatment heterogeneity confirming that the majority of the heterogeneity was between HFrEF and HFmrEF/HFpEF (Figure 27).

### Safety

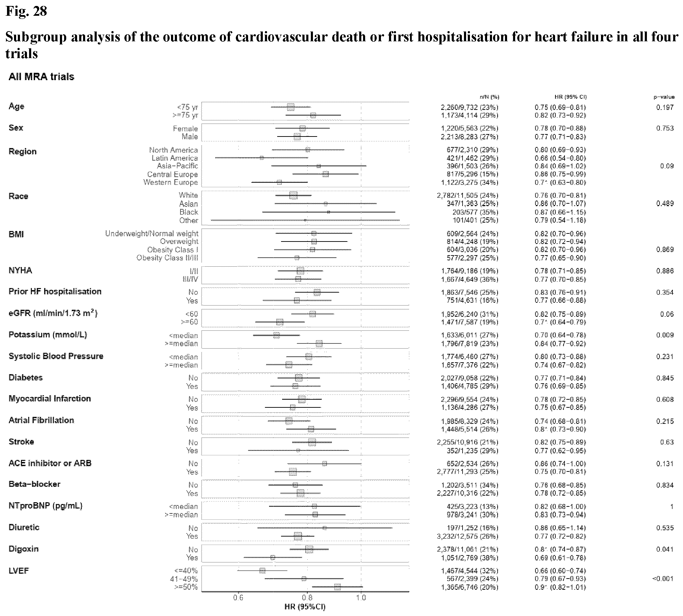

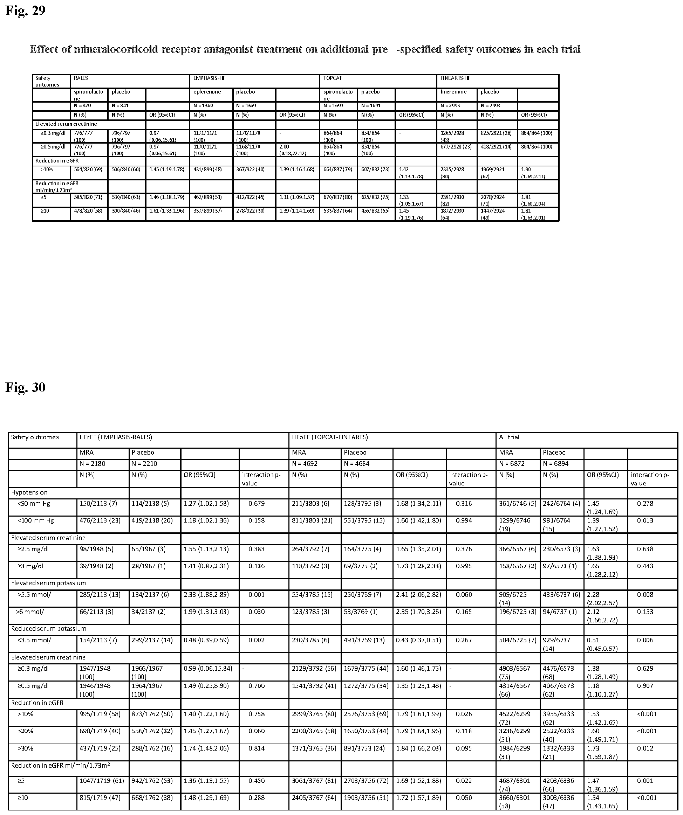

Safety outcomes in each trial and treatment arm in patients receiving trial treatment are shown in Table 3 (appendix page 18). The risk of hyperkalaemia defined as serum potassium > 5.5 (moderate) or > 6 mmol/L (severe) was twice that of the placebo group. However, the absolute risk of severe hyperkalaemia was low at approximately 2.9% in the MRA group and 1.4% in the placebo group. There was little heterogeneity in the risk of any hyperkalaemia when rates were examined by trials defined by ejection fraction or by type of MRA (Figure 25, Figure 28, Figure 29, Figure 30,), however, this analysis did not differentiate between on- and off-treatment hyperkalemia. Conversely, the risk of hypokalaemia (potassium <3.5 mmol/L) was halved in MRA-treated patients compared to placebo-treated patients (7% versus 14%, respectively) across all trials (Figure 30).

Although hypotension was more common in the low ejection fraction trials (Table 3) we found no statistical heterogeneity for the effect of treatment on a systolic blood pressure <100 mmHg by trial group (7% versus 5% in the HFrEF trials and 5% versus 3% in the HFmrEF/HFpEF trials).

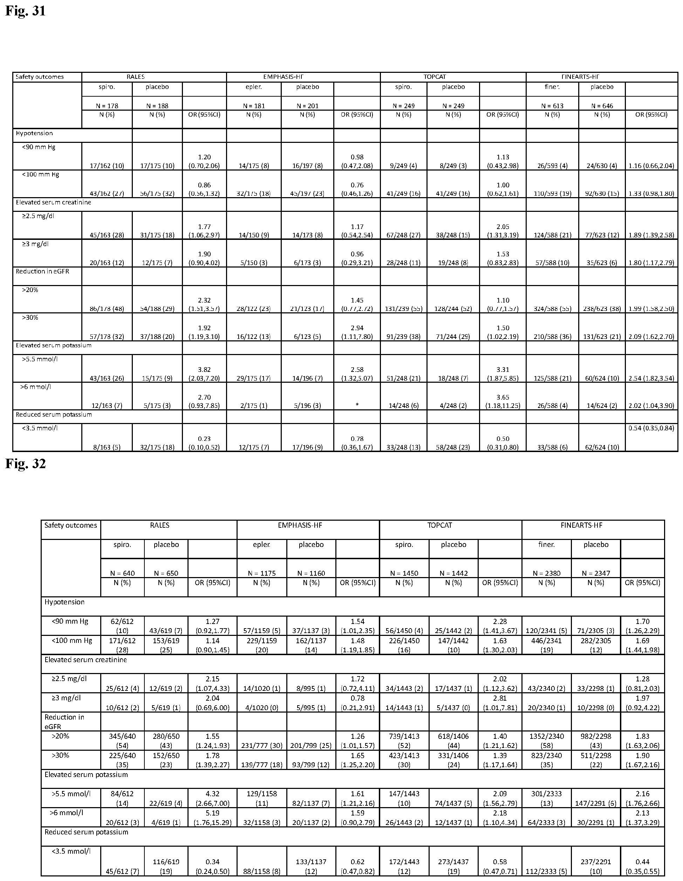

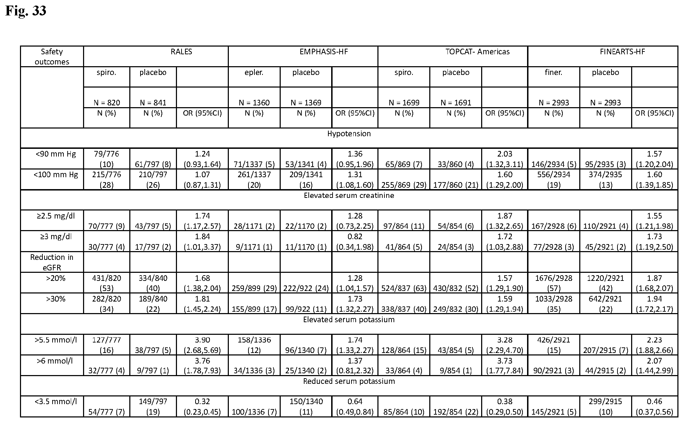

Additional safety outcomes are given in the appendix including by baseline eGFR and using TOPCAT America (Figure 31, Figure 32, Figure 33).

### Discussion

Before this meta-analysis, there was strong evidence of the benefits of the steroidal MRAs spironolactone and eplerenone in patients with heart failure and a substantially reduced left ventricular ejection fraction but uncertainty about the efficacy of MRAs in patients with mildly reduced or preserved ejection fraction, especially as spironolactone failed to show significant benefit the TOPCAT trial. With completion of the FINEARTS-HF trial using finerenone, there is now evidence that blocking the mineralocorticoid receptor with a non-steroidal MRA is beneficial in people with an ejection fraction of 40 percent or above. In this meta-analysis, collectively, all four major trials demonstrate the value of steroidal and non-steroidal MRAs in heart failure irrespective of ejection fraction phenotype.

However, this meta-analysis also shows that there was significant heterogeneity between the treatment effect on efficacy outcomes in the trials that included patients with reduced left ventricular ejection fraction compared to the trials in people with mildly reduced or preserved ejection fraction, with smaller relative risk reductions in the latter participants. This variation in efficacy according to ejection fraction phenotype was evident for both heart failure hospitalisation and mortality, although there was a significant reduction in heart failure hospitalisation with MRAs across all ejection fraction phenotypes. By contrast, cardiovascular death was not reduced significantly in patients with mildly reduced or preserved ejection fraction and this conclusion was not altered by using different definitions of cardiovascular death (i.e., whether deaths of unknown cause were included or excluded) or including only TOPCAT patients enrolled in the Americas, although in the latter sensitivity analysis, the HR was 0.86 with 95%CI 0.75-1.00. This differential effect on fatal and non-fatal outcomes was also observed in the sodium-glucose co-transporter 2 trials 10 and with sacubitril/valsartan. The lack of effect of all of these treatments on all-cause death in patients with a higher ejection fraction may be due to the much larger proportion of non-cardiovascular deaths in this group compared to people with lower ejection fraction, with non-cardiovascular deaths unlikely to be reduced by treatments such as an MRA.

Among patients with mildly reduced or preserved ejection fraction, other treatments acting through neurohumoral mechanisms show attenuated efficacy in patients with a completely normal ejection fraction. While our analyses by ejection fraction category or using ejection fraction as a continuous variable might suggest this visually, formal interaction testing did not show that ejection fraction modified the effects of MRAs in patients with mildly reduced or preserved ejection fraction.

Examination of other subgroups including by age, comorbidity, and laboratory and other physiological variables showed mainly consistent results for the effect of an MRA on the primary outcome in both the reduced ejection fraction trials and, separately, in the mildly reduced or preserved ejection fraction trials (and, therefore, across all four trials). Importantly, the meta-analysis included a substantial number of patients with significantly impaired kidney function and the benefit of MRA treatment was consistent in these patients as well as those with better baseline kidney function.

Hyperkalaemia is a safety concern with MRAs, and the risk of both modest and severe hyperkalaemia was approximately doubled with MRA treatment in the reduced ejection fraction trials and this risk was elevated to a similar extent in the mildly reduced/preserved ejection fraction trials. However, the absolute risk of serious hyperkalaemia (potassium >6 mmol/L) was low (around 3 percent in the MRA treatment group compared with 1 to 1.5 percent in the placebo group). We did not find heterogeneity between trials or between MRAs for hyperkalaemia. Hypokalaemia is a more common problem than hyperkalaemia in heart failure because of the use of diuretics and it is at least as important a safety concern. The overall risk of hypokalaemia (potassium <3.5 mmol/L) was halved by MRA treatment (7% in the MRA group versus 14% in the placebo group).

Initiation of an MRA may also lead to a decline in estimated glomerular filtration rate although decreases in glomerular filtration rate are extremely common in patients with heart failure as evidenced by the rate of these changes in the placebo group in all four trials. The risk of a 20 or 30 percent decrease in glomerular filtration rate was between one and a half and two times as common with mineralocorticoid receptor antagonist treatment compared to placebo.

Despite possible concerns about hypotension, a systolic blood pressure below 90 mmHg was uncommon overall and the difference in episodes of low systolic blood pressure between MRA treatment and placebo was small.

The results of these analyses should be interpreted with some limitations in mind. Certain variables were not available in individual trials e.g., body mass index in RALES. In RALES the time to hospitalisation was only available for the first event and therefore a negative binomial model had to be used to analyse repeat events. The FINEARTS-HF trial included episodes of worsening heart failure requiring urgent treatment as an equivalent to hospitalisation. These were not collected in the older trials but have been validated endpoints in recent trials because thresholds for hospital admission have changed (although they constituted a minority of events). Our analysis was restricted to randomised controlled trials with more than 1000 participants and the exclusion of smaller trials might affect the generalisability of this meta-analysis, as the included trials had relatively homogenous exclusion criteria. While we tried to control for the difference in baseline risk by stratifying by trial in our models the level of heterogeneity was high as demonstrated both in the individual patient-level analysis and our two-stage meta-analysis. However, this meant that we were not able to perform a direct comparison between steroidal MRAs of spironolactone and eplerenone with the non-steroidal MRA finerenone. The meta-analysis includes trials conducted over several decades with changes in background care in that period. Sodium-glucose co-transporter 2 inhibitors are now considered a foundational treatment for heart failure but were not developed or indicated at the time of the older trials and we cannot comment on the concurrent use of an MRA and these drugs that were introduced as a treatment for heart failure during the conduct of the FINEARTS-HF trial.

In conclusion, this systematic review and meta-analysis of nearly 14,000 patients across 4 large clinical trials provides comprehensive evidence that steroidal and non-steroidal MRAs improve cardiovascular outcomes in patients with heart failure, regardless of ejection fraction phenotype. These benefits were consistent across patient subgroups. Treatment with an MRA should be considered in all patients with heart failure without a contraindication to this treatment.

**Table 15: Markers of Cardiorenal Risk and Health Status by Presence of Cardio-Renal-Metabolic Conditions**

| **# of Cardio-Renal-Metabolic Conditions** | | | | | | | | | |
|---|---|---|---|---|---|---|---|---|---|
| | **0** | | **1** | | | **2** | | **3** | |
| | **No ASCVD, no DM, no CKD** | **ASCVD, no DM, no CKD** | **DM, no ASCVD, no CKD** | **CKD, no ASCVD, no DM** | **ASCVD, DM, no CKD no CKD** | **ASCVD, CKD, no DM** | **DM, CKD, no ASCVD** | **ASCVD, DM, CKD** | **5** |
| | **n=1081** | **n=894** | **n=502** | **n=860** | **n=636** | **n=730** | **n=549** | **n=749** | |
| **UACR (mg/g)** | 12 [5,34] | 11 [5,30] | 20 [8, 87] | 18 [8, 56] | 23 [7, 75] | 18 [6, 52] | 42 [13, 190 ] | 51 [14,248 ] | p<0.001 |
| **NT-proBNP (pg/mL)** | 1352 [472, 2500]¹⁾ | 987 [390, 2093]¹⁾ | 1127 [365, 2279]¹⁾ | 2110 [1017, 3844] ¹⁾ | 1008 [377, 2034] ¹⁾ | 2004 [750, 4119] ¹⁾ | 2114 [898, 3741]¹⁾ | 1822 [654, 3983] ¹⁾ | p<0.001 |
| **KCCQ TSS** | 68.7 ± 23.1 | 71.2 ± 22.5 | 66.0 ± 24.5 | 65.3 ± 23.8 | 68.5 ± 23.7 | 67.8 ± 22.7 | 62.3 ± 25.4 | 63.8 ± 25.5 | p<0.001 10 |

| | | | | | | | | | |
|---|---|---|---|---|---|---|---|---|---|
| Definition of ¹⁾ in Example 1b. | | | | | | | | | |

## Claims

1. The compound according to formula (I) 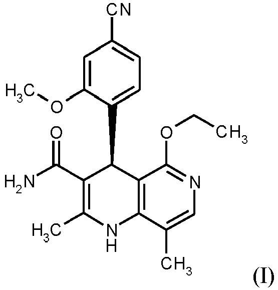
or a hydrate, solvate, pharmaceutically acceptable salt thereof, co-crystal or a polymorph thereof, for use in the prevention or treatment of heart failure,
wherein the heart failure is selected from symptomatic heart failure, heart failure with improved ejection fraction (HFimpEF), heart failure with mid-range ejection fraction (HFmrEF), heart failure preserved ejection fraction (HFpEF), heart failure with reduced ejection fraction (HFrEF), chronic heart failure (CHF), congestive heart failure, acute heart failure, chronic heart failure, worsening chronic heart failure (WCHF), hospitalization for heart failure,
wherein the patient receives a CYP3A4 inhibitor or a CYP3A4 inducer.

2. The compound for use according to claim 1, wherein the patient receives a CYP3A4 inhibitor.

3. The compound for use according to claim 1, wherein the patient receives a CYP3A4 inducer.

4. The compound for use according to any one of claims 1 to 3, wherein compound (I) is administered within a dosage range of 0.5 to 80 mg.

5. The compound for use according to any one of claims 1 to 4, wherein the patient receives a dose selected from 80 mg, 60 mg, 40 mg, 30 mg, 20 mg, and 10 mg.

6. The compound for use according to any one of claims 1 to 5, wherein the dose the patient receives is down-titrated to 60 mg, 40 mg, 30 mg, 20 mg, 10 mg or 5 mg.

7. The compound for use according to any one of claims 1 to 6, wherein the dose the patient receives is 30 mg, 20 mg, 10 mg or 5 mg of the compound according to formula (I).

8. The compound for use according to any one of claims 1 to 7, wherein the dose the patient receives is up-titrated to 80 mg, 60 mg, 40 mg, 30 mg, 20 mg or 10 mg.

9. The compound for use according to any one of claims 1 to 8, wherein the patient further receives any one of the medicaments or comedications selected from loop diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), glucagon-like peptide-1 (GLP-1) agonistsand mixtures thereof.

10. The compound for use according to any one of claims 1, 2, or 4 to 9, wherein the patient receives
- a CYP3A4 inducer and
- the compound according to formula (I) in an amount selected from 5 mg, 10 mg, 20 mg, 30 mg, 40 mg, and 60 mg
wherein the dose is adjusted by up-titration to a dose selected from 10 mg, 20 mg, 30 mg, 40 mg, 60 mg and 80 mg.

11. The compound for use according to any one of claims 1 or 3 to 9, wherein the patient receives
- a CYP3A4 inhibitor and
- the compound according to formula (I) in an amount selected from 10 mg, 20 mg, 30 mg, 40 mg, 60 mg and 80 mg.
wherein the dose is adjusted by down-titration to a dose selected from 5 mg, 10 mg, 20 mg, 30 mg, 40 mg, and 60 mg.

12. The compound for use according to any one of claims 1 to 11, wherein the dose the patient receives is adjusted based on the factors selected from LVEF, NYAH, eGFR, UACR, potassium level, sodium level, chloride level, urea level, blood urea nitrogen (BUN), high-sensitivity Troponin T level, natriuretic peptide (NT-proBNP), blood pressure (BP), systolic blood pressure, average heart rate (resting), previous diseases, prior MRA use, heart rate (or pulse rate), KCCQ-TSS, body mass index (BMI), a comedications selected from loop diuretics, beta-blocker, ACE inhibitor (ACEi), angiotension-receptor-blocker (ARB), Angiotensin Receptor-Neprilysin Inhibitor (ARNI), Calcium Channel Blockers, Sodium-glucose Cotransporter-2 Inhibitor (SGLT-2i), CYP3A4 inducer, CYP3A4 inhibitor, and mixtures thereof.

13. The compound (I) 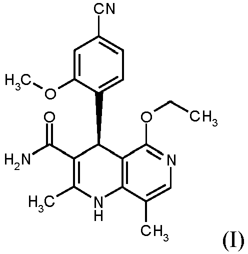
or a hydrate, solvate, pharmaceutically acceptable salt thereof, co-crystal, or a polymorph thereof, for reducing the risk of cardiovascular death, hospitalization for heart failure, and/or urgent heart failure visits in patients with heart failure, wherein
- the patient receives a therapeutically effective amount of the compound,
- and a CYP3A4 inhibitor or CYP3A4 inducer,
wherein the use is defined accdoring to any one of claims 2 to 12.

14. The compound (I) 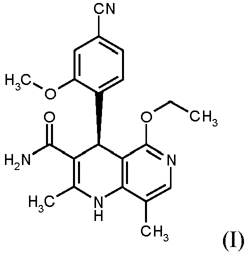
or a hydrate, solvate, pharmaceutically acceptable salt thereof, co-crystal, or a polymorph thereof, for improving symptoms or preventing worsening of symptoms of heart failure in patients with heart failure, wherein
- the patient receives a therapeutically effective amount of the compound,
- and a CYP3A4 inhibitor or CYP3A4 inducer,
wherein the use is defined accdoring to any one of claims 2 to 12.

15. The compound for use according to any one of claims 1 to 14, wherein the compound of the formula (I) in crystalline form of polymorph I 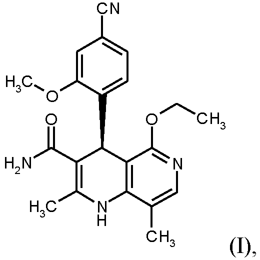 wherein the x-ray diffractogram of the compound exhibits peak maxima of the 2 theta angle at 8.5, 14.1, and 19.0.
